# EUROPEAN PATENT SPECIFICATION

(11) **EP 2 958 586 B1**
(45) Date of publication and mention of the grant of the patent: **05.09.2018**
(21) Application number: 13707329.2
(22) Date of filing: 21.02.2013
(51) Int. Cl.: A61K 39/145

(54) **H5 PROTEINS OF H5N1 INFLUENZA VIRUS FOR USE AS A MEDICAMENT**
H5-PROTEINE AUS DEM H5N1-INFLUENZAVIRUS ZUR VERWENDUNG ALS ARZNEIMITTEL
PROTÉINES H5 DU VIRUS DE LA GRIPPE H5N1 POUR UTILISATION EN TANT QUE MÉDICAMENT

(43) Date of publication of application: 30.12.2015
(73) Proprietor: Boehringer Ingelheim Vetmedica GmbH, 55216 Ingelheim am Rhein (DE)
(72) Inventor: MUNDT, Egbert, 55216 Ingelheim am Rhein (DE)
(74) Representative: Schmidt, Stefan
(86) International application number: PCT/EP2013/053505
(87) International publication number: WO 2014/127825

(56) References cited:
- WO-A1-2011/136738
- WO-A1-2013/024113
- WO-A2-2008/052173
- HESSEL A ET AL: "Vectors Based on Modified Vaccinia Ankara Expressing Influenza H5N1 Hemagglutinin Induce Substantial Cross-Clade Protective Immunity", PLOS ONE, vol. 6, no. 1, 1 January 2011 (2011-01-01) , pages e16247-e16247, XP055013626, ISSN: 1932-6203, DOI: 10.1371/journal.pone.0016247
- DINAPOLI J M ET AL: "Newcastle disease virus-vectored vaccines expressing the hemagglutinin or neuraminidase protein of H5N1 highly pathogenic avian influenza virus protect against virus challenge in monkeys", JOURNAL OF VIROLOGY, AMERICAN SOCIETY FOR MICROBIOLOGY, US, vol. 84, no. 3, 1 February 2010 (2010-02-01), pages 1489-1503, XP009130182, ISSN: 1098-5514
- KREIJTZ J ET AL: "MVA-Based H5N1 Vaccine Affords Cross-Clade Protection in Mice against Influenza A/H5N1 Viruses at Low Doses and after Single Immunization", PLOS ONE, vol. 4, no. 11, 1 January 2009 (2009-01-01), pages e7790-e7790, XP055002466, ISSN: 1932-6203, DOI: 10.1371/journal.pone.0007790
- LARDINOIS A ET AL: "Potency of a Recombinant NDV-H5 Vaccine Against Various HPAI H5N1 Virus Challenges in SPF Chickens", AVIAN DISEASES, vol. 56, no. 4, Suppl. S, December 2012 (2012-12), pages 928-936, XP009173366,
- SANTIAGO F W ET AL: "Vaccination with drifted variants of avian H5 hemagglutinin protein elicits a broadened antibody response that is protective against challenge with homologous or drifted live H5 influenza virus", VACCINE, ELSEVIER, AMSTERDAM, NL, vol. 29, no. 48, 16 September 2011 (2011-09-16), pages 8888-8897, XP028326018, ISSN: 0264-410X, DOI: 10.1016/J.VACCINE.2011.09.069 [retrieved on 2011-09-21]
- HOFFMANN E ET AL: "ROLE OF SPECIFIC HEMAGGLUTININ AMINO ACIDS IN THE IMMUNOGENICITY AND PROTECTION OF H5N1 INFLUENZA VIRUS VACCINES", PROCEEDINGS NATIONAL ACADEMY OF SCIENCES, NATIONAL ACADEMY OF SCIENCES, US, vol. 102, no. 36, 1 September 2005 (2005-09-01), pages 12915-12920, XP009084967, ISSN: 0027-8424, DOI: 10.1073/PNAS.0506416102
- SU Y ET AL: "Analysis of a point mutation in H5N1 avian influenza virus hemagglutinin in relation to virus entry into live mammalian cells", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 153, no. 12, 20 November 2008 (2008-11-20), pages 2253-2261, XP019722818, ISSN: 1432-8798
- OLIVEIRA CAVALCANTI M ET AL: "A genetically engineered H5 protein expressed in insect cells confers protection against different clades of H5N1 highly pathogenic avian influenza viruses in chickens", AVIAN PATHOLOGY, vol. 46, no. 2, 27 January 2017 (2017-01-27), pages 224-233, XP055413180, GB ISSN: 0307-9457, DOI: 10.1080/03079457.2016.1250866

## Description

### FIELD OF THE INVENTION

The present disclosure relates to the field of medicine, preferably to the field of infectious diseases. In particular the present disclosure relates to influenza proteins and vaccines. Most particularly, the present disclosure relates to the use of any of such proteins or vaccines for the treatment and prevention of influenza infections, furthermore for the prevention of intra- and inter-species transmission of influenza virus.

### BACKGROUND OF THE INVENTION

Influenza infection remains an important infection in animals and humans. Influenza is caused by viruses that undergo continuous antigenic changes/modifications and that possess an animal reservoir. Thus new epidemics and pandemics may occur in the future, and eradication of the disease will be difficult to achieve. Influenza viruses are well known in the art and described more in detail for example by P. Palese, Nature Medicine, vol. 10, no. 12, pp. S 82 to S 86 of December 2004*,* with further references. Briefly, the genome of the influenza A virus consists of eight single-stranded segments, and the viral particles has two major glycoproteins on its surface: hemagglutinin (H) and neuraminidase (N). With at least 16 different hemagglutinin (H1 to H16) and 9 different neuraminidase (N1 to N9) subtypes, there is a considerable antigenic variation among influenza viruses.

Influenza virus of type H5N1 Fowl Plague virus has been demonstrated to infect poultry, pigs and man. The viruses can also be transmitted directly from avian species to humans (Claas et al., Lancet 1998, 351: 472*;* Suarez et al., J. Virol. 1998, 72: 6678*;* Subbarao et al., Science 1998, 279: 393*;* Shortridge, Vaccine 1999, 17 (Suppl. 1): S26-S29*).* Mortality in known human clinical cases approaches about 50%.

Over the last century pigs have been an important vector for influenza pandemics. Pigs, camels, and seals, preferably pigs, can serve as a 'mixing chamber' for avian influenza viruses, and therefore represent a potential risk factor for overcoming the species hurdles from poultry, the naturally reservoir of influenza viruses, to mammals. This normally occurs by double infections of the susceptible animals, e.g. pig, with both, an established mammalian (porcine), as well as an avian influenza virus. This double infection may create new recombinant viruses that may be the cause of human or porcine pandemics. Recent evidence would, however, indicate that a recombination of current avian H5 strains with mammalian influenza viruses will not result in highly virulent recombinants. On the other hand, avian influenza virus can infect pigs and by spontaneous mutations can become adapted to pigs. The critical hurdle will be overcome as soon as the virus can cause horizontal infections within a pig (or other mammalian) population.

Yet, a major part of Southeast Asian pigs have been infected with avian (H5) influenza virus strains originating from neighbouring poultry husbandry. As those infections have so far been sub-clinical, they can only be diagnosed by laboratory methods and thus are frequently overlooked. There is a high risk that those sub-clinically-infected pigs will serve as an opportunity for the virus to adapt to the mammalian system, spread within the porcine population, and also infect human beings.

Current influenza vaccines include a subunit vaccine (Babai et al., Vaccine 1999, 17(9-10):1223-1238*;* Crawford et al., Vaccine 1999, 17(18):2265-2274*;* Johansson et al., Vaccine 1999, 17(15-16):2073-2080*)* attenuated vaccine (Horimoto et al., Vaccine 2004, 22(17-18):2244-2247), DNA vaccine (Watabe et al., Vaccine 2001, 19(31):4434-4444) and inactivated influenza vaccine (Cao et al., Vaccine 1992, 10(4):238-242), with the latter being the most widely used on a commercial scale (Lipatov et al., J Virol 2004, 78(17):8951-8959*).*

Subunit vaccines, recombinant hemagglutinin and neuraminidase (Babai et al., Vaccine 1999, 17(9-10):1223-1238*;* Crawford et al., Vaccine 1999, 17(18):2265-2274*;* Johansson et al., Vaccine 1999, 17(15-16):2073-2080) may be an attractive alternative to the inactivated vaccine, although none are currently in use as commercial vaccines. The preparation of such vaccines is obviously safer than for an inactivated vaccine. Moreover, subunit vaccines do not generate antibody responses to internal influenza viral proteins and thus allow distinction between vaccinated and infected animals (Crawford et al., Vaccine 1999, 17(18):2265-2274)*.*

Hemagglutinin protein is the receptor-binding and membrane fusion glycoprotein of influenza virus and the target for infectivity-neutralizing antibodies. The entire hemagglutinin protein (HA) from the H5N1 is composed of 568 amino acids, with a molecular weight of 56 kDa. The HA molecule consists of HA1 and HA2 subunits, with the HA1 subunit mediating initial contact with the cell membrane and HA2 being responsible for membrane fusion (Chizmadzhev, Bioelectrochemistry 2004, 63(1-2):129-136).

Baculovirus/insect cell systems have been used to express hemagglutinin genes isolated from avian influenza subtypes (Babai et al., Vaccine 1999, 17(9-10):1223-1238*;* Crawford et al., Vaccine 1999, 17(18):2265-2274*;* Johansson et al., Vaccine 1999, 17(15-16):2073-2080*);* Nwe et al., BMC Mircobiology 2006, 6(16):doi:10.1186/1471-2180-6-16)*.* However, those recombinant proteins seem not to be protective in any case, or only less effective at least for some species (Treanor et al., Vaccine 2001, 19: 1732-1737).

The document Lin et al. (J Vet Med Sci. 2008 70(11): 1147-52) discloses the use of a baculovirus/insect cell system for the production of H5 protein of clade 2 H5N1 virus A/duck/China/E319-2/03, which is usable for a prime-booster vaccination for preventing an infection with the clade 2 virus A/duck/China/E319-2/03.

Bright et al. (PLoS One. 2008 30;3(1):e1501) describes the use of a baculovirus/insect cell system for generating virus-like particles (VLPs) which include neuraminidase, hemagglutinin and matrix 1 protein from clade 2 H5N1 virus for inducing a cross-clade protective immune response against a challenge with clade 1 H5N1 virus A/VN/1203/2004 in mice. However, the production of VLPs is not without problems, since in order to generate a functional VLP that effectively mimic a real virus, multiple virus structural proteins are needed which must then be correctly assembled into a particle that reproduces the confirmation of the outer shell (capsid) of the infectious virus. Further, study also reals that in vitro assembly of VLPs competes with aggregation (Ding et al. Biotechnology and Bioengineering 107 (3): 550-560).

WO 2008/052173 A2 discloses a subunit vaccine, but does not disclose any cross protection against different H5N1 clades. Santiago et al 2011 (Vaccine, vol 29, pages 8888-8897) discloses that vaccination of mice with a H5 HA protein induces the production of some cross reacting antibodies.

Thus, there is a need to increase availability of improved vaccines and new vaccination approaches to provide better approaches to control influenza infections and to have a positive impact on disease load. In particular, there is a strong need for a simple, effective and easy-to-handle system inducing, preferably by a single-shot vaccination, a cross-clade protective immune response to influenza viruses with H5N1 HA.

### DESCRIPTION OF THE INVENTION

Before the embodiments of the present invention it shall be noted that as used herein and in the appended claims, the singular forms "a", "an", and "the" include plural reference unless the context clearly dictates otherwise. Thus, for example, reference to "a preparation" includes a plurality of such preparations; reference to the "carrier" is a reference to one or more carriers and equivalents thereof known to those skilled in the art, and so forth. Unless defined otherwise, all technical and scientific terms used herein have the same meanings as commonly understood by one of ordinary skill in the art to which this invention belongs. All given ranges and values may vary by 1 to 5 % unless indicated otherwise or known otherwise by the person skilled in the art, therefore, the term "about" was omitted from the description. Although any methods and materials similar or equivalent to those described herein can be used in the practice or testing of the present invention, the preferred methods, devices, and materials are now described. All publications mentioned herein by reference for the purpose of describing and disclosing the substances, excipients, carriers, and methodologies as reported in the publications which might be used in connection with the invention. Nothing herein is to be construed as an admission that the invention is not entitled to antedate such disclosure by virtue of prior invention.

The solution to the above technical problem is achieved by the description and the embodiments characterized in the claims.

### Influenza proteins and nucleic acid molecules coding for those

The present invention overcomes the problems present in the prior art by providing an H5 protein (1) of clade 1 H5N1 virus for use in a method of treating or preventing infections with H5N1 virus of a different clade, wherein said H5 protein (1) comprises or consists of the polypeptide sequence of SEQ ID NO:5, wherein said H5N1 virus of a different clade is a H5N1 virus comprising a H5 protein (2) of influenza virus, wherein said H5 protein (2) consists of or comprises an amino acid sequence which has at least 98% sequence identity with any one of the sequences as set forth in SEQ ID NOs: 102 to 110, 116, 117, 119 to 126 and 128 to 137.

The present disclosure is based on the surprising finding that H5 protein of clade 1 H5N1 induces, in particular by a single-shot vaccination, a cross-clade protective immune response to influenza viruses with H5N1 HA. As one feature, the H5 protein of clade 1 H5N1 virus, which is for reasons of clarity also termed "H5 protein (1)" herein, comprises or consists of a polypeptide sequence having at least 98% sequence identity with the polypeptide sequence set forth in SEQ ID NO:1.

Sequence identity with a sequence, as mentioned herein, in particular means sequence identity over the length of said sequence or sequence identity over the whole length of said seuqence, respectively.

A "single-shot vaccination" refers to an immunogenic composition that is effective at reducing the incidence of or severity of infection after a single dose thereof, without the need for a booster.

Preferably, the disclosure is thus directed to H5 protein (1) of clade 1 H5N1 virus for use in a method of treating or preventing infections with H5N1 virus of a different clade, namely of a clade different from clade 1 or from any clade with the exception of clade 1, respectively, wherein said H5 protein (1) comprises or consists of a polypeptide sequence having at least 98% sequence identity with the polypeptide sequence of SEQ ID NO:1.

The term "clade" or "clades" as used herein relates to the clade(s) of the WHO Nomenclature System for the highly Pathogenic Avian Influenza Virus (H5N1), which is summarized at the WHO website URL:
who.int/csr/disease/avian_influenza/guidelines/nomenclature/en/ (12.08.2011).

10 distinct initial clades of viruses (numbered 0-9) are defined (WHO/OIE/FAO H5N1 Evolution Working Group, 2008), which are called first order clades. Clades are strictly defined on the nucleotide level as meeting the following three specific clade definition criteria developed by the WHO/OIE/FAO H5N1 Evolution Working Group:
- sharing of a common (clade-defining) node;
- monophyletic grouping with a bootstrap value of ≥60 at the clade-defining node (after 1000 neighbor-joining bootstrap replicates); and
- average percentage pairwise nucleotide distances between and within clades of >1.5% and <1.5%, respectively.

As the viruses within these 10 clades continue to evolve, new sublineages (potential H5N1 clades) periodically emerge. Once these sublineages meet the same three specific clade definition criteria as the initial 10 clades (numbered 0-9), they are designated as separate clades (WHO/OIE/FAO H5N1 Evolution Working Group Emerg. Inf. Dis. 14, 7 (2008). These new clades are defined as second (or third, etc) order clades and assigned a numerical 'address' which links them to their original clade using a hierarchical decimal numbering system. For example, within the antigenically distinct clade 2.3, third order clades meeting the clade definition are designated as clades 2.3.1 and 2.3.2 and so on. This logical hierarchal numbering system is objectively related to HA phylogeny.

The criteria used for the clade designation according to the WHO Nomenclature System for H5N1are:
1 Maintain previously designated clade numbers where possible (i.e., clade 2.2 remains 2.2 and clade 1 remains 1)
2 New clade designations based on phylogenetic tree topology derived from all available sequences (the large tree)
   H5N1 progenitors (closest to Gs/Guangdong/1/96) re-designated as clade 0
   Subsequent clades numbered starting from clade 3 (i.e., clades 3-9)
   Clades designated by presence of a distinct common node shared by at least 4 isolates (in a monophyletic group)
   Additional branches designated as a single clade evolves into more than one distinct lineage (i.e., clade 2.2 or clade 2.3.1; based on sharing of a common node and monophyletic grouping)
3 Average percentage pairwise distances between and within clades (using Kimura 2-parameter)
   Distinct clades should have >1.5% average distances between other clades
   Distinct clades should have <1.5% average distances within the clade (may be slightly higher in clades with highly evolved outliers; i.e., Ck/Shanxi/2/2006 in clade 7)
4 Bootstrap (based on 1,000 neighbor-joining bootstrap replicates) ≥60% bootstrap value at clade-defining node
   (taken from Table 1 of: WHO/OIE/FAO H5N1 Evolution Working Group Emerg. Inf. Dis. 14, 7 (2008)).

The prototype strain for each clade is listed in the following Table:

| Clade | Prototype strain |
|---|---|
| 0 | Gs/Guangdong/1/96 |
| 3 | Ck/Hong Kong/YU562/2001 |
| 4 | Gs/Guiyang/337/2006 |
| 5 | Gs/Guangxi/914/2004 |
| 6 | Ck/Hunan/01/2004 |
| 7 | Ck/Shanxi/2/2006 |
| 8 | Ck/Hong Kong/YU777/2002 |
| 9 | Dk/Guangxi/2775/2005 |
| 1 | Vietnam/1203/2004 |
| 2.1.1 | Ck/Indonesia/BL/2003 |
| 2.1.2 | Indonesia/538H/2006 |
| 2.1.3 | Indonesia/5/2005 |
| 2.2 | BHGs/Qinghai/1A/2005 |
| 2.3.1 | Dk/Hunan/303/2004 |
| 2.3.2 | Ck/Guangxi/2461/2004 |
| 2.3.3 | Ck/Guiyang/3055/2005 |
| 2.3.4 | Dk/Fujian/1734/2005 |
| 2.4 | Ck/Yunnan/115/2004 |
| 2.5 | Ck/Korea/ES/2003 |
| 2.5 | Ck/Korea/ES/2003 |

(taken from Table 2 of: WHO/OIE/FAO H5N1 Evolution Working Group Emerg. Inf. Dis. 14, 7 (2008)).

The publication WHO/OIE/FAO H5N1 Evolution Working Group Emerg. Inf. Dis. 14, 7 (2008) is found at the CDC website URL: cdc.gov/EID/content/14/7/e1.htm (12.08.2011).

An overview of the clade classification of known H5N1 viruses is provided by the phylogenetic tree at the WHO website URL: who.int/csr/disease/avian_influenza/H5CompleteTree.pdf (15.08.2011).

For determining the clade of a H5 protein of H5N1, for example, the web based tool "Highly Pathogenic Avian Influenza (HPAI) H5N1 HA clade prediction" can be used, which is described by Lu, Davis, Rowley, and Donis: "A Web-based tool for the clade designation of highly pathogenic avian influenza H5N1 viruses" in Options for the Control of Influenza VI. J.M. Katz, N. Cox & A.W. Hampson (Eds.) London: Blackwell, 2007 and which is found at the website URL: h5n1.flugenome.org/grouping.php (12.08.2011).

For example, a H5 protein of clade 1 H5N1 virus (H5 protein (1)) is thus a HA with an amino acid sequence encoded by a nucleotide sequence of a clade 1 according to the above-mentioned WHO Nomenclature System for H5N1.

A clade 2.3.1 H5N1 virus, for instance, is hence a H5N1 falling under the criteria of a clade 2.3.1 according to the above-mentioned WHO Nomenclature System for H5N1.

Preferably, the H5 protein (1) according to the disclosure, namely the H5 protein of clade 1 H5N1 virus as described herein, comprises or consists of a polypeptide sequence having at least 98.1%, preferably at least 98.2%, more preferably at least 98.3%, and most preferably at least 98.4% sequence identity with the polypeptide sequence of SEQ ID NO:1.

Sequence identity in the context of the invention is understood as being based on determined pairwise similarity between protein sequences. The determination of percent similarity between two sequences is preferably accomplished using a computational algorithm, in particular the well-known Basic Local Alignment Search Tool (Altschul SF, Gish W, Miller W, Myers EW, Lipman DJ: Basic local alignment search tool. J Mol Biol 1990, 215(3):403-410). For purposes of the present invention, percent sequence identity of an amino acid sequence is determined using the BLAST blastp homology search algorithm using the following parameters: an expected threshold of 10, word size 3, BLOSUM62 matrix, gap opening penalty of 11, a gap extension penalty of 1, and conditional compositional score matrix adjustment. The database to search against is the set of non-redundant protein sequences (nr). The BLAST homology search algorithm is described in Altschul SF (1990),J Mol Biol 1990, 215(3):403-410.

A variant may, for example, differ from the reference accession number BAE07201 molecule without signal pepdtide (N-terminal 16 amino acid residues are not shown in SEQ ID NO:1) by as few as 1 to 15 amino acid residues, as few as 1 to 10 amino acid residues, such as 6-10, as few as 5, as few as 4, 3, 2, or even 1 amino acid residue.

Preferably, the H5 protein (1) according to the disclosure, i.e. the H5 protein (1) of clade 1 H5N1 virus for use in a method of treating or preventing infections with H5N1 virus of a different clade, is preferably a H5 protein of influenza virus, wherein the H5 protein having the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted. Said preferred H5 protein (1) is also termed Mut k+ or mutK+ in the following. Preferably, such H5 protein and any further H5 protein according to the disclosure is an isolated H5 protein.

The term "H5 protein (1) of clade 1 H5N1", as used herein, preferably means "H5 protein (1) as single antigen of clade 1 H5N1 virus" or in particular "H5 protein (1) as single antigen".

The terms "hemagglutinin 5 (H5)" or "H5 of avian influenza virus" or "H5 protein" as used herein are equivalent and mean, but are not limited to any naturally occurring H5 protein and any modified forms of H5 protein, including any deletion, substitution and/or insertion mutant of H5 protein.

The numbering of the amino acid positions of the H5 protein (1) Mut k+ as used herein refers to the amino acid position as exemplarily given in SEQ ID NO:2. SEQ ID NO:2 represents the amino sequence of the hemagglutinin of strain duck/China/E319-2/03 but lacking the amino terminal signal peptide. In other words, if reference is made to the amino acid at position 223 (amino acid 223), the amino acid residue is meant which corresponds to amino acid 223 of SEQ ID NO:2. However, this does not mean that the H5 protein Mut k+ according to the disclosure has the identical amino acid sequence with SEQ ID NO:2. It only says, that the corresponding amino acids of the H5 proteins according to the disclosure code for the amino acid residue, as explicitly mentioned. In the current case, amino acid 223 would be Serine (S). The terms "223N", or "155N" exemplarily mean, that the amino acid at positions 223 and 155, respectively - numbering according to the amino acid positions of SEQ ID NO:2 -,that shall code for the amino acid Asparagine (N). In other words, if reference is made to "H5 protein (1) having the amino acid 223N", a H5 amino acid molecule that normally codes for Serine at amino acid position 223 - numbering according to the amino acid positions of SEQ ID NO:2 - that amino acid shall be substituted by an Asparagine (N). The term "328K+" or "modification 328K+" means, that at amino acid position 328 of H5 protein - numbering according to the amino acid positions of SEQ ID NO:2 - , a second Lysine (K+) is inserted. In cases were amino acids sequences at positions 328 and 329 naturally codes for Lysine-Lysine, no further Lysine (K) shall be inserted. However, most of the known H5 sequences code at amino acid positions 328 and 329 for Lysine-Arginine. In any such cases, the term 328K+ modification means, that a second Lysine (K) shall be inserted between Lysine at position 328 and Arginine at position 329. The modified sequence would read then Lysine-Lysine-Arginine (KKR).

Regarding the present example, the hemagglutinin of strain duck/China/E319-2/03 is shifted to a H5 protein (1) of clade 1 H5N1, since it resembles the H5 sequence of the clade 1 H5N1 virus A/HongKong/213/2003, the year/location/host of this HK isolate, and shows reactivity with clade-1-specific antibodies. Hence the Mut K+ sequence is classified as a H5 sequence of a clade 1 H5N1. Within the context of the disclosure, the designed Mut K+ sequence is thus understood and defined to be a H5 protein of clade 1 H5N1 virus.

Thus, in particular also any designed H5 protein is understood and defined as a H5 protein of clade 1 H5N1 virus according to the disclosure, if it is encoded by a nucleotide sequence which fulfils the criteria of a nucleotide sequence of a clade 1 according to the above-mentioned WHO Nomenclature System for H5N1.

Disclosed is a H5 protein and any modified forms of H5 protein, including any deletion, substitution and/or insertion mutant of H5 protein, wherein those H5 proteins having the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted. It is self-explanatory, that any of the H5 proteins as provided herewith are antigenic, which mean they show antigenic properties in a standard hemagglutinin inhibition assay for influenza viruses.

The present disclosure also relates to any part of the H5 protein (1), which means any peptide-fragment which shows antigenic properties in an standard hemagglutinin inhibition assay, having in one aspect at least the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted.

A H5 protein (1) shows antigenic properties if it inhibits hemagglutination in a standard hemagglutinin inhibition assay, for example, as described in Example 2. Normally said antigenic part of H5 protein (1) comprises 200, 180, 160, 150, 140, 130, 120, 110 or most preferably 105 contiguous amino acids of the amino acid sequence that codes for the H5 protein as mentioned above, modified or non-modified, which shows antigenic properties in an standard hemagglutinin inhibition assay as described in Example 2. A standard hemagglutinin inhibition assay for example is also described in Stephenson et al., Virus Research vol. 103, pp. 91-95 (2004) with further references. However, the HI assay as described in Example 2 shall be understood to be the relevant reference assay in connection with all aspects of the disclosure as described herein:
Briefly, HI assay was performed to detect the presence of HA-specific antibodies. A heterologous H5N2 virus, A/chicken/Mexico/232/94, was used at a concentration of four hemagglutinating units [4 HA units] in the HI assay. In U-bottomed microtiter plates serial two-fold serum dilutions in PBS were subsequently mixed with equal volumes (25 µL) containing 4 HA units of virus, and incubated at room temperature (about 25°C) for 30 min. Chicken red blood cells, at a concentration of 0.5% in PBS, were added to the serum-virus containing wells and incubated for 40 min at room temperature. The HI titers were determined as reciprocals of the highest serum dilutions in which inhibition of hemagglutination was observed.

Of note, *Haesebrouck and Pensaert (1986)* found "that there may exist a correlation between the HI titers against the challenge virus and protection from challenge". *Haesebrouck and Pensaert (1986)* also determined that pigs with HI titers of ≥40 were "completely resistant to challenge and no replication of the virus occurred in the respiratory tract at challenge". Thus, the development of HI titers ≥40 in the vaccinated swine would correlate to protection. (*F. Haesebrouck and M.B. Pensaert, 1986*). Effect of intratracheal challenge of fattening pigs previously immunized with an inactivated influenza H1N1 vaccine (Veterinary Microbiology, 11 (1986) 239--249*.* It has to assume that equivalent or at least nearly equivalent H5 HI titers will also result in a complete immune protection of swine against avian influenza virus. Lower titers, at least result in a seroconversion of the vaccinated animals and result in partial immune protection of those animals, which also can dramatically reduce the risk of a pandemics.

Moreover, an antigenic part of the H5 protein (1) according to the disclosure includes, but is not limited to deletion mutants of H5 protein, which comprises:
i. at least 35, 30, 25, 20, 18, 15, 13, 10, 9, or most preferably 8 contiguous amino acids of the amino acid sequence that surrounds and includes the amino acid 223N; and
ii. at least 35, 30, 25, 20, 18, 15, 13, 10, 9, or most preferably 8 contiguous amino acids of the amino acid sequence that surrounds and includes the amino acid modification 328K+, and
iii. wherein any of such antigenic part of H5 protein shows hemagglutinin inhibition in a standard hemagglutinin inhibition assay as described in Example 2.

Preferably, those surrounding amino acids of amino acid 223N and/or 328K+ are encoded by SEQ ID NO:2 or SEQ ID NO:5.

Furthermore preferred H5 proteins (1) according to the disclosure are:
i. any of those mentioned above having the amino acid 223N and the modification 328K+;
ii. any of those mentioned above having the amino acid 94N/223N and the modification 328K+;
iii. any H5 protein of avian origin having the amino acid 223N, and the modification 328K+, wherein avian origin means that the H5 sequence derived form a virus isolate that was originally isolated from a poultry infected with avian influenza virus type 5; or
iv. any H5 protein of avian origin having the amino acids 94N/223N and the modification 328K+, wherein avian origin means that the H5 sequence derived from a virus isolate that was originally isolated from poultry infected with avian influenza virus type 5; or
v. any H5 protein of avian origin having the amino acids 155N/223N and the modification 328K+, wherein avian origin means that the H5 sequence derived from a virus isolate that was originally isolated from poultry infected with avian influenza virus type 5; or
vi. any H5 protein of avian origin having the amino acid 120N/155N/223N and the modification 328K+, wherein avian origin means that the H5 sequence derived from a virus isolate that was originally isolated from poultry infected with avian influenza virus type 5; or
vii. any H5 protein having the modifications 94N/223N and the modification 328K+; or
viii. any H5 protein having the modifications 94N/155N/223N and the modification 328K+; or;
ix. any H5 protein having the modifications 94N/120N/155N/223N and the modification 328K+; or
x. any H5 protein having the modifications 223N, the modification 328K+, and one or more of the following amino acid clusters selected from the group consisting of:
   a. aa 93 - 95: GNF
   b. aa 123 - 125: SDH
   c. aa 128 - 130: SSG
   d. aa 138 - 140: GSS
   e. aa 226 - 228: MDF
   f. aa 270 - 272: EVE
   g. aa 309 - 311: NKL; or
xi. any H5 protein having the amino acid 223N, and the modification 328K+, and one or more of the following amino acid clusters selected from the group consisting of:
   a. aa 93 - 95: GNF
   b. aa 128 - 130: SSG
   c. aa 138 - 140: GSS; or
xii. any H5 protein having the amino acid sequence of SEQ ID NO:5.

Furthermore preferred H5 proteins (1) as disclosed herewith include the H5 proteins as described by Hoffmann et al, PNAS, vol. 106, no.36, pp. 12915-12920 of September 6, 2005*,* wherein that H5 proteins includes one or more of the modifications as described above, at least the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted.

Furthermore preferred H5 proteins (1) as disclosed herewith include H5 proteins which comprise a peptide that comprises the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted, and:
i. the amino acid sequences of SEQ ID NO:2, SEQ ID NO:3, SEQ ID NO:4; SEQ ID NO:5; SEQ ID NO:6 or SEQ ID NO:7 or;
ii. any peptide that has at least 85% sequence homology, more preferably at least about 90% sequence homology, still more preferably at least about 95% sequence homology, even more preferably at least about 97% sequence homology, still even more preferably at least about 98% sequence homology, and even more preferably at least about 99% sequence homology to the polypeptide of i) that comprises hemagglutinin inhibition in a standard hemagglutinin inhibition as described above; or
iii. any antigenic part of the polypeptides of i) or ii) comprising at least 35, 30, 25, 20, 18, 15, 13, 10, 9, or most preferably 8 contiguous amino acids of any of peptides of i) or ii).
iv. any peptides of i), ii) or iii) having the amino acids 36T, 36K, 83A, 83T, 83D, 86A, 86V, 120N, 120S, 155N, 155S, 156A, 156T, 189R, 189K, 212K, 212R, 212E, 223N, 223N, or 120N/155N.
v. any peptide of i), ii), iii) or iv) having one or more of the following amino acid clusters selected from the group consisting of:
   a. aa 93 - 95: GNF
   b. aa 123 - 125: SDH
   c. aa 128 - 130: SSG
   d. aa 138 - 140: GSS
   e. aa 226 - 228: MDF
   f. aa 270 - 272: EVE
   g. aa 309 - 311: NKL; or
vi. any peptide of i), ii) iii) or iv) having one or more of the following amino acid clusters selected from the group consisting of:
   a. aa 93 - 95: GNF
   b. aa 128 - 130: SSG
   c. aa 138 - 140: GSS.

"Sequence homology", as used herein, refers to a method of determining the relatedness of two sequences. To determine sequence homology, two or more sequences are optimally aligned, and gaps are introduced if necessary. In contrast to sequence identity, conservative amino acid substitutions are counted as a match when determining sequence homology. In other words, to obtain a polypeptide or polynucleotide having 95% sequence homology with a reference sequence, 85%, preferably 90%, even more preferably 95% of the amino acid residues or nucleotides in the reference sequence must match or comprise a conservative substitution with another amino acid or nucleotide, or a number of amino acids or nucleotides up to 15%, preferably up to 10%, even more preferably up to 5% of the total amino acid residues or nucleotides, not including conservative substitutions, in the reference sequence may be inserted into the reference sequence. Preferably the homolog sequence comprises at least a stretch of 50, even more preferred of 100, even more preferred of 250, even more preferred of 500 nucleotides. Upon such alignment, sequence homology is ascertained on a position-by-position basis, e.g., the sequences are "homolog" at a particular position if at that position, the nucleotides or amino acid residues are identical. The total number of such position identities is then divided by the total number of nucleotides or amino acid residues in the reference sequence to give % sequence homology. Sequence homology can be readily calculated by known methods, including but not limited to, those described in Computational Molecular Biology, Lesk, A. N., ed., Oxford University Press, New York (1988), Biocomputing: Informatics and Genome Projects, Smith, D.W., ed., Academic Press, New York (1993); Computer Analysis of Sequence Data, Part I, Griffin, A.M., and Griffin, H. G., eds., Humana Press, New Jersey (1994); Sequence Analysis in Molecular Biology, von Heinge, G., Academic Press (1987); Sequence Analysis Primer, Gribskov, M. and Devereux, J., eds., M. Stockton Press, New York (1991); and Carillo, H., and Lipman, D., SIAM J. Applied Math., 48: 1073 (1988). Preferred methods to determine the sequence homology are designed to give the largest match between the sequences tested. Methods to determine sequence homology are codified in publicly available computer programs which determine sequence identity between given sequences. Examples of such programs include, but are not limited to, the GCG program package (Devereux, J., et al., Nucleic Acids Research, 12(1):387 (1984)), BLASTP, BLASTN and FASTA (Altschul, S. F. et al., J. Molec. Biol., 215:403-410 (1990). The BLASTX program is publicly available from NCBI and other sources (BLAST Manual, Altschul, S. et al., NCVI NLM NIH Bethesda, MD 20894, Altschul, S. F. et al., J. Molec. Biol., 215:403-410 (1990)). These programs optimally align sequences using default gap weights in order to produce the highest level of sequence homology between the given and reference sequences.

Furthermore preferred H5 proteins (1) include H5 proteins which comprise the 328K+ modification as mentioned above, and the amino acid sequence provided in TABLE 1, or any immunogenic part thereof:

**TABLE 1 H5 antigens**

| **Sequence name** | **Basic- sequence** | **Amino acid positions^{#}** | | | | | | | | | |
|---|---|---|---|---|---|---|---|---|---|---|---|
| | | **36** | **83** | **86** | **120** | **155** | **156** | **189** | **212** | **223** | **263** |
| 223N/328K+ | any HA H5 | - | - | - | - | - | - | - | - | N | - |
| 36T/223N/328K+ | any HA H5 | T | - | - | - | - | - | - | - | N | - |
| 36K/223N/328k+ | any HA H5 | K | - | - | - | - | - | - | - | N | - |
| 83A/223N/328k+ | any HA H5 | - | A | - | - | - | - | - | - | N | - |
| 83T/223N/328k+ | any HA H5 | - | T | - | - | - | - | - | - | N | - |
| 83D/223N/328k+ | any HA H5 | - | D | - | - | - | - | - | - | N | - |
| 86A/223N/328k+ | any HA H5 | - | - | A | - | - | - | - | - | N | - |
| 86V/223N/328k+ | any HA H5 | - | - | V | - | - | - | - | - | N | - |
| 120N/223N/328k+ | any HA H5 | - | - | - | N | - | - | - | - | N | - |
| 120S/223N/328k+ | any HA H5 | - | - | - | S | - | - | - | - | N | - |
| 155N/223N/328k+ | any HA H5 | - | - | - | - | N | - | - | - | N | - |
| 155S/223N/328k+ | any HA H5 | - | - | - | - | S | - | - | - | N | - |
| 156A/223N/328k+ | any HA H5 | - | - | - | - | - | A | - | - | N | - |
| 156T/223N/328k+ | any HA H5 | - | - | - | - | - | T | - | - | N | - |
| 189R/223N/328k+ | any HA H5 | - | - | - | - | - | - | R | - | N | - |
| 189K/223N/328k+ | any HA H5 | - | - | - | - | - | - | K | - | N | - |
| 212K/223N/328k+ | any HA H5 | - | - | - | - | - | - | - | K | N | - |
| 212R/223N/328k+ | any HA H5 | - | - | - | - | - | - | - | R | N | - |
| 212E/223N/328k+ | any HA H5 | - | - | - | - | - | - | - | E | N | - |
| 223N/263A/328k+ | any HA H5 | - | - | - | - | - | - | - | - | N | A |
| 223N/263T/328k+ | any HA H5 | - | - | - | - | - | - | - | - | N | T |
| 120N/155N/223N/328k+ | any HA H5 | - | - | - | N | N | - | - | - | N | - |
| A/duck/China/E319-2/03/328k+ | AAR99628 | T | A | A | S | D | A | R | K | N | A |
| A/duck/China/E319-2/03_223N/328k+ | AAR99628 | T | A | A | S | D | A | R | K | N | A |
| A/duck/China/E319-2/03_120N/223N/328k+ | AAR99628 | T | A | A | N | D | A | R | K | N | A |
| A/duck/China/E319-2/03_155N/223N/328k+ | AAR99628 | T | A | A | S | N | A | R | K | N | A |
| A/duck/China/E319-2/03_120N/155N/223N/32 8k+ | AAR99628 | T | A | A | S | N | N | R | K | N | A |
| HA/HK/213/03/328k+ | AY518362 | T | A | A | N | N | A | R | K | N | A |
| HA/Vietnam/1203/04 | | K | T | V | S | S | T | K | R | N | T |
| HA/Vietnam/1203/04 _223N/328k+ | | K | T | V | S | S | T | K | R | N | T |
| HA//Vietnam/3046/04 _223N/328k+ | | T | A | V | S | S | T | K | R | N | T |
| HA/Vietnam/3062/04 _223N/328k+ | | T | A | V | S | S | T | K | R | N | T |
| HA/chicken/Vietnam/ 39/04_223N/328k+ | | T | A | V | S | S | T | K | R | N | T |
| HA/falcon/HK-D0028/04_223N/328k+ | | T | A | A | S | S | A | K | E | N | A |
| HA/duck/Singapore/3/97 223N/328k+ | | T | D | V | S | N | A | K | E | N | A |
| HA/HK/156/97/328k+ | | T | A | A | S | S | A | K | E | N | T |

| | | | | | | | | | | | |
|---|---|---|---|---|---|---|---|---|---|---|---|
| ^{#} the amino acid positions given in TABLE 1 refers to the positions as exemplarily defined in SEQ ID NO:2. In other words amino acid 223 of TABLE 1 refers to the amino acid 223 of the sequence of SEQ ID NO:2. - means that the amino acids at this positions are variable as compared to the reference sequence. | | | | | | | | | | | |

Furthermore, the present disclosure also relates to H5 proteins (1) having at least the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted, and comprises:
i. a peptide having the sequences of NCBI Accession No. AAT65209, CAJ32556, ABC47656, CAF21874, CAF21870, AAC58998, AAC58997, AAC58996, AAC58994, AAC58993, AAC58992, AAC58991, AAC58990, AAC58995, AAS45134, AAN 17270, AAN 17269, AAN 17268, AAN 17267, AAN 17266, AAN 17265, AAN 17264, AAN 17263, AAN 17262, AAN 17261, AAN 17260, AAN 17259, AAN 17257, AAN 17256, AAN 17255, AAN 17254, AAA43083, AAA43082, AAB19079, BAE48696, BAE48693, BAE48696, BAE48695, BAE48694, BAE48692, BAE48691, BAE48690, BAE48689, BAE48688, BAE48687, BAE48686, BAE48685, BAE48684, BAE48683, AAC58999, ABC72082, AAV91149, AAP71993, AAP71992, AAP71991, AAP71990, AAP71989, AAP72011, AAP72010, AAP72009, AAP72008, AAP72007, AAP72006, AAP72005, AAP72004, AAP72003, AAP72002, AAP72001, AAP72000, AAP71999, AAP71998, AAP71997, AAP71996, AAP71995, AAP71994, AAF99718, ABF58847, AAG38534, AAC32102, AAC32099, AAL75847, AAC32101, AAC32098, AAC32088, AAC32078, AAR99628, AAC32100, AAM49555, AAL75843, AAL75839, AAD13573, AAD13568, AAF04720, AAF04719, AAC34263, AAR16155, AAD13574, AAD13570, AAD13575, AAD13572, AAD13569, AAD13567, AAD13566, AAK57506, AAG01225, AAG01215, AAG01205, AAG01195, or ABD83813 modified in a manner described above, which means that those sequences include the above-mentioned modifications 223N and 328 K+ which are not part of the wild-type sequences; or
ii. any peptide that has at least 85% sequence homology, more preferably at least about 90% sequence homology, still more preferably at least about 95% sequence homology, even more preferably at least about 97% sequence homology, still even more preferably at least about 98% sequence homology, and even more preferably at least about 99% sequence homology to the polypeptide of i) and that show hemagglutinin inhibition in a standard hemagglutinin inhibition as described above;
iii. any of the peptides of i) or ii) having the amino acids 36T, 36K, 83A, 83T, 83D, 86A, 86V, 120N, 120S, 155N, 155S, 156A, 156T, 189R, 189K, 212K, 212R, 212E, 263A, 263T, or 120N/155N; or
iv. any of such peptides of i), ii), or iii) having one or more of the following amino acid clusters selected from the group consisting of:
   a. aa 93 - 95: GNF
   b. aa 123 - 125 SDH
   c. aa 128 - 130: SSG
   d. aa 138 - 140: GSS
   e. aa 226 - 228: MDF
   f. aa 270 - 272: EVE
   g. aa 309 - 311: NKL; or
v. any peptide of i), ii) iii) or iv) having one or more of the following amino acid clusters selected from the group consisting of:
   a. aa 93 - 95: GNF
   b. aa 128 - 130: SSG
   c. aa 138 - 140: GSS

Preferably, the H5 protein (1) for use in a method of treating or preventing infections with H5N1 virus of a different clade is recombinantly expressed and/or produced by a baculovirus expression system, preferably in cultured insect cells.

The term "H5 protein (1)" as mentioned herein is thus, in particular, equivalent to the term "recombinant H5 protein" used herein.

Regarding the H5N1 virus of a different clade, as mentioned herein, said H5N1 virus of a different clade is preferably selected from the group consisting of clade 0 H5N1 virus, clade 2 H5N1 virus, clade 3 H5N1 virus, clade 4 H5N1 virus, clade 5 H5N1 virus, clade 6 H5N1 virus, clade 7 H5N1 virus, clade 8 H5N1 virus and clade 9 H5N1 virus.

Preferably, the H5N1 virus of a different clade is clade 2.2 H5N1 virus or a clade 2.3 H5N1 virus.

Preferably, the H5N1 virus of a different clade is a clade 2.2.1 H5N1 virus or a clade 2.3.2 H5N1 virus.

For reasons of clarity, H5 protein of the H5N1 virus of a different clade is termed "H5 protein (2)" hereinafter. Hence, H5 protein (2) as mentioned herein is in particular a H5 protein coded by the genome of a H5N1 of any clade with the exception of clade 1.

Preferably, the H5N1 virus of a different clade is a H5N1 virus of North African or of Vietnamese origin, wherein said H5N1 virus of North African origin is preferabyl a H5N1 virus comprising a H5 protein (2) of influenza virus,
wherein said H5 protein (2) has
(a) the amino acids 113D, 126H, 145(-), 156R, 160F, 167T, and 181N, wherein the modification 145(-) means that amino acid position 145 of H5 is deleted, or
(b) the amino acids 87P, 145L, 172T, 201E, 206I, 208K, 254T, 341G and 421K, or
(c) the amino acids 145L, 172T, and 254V,
and wherein the numbering of the amino acid positions of the H5 protein (2) refers to the amino acid position as exemplarily given in SEQ ID NO:8;
or wherein said H5 protein (2) consists of or comprises an amino acid sequence which is at least 95%, preferably at least 96%, more preferably at least 97%, still more preferably at least 98%, yet more preferably at least 99%, or in particular preferred 100% homolog with any one of the sequences as set forth in SEQ ID NOs: 62 to 137 or 9 to 46.

Preferably, the H5N1 virus of a different clade is a H5N1 virus of North African or of Vietnamese origin, wherein said H5N1 virus of North African origin is preferabyl a H5N1 virus comprising a polynucleotide encoding a H5 protein (2) of influenza virus,
wherein said H5 protein (2) has
(a) the amino acids 113D, 126H, 145(-), 156R, 160F, 167T, and 181N, wherein the modification 145(-) means that amino acid position 145 of H5 is deleted, or
(b) the amino acids 87P, 145L, 172T, 201E, 206I, 208K, 254T, 341G and 421K, or
(c) the amino acids 145L, 172T, and 254V,
and wherein the numbering of the amino acid positions of the H5 protein (2) refers to the amino acid position as exemplarily given in SEQ ID NO:8;
or wherein said H5 protein (2) consists of or comprises an amino acid sequence which is at least 95%, preferably at least 96%, more preferably at least 97%, still more preferably at least 98%, yet more preferably at least 99%, or in particular preferred 100% homolog with any one of the sequences as set forth in SEQ ID NOs: 62 to 137 or 9 to 46.

In the context of the disclosure, said H5 protein (2) according to (a) is a Subclade A protein, and said H5 protein according to (b) or (c) is a Subclade B protein.

It is thus understood that such a H5 protein (2) of North African origin could be, for example, a sequence as set forth in SEQ ID NO: 140, which corresponds to SEQ ID NO: 8 without signal peptide (the N-terminal 16 amino acid residues of SEQ ID NO: 8 are not included in SEQ ID NO: 140), and thus starts with the N-terminal amino acid D17, but wherein said sequence has
(a) the amino acids 113D, 126H, 145(-), 156R, 160F, 167T, and 181N, wherein the modification 145(-) means that amino acid position 145 of H5 is deleted, or
(b) the amino acids 87P, 145L, 172T, 201E, 206I, 208K, 254T, 341G and 421K, or
(c) the amino acids 145L, 172T, and 254V,
wherein the numbering as exemplarily given in SEQ ID NO:8 is used.

As described herein it is understood that the term "H5N1 virus comprising a polynucleotide encoding a H5 protein (2)" is equivalent to "H5N1 virus comprising a polynucleotide which comprises a sequence encoding a H5 protein (2)" or to "H5N1 virus comprising a polynucleotide comprising a sequence which encodes a H5 protein (2)", respectively.

Within the context of the invention, it is understood that the term "amino acid" in particular refers to an amino acid residue or, respectively, to an amino acid which has been covalently linked via peptide bonds to two further amino acids or, if the amino acid is N- or C-terminally located in the peptide sequence, to one further amino acid.

The H5 protein (2), as described herein, in particular consists of or comprises an amino acid sequence which is at least 95%, preferably 96%, more preferably 97%, still more preferably 98%, yet more preferably 99% or in particular preferrably 100% homologous with any one of the sequences as set forth in SEQ ID NOs: 9 to 47. Preferably, the H5 protein (2) consists of or comprises an amino acid sequence which is at least 95%, preferably 96%, more preferably 97%, still more preferably 98%, yet more preferably 99% or in particular preferrably 100% homologous with any one of the sequences as set forth in SEQ ID NOs: 15, 20, or 47. In particular, H5 proteins (2) that comprise or consist of the amino acid sequence set forth in SEQ ID NO: 20 are preferred.

Preferably, the H5 protein (2), as described herein, has the amino acid 239N. The term "239N" exemplarily means, that the amino acid at position 239-numbering according to the amino acid positions of SEQ ID NO:8-shall code for the amino acid Asparagine (N). In other words, if reference is made to "H5 protein (2) having the amino acid 239N", a H5 amino acid molecule that normally codes for Serine at amino acid position 239-numbering according to the amino acid positions of SEQ ID NO:8-that amino acid shall be substituted by an Asparagine (N).

Preferably, the H5 protein (2), as described herein, does not comprise the influenza A H5N1 hemagglutinin signal sequence, wherein the H5 protein (2) influenza A H5N1 hemagglutinin signal sequence preferably comprises or consists of the first 16 N-terminal amino acids (one letter code) MEKIVLLLAIVSLVKS (SEQ ID NO:51) or M1 E2 K3 I4 V5 L6 L7 L8 A9 I10 V11 S12 L13 V14 K15 S16, respectively, wherein the numbering of said first N-terminal 16 amino acid positions refers to the amino acid positions 1 to 16 as exemplarily given in SEQ ID NO 8, namely referring to the sequence (three letter code) Met Glu Lys Ile Val Leu Leu Leu Ala Ile Val Ser Leu Val Lys Ser (SEQ ID NO:51) as set forth in SEQ ID NO:8.

Preferably, the H5 protein (2), as described herein, thus consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 9 to 47, wherein the sequences as set forth in SEQ ID NOs: 9 to 47 start with amino acid D17, and wherein the numbering of the first N-terminal amino acid position (D17) refers to the amino acid position 17 as exemplarily given in SEQ ID NO 8.

Table B provides the respective sequences SEQ ID NOs: 62 to 99 that correspond to variants of the sequences as set forth in SEQ ID NOs: 9 to 46, wherein SEQ ID NOs: 62 to 99 start with the respective amino acid D17 of SEQ ID NOs: 9 to 46, as mentioned herein, and Table D provides the respective variant of sequence SEQ ID NO: 47, i.e. SEQ ID NO: 138, wherein the variant sequence starts with amino acid D17, as mentioned herein.

In one example, the H5 protein (2), as described herein, thus preferably consists of or comprises an amino acid sequence which is at least 95%, preferably 96%, more preferably 97%, still more preferably 98%, yet more preferably 99% or in particular preferably 100% homologous with the polypeptide sequence DQICIGYHANNSTEQVDTIMEKNVTVTHAQDILEKTHNGKLCNLDGVKPLILRDCSVAGWLLGNPMCDEFLNVPEWSYIVEKINPTNDLCYPGNFNDYEELKHLLSRINHFEKIQIIPKNSWSDHEASGVSSACPYQGRSSFFRNVVWLTKKNNAYPTIKKSYNNTNQEDLLVLWGIHHPNDAAEQTRLYQNPTTYISVGTSTLNQRLVPKIATRSKVNGQSGRMEFFWTILKSNDAINFESNGNFIAPENAYKIVKKGDSTIMKSELEYGDCNTKCQTPIGAINSSMPFHNIHPLTIGECPKYVKSNRLVLATGLRNSPQGERRRKKRGLFGAIAGFIEGGWQGMVDGWYGYHHSNEQGSGYAADKESTQKAIDGVTNKVNSIIDKMNTQFEAVGREFNNLERRIENLNKKMEDGFLDVWTYNAELLVLMENERTLDFHDSNVKNLYDKVRLQLRDNAKELGNGCFEFYHRCDNECMESVRNGTYDYPQYSEEARLKREEISGVKLESIGTYQILSIYSTVASSLALAIMV (SEQ ID NO:52), wherein this sequence corresponds to a variant of SEQ ID NO:15, wherein the variant starts with amino acid D17 of SEQ ID NO: 15, wherein the numbering of the N-terminal amino acid position (D17) refers to the amino acid position 17 as exemplarily given in SEQ ID NO 8.

In another example, the H5 protein (2), as described herein, thus preferably consists of or comprises an amino acid sequence which is at least 95%, preferably 96%, more preferably 97%, still more preferably 98%, yet more preferably 99% or in particular preferably 100% homologous with the polypeptide sequence DQICIGYHANNSTEQVDTIMEKNVTVTHAQDILEKTHNGKLCDLGGVKPLILRDCSVAGWLLGNPMCDEFPNVSEWSYIVEKINPANDLCYPGNFNNYEELKHLLSRINRFEKIQIIPKSSWPDHEASLGVSSACPYQGGPSFYRNVVWLIKKNDTYPTIKESYHNTNQEDLLVLWGIHHPNNEEEQKRIYKNPTTYVSVGTSTLNQRLVPKIATRSKVNGQSGRVEFFWTILKSNDTINFESNGNFIAPENAYKIVKKGDSTIMKSELEYGNCSTKCQTPIGAINTSMPFHNIHPLTIGECPKYVKSNRLVLATGLRNSPQGEGRRKKRGLFGAIAGFIEGGWQGMVDGWYGYHHSNEQGSGYAADKESTQKAIDGVTNKVNSIIDKMNTQFEAVGREFNNLEKRIENLNKKMEDGFLDVWTYNAELLVLMENERTLDFHDSNVRNLYDKVRLQLRDNAKELGNGCFEFYHRCDNECME SVRNGTYDYPQYSEEARLKREEISGVKLESIGTYQILSIYSTVASSLALAIMVAGLFLWMCSNGSLQCRICI, (SEQ ID NO: 53) wherein the sequence corresponds to SEQ ID NO:20 starting with amino acid D17, and wherein the numbering of the first N-terminal amino acid position (D17) refers to the amino acid position 17 as exemplarily given in SEQ ID NO: 8.

It is thus understood, that the term "variants of SEQ ID NOs: 9 to 47" is preferably also directed to the variant sequences that start with amino acid D17 of SEQ ID NOs: 9 to 47, and wherein the variant sequences of SEQ ID NOs 9 to 46 starting with amino acid D17 are given in Table B and correspond to SEQ ID NOs: 62 to 99, and wherein the sequence of SEQ ID NO: 47 starting with amino acid D17 has the sequence

Preferably, the H5 protein (2), as described herein, comprises a full length C-terminal sequence, wherein the full length C-terminal sequence preferably corresponds to amino acids 527 to 568, wherein the numbering of said amino acid positions refers to the amino acid positions 527 to 568 as exemplarily given in SEQ ID NO 47. The full length C-terminal sequence is thus preferably the sequence IGTYQILSIYSTVASSLALAIMVAGLFLWMCSNGSLQCRICI (SEQ ID NO: 55), wherein in a further preferred aspect the Phenyl alanine residue (F) is a Serine (S) residue or wherein in another preferred aspect the sequence IMVA (SEQ ID NO: 56) is subsituted by the sequence IMMA (SEQ ID NO: 57). Hence the full length C-terminal
sequence is thus preferably the sequence
IGTYQILSIYSTVASSLALAIMVAGLFLWMCSNGSLQCRICI (SEQ ID NO: 58) or the sequence IGTYQILSIYSTVASSLALAIMVAGLSLWMCSNGSLQCRICI (SEQ ID NO: 59) or the sequence IGTYQILSIYSTVASSLALAIMMAGLSLWMCSNGSLQCRICI (SEQ ID NO: 60).

Preferably, the H5 protein (2), as described herein, thus consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 9 to 47, sequences as set forth in SEQ ID NOs: 54, 100-137 that correspond to variants of SEQ ID NOs: 9 to 47 that start with amino acid D17 and additionally comprise a full length C-terminal sequence, wherein the numbering of the first N-terminal amino acid position (D17) refers to the amino acid position 17 as exemplarily given in SEQ ID NO 8, and wherein the full length C-terminal sequence corresponds to amino acids 527 to 568, wherein the numbering of these amino acid positions refers to the amino acid positions 527 to 568 as exemplarily given in SEQ ID NO 47.

Table C provides the respective sequences SEQ ID NOs 100 to 137, wherein the sequences start with amino acid D17 and comprise a full-length C-terminal sequence, as described herein, and Table D provides the respective sequence SEQ ID NO: 138, wherein the sequence starts with amino acid D17 of SEQ ID NO: 47 and comprises a full-length C-terminal sequence, as described herein.

In one example, the H5 protein (2), as described herein, thus preferably consists of or comprises an amino acid sequence which is at least 95%, preferably 96%, more preferably 97%, still more preferably 98%, yet more preferably 99% or in particular preferably 100% homologous with the polypeptide sequence DQICIGYHANNSTEQVDTIMEKNVTVTHAQDILEKTHNGKLCNLDGVKPLILRDCSVAGWLLGNPMCDEFLNVPEWSYIVEKINPTNDLCYPGNFNDYEELKHLLSRINHFEKIQIIPKNSWSDHEASGVSSACPYQGRSSFFRNVVWLTKKNNAYPTIKKSYNNTNQEDLLVLWGIHHPNDAAEQTRLYQNPTTYISVGTSTLNQRLVPKIATRSKVNGQSGRMEFFWTILKSNDAINFESNGNFIAPENAYKIVKKGDSTIMKSELEYGDCNTKCQTPIGAINSSMPFHNIHPLTIGECPKYVKSNRLVLATGLRNSPQGERRRKKRGLFGAIAGFIEGGWQGMVDGWYGYHHSNEQGSGYAADKESTQKAIDGVTNKVNSIIDKMNTQFEAVGREFNNLERRIENLNKKMEDGFLDVWTYNAELLVLMENERTLDFHDSNVKNLYDKVRLQLRDNAKELGNGCFEFYHRCDNECMESVRNGTYDYPQYSEEARLKREEISGVKLESIGTYQILSIYSTVASSLALAIMVAGLFLWMCSNGSLQCRICI (SEQ ID NO:52), wherein said sequence corresponds to SEQ ID NO:15 starting with amino acid D17, and additionally comprising a full length C-terminal sequence, wherein the numbering of said first N-terminal amino acid position (D17) refers to the amino acid position 17 as exemplarily given in SEQ ID NO 8, and wherein the full length C-terminal sequence corresponds to amino acids 527 to 568, more particular to amino acids 534 to 568, wherein the numbering of said amino acid positions refers to the amino acid positions 527 to 568, or more particular to amino acid positions 534 to 568, as exemplarily given in SEQ ID NO 47. Herein, this sequence, SEQ ID NO: 52, and sequences having different C-terminal sequences may be referred to as a "variant of SEQ ID NO: 15".

It is thus further understood, that the term "variants of SEQ ID NOs 9 to 47" is preferably also directed to the sequences SEQ ID NOs: 9 to 47 starting with amino acid D17 and having the full length C-terminal sequence, and wherein said sequences SEQ ID No 9 to 46 starting with amino acid D17 and having the full length C-terminal sequence are given in Table C, and said sequence SEQ ID NO: 47 starting with amino acid D17 and having the full length C-terminal sequence is given in Table D.

The terms "variant sequences as set forth in SEQ ID NOs: 9 to 47" or "variant sequences of SEQ ID NOs: 9 to 47", as used herein, preferably also relate to the sequences as set forth in Table B or Table C, respectively, and, additionally the sequence as set forth in Table D, respectively.

Preferably, the H5N1 virus of a different clade comprises H5 protein (2) having
(a) the amino acids 87L, 113D, 126H, 145(-), 156R, 160F, 167T, and 181N, or
(b) the amino acids 87P, 113N, 126R, 145L, 160Y, 172T, 181H, 201E, 206I, 208K, 254T, 341G and 421K, or
(c) the amino acids 87L, 113N, 126R, 145L, 156G, 160Y, 172T, 181H, and 254V,
and/or
wherein such H5 protein (2) comprises a peptide that comprises:
i. any one of the amino acid sequences of SEQ ID NOs: 62 to 137 or 9 to 46;
ii. any peptide that has at least 85%, preferably at least 95%, even more preferably at least 96%, even more preferably at least 97%, even more preferably at least 98%, even more preferably at least 99%, most preferably 100% sequence homology to the polypeptide of i) and that comprises hemagglutinin inhibition in a standard hemagglutinin inhibition assay; or
iii. any part of the polypeptides of i) or ii) comprising at least 334 contiguous amino acids of any of such peptides of i) or ii) and wherein any of such peptide comprises hemagglutinin inhibition in a standard hemagglutinin inhibition assay,
and/or
wherein such H5 protein (2) consists of or comprises a contiguos amino acid sequence which has at least 95% even more preferably at least 96%, even more preferably at least 97%, even more preferably at least 98%, even more preferably at least 99%, most preferably 100% sequence identity with any one of the sequences as set forth in SEQ ID NOs: 62 to 137 or 9 to 46.

Preferably, the H5N1 virus of a different clade comprises a polynucleotide encoding a H5 protein (2) having:
(a) the amino acids 87L, 113D, 126H, 145(-), 156R, 160F, 167T, and 181N, or
(b) the amino acids 87P, 113N, 126R, 145L, 160Y, 172T, 181H, 201E, 206I, 208K, 254T, 341G and 421K, or
(c) the amino acids 87L, 113N, 126R, 145L, 156G, 160Y, 172T, 181H, and 254V,
and/or
wherein such H5 protein (2) comprises a peptide that comprises:
i. any one of the amino acid sequences of SEQ ID NOs: 62 to 137 or 9 to 46;
ii. any peptide that has at least 85%, preferably at least 95%, even more preferably at least 96%, even more preferably at least 97%, even more preferably at least 98%, even more preferably at least 99%, most preferably 100% sequence homology to the polypeptide of i) and that comprises hemagglutinin inhibition in a standard hemagglutinin inhibition assay; or
iii. any part of the polypeptides of i) or ii) comprising at least 334 contiguous amino acids of any of such peptides of i) or ii) and wherein any of such peptide comprises hemagglutinin inhibition in a standard hemagglutinin inhibition assay,
and/or
wherein such H5 protein (2) consists of or comprises a contiguos amino acid sequence which has at least 95% even more preferably at least 96%, even more preferably at least 97%, even more preferably at least 98%, even more preferably at least 99%, most preferably 100% sequence identity with any one of the sequences as set forth in SEQ ID NOs: 62 to 137 or 9 to 46.

More particular, the H5N1 virus of a different clade preferably comprises H5 protein (2) which consists of or comprises an amino acid sequence which is at least 95%, preferably at least 96%, more preferably at least 97%, still more preferably at least 98%, yet more preferably at least 99%, or in particular preferred 100% homolog with any one of the sequences as set forth in SEQ ID NOs: 68, 73, 106, 111, 15, or 20, and wherein such H5 protein (2) comprising or consisting of the amino acid sequence set forth in SEQ ID NO: 73, 111, or 20 are in particular more preferred.

Preferably, the H5N1 virus of a different clade in particular comprises a polynucleotide encoding a H5 protein (2) which consists of or comprises an amino acid sequence which is at least 95%, preferably at least 96%, more preferably at least 97%, still more preferably at least 98%, yet more preferably at least 99%, or in particular preferred 100% homolog with any one of the sequences as set forth in SEQ ID NOs: 68, 73, 106, 111, 15, or 20, and wherein such H5 protein (2) comprising or consisting of the amino acid sequence set forth in SEQ ID NO: 73, 111, or 20 are in particular more preferred.

In particular, the present disclosure is directed to the H5 protein (1) described herein for use in a method of treating or preventing infections
(A) with Subclade A H5N1 virus of North African origin, namely an infection with a H5N1 virus comprising a H5 protein (2) having the amino acids according to alternative (a) as described herein, in particular according to (a) of claim 15 or 16, or comprising a H5 protein according to claim 15, 16 or 17 relating to any one of the sequences as set forth in SEQ ID NOs: 62 to 72, 100 to 110, 9 to 19, or 95 or 96, 133 or 134, 42 or 43,
   or
(B) with Subclade B H5N1 virus of North African origin, namely an infection with a H5N1 virus comprising a H5 protein having the amino acids according to alternative (b) or (c) as described herein, in particular according to (b) or (c) of claim 15 or 16, or comprising a H5 protein according to claim 15, 16 or 17 relating to any one of the sequences as set forth in SEQ ID NOs: 82 to 94, 111 to 132, 20 to 41, or 97 to 99, 135 to 137, or 44 to 46.

Particularly, the present disclosure is directed to the H5 protein (1) described herein for use in a method of treating or preventing infections
(A) with Subclade A H5N1 virus of North African origin, namely an infection with a H5N1 virus comprising a polynucleotide encoding a H5 protein (2) having the amino acids according to alternative (a) as described herein, in particular according to (a) of claim 15 or 16, or an infection with a H5N1 virus comprising a polynucleotide encoding a H5 protein (2) according to claim 15, 16 or 17 relating to any one of the sequences as set forth in SEQ ID NOs: 62 to 72, 100 to 110, 9 to 19, or 95 or 96, 133 or 134, 42 or 43,
   or
(B) with Subclade B H5N1 virus of North African origin, namely an infection with a H5N1 virus comprising a polynucleotide encoding a H5 protein (2) having the amino acids according to alternative (b) or (c) as described herein, in particular according to (b) or (c) of claim 15 or 16, or an infection with a H5N1 virus comprising a polynucleotide encoding a H5 protein (2) according to claim 15, 16 or 17 relating to any one of the sequences as set forth in SEQ ID NOs: 82 to 94, 111 to 132, 20 to 41, or 97 to 99, 135 to 137, or 44 to 46.

Preferably, the present disclosure also relates to nucleic acid molecules, which code for any of the H5 proteins (1), as described *supra,* for use in a method of treating or preventing infections with H5N1 virus of a different clade. Preferably, those nucleic acid molecules are RNA, DNA or copy (c)DNA molecules. Thus, the present disclosure relates to a nucleic acid molecule, preferably a cDNA molecule coding for a H5 protein or any modified forms of H5 protein, including any deletion, substitution and/or insertion mutant of H5 protein, wherein those H5 proteins having the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted.

Preferably, the present disclosure also relates to a nucleic acid molecule, preferably a cDNA molecule coding for any part of the H5 protein (1), which means encoding for any peptide-fragment which shows antigenic properties in an standard hemagglutinin inhibition assay as described *supra,* and having at least the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted. Normally such nucleic acid molecules, which code for an antigenic part of H5 protein, comprise 600, 540, 480, 450, 420, 390, 360, 330 or most preferably 315 contiguous nucleotides of the nucleotide sequence that codes for the H5 protein as mentioned above, modified or non-modified, and which shows antigenic properties in an standard hemagglutinin inhibition assay as described herein.

Further antigenic parts of the H5 protein (1) are described *supra.* It is in the common knowledge of a person skilled in the art to construct any such nucleic acid molecules, preferably cDNA molecules which codes for the antigenic part of the H5 protein as described *supra.* This also include but is not limited to the construction of nucleic acid molecules, preferably of cDNA molecules, which codes for antigenic parts of the H5 protein as mentioned above including deletion mutants of H5 protein, which comprises:
i. at least 105, 90, 75, 60, 48, 45, 39, 30, 27, or most preferably 24 contiguous amino nucleotides of the nucleotide sequence that surrounds and includes the coding sequence that codes for amino acid 223N; and
ii. at least 105, 90, 75, 60, 48, 45, 39, 30, 27, or most preferably 24 contiguous amino nucleotides of the nucleotide sequence that surrounds and includes the coding sequence that codes for modification 328K+, and
iii. wherein any of such antigenic part of H5 protein show hemagglutinin inhibition in a standard hemagglutinin inhibition assay as described in Example 2.

Preferably, those surrounding nucleotides of the nucleotides, which code for amino acids 223N and/or 328K+, coding for SEQ ID NO:2 or SEQ ID NO:5.

Furthermore preferred nucleic acid molecules encoding for the H5 protein (1) according to the disclosure are:
i. any of those mentioned *supra* encoding for the amino acid 223N and the modification 328K+;
ii. any of those mentioned *supra* encoding for the amino acid 94N/223N and the modification 328K+;
iii. any nucleic acid molecules of avian origin encoding for the amino acid 223N, and the modification 328K+, wherein avian origin means that the H5 sequence derived from a virus isolate that was originally isolated from poultry infected with avian influenza virus type 5; or
iv. any nucleic acid molecules of avian origin encoding for the amino acids 94N/223N and the modification 328K+, wherein avian origin means that the H5 sequence derived from a virus isolate that was originally isolated from poultry infected with avian influenza virus type 5; or.
v. any nucleic acid molecules of avian origin encoding for the amino acids 155N/223N and the modification 328K+, wherein avian origin means that the H5 sequence derived from a virus isolate that was originally isolated from poultry infected with avian influenza virus type 5; or
vi. any nucleic acid molecule encoding for H5 protein of avian origin having the amino acid 120N/155N/223N and the modification 328K+, wherein avian origin means that the H5 sequence derived from a virus isolate that was originally isolated from poultry infected with avian influenza virus type 5; or
vii. any nucleic acid molecule encoding for H5 protein having the modifications 94N/223N and the modification 328K+; or
viii.any nucleic acid molecule encoding for H5 protein having the modifications 94N/155N/223N and the modification 328K+; or;
ix. any nucleic acid molecule encoding for H5 protein having the modifications 94N/120N/155N/223N and the modification 328K+; or
x. any nucleic acid molecule encoding for H5 protein having the modifications 223N, the modification 328K+, and one or more of the following amino acid clusters selected from the group consisting of:
   a. aa 93 - 95: GNF
   b. aa 123 - 125: SDH
   c. aa 128 - 130: SSG
   d. aa 138 - 140: GSS
   e. aa 226 - 228: MDF
   f. aa 270 - 272: EVE
   g. aa 309 - 311: NKL; or
xi. any nucleic acid molecule encoding for H5 protein having the amino acid 223N, the modification 328K+, and one or more of the following amino acid clusters selected from the group consisting of:
   a. aa 93 - 95: GNF
   b. aa 128 - 130: SSG
   c. aa 138 - 140: GSS; or
xii. any nucleic acid molecule encoding for H5 protein having the amino acid sequence of SEQ ID NO:5.

Furthermore preferred H5 proteins (1) as disclosed herewith include the H5 proteins as described by Hoffmann et al, PNAS, vol. 106, no.36, pp. 12915-12920 of September 6, 2005*,* wherein that H5 proteins includes one or more of the modifications as described above, at least the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted. The present disclosure also relates to any nucleic acid molecule, preferably a cDNA molecule coding for any of such proteins described by Hoffmann et al, PNAS, vol. 106, no.36, pp. 12915-12920 of September 6, 2005*,* wherein that H5 proteins includes one or more of the modifications as described above, at least the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted.

Methods, of how to introduce any of the above-mentioned modifications within the nucleotide sequence, including the encoding sequence of the H5 protein of an influenza virus, are well known in the art. The genomic sequence of the entire influenza virus can be modified according to the disclosure, for example according to the methods described in US 6,951,754, with further references.

Furthermore, there may be employed conventional molecular biology, microbiology, and recombinant DNA techniques within the skill of the art to modify a nucleic acid sequence coding for an antigen as described herein. Such techniques are explained fully in the literature. See, e.g., Sambrook et al., Molecular Cloning: A Laboratory Manual, Second Edition (1989) Cold Spring Harbor Laboratory Press, Cold Spring Harbor, N.Y*.;* DNA Cloning: A Practical Approach, Volumes I and II (D. N. Glover ed. 1985*);* Oligonucleotide Synthesis (M. J. Gait ed. 1984*);* Nucleic Acid Hybridization [B. D. Hames & S. J. Higgins eds.(1985*)];* Transcription And Translation [B. D. Hames & S. J. Higgins, eds. (1984*)];* Animal Cell Culture [R. I. Freshney, ed. (1986*)];* Immobilized Cells And Enzymes [IRL Press, (1986*)];* B. Perbal, A Practical Guide To Molecular Cloning (1984*);* F. M. Ausubel et al. (eds.), Current Protocols in Molecular Biology, John Wiley & Sons, Inc. 1994*).*

The present disclosure also relates to a vector that comprises any of such nucleic acid molecules as described *supra.* In other words, the present disclosure relates to a vector, that includes the coding sequence of any such H5 protein (1), or part thereof as described *supra.* Preferably, said vector is an expression vector, which allows the expression of any such H5 protein (1) or part thereof as described *supra.* Vectors according to the disclosure are those which are suitable for the transfection or infection of bacterial, yeast or animal cells, *in vitro* or *in vivo.*

Vectors and methods for making and/or using vectors (or recombinants) for expression can be by or analogous to the methods disclosed in: U.S. Patent Nos. 4,603,112, 4,769,330, 5,174,993, 5,505,941, 5,338,683, 5,494,807, 4,722,848, 5,942,235, 5,364,773, 5,762,938, 5,770,212, 5,942,235, 382,425, PCT publications WO 94/16716, WO 96/39491, WO 95/30018, Paoletti, "Applications of pox virus vectors to vaccination: An update, "PNAS USA 93: 11349-11353, October 1996, Moss, "Genetically engineered poxviruses for recombinant gene expression, vaccination, and safety," PNAS USA 93: 11341-11348, October 1996, Smith et al., U. S. Patent No. 4,745,051, (recombinant baculovirus), Richardson, C.D. (Editor), Methods in Molecular Biology 39, "Baculovirus Expression Protocols" (1995 Humana Press Inc.), Smith et al., "Production of Human Beta Interferon in Insect Cells Infected with a Baculovirus Expression Vector", Molecular and Cellular Biology, Dec., 1983, Vol. 3, No. 12, p. 2156-2165; Pennock et al., "Strong and Regulated Expression of Escherichia coli B-Galactosidase in Infect Cells with a Baculovirus vector, "Molecular and Cellular Biology Mar. 1984, Vol. 4, No. 3, p. 399-406; EPA0 370 573, U. S. application No. 920,197, filed October 16,1986, EP Patent publication No. 265785, U. S. Patent No. 4,769,331 (recombinant herpesvirus), Roizman, "The function of herpes simplex virus genes: A primer for genetic engineering of novel vectors," PNAS USA 93:11307-11312, October 1996, Andreansky et al., "The application of genetically engineered herpes simplex viruses to the treatment of experimental brain tumors," PNAS USA 93: 11313-11318, October 1996, Robertson et al. "Epstein-Barr virus vectors for gene delivery to B lymphocytes", PNAS USA 93: 11334-11340, October 1996, Frolov et al., "Alphavirus-based expression vectors: Strategies and applications," PNAS USA 93: 11371-11377, October 1996, Kitson et al., J. Virol. 65,3068-3075,1991; U. S. Patent Nos. 5,591,439, 5,552,143, WO 98/00166, allowed U. S. applications Serial Nos. 08/675,556, and 08/675,566 both filed July 3,1996 (recombinant adenovirus), Grunhaus et al., 1992,"Adenovirus as cloning vectors," Seminars in Virology (Vol. 3) p. 237-52, 1993, Ballay et al. EMBO Journal, vol. 4, p. 3861-65,Graham, Tibtech 8,85-87, April, 1990, Prevec et al., J. Gen Virol. 70,42434, PCT WO 91/11525, Felgner et al. (1994), J. Biol. Chem. 269,2550-2561, Science, 259: 1745-49,1993 and McClements et al., "Immunization with DNA vaccines encoding glycoprotein D or glycoprotein B, alone or in combination, induces protective immunity in animal models of herpes simplex virus-2 disease", PNAS USA 93: 11414-11420, October 1996, and U. S. Patent Nos. 5,591,639, 5,589,466, and 5,580,859, as well as WO 90/11092, WO93/19183, WO94/21797, WO95/11307, WO95/20660, Tang et al., Nature and Furth et al. Analytical Biochemistry, relating to DNA expression vectors, inter alia. See also WO 98/33510; Ju et al., Diabetologia, 41: 736-739,1998 (lentiviral expression system); Sanford et al., U. S. Patent No. 4,945,050; Fischbachet al. (Intracel), WO 90/01543; Robinson et al., seminars in Immunology vol. 9, pp. 271-283 (1997), (DNA vector systems); Szoka et al., U. S. Patent No. (method of inserting DNA into living cells); McCormick et al., U. S. Patent No. 5,677,178 (use of cytopathic viruses); and U. S. Patent No. 5,928,913 (vectors for gene delivery), as well as other documents cited herein.

A viral vector, for instance, selected from pig herpes viruses, such as Aujeszky's disease virus, porcine adenovirus, poxviruses, especially vaccinia virus, avipox virus, canarypox virus, and swinepox virus, as well as DNA vectors (DNA plasmids) are advantageously employed in the practice of the disclosure.

### Methods of producing the H5 proteins (1) according to the present invention

The present disclosure provides methods of producing and/or recovering high amounts of recombinant H5 protein: i) by permitting infection of susceptible cells in culture with a recombinant viral vector containing H5 DNA coding sequences, wherein H5 protein is expressed by the recombinant viral vector, and ii) thereafter recovering the H5 protein from cell culture. High amounts of H5 protein means, but are not limited to, more than about 20 µg/mL cell culture, preferably more than about 25 µg/mL, even more preferred more than about 30 µg/mL, even more preferred more than about 40 µg/mL, even more preferred more than about 50 µg/mL, even more preferred more than about 60 µg/mL, even more preferred more than about 80 µg/mL, even more preferred more than about 100 µg/mL, even more preferred than about 150 µg/mL, most preferred more than about 190 µg/mL.

Preferably, the H5 protein (1) is recovered by harvesting the whole (i.e. intact) SF+ cells expressing the H5 protein.

Preferred cells are those susceptible for infection with an appropriate recombinant viral vector, containing a H5 DNA and expressing the H5 protein (1). Preferably the cells are insect cells, and more preferably, they include the insect cells sold under the trademark SF+ insect cells (Protein Sciences Corporation, Meriden, CT). Preferred cell cultures have a cell count between about 0.3 - 2.0 x 10⁶ cells/mL, more preferably from about 0.35 - 1.9 x 10⁶ cells/mL, still more preferably from about 0.4 - 1.8 x 10⁶ cells/mL, even more preferably from about 0.45 - 1.7 x 10⁶ cells/mL, and most preferably from about 0.5 - 1.5 x 10⁶ cells/mL.

Preferred viral vectors include baculovirus such as BaculoGold (BD Biosciences Pharmingen, San Diego, CA), in particular provided that the production cells are insect cells. Although the baculovirus expression system is preferred, it is understood by those of skill in the art that other expression systems will work for purposes of the present disclosure, namely the expression of H5 into the supernatant of a cell culture. Such other expression systems may require the use of a signal sequence in order to cause H5 expression into the media.

Appropriate growth media will also be determinable by those of skill in the art with a preferred growth media being serum-free insect cell media such as Excell 420 (JRH Biosciences, Inc., Lenexa, KS) and the like.

The recombinant viral vector containing the H5 DNA sequences has a preferred multiplicity of infection (MOI) of between about 0.03 - 1.5, more preferably from about 0.05 - 1.3, still more preferably from about 0.09 - 1.1, and most preferably from about 0.1 - 1.0, when used for the infection of the susceptible cells. Preferably the MOIs mentioned above relates to one mL of cell culture fluid. Preferably, the method described herein comprises the infection of 0.35 - 1.9 x 10⁶ cells/mL, still more preferably of about 0.4 - 1.8 x 10⁶ cells/mL, even more preferably of about 0.45 - 1.7 x 10⁶ cells/mL, and most preferably of about 0.5 - 1.5 x 10⁶ cells/mL with a recombinant viral vector containing a H5 DNA and expressing the H5 protein having a MOI (multiplicity of infection) of between about 0.03 - 1.5, more preferably from about 0.05 - 1.3, still more preferably from about 0.09 - 1.1, and most preferably from about 0.1 - 1.0.

The infected cells are then incubated over a period of up to ten days, more preferably from about two days to about ten days, still more preferably from about four days to about nine days, and most preferably from about five days to about eight days. Preferred incubation conditions include a temperature between about 22 - 32°C, more preferably from about 24 - 30°C, still more preferably from about 25 - 29°C, even more preferably from about 26 - 28°C, and most preferably about 27°C. Preferably, the SF+ cells are observed following inoculation for characteristic baculovirus-induced changes. Such observation may include monitoring cell density trends and the decrease in viability during the post-infection period. It was found that peak viral titer is observed 3-5 days after infection and peak H5 protein expression in the cells is obtained between days 5 and 8, and/or when cell viability decreases to less than 10%.

Thus, one aspect of the present disclosure provides a method of producing and/or recovering recombinant H5 protein, preferably in amounts described above, by i) permitting infection of a number of susceptible cells (see above) in culture with a recombinant viral vector with a MOI as defined above, ii) expressing H5 protein by the recombinant viral vector, and iii) thereafter recovering the H5 protein from the cells obtained between days 5 and 8 after infection and/or cell viability decreases to less then 10%. Preferably, the recombinant viral vector is a recombinant baculovirus containing H5 DNA coding sequences and the cells are SF+ cells. Additionally, it is preferred that the culture be periodically examined for macroscopic and microscopic evidence of contamination or for atypical changes in cell morphology during the post-infection period. Any culture exhibiting any contamination should be discarded.

For recovery of H5 protein (1) that will be used in an immunogenic or immunological composition such as a vaccine, the inclusion of an inactivation step is preferred in order to inactivate the viral vector.

An "immunogenic or immunological composition" refers to a composition of matter that comprises at least one antigen which elicits an immunological response in the host of a cellular and/ or antibody-mediated immune response to the composition or vaccine of interest. Usually, an "immunological response" includes but is not limited to one or more of the following effects: the production or activation of antibodies, B cells, helper T cells, suppressor T cells, and/or cytotoxic T cells and/or gamma-delta T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

As used herein, "vaccine" refers to that term as it is used by those of skill in the art. More particularly, "vaccine" refers to an immunogenic composition that, when administered to an animal in need thereof, results in a reduction in the incidence of or severity of clinical signs of influenza infection up to an including the complete prevention of such clinical signs. Preferably, the reduction in incidence or severity is at least 10%, more preferably at least 20%, still more preferably at least 30%, even more preferably at least 40%, more preferably at least 50%, still more preferably at least 60%, even more preferably at least 70%, more preferably at least 80%, still more preferably at least 90%, even more preferably at least 95%, and most preferably 100% in comparison to an animal or group of animals that did not receive the compositions of the present disclosure but that were exposed to infectious levels of influenza virus that would normally result in influenza infection resulting in exhibiting clinical signs.

Thus, the present disclosure also relates to a method of producing and/or recovering recombinant H5 protein, preferably in amounts described above, by i) permitting infection of a number of susceptible cells (see above) in culture with a recombinant viral vector with a MOI as defined above, ii) expressing H5 protein by the recombinant viral vector, iii) recovering the H5 expressed in cells obtained between days 5 and 8 after infection and/or cell viability decreases to less then 10%, and iv) inactivating the recombinant viral vector.

Preferably, this inactivation is done either just before or just after the filtration step, with after the filtration step being the preferred time for inactivation. Any conventional inactivation method can be used for purposes of the present invention. Thus, inactivation can be performed by chemical and/or physical treatments. In preferred forms, the volume of harvest fluids is determined and the temperature is brought to between about 32 - 42°C, more preferably between about 34 - 40°C, and most preferably between about 35 - 39°C. Preferred inactivation methods include the addition of cyclized binary ethylenimine (BEI), preferably in a concentration of about 1 to about 20 mM, preferably of about 2 to about 10 mM, still more preferably of about 2 to about 8 mM, still more preferably of about 3 to about 7 mM, most preferably of about 5 mM. For example the inactivation includes the addition of a solution of 2-bromoethyleneamine hydrobromide , preferably of about 0.4M, which has been cyclized to 0.2M binary ethylenimine (BEI) in 0.3N NaOH, to the fluids to give a final concentration of about 5mM BEI. Preferably, the fluids are then stirred continuously for 72 - 96 hours and the inactivated harvest fluids can be stored frozen at -40°C or below or between about 1 - 7°C. After inactivation is completed a sodium thiosulfate solution, preferably at 1.0M is added to neutralize any residual BEI. Preferably, the sodium thiosulfate is added in equivalent amount as compared to the BEI added prior to for inactivation. For example, in the event BEI is added to a final concentration of 5mM, a 1.0M sodium thiosulfate solution is added to give a final minimum concentration of 5 mM to neutralize any residual BEI.

Thus, one further aspect of the present disclosure relates to a method of producing recombinant H5 protein, preferably in amounts described above, by i) permitting infection of a number of susceptible cells (see above) in culture with a recombinant viral vector with a MOI as defined above, ii) expressing H5 protein by the recombinant viral vector, iii) recovering the H5 expressed in the cells obtained between days 5 and 8 after infection and/or cell viability decreases to less then 10%, and iv) inactivating the recombinant viral vector. Preferably, the recombinant viral vector is a baculovirus containing H5 DNA coding sequences and the cells are SF+ cells. Preferred inactivation steps are those described above. Preferably, inactivation is performed between about 35 - 39°C and in the presence of 2 to 8 mM BEI, still more preferred in the presence of about 5 mM BEI.

According to one further aspect of the present disclosure, the method described above also includes a neutralization step after step iv). This step v) comprises adding of an equivalent amount of an agent that neutralizes the inactivation agent within the solution. Preferably, if the inactivation agent is BEI, addition of sodium thiosulfate to an equivalent amount is preferred. Thus, according to a further aspect, step v) comprises adding of a sodium thiosulfate solution to a final concentration of about 1 to about 20 mM, preferably of about 2 to about 10 mM, still more preferably of about 2 to about 8 mM, still more preferably of about 3 to about 7 mM most preferably of about 5 mM, when the inactivation agent is BEI.

In preferred forms and especially in forms that will use the recombinant H5 protein in an immunogenic composition such as a vaccine, each lot of harvested H5 protein will be tested for inactivation by passage in the anchorage dependent, baculovirus susceptible insect cells, such as Sf9 cells. In a preferred form of this testing, 150 cm² of appropriate cell culture monolayer is inoculated with 1.0 mL of inactivated H5 fluids and maintained at 25 - 29°C for 14 days with at least two passages. At the end of the maintenance period, the cell monolayers are examined for cytopathogenic effect (CPE) typical of H5 baculovirus. Preferably, positive virus controls are also used. Such controls can consist of one culture of Sf9 cells inoculated with a non-inactivated reference H5 baculovirus and one flask of Sf9 cells that remain non-inoculated. After incubation and passage, the absence of virus-infected cells in the BEI treated viral fluids would constitute a satisfactory inactivation test. The control cells inoculated with the reference virus should exhibit CPE typical of H5 baculovirus and the non-inoculated flask should not exhibit any evidence of H5 baculovirus CPE. Alternatively, at the end of the maintenance period, the supernatant samples could be collected and inoculated onto a Sf9 96 well plate, which has been loaded with Sf9 cells, and then maintained at 25 - 29°C for 5 - 6 days. The plate is then fixed and stained with anti-H5 antibody conjugated to FITC or any labeled antibody directed to baculovirus specific proteins (i.e. gp64). The absence of CPE, H5 expression, or expression of baculovirus specific proteins (i.e. gp64) in the BEI treated viral fluids constitutes a satisfactory inactivation test. The control cells inoculated with the reference virus should exhibit CPE and IFA activity and the non-inoculated flask should not exhibit any evidence of H5 baculovirus CPE and contain no IFA activity.

Thus a further aspect described herein, relates to an inactivation test for determining the effectiveness of the inactivation of the recombination viral vector expressing H5 protein (1), comprises the steps: i) contacting at least a portion of the culture fluid containing the recombinant viral vector with an inactivating agent, preferably as described above, ii) adding a neutralization agent to neutralize the inactivation agent, preferably as described above, and iii) determining the residual infectivity by the assays as described above.

After inactivation, the relative amount of recombinant H5 protein in a sample can be determined in a number of ways. Preferred methods of quantitation include SDS-PAGE densitometry, ELISA, and animal vaccination studies that correlate known quantities of vaccine with clinical outcomes (serology, etc.). When SDS-PAGE is utilized for quantitation, the sample material containing an unknown amount of recombinant H5 protein is run on a gel, together with samples that contain different known amounts of recombinant H5 protein. A standard curve can then be produced based on the known samples and the amount of recombinant H5 in the unknown sample can be determined by comparison with this standard curve. Because ELISAs are generally recognized as the industry standard for antigen quantitation, they are preferred for quantitation.

### Vaccines comprising H5 proteins (1) or nucleic acid molecules or vectors coding for those

Disclosed is a combination of
(a) the H5 protein (1) described herein
   and
(b) an inactivted Newcastle disease virus
   for use in a method of treating or preventing infections with H5N1 virus of a different clade, in particular for use in any method of treating or preventing infections with H5N1 virus of a different clade as described herein.

Said combination is also termed "the combination described herein" hereinafter.

According to the disclosure it is understood that the combination described herein is preferably included in a multivalent combination vaccine or the combination described herein is in particular directed to a combined vaccination, more particular to an administration of the H5 protein (1) described herein and of the inactivted Newcastle disease virus within a maximum of 24 hours to an animal, in particular poultry, or human being in need thereof.

Preferably, the inactivated Newcastle disease virus is an inactivated whole Newcastle disease virion.

Preferably, the inactivated Newcastle disease virus is an inactivated Newcastle disease virus obtained by inactivation of a Newcastle disease virus comprising a RNA polynucleotide having at least 70%, preferably at least 80%, more preferably at least 90%, still more preferably at least 95% or in particular 100% sequence identity with a RNA copy of the polynucleotide set forth in SEQ ID NO: 139 (cDNA sequence of LaSota strain virus), which has been inactivated.

In particular, the inactivated Newcastle disease virus is an inactivated Newcastle disease LaSota strain virus.

Preferably, the inactivated Newcastle Disease Virus is a Newcastle Disease Virus which has been inactivated with a reagent selected from the group consisting of Formaldehyde, binary ethyleneimine (BEI), Beta-Propio-Lactone (BPL), and combinations thereof.

The amount of inactivated Newcastle disease virus in the combination described herein is preferably between 10² and 10¹⁰ equivalents of egg infectious doses (EID50), preferably between 10⁶ and 10⁹ EID50, in particular preferably between 10⁷ and 10⁹ EID50. The amount of the H5 protein (1) in the combination described herein is preferably the same as mentioned hereinafter.

The amount of the H5 protein (1) according to the disclosure is preferably between 10 and 1000 Hemagglutination units (HAU's) per dose, more preferably between 50 and 950 HAU's per dose, even more preferably between 100 and 900 HAU's per dose, even more preferably between 200 and 800 HAU's per dose, even more preferably between 300 and 700 HAU's per dose, still more preferably between 300 and 500 HAU's per dose.

According to a further aspect, the present disclosure relates to vaccines or pharmaceutical compositions in general, that comprises,
i. one or more of the H5 proteins (1) as described herein or the combination described herein;
ii. one or more of the nucleic acid molecules as described herein, coding for any such H5 proteins (1); and/or
iii. one or more of the vectors as described herein, including any such nucleic acid molecules and coding for any such H5 proteins (1) as described herein; and
iv. a pharmaceutical acceptable carrier and/or excipient.

The term "pharmaceutical composition" "Pharmaceutical/vaccine composition" as described herein, includes but is not limited to, vaccines for the reduction or prevention of an infection or to a composition of matter for the treatment and lessening of an infection.

The preparation of nucleic acid based vaccines, preferably cDNA vaccines, coding for influenza hemagglutinin are described for example in Deck et al, Vaccine 1997; 15(1):71-78*;* Ulmer et al., Science 1993; 259:1745-1749*;* Ulmer et al., Vaccine 1994;12(16):1541-1544*.* Any of those methods can be used for the production of nucleic acid based vaccines, preferably cDNA vaccines, coding for an influenza H5 protein as described herein.

Moreover, a vaccine, which comprises H5 protein (1) or parts thereof as described herein, can be produced by conventional approaches, e.g. by recombinant expression techniques or by biochemical purification and separation techniques. Recombinant expression techniques, including the expression in insect cells are well known in the art, and described for example in Sambrook et al., Molecular Cloning: A Laboratory Manual, Second Edition (1989) Cold Spring Harbor Laboratory Press, Cold Spring Harbor, N.Y*.;* DNA Cloning: A Practical Approach, Volumes I and II (D. N. Glover ed. 1985*);* Oligonucleotide Synthesis (M. J. Gait ed. 1984*);* Nucleic Acid Hybridization [B. D. Hames & S. J. Higgins eds.(1985*)];* Transcription And Translation [B. D. Hames & S. J. Higgins, eds. (1984*)];* Animal Cell Culture [R. I. Freshney, ed. (1986*)];* Immobilized Cells And Enzymes [IRL Press, (1986*)];* B. Perbal, A Practical Guide To Molecular Cloning (1984*);* F. M. Ausubel et al. (eds.), Current Protocols in Molecular Biology, John Wiley & Sons, Inc. 1994*).* Further examples of well established recombinant expression systems are bacterial expression systems such as *E. coli* or *B. subtilis,* yeast-based expression systems such as *S*. *cerevisiae* or S. *pombe,* or mammalian cell expression systems such as the BHK-, CHO- and/or NS0-based expression systems. Such systems are well known in the art and generally available, e.g. commercially through Clontech Laboratories, Inc. 4030 Fabian Way, Palo Alto, California 94303-4607, USA. Further expression strategies are for example described in Lüschow et al., Vaccine no. 19 (2001), pp. 4249-4259*,* or Veit et al., PNAS vol. 103 (2006), pp. 8197-8202*.* Furthermore, recombinant adeno-associated virus systems are well established and for example described in US 5,436,146 or WO200203872 with further references. Moreover, vaccinia (pox) virus based expression systems, for example as described in US 6,265,183 with further references, are also well established and suitable to produce recombinant antigen(s), antigenic composition(s) as used according to the disclosure. Further suitable expression systems make use of recombinant popova viruses, such as SV40, fowl pox virus, pseudorabies viruses and retroviruses.

The relevant pharmaceutical/vaccine compositions as described herein, can also comprise inactivated virus which comprises H5 protein (1) as described herein, an apathogenic version of a live virus comprising H5 protein (1) as described herein, preparation and/or fragments of a virus, wherein said preparation and/or fragment comprise the H5 protein (1) as described herein.

The skilled person knows additional components which may be comprised in said compositions/vaccines together with antigen (see for example, Remington's Pharmaceutical Sciences. (1990). 18th ed. Mack Publ., East*on).* The expert may use known injectable, physiologically acceptable sterile solutions. For preparing a ready-to-use solution, aqueous isotonic solutions, such as e.g. saline or corresponding plasma protein solutions, are readily available. The pharmaceutical composition/vaccine may be present as lyophylisates or dry preparations, which can be reconstituted with a known injectable solution directly before use under sterile conditions, e.g. as a kit of parts.

In addition the pharmaceutical/vaccine compositions of the present disclosure can include one or more veterinary-acceptable carriers. As used herein, "a veterinary-acceptable carrier" includes but is not limited to any and all solvents, dispersion media, coatings, adjuvants, stabilizing agents, diluents, preservatives, antibacterial and antifungal agents, isotonic agents, adsorption delaying agents, and the like.

Diluents can include water, saline, dextrose, ethanol, glycerol, and the like. Isotonic agents can include sodium chloride, dextrose, mannitol, sorbitol, and lactose, among others. Stabilizers include albumin and alkali salts of ethylendiamintetracetic acid, among others.

A preservative as used herein, refers to an anti-microbiological active agent, such as for example Gentamycin, Merthiolate, and the like. In particular adding of a preservative is most preferred for the preparation of a multi-dose composition. Those anti-microbiological active agents are added in concentrations effective to prevent the composition of interest for any microbiological contamination or for inhibition of any microbiological growth within the composition of interest.

"Adjuvants" as used herein, can include aluminum hydroxide and aluminum phosphate, saponins e.g., Quil A, QS-21 (Cambridge Biotech Inc., Cambridge MA), GPI-0100 (Galenica Pharmaceuticals, Inc., Birmingham, AL), water-in-oil emulsion, oil-in-water emulsion, water-in-oil-in-water emulsion.

The emulsion can be based in particular on light liquid paraffin oil (European Pharmacopoeia type); isoprenoid oil such as squalane or squalene ; oil resulting from the oligomerization of alkenes, in particular of isobutene or decene; esters of acids or of alcohols containing a linear alkyl group, more particularly plant oils, ethyl oleate, propylene glycol di-(caprylate/caprate), glyceryl tri-(caprylate/caprate) or propylene glycol dioleate; esters of branched fatty acids or alcohols, in particular isostearic acid esters. The oil is used in combination with emulsifiers to form the emulsion. The emulsifiers are preferably nonionic surfactants, in particular esters of sorbitan, of mannide (e.g. anhydromannitol oleate), of glycol, of polyglycerol, of propylene glycol and of oleic, isostearic, ricinoleic or hydroxystearic acid, which are optionally ethoxylated, and polyoxypropylene-polyoxyethylene copolymer blocks, in particular the Pluronic products, especially L121. See Hunter et al., The Theory and Practical Application of Adjuvants (Ed.Stewart-Tull, D. E. S.). John Wiley and Sons, NY, pp51-94 (1995) and Todd et al., Vaccine 15:564-570 (1997). Examples for suitable oil-in water emulsions are Emulsigen-based adjuvants, such as EMULSIGEN®, EMULSIGEN-D®, EMULSIGEN-P®, EMULSIGEN-75® (MVP Laboratories, Inc. Omaha, NE, USA). It has been surprisingly found, that pharmaceutical/vaccine compositions that comprise H5 protein, preferably recombinant H5 protein as described herein, have been effectively adjuvanted with oil-in water emulsions, preferably with such Emulsigen-based adjuvants, more preferably with EMULSIGEN® and EMULSIGEN-D®

Moreover, it is possible to use the SPT emulsion described on page 147 of "Vaccine Design, The Subunit and Adjuvant Approach" edited by M. Powell and M. Newman, Plenum Press, 1995, and the emulsion MF59 described on page 183 of this same book.

A further instance of an adjuvant is a compound chosen from the polymers of acrylic or methacrylic acid and the copolymers of maleic anhydride and alkenyl derivative. Advantageous adjuvant compounds are the polymers of acrylic or methacrylic acid which are cross-linked, especially with polyalkenyl ethers of sugars orpolyalcohols. These compounds are known by the term carbomer (Phameuropa Vol. 8, No. 2, June 1996). Persons skilled in the art can also refer to U. S. Patent No. 2,909,462 which describes such acrylic polymers cross-linked with a polyhydroxylated compound having at least 3 hydroxyl groups, preferably not more than 8, the hydrogen atoms of at least three hydroxyls being replaced by unsaturated aliphatic radicals having at least 2 carbon atoms. The preferred radicals are those containing from 2 to 4 carbon atoms, e.g. vinyls, allyls and other ethylenically unsaturated groups. The unsaturated radicals may themselves contain other substituents, such as methyl. The products sold under the name Carbopol ; (BF Goodrich, Ohio, USA) are particularly appropriate. They are cross-linked with an allyl sucrose or with allyl pentaerythritol. Among then, there may be mentioned Carbopol 974P, 934P and 971P. Most preferred is the use of Carbopol 971P. Among the copolymers of maleic anhydride and alkenyl derivative, the copolymers EMA (Monsanto) which are copolymers of maleic anhydride and ethylene. The dissolution of these polymers in water leads to an acid solution that will be neutralized, preferably to physiological pH, in order to give the adjuvant solution into which the immunogenic, immunological or vaccine composition itself will be incorporated.

Further suitable adjuvants include, but are not limited to, the RIBI adjuvant system (Ribi Inc.), Block co-polymer (CytRx, Atlanta GA), SAF-M (Chiron, Emeryville CA), monophosphoryl lipid A, Avridine lipid-amine adjuvant, heat-labile enterotoxin from E. coli (recombinant or otherwise), cholera toxin, or muramyl dipeptide among many others.

Preferably, the adjuvant is added in an amount of about 100 µg to about 10 mg per dose. Even more preferred the adjuvant is added in an amount of about 100 µg to about 10 mg per dose. Even more preferred the adjuvant is added in an amount of about 500 µg to about 5 mg per dose. Even more preferred the adjuvant is added in an amount of about 750 µg to about 2,5 mg per dose. Most preferred the adjuvant is added in an amount of about 1 mg per dose.

The pharmaceutical/vaccine compositions, can further include one or more other immunomodulatory agents such as, e. g., interleukins, interferons, or other cytokines. The pharmaceutical/vaccine compositions can also include Gentamicin and Merthiolate. While the amounts and concentrations of adjuvants and additives useful can readily be determined by the skilled artisan, preferably compositions comprise from about 50 µg to about 2000 µg of adjuvant and preferably about 250 ug/1 ml dose of the vaccine composition. The present disclosure contemplates vaccine compositions comprising from about 1 ug/ml to about 60 µg/ml of antibiotics, and more preferably less than about 30 µg/ml of antibiotics.

The present disclosure also relates to a pharmaceutical/vaccine composition comprising
i. a therapeutically effective amount of any one of the H5 proteins of influenza virus as described herein, wherein the H5 protein having the amino acid 223N and the modification 328K+, wherein numbering of the amino acid positions of the H5 protein refers to the amino acid position as exemplarily given in SEQ ID NO:2 and wherein the modification 328K+ means that at amino acid position 328 of H5 protein a second Lysine (K+) is inserted; and
ii. a pharmaceutically acceptable adjuvants as described above.

Preferably, the adjuvant is selected from the group consisting of:
a) EMULSIGEN®, a oil-in-water emulsion (o/w);
b) EMULSIGEN-D®, a oil-in-water (o/w) with dimethyldioctadecylammonum bromide (DDA);
c) a Polygen, a copolymer
d) EMULSIGEN-P®, a oil-in-water (o/w) with a proprietary immunostimulant
e) Carbigen is a cross-linked polymer
f) EMULSIGEN-75®, a double adjuvants comprise of a oil-in-water (o/w) with a cross-linked polymer
g) ISA 70 is a water-in-oil (w/o)

Most preferably, the adjuvants is a oil-in-water emulsion such as an emulsigen-based adjuvant selected from the group consisting of EMULSIGEN®, EMULSIGEN-D®, EMULSIGEN-P®, EMULSIGEN-75®, EMULSIGEN® and EMULSIGEN-P® Most preferably EMULSIGEN® and EMULSIGEN-P® are used in the formulation of the current invention.

According to a further aspect, the pharmaceutical/vaccine compositions as disclosed herewith, comprise one or more antigen. Preferably, that further antigen is an antigen of a poultry or mammalian pathogen. Preferably, that additional antigen is an further influenza antigen such as hemagglutinin H5, H7, H9, or any other hemagglutinin of influenza virus, wherein the H5 is preferably a H5 protein of a H5N1 virus of a clade different than clade 1, in particular of a H5N1 virus of North African origin, such as the H5 protein (2) described herein. The additional antigen(s) can be added in a purified form, as part of an antigenic preparation, in the form of a killed microorganism or in the form of a modified live microorganism.

The term "antigen", as used herein means, but is not limited to, peptides, polypeptides, glycopeptides, or polysaccharides which are capable of specifically interacting with an antigen recognition molecule of the immune system, such as an immunoglobulin (antibody) or T cell antigen receptor in order to elicit, activate or stimulate an immune response directed to said antigen in a host to which said antigen is administered. The term "antigen" also refers to nucleic acid molecules, preferably DNA- or RNA-molecules, each of which codes for and express a peptide, polypeptide, or glycopeptide that is capable of specifically interacting with an antigen recognition molecule of the immune system, such as an immunoglobulin (antibody) or T cell antigen receptor in order to elicit, activate or stimulate an immune response against the antigen that is coded by the nucleic acid molecule. The antigen used for the preparation of the pharmaceutical composition which is used according to the invention is a microorganism or an antigenic part and/or preparation of said microorganism. In this connection, the term "immunization", as used herein, means but is not limited to, any cause or enhancement of an immune response. The term "immune response" is already described *supra.*

Administration strategies for influenza vaccines are well known in the art. Mucosal vaccination strategies for inactivated and attenuated virus vaccines are contemplated. While the mucosa can be targeted by local delivery of a vaccine, various strategies have been employed to deliver immunogenic proteins to the mucosa.

Preferably, the vaccine can be administered in an admixture with, or as a conjugate or chimeric fusion protein with, cholera toxin, such as cholera toxin B or a cholera toxin A/B chimera (Hajishengallis , J Immunol., 154:4322-32, 1995*;* Jobling and Holmes, Infect Immun., 60:4915-24, 1992*).* Mucosal vaccines based on use of the cholera toxin B subunit have been described (Lebens and Holmgren, Dev Biol Stand 82:215-27, 1994). Preferably, an admixture with heat labile enterotoxin (LT) can be prepared for mucosal vaccination.

Other mucosal immunization strategies include encapsulating the virus in microcapsules (US 5,075,109, US 5,820,883, and US 5,853,763) and using an immunopotentiating membranous carrier (WO 98/0558). Immunogenicity of orally administered immunogens can be enhanced by using red blood cells (rbc) or rbc ghosts (US 5,643,577), or by using blue tongue antigen (US 5,690,938).

According to another aspect, the present disclosure relates to a method for preparing a pharmaceutical /vaccine composition as described above, preferably a method for producing a vaccine which comprises a recombinant, baculovirus expressed H5 protein as described *supra.* Generally, this method includes the steps of transfecting a construct into a virus, wherein the construct comprises i) recombinant H5 cDNA as described herein, ii) infecting cells in growth media with the transfected virus, iii) causing the virus to express the recombinant H5 protein as described herein iv) recovering the expressed H5 protein from the culture v) and preparing the composition by blending the expressed H5 protein with a suitable adjuvant and/or other pharmaceutically acceptable carrier.

Preferred adjuvants are those described above. Thus according to a further aspect, the method for preparing an antigenic composition, such as for example a vaccine, for invoking an immune response against influenza infections comprises i) preparing and recovering H5 protein, and ii) admixing this with a suitable adjuvants.

In addition, the vaccine composition of the present disclosure can also include diluents, isotonic agents, stabilizers, an/or preservatives. Diluents can include water, saline, dextrose, ethanol, glycerol, and the like. Isotonic agents can include anorganic or organic salts, e.g. sodium chloride, dextrose, mannitol, sorbitol, and lactose, saccharides, trehalose, mannitol, saccharose among others. Stabilizers include albumin and alkali salts of ethylendiamintetracetic acid, among others. Suitable adjuvants, are those described above.

### Medicinal use of any of such H5 proteins (1), nucleic acid molecules, vectors, vaccines, and combinations described herein

The H5 proteins (1) as disclosed herewith, the nucleic acid molecules coding for any such H5 proteins (1), the vectors comprising any such nucleic acid molecules coding for any such H5 proteins (1) as described herein, and any pharmaceutical/vaccine composition comprising any of such H5 protein (1), nucleic acid molecule or vector or the combination described herein can be used as a medicine, preferably for the treatment and prophylaxis of infections, caused by influenza virus, most preferably by influenza A virus. The H5 proteins (1) as disclosed herewith, the nucleic acid molecules encoding for any such H5 proteins, the vectors comprising any such nucleic acid molecules encoding for any such H5 proteins (1) as described herein, and any pharmaceutical/vaccine composition comprising any of such H5 protein (1), nucleic acid molecule or vector, as described herein, or the combination described herein can be used for the treatment or prophylaxis of human beings as well as in veterinary medicine. When used in veterinary medicine, the treatment of poultry, preferably bird, chicken, duck, turkey and the like as well as mammals, preferably pigs, cattle, horses, seals, camels, dogs, cats, hamsters, mice and the like, is preferred.

In terms of the present invention, "prophylaxis" refers to the reduction in the incidence of or severity of clinical signs of influenza infection up to an including the complete prevention of such clinical signs. Preferably, the reduction in incidence or severity is at least 10%, more preferably at least 20%, still more preferably at least 30%, even more preferably at least 40%, more preferably at least 50%, still more preferably at least 60%, even more preferably at least 70%, more preferably at least 80%, still more preferably at least 90%, even more preferably at least 95%, and most preferably 100% in comparison to an animal or group of animals that did not receive the compositions of the present invention but that were exposed to infectious levels of influenza virus that would normally result in influenza infection resulting in exhibiting clinical signs.

Thus, according to another aspect the present disclosure relates to the use of H5 proteins (1) as disclosed herewith, the nucleic acid molecules encoding for any such H5 proteins (1), the vectors comprising any such nucleic acid molecules encoding for any such H5 proteins (1) as described herein and any pharmaceutical/vaccine compositions comprising any of such H5 protein (1), nucleic acid molecule or vector as described herein or the combination described herein, can be used as a medicine, preferably as a medicine for human beings and/or as veterinary medicine, preferably for poultry, in particular for chicken.

Moreover, H5 proteins (1) as disclosed herewith, the nucleic acid molecules coding for any such H5 proteins (1), the vectors comprising any such nucleic acid molecules coding for any such H5 protein (1), as described herein, or the combination described herein can be used for the preparation of a pharmaceutical composition, as described herein, prefereably of a single-shot vaccine or a one dose vaccine, for the prophylaxis or treatment of infections caused by H5N1 virus of a clade other than clade 1, wherein said H5N1 virus of a clade other than clade 1 is preferably the H5N1 virus of a different clade as descibed herein. As mentioned above, those pharmaceutical compositions/vaccine compositions can be used for the treatment and/or prophylaxis of human beings as well as for the treatment and/or prophylaxis of animals, such as poultry, preferably bird, chicken, duck, turkey and the like as well as mammals, preferably pigs, cattle, horses, seals, camels, dogs, cats, hamsters, mice and the like.

According to a further aspect, the present disclosure also relates to a method for the treatment or prophylaxis of influenza virus infections caused by H5N1 virus of a clade other than clade 1, wherein said H5N1 virus of a clade other than clade 1 is preferably the H5N1 virus of a different clade as described herein, wherein the method comprising administration of a therapeutically effective amount of the H5 protein (1) as described herein or of the combination described herein, to a subject in need of such a treatment. Moreover, the present disclosure also relates to a method for the treatment or prophylaxis of influenza virus infections caused by H5N1 virus of a clade other than clade 1, wherein said H5N1 virus of a clade other than clade 1 is preferably the H5N1 virus of a different clade as described herein, wherein the method comprising administration of a therapeutically effective amount of any H5 nucleic acid molecule or vector as described herein, that codes for any H5 protein (1) as described herein, to a subject in need of such a treatment. Furthermore, the present disclosure also relates to a method for the treatment or prophylaxis of influenza virus infections caused by H5N1 virus of a clade other than clade 1, wherein said H5N1 virus of a clade other than clade 1 is preferably the H5N1 virus of a different clade described herein, wherein the method comprising administration of a therapeutically effective amount of the vaccine comprising any such H5 protein (1), nucleic acid molecule or vector, as described herein, to a subject in need of such a treatment. The subject in need thereof can be a human being as well as an animal, preferably poultry, even more preferably bird, chicken, duck, turkey or a mammal, preferably pig, cattle, horse, seal, camel, dog, cat, hamster, mouse and the like.

Preferably, the administration, as described herein, is a single-shot administration or a one dose administration.

Preferably, when chicken are vaccinated, the H5 protein as described herein can be used for vaccination at day 1 of age or later, e.g. at day 10, or at day 1 to 10, or at day 10 or later.

Preferably the influenza infection that can be treated by the administration of any H5 protein (1), the nucleic acid molecule or vector encoding for any such H5 protein, or any pharmaceutical / vaccine compositions as described herein, is caused by H5N1 virus of a clade other than clade 1, wherein said H5N1 virus of a clade other than clade 1 is preferably the H5N1 virus of a different clade as described herein and, as the case may be, also in combination with with another avian, swine or human influenza virus or any combination or hybrid thereof.

A further advantage of the present disclosure is that it benefits a "DIVA" (Differentiation of Infected and Vaccinated Animals) concept with specific Elisa Kits for differentiating between vaccinated human beings or animals and human beings or animals infected with H5N1 virus.

According to another aspect, the present disclosure relates to a kit of parts, that comprises i) any of such H5 protein (1) as described herein, the nucleic acid molecule or vector encoding for any such H5 protein, or any pharmaceutical / vaccine composition comprising any of such H5 protein, nucleic acid molecule or vector as described herein, and ii) a package leaflet indicating the use of such H5 protein, nucleic acid molecule, vector or vaccine for the treatment or prophylaxis of infections caused by H5N1 virus of a clade other than clade 1, wherein said H5N1 virus of a clade other than clade 1 is preferably the H5N1 virus of a different clade as described herein. When chicken are vaccinated, the H5 protein (1) as described herein can be used for vaccination at day 1 on age or later.

It is thus understood that the kit of parts as mentioned herein is for the use, or is used, respectively, for the treatment or prophylaxis of infections caused by H5N1 virus of a clade other than clade 1, wherein said H5N1 virus of a clade other than clade 1 is preferably the H5N1 virus of a different clade as described herein.

Preferably, that kit in parts comprises at least one further antigen of a poultry or mammalian pathogen and the information indication the medicinal, human or veterinary use of that additional antigen, in particular the further antigen as mentioned above.

Disclosed is a method for reducing viral shedding in a subject, comprising administering the H5 protein (1) described herein or the combination as described herein to a subject infected with or at risk of a viral infection with H5N1 virus of a clade other than clade 1, wherein said H5N1 virus of a clade other than clade 1 is preferably the H5N1 virus of a different clade as described herein.

The disclosure also relates to the H5 protein (1) described herein or the combination as described herein for use in a method for reducing viral shedding in a subject, wherein said H5 protein (1) or said combination is to be administered to a subject infected with or at risk of a viral infection with H5N1 virus of a clade other than clade 1, and wherein said H5N1 virus of a clade other than clade 1 is preferably the H5N1 virus of a different clade as described herein.

Disclosed is the use of the H5 protein (1) described herein or of the combination as described herein for the preparation of a medicament for reducing viral shedding in a subject infected with or at risk of a viral infection with H5N1 virus of a clade other than clade 1, wherein said H5N1 virus of a clade other than clade 1 is preferably the H5N1 virus of a different clade as described herein.

Preferably, the H5 protein (1) according to the disclosure, the combination described herein, the vaccine as described herein or the kit mentioned herein is for use as a single-shot vaccine or in a one-dose vaccination.

### EXAMPLES

The following examples set forth preferred materials and procedures in accordance with the present invention. It is to be understood, however, that these examples are provided by way of illustration only, and nothing therein should be deemed a limitation upon the overall scope of the invention.

### EXAMPLE 1

### Construction of a recombinant baculoviruses coding for and expressing HA H5 antigens

The recombinant baculovirus containing the H5 HA antigen was generated as follows: the coding sequences of the H5 HA (SEQ ID NO:3) was chemically synthesized and subcloned into the transfer vector pVL1392 (BD Biosciences Pharmingen, San Diego, CA). The H5 HA MutK+ (SEQ ID NO:5) was generated by using oligonucleotide primers and the QuikChange® Site-Directed Mutagenesis Kit (Stratagene, La Jolla, CA) and subcloned into the transfer vector pVL1392 (BD Biosciences Pharmingen, San Diego, CA). The pVL1392 plasmids containing the genes coding for H5 HA antigen (SEQ ID The recombinant baculovirus containing the H5 HA antigen was generated as follows: the coding sequences of the H5 HA (SEQ ID NO:3) was chemically synthesized and subcloned into the transfer vector pVL1392 (BD Biosciences Pharmingen, San Diego, CA). The H5 HA MutK+ (SEQ ID NO:5) was generated by using oligonucleotide primers and the QuikChange® Site-Directed Mutagenesis Kit (Stratagene, La Jolla, CA) and subcloned into the transfer vector pVL1392 (BD Biosciences Pharmingen, San Diego, CA). The pVL1392 plasmids containing the genes coding for H5 HA antigen (SEQ ID NO:3) and H5 HA MutK+ (SEQ ID NO:5) were then co-transfected with DiamondBac® (Sigma) baculovirus DNA into Sf9 insect cells (BD Biosciences Pharmingen) to generate the recombinant baculovirus containing the genes H5 HA coding for SEQ ID NO:3 and H5 HA mutK+ coding for SEQ ID NO:5. The recombinant baculoviruses containing the genes coding for H5 HA (SEQ ID NO:3) and H5 HA MutK+ (SEQ ID NO:5) were plaque-purified and Master Seed Viruses (MSVs) were propagated on the SF+ cell line, aliquoted, and stored at -70°C. Insect cells infected with H5 HA baculoviruses as described above to generate MSV or Working Seed Viruses express H5 HA antigen (SEQ ID NO:3) and H5 HA MutK+ (SEQ ID NO:5) antigen as detected by polyclonal serum or monoclonal antibodies in an indirect fluorescent antibody assay or Western blot.

After being seeded with the appropriate amounts of recombinant baculoviruses (H5 HA and H5 HA MutK+, respectively), spinner flasks containing SF+ cells (Protein Sciences, Inc., Meriden, CT) were then incubated at 27 ± 2°C for 7 days and with stirring 100rpm during that time. The flasks used ventilated caps to allow for air flow. The crude whole cell culture containing baculovirus infected SF+ cells and the cell culture supernatents of each culture were harvested.

### EXAMPLE 2

### Preparation of pharmaceutical compositions (vaccines) comprising HA H5 antigens

The crude whole cell H5 HA protein and H5 HA Mutk+ protein expressed in insect cells by baculovirus-based expression system were harvested. Baculoviruses were inactivated in the presence of 5 mM cyclized binary ethylenimine (BEI) (final concentration) between about 32 and 39°C for 72 to 96 hours. After inactivation is completed, a 0.3 M sodium thiosulfate solution was added to a final concentration of 5mM to neutralize any residual BEI. After neutralization, various adjuvants were added and the following vaccine/ pharmaceutical compositions were generated.

### VACCINES

| | |
|---|---|
| **Generic product name** | 501 |
| **Antigen** | Crude whole-cell H5 HA protein expressed in insect cells by a baculovirus-based expression system. |
| **Formulation** | An experimental vaccine comprised of cultured insect cells and supernatant expressing recombinant H5 HA. The vaccine was adjuvanted with Emulsigen. |

| | |
|---|---|
| **Generic product name** | 502 |
| **Antigen** | Crude whole-cell H5 HA protein expressed in insect cells by a baculovirus-based expression system. |
| **Formulation** | An experimental vaccine comprised of cultured insect cells and supernatant expressing recombinant H5 HA. The vaccine was adjuvanted with Emulsigen-D. |

| | |
|---|---|
| **Generic product name** | 503 |
| **Antigen** | Crude whole-cell H5 HA protein expressed in insect cells by a baculovirus-based expression system. |
| **Formulation** | An experimental vaccine comprised of cultured insect cells and supernatant expressing recombinant H5 HA. The vaccine was adjuvanted with Polygen. |

| | |
|---|---|
| **Generic product name** | 504 |
| **Antigen** | Crude whole-cell H5 HA protein expressed in insect cells by a baculovirus-based expression system. |
| **Formulation** | An experimental vaccine comprised of cultured insect cells and supernatant expressing recombinant H5 HA. The vaccine was adjuvanted with Emulsigen-P. |

| | |
|---|---|
| **Generic product name** | 505 |
| **Antigen** | Crude whole-cell H5 HA protein expressed in insect cells by a baculovirus-based expression system. |
| **Formulation** | An experimental vaccine comprised of cultured insect cells and supernatant expressing recombinant H5 HA. The vaccine was adjuvanted with Carbigen. |

| | |
|---|---|
| **Generic product name** | 506 |
| **Antigen** | Crude whole-cell H5 HA protein expressed in insect cells by a baculovirus-based expression system. |
| **Formulation** | An experimental vaccine comprised of cultured insect cells and supernatant expressing recombinant H5 HA. The vaccine was adjuvanted with Emulsigen-75. |

| | |
|---|---|
| **Generic product name** | 507 |
| **Antigen** | Crude whole-cell H5 HA protein expressed in insect cells by a baculovirus-based expression system. |
| **Formulation** | An experimental vaccine comprised of cultured insect cells and supernatant expressing recombinant H5 HA. The vaccine was adjuvanted with ISA 70. |

| | |
|---|---|
| **Generic product name** | 508 |
| **Antigen** | Crude whole-cell H5 HA mutK+ protein expressed in insect cells by a baculovirus-based expression system. |
| **Formulation** | An experimental vaccine comprised of cultured insect cells and supernatant expressing recombinant H5 HA. The vaccine was adjuvanted with Emulsigen. |

| | |
|---|---|
| **Generic product name** | 509 |
| **Antigen** | Crude whole-cell H5 HA mutK+ protein expressed in insect cells by a baculovirus-based expression system. |
| **Formulation** | An experimental vaccine comprised of cultured insect cells and supernatant expressing recombinant H5 HA. The vaccine was adjuvanted with Emulsigen-D. |

| | |
|---|---|
| **Generic product name** | 510 |
| **Antigen** | Crude whole-cell H5 HA mutK+ protein expressed in insect cells by a baculovirus-based expression system. |
| **Formulation** | An experimental vaccine comprised of cultured insect cells and supernatant expressing recombinant H5 HA. The vaccine was adjuvanted with Polygen. |

| | |
|---|---|
| **Generic product name** | 511 |
| **Antigen** | Crude whole-cell H5 HA mutK+ protein expressed in insect cells by a baculovirus-based expression system. |
| **Formulation** | An experimental vaccine comprised of cultured insect cells and supernatant expressing recombinant H5 HA. The vaccine was adjuvanted with Emulsigen-P. |

| | |
|---|---|
| **Generic product name** | 512 |
| **Antigen** | Crude whole-cell H5 HA mutK+ protein expressed in insect cells by a baculovirus-based expression system. |
| **Formulation** | An experimental vaccine comprised of cultured insect cells and supernatant expressing recombinant H5 HA. The vaccine was adjuvanted with Carbigen. |

| | |
|---|---|
| **Generic product name** | 513 |
| **Antigen** | Crude whole-cell H5 HA mutK+ protein expressed in insect cells by a baculovirus-based expression system. |
| **Formulation** | An experimental vaccine comprised of cultured insect cells and supernatant expressing recombinant H5 HA. The vaccine was adjuvanted with Emulsigen-75. |

| | |
|---|---|
| **Generic product name** | 514 |
| **Antigen** | Crude whole-cell H5 HA K+ protein expressed in insect cells by a baculovirus-based expression system. |
| **Formulation** | An experimental vaccine comprised of cultured insect cells and supernatant expressing recombinant H5 HA. The vaccine was adjuvanted with ISA 70. |

### EXAMPLE 3

### Vaccination of chicken against avian influenza

A combination vaccine comprising H5 HA Mutk+ (Fraction 1) and inactivated Newcastle disease virus (Fraction 2), named "BACULO Al + ND KV" has been evaluated in animal trials. The vaccine was formulated with the haemmagglutinin H5 produced in the Baculovirus expression system based on the MutK+ construct (Examples 1 and 2). The origin of the Newcastle Disease (ND) virus fraction is the whole virus.

### Fraction 1:

Recombinant, baculovirus-expressed, H5 hemagglutinin (H5 HA) from Avian Influenza H5N1 virus. **Avian Influenza (Al) fraction.**

Al fraction is inactivated with binary ethyleneimine (BEI). No residual infectivity coming from Baculovirus vector is allowed.

### Fraction 2:

Whole virion, Newcastle Disease Virus (ND), LaSota Strain. **Newcastle Disease fraction.**

ND fraction is inactivated with Formaldehyde, BEI or Beta-Propio-Lactone (BPL). No residual infectivity coming from ND virus is allowed.

### Formula composition:

Inactivated harvest material from H5 HA protein and ND are blended into a water/oil emulsion. The mixture includes mineral oil as an adjuvant.

For evaluation of vaccine efficacy, three clinical parameters were considered: 1) Morbility/mortality. 2) Antibodies levels. 3) Viral shedding.

In all studies SPF chickens were vaccinated, administration of the vaccine was by subcutaneous route, in the back of the neck. A dose of 0.5 ml was administered unless otherwise stated.

Chickens were maintained inside isolator units during the whole duration of the studies. Studies were compliant with OIE international guidelines for evaluation of Avian Influenza vaccines.

Challenge was conducted to evaluate the Avian Influenza (AI) antigenic fraction. Chickens were inoculated 3 weeks after vaccination by the intra-nasal (50 µl) and oral (50 µl) route administering a total of 100 µl of allantoic fluid containing 10⁶ EID₅₀ of the challenge virus.

To evaluate protection from challenge against HPAI H5N1 two studies were conducted:
1) Protectotypes study, using a single or double vaccination (evaluating boosting effect), ages of 1 day old or 10 days old chickens (evaluating age effect), and doses of 0.5 or 0.2 ml (evaluating dose effect).
   Two different challenge strains were used for this study: a) A subclade 2.3.2 Vietnamese strain (isolated in 2006) which has been recently causing disease in South-East-Asia (China, Vietnam) Poultry production. b) A subclade 2.2.1 group B1 Egyptian strain (isolated in 2010), which has been recently causing disease in Egyptian Poultry production. Challenge strains are not genetically close to the vaccine baculovirus construct (MutK+). Results are interpreted in the context of protectotypes as broadening up the protection conferred for two immunizations with similar or different vaccines.
   Conclusions:
   1) Protection between 80 and 100 % was observed depending on the age or dose. 100 % protection was observed when administered as 0.5 ml dose at 10 days old of the bivalent formulation.
   2) When administered as a single 0.5 ml immunization of BACULO AI + ND KV at 10 days of age, the same protection is observed than administering two shots of the inactivated traditionally-produced comercial Volvac AI KV vaccine.
   3) When administered as a single 0.5 ml immunization of BACULO AI + ND KV at 10 days of age, similar level of H5-specific antibodies were detected in comparison with administering two shots of the inactivated traditionally-produced comercial Volvac AI KV vaccine.
   4) Low levels of viral shedding were observed until 3 days post-challenge, when the vaccine was administered as a single 0.5 ml immunization of BACULO Al + ND KV at 10 days of age.
2) BACULO efficacy study, using a single, unique vaccination at 10 days of age.
   Three different challenge strains were used for this study: a) A subclade 2.2.1 Egyptian strain (isolated in 2008). b) A subclade 2.2.1 group A1 Egyptian strain (isolated in 2010). c) A subclade 2.2.1 group B1 Egyptian strain (isolated in 2010). The last two have been recently causing disease in Egyptian Poultry production.
   Conclusions:
   1) Protection between 90 and 100 % was observed.
   2) Vaccine BACULO Al + ND KV showed performance compliant with European Medicine Agency (EMA) guidelines for vaccines against HPAI virus in birds.
   3) This is the first report available demonstrating efficacy with a single shot administration for a baculovirus-based vaccine including a hemagglutinin genetically distant from those of the viruses used for challenge.

### EXAMPLE 4

### 1. Experimental design.

This experiment was designed and conducted similar to the above described Example 3: For evaluation of vaccine efficacy, three clinical parameters were considered: 1) Morbility/mortality. 2) Antibodies levels. 3) Viral shedding.

In all studies SPF chickens were vaccinated, the administration of the vaccine was by subcutaneous route, in the back of the neck. A vaccine prototype containing a clade 1 H5 protein was used (called Mut K+) formulated as a bivalent product with a second, ND (Newcastle disease virus) antigenic fraction.

A dose of 0.5 ml was administered unless otherwise stated. Animals were vaccinate at 10 days of age.

Chickens were maintained inside isolator units during the whole duration of the studies. Studies were compliant with OIE international guidelines for evaluation of Avian Influenza vaccines.

Challenge was conducted to evaluate the Avian Influenza (AI) antigenic fraction. Chickens were inoculated 3 weeks after vaccination by the intra-nasal (50 µl) and oral (50 µl) route administering a total of 100 µl of allantoic fluid containing 10⁶ EID₅₀ of the challenge virus.

This is also summarized in the table (Table a) below (Vaccination was performed at 10 days of age, column 1 (ID of experimental groups according to the vaccine applied), column 2 (Vaccine dose), and column 3 (Challenge age)).

Challenge virus was A/Chicken/Egypt/1063/2010, which is classified as subclade 2.2.1.1 HP AIV H5N1 subtype. This is the official challenge strain used in Egypt for evaluation of vaccine batches. The challenge dose was 10⁶ EID₅₀.

### 2. Results & Data analysis.

Results & Data analysis are summarized in the table below (Table a): Column 4 (HI GMT (Geometric Mean Titre) 3 weeks post-vaccination, pre-challenge), column 5 (Percentage of survival, 2 weeks post-challenge), and column 6 (Detection of viral shedding, RT-PCR positive samples).

**Table a: Summary of the experimental design and of the results and data analysis of Example 4.**

| **Experimental group (10 chickens each) -Vaccine ID-** | **Vaccine Dose (age)** | **Challenge dose (age) -Strain 1063-** | **GMT measured at 31 days of age** | | **Percentage of survival post- challenge (%)** | **Viral Shedding- Detection of viral RNA using RT- PCR- (#positives/total)** |
|---|---|---|---|---|---|---|
| | | | Homologous (vaccine strain) | Heterologous (challenge virus) | | |
| Mut K+ | 0.5 ml (10 days of age) | 10⁶ EID₅₀ (31 days of age) | 9.1 | 0.9 | 100 | 2/10 |
| No vaccine | | | - | - | 0 | 10/10 |

### 3. Conclusions

- The vaccinated group survived the challenge. The vaccine prototype triggered an efficient immune response, as measured as HI titration using the homologous antigen.
- The Mut K+ vaccine prototype provided good virological protection, as measured as ability to reduce viral shedding. RT-PCR Ct values were far low to represent infectious virus but only residual genetical material instead.

### EXAMPLE 5

### Experimental design.

The described experiment is designed to show the efficacy of a vaccine prototype only containing a clade 1 H5 protein (called Mut K+).

For evaluation of vaccine efficacy, three clinical parameters are considered: 1) Morbility/mortality. 2) Antibodies levels. 3) Viral shedding.

In all studies SPF chickens are vaccinated, the administration of the vaccine is by the subcutaneous route, in the back of the neck. A vaccine prototype containing a clade 1 H5 protein is used (called Mut K+) formulated as monovalent product.

A dose of 0.5 ml is administered unless otherwise stated. Animals are vaccinated at 10 days of age.

Chickens are maintained inside isolator units during the whole duration of the studies. Studies are compliant with OIE international guidelines for evaluation of Avian Influenza vaccines.

Challenge is conducted to evaluate the efficacy of the vaccine in regard to the Avian Influenza (AI) antigenic fraction. Chickens are inoculated 3 weeks after vaccination by the intra-nasal (50 µl) and oral (50 µl) route administering a total of 100 µl of allantoic fluid containing 10⁶ EID₅₀ of the challenge virus.

This is also summarized in the tables (Table b and Table c) below (Vaccination was performed at 10 days of age, column 1 (ID of experimental groups according to the vaccine applied), column 2 (Vaccine dose), and column 3 (Challenge age)).

Challenge virus is either A/Chicken/Egypt/1063/2010 (classified as subclade B or subclade 2.2.1.1 HP AIV H5N1 subtype) orA/chicken/Egypt/3982-8/2010 (classified as subclade A or subclade 2.2.1 HP AIV H5N1 subtype)

The A/Chicken/Egypt/1063/2010 was the official challenge strain used in Egypt for evaluation of vaccine batches until the end of 2012. The challenge dose is in any case 10⁶ EID₅₀.

**Table b Summary of the experimental design of Example 5.**

| **1** | **2** | **3** |
|---|---|---|
| **Experiment al group (10 chickens each) -Vaccine ID**- | **Vaccine Dose (age)** | **Challen ge dose (age) -Strain 1063-** |
| Mut K+ | 0.5 ml (10 days of age) | 10⁶ EID₅₀ (31 days of age) |
| No vaccine | | |

**Table c: Summary of the experimental design of Example 5.**

| **1** | **2** | **3** |
|---|---|---|
| **Experiment al group (10 chickens each) -Vaccine ID-** | **Vaccine Dose (age)** | **Challen ge dose (age) -Strain** 3982-8 |
| Mut K+ | 0.5 ml (10 days of age) | 10⁶ EID₅₀ (31 days of age) |
| No vaccine | | |

- The chickens of the vaccinated group (Table c) survive the challenge with A/chicken/Egypt/3982-8/2010 and nearly all chickens of the vaccinated group (Table b) survive the challenge with A/Chicken/Egypt/1063/2010. The vaccine prototype induces an efficient immune response, as measured by determination of the HI titer using the homologous antigen.
- The Mut K+ vaccine prototype provides sufficient protection in regard to shedding of the challenge virus, as measured as ability to reduce viral shedding. The RT-PCR Ct values are very low and represent rather only residual virus genetic material instead infectious virus.

In the sequence listing (SEQ ID NOs: 1 to 50 and 139):
SEQ ID NO: 1 corresponds to H5 of A/Hong Kong/213/2003(H5N1) without signal peptide,
SEQ ID NOs: 2-7 correspond to SEQ ID NOs: 1-6 of the international (PCT) application number PCT/US2007/082699,
SEQ ID NO: 8 corresponds to H5 sequence of H5N1 "1709-6",
SEQ ID NO: 9 corresponds to H5 sequence of H5N1 "1553-1/A1",
SEQ ID NO: 10 corresponds to H5 sequence of H5N1 "1553-15/A1",
SEQ ID NO: 11 corresponds to H5 sequence of H5N1 "2095-50/A1",
SEQ ID NO: 12 corresponds to H5 sequence of H5N1 "3982-2/A1",
SEQ ID NO: 13 corresponds to H5 sequence of H5N1 "3982-5/A1",
SEQ ID NO: 14corresponds to H5 sequence of H5N1 "3982-7/A1",
SEQ ID NO: 15 corresponds to H5 sequence of H5N1 "3982-8/A1",
SEQ ID NO: 16 corresponds to H5 sequence of H5N1 "3982-9/A1",
SEQ ID NO: 17 corresponds to H5 sequence of H5N1 "3982-12/A1",
SEQ ID NO: 18 corresponds to H5 sequence of H5N1 "3982-20/A1",
SEQ ID NO: 19 corresponds to H5 sequence of H5N1 "3982-44/A1",
SEQ ID NO: 20 corresponds to H5 sequence of H5N1 "1553-2/B1",
SEQ ID NO: 21 corresponds to H5 sequence of H5N1 "1553-6/B1",
SEQ ID NO: 22 corresponds to H5 sequence of H5N1 "1553-13/B2",
SEQ ID NO: 23 corresponds to H5 sequence of H5N1 "1553-26/B2",
SEQ ID NO: 24 corresponds to H5 sequence of H5N1 "1553-28/B1",
SEQ ID NO: 25 corresponds to H5 sequence of H5N1 "2095-39/B2",
SEQ ID NO: 26 corresponds to H5 sequence of H5N1 "2095-46/B1",
SEQ ID NO: 27 corresponds to H5 sequence of H5N1 "2095-49/B1",
SEQ ID NO: 28 corresponds to H5 sequence of H5N1 "2095-65/B1",
SEQ ID NO: 29 corresponds to H5 sequence of H5N1 "2095-68/B2",
SEQ ID NO: 30 corresponds to H5 sequence of H5N1 "2095-70/B2",
SEQ ID NO: 31 corresponds to H5 sequence of H5N1 "2095-73/B2",
SEQ ID NO: 32 corresponds to H5 sequence of H5N1 "2095-75/B2",
SEQ ID NO: 33 corresponds to H5 sequence of H5N1 "3982-3/B1",
SEQ ID NO: 34 corresponds to H5 sequence of H5N1 "3982-4/B1",
SEQ ID NO: 35 corresponds to H5 sequence of H5N1 "3982-13/B1",
SEQ ID NO: 36 corresponds to H5 sequence of H5N1 "3982-14/B2",
SEQ ID NO: 37 corresponds to H5 sequence of H5N1 "3982-19/B3",
SEQ ID NO: 38 corresponds to H5 sequence of H5N1 "3982-21/B2",
SEQ ID NO: 39 corresponds to H5 sequence of H5N1 "3982-43/B1",
SEQ ID NO: 40 corresponds to H5 sequence of H5N1 "3982-50/B1",
SEQ ID NO: 41 corresponds to H5 sequence of H5N1 "3982-52/B1",
SEQ ID NO: 42 corresponds to H5 sequence of H5N1 "3982-55/A1",
SEQ ID NO: 43 corresponds to H5 sequence of H5N1 "3982-56/A1",
SEQ ID NO: 44 corresponds to H5 sequence of H5N1 "3982-78/B2",
SEQ ID NO: 45 corresponds to H5 sequence of H5N1 "4794-17/B ",
SEQ ID NO: 46 corresponds to H5 sequence of H5N1 "4794-18/B",
SEQ ID NO: 47 corresponds to H5 sequence translated from SEQ ID NO: 50,
SEQ ID NO: 48 codes for a H5 sequence of H5N1 "3982-8/A1" (SEQ ID NO: 15),
SEQ ID NO: 49 codes for a H5 sequence of H5N1 "1553-2/B1" (SEQ ID NO: 20),
SEQ ID NO: 50 corresponds to the consensus sequence obtained after analysis of the 38 H5 HA gene sequences coding for SEQ ID NOs: 9 to 46,
SEQ ID NO: 139 corresponds to the cDNA of Newcastle Disease Virus LaSota strain.

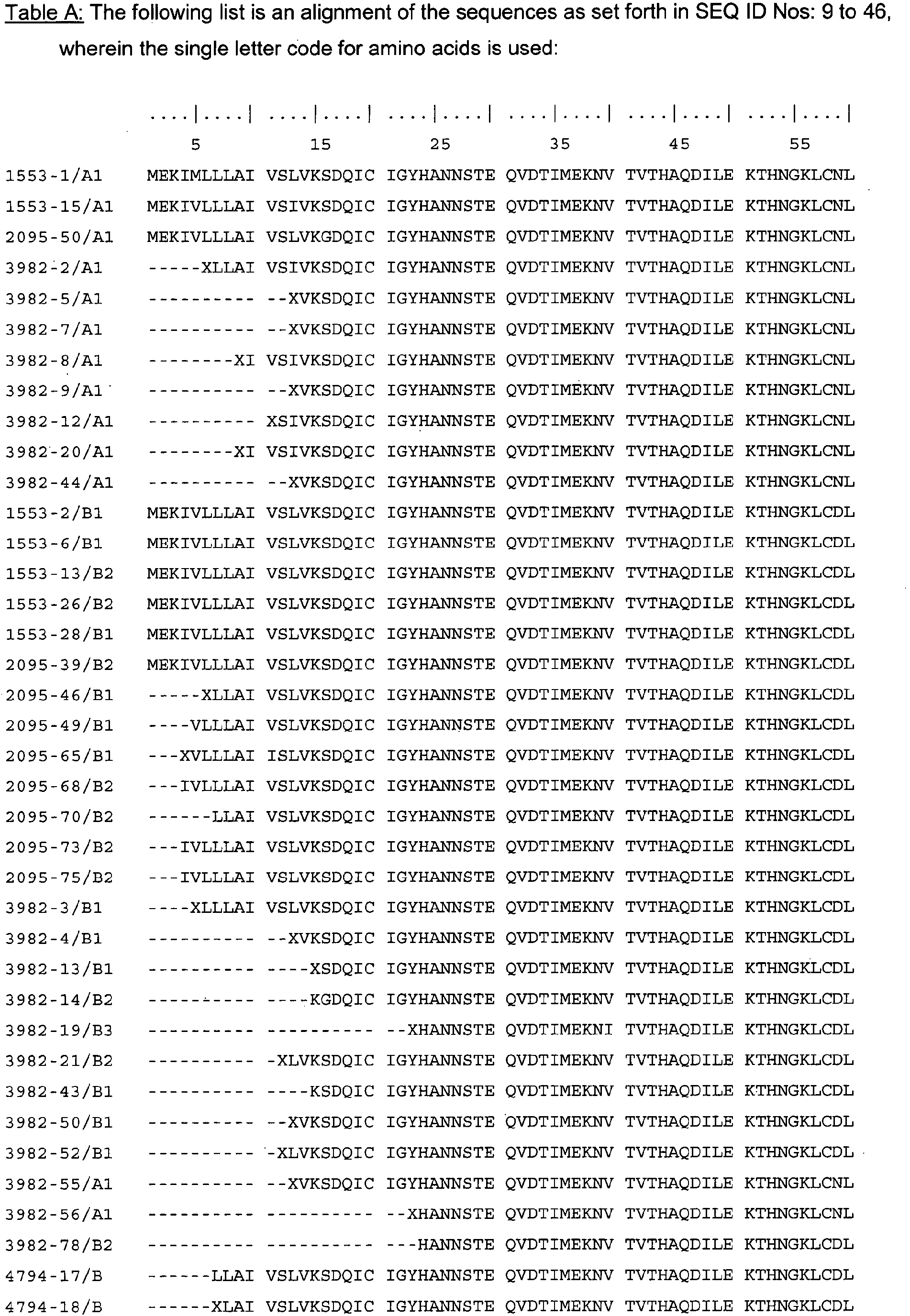

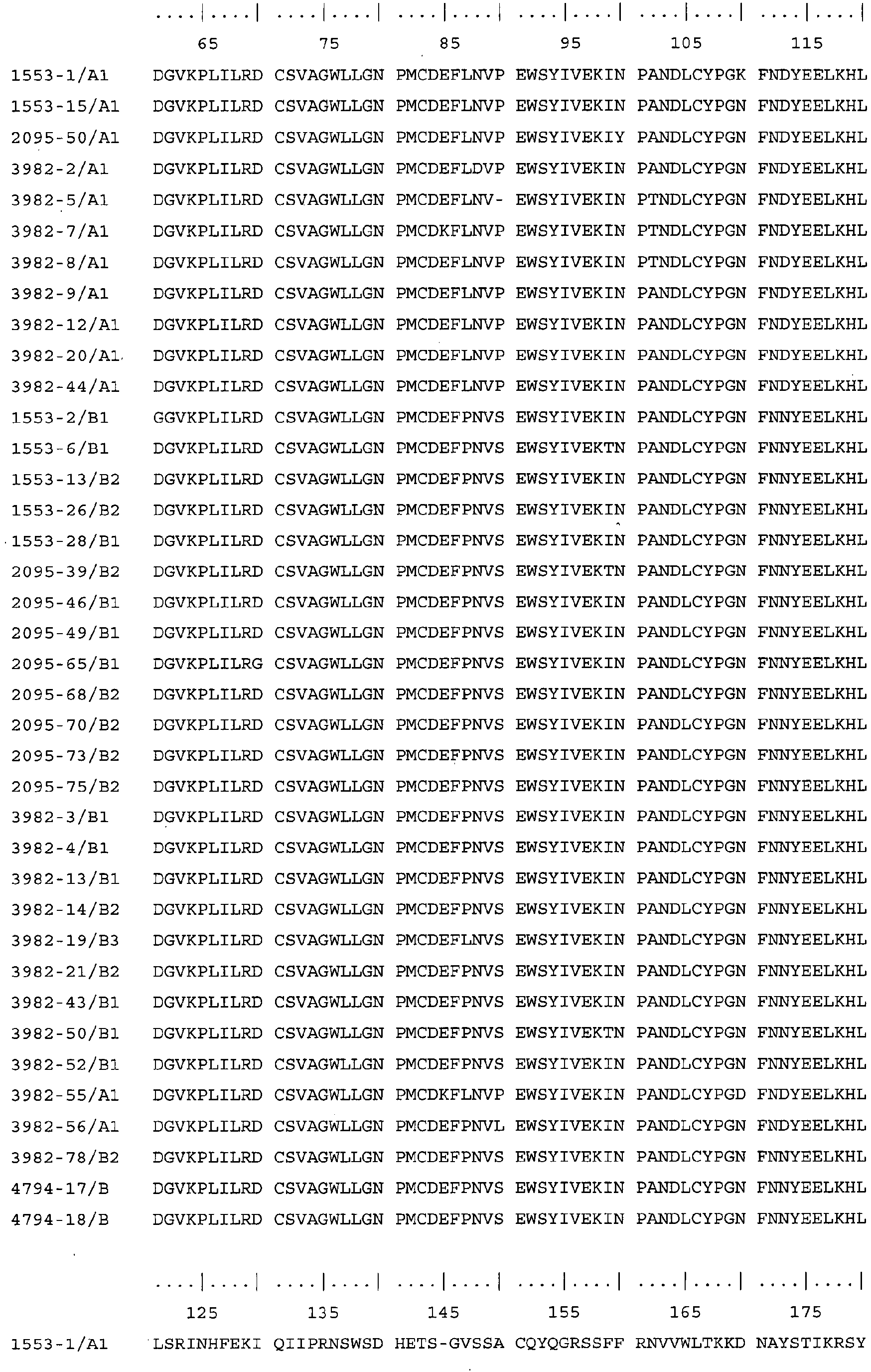

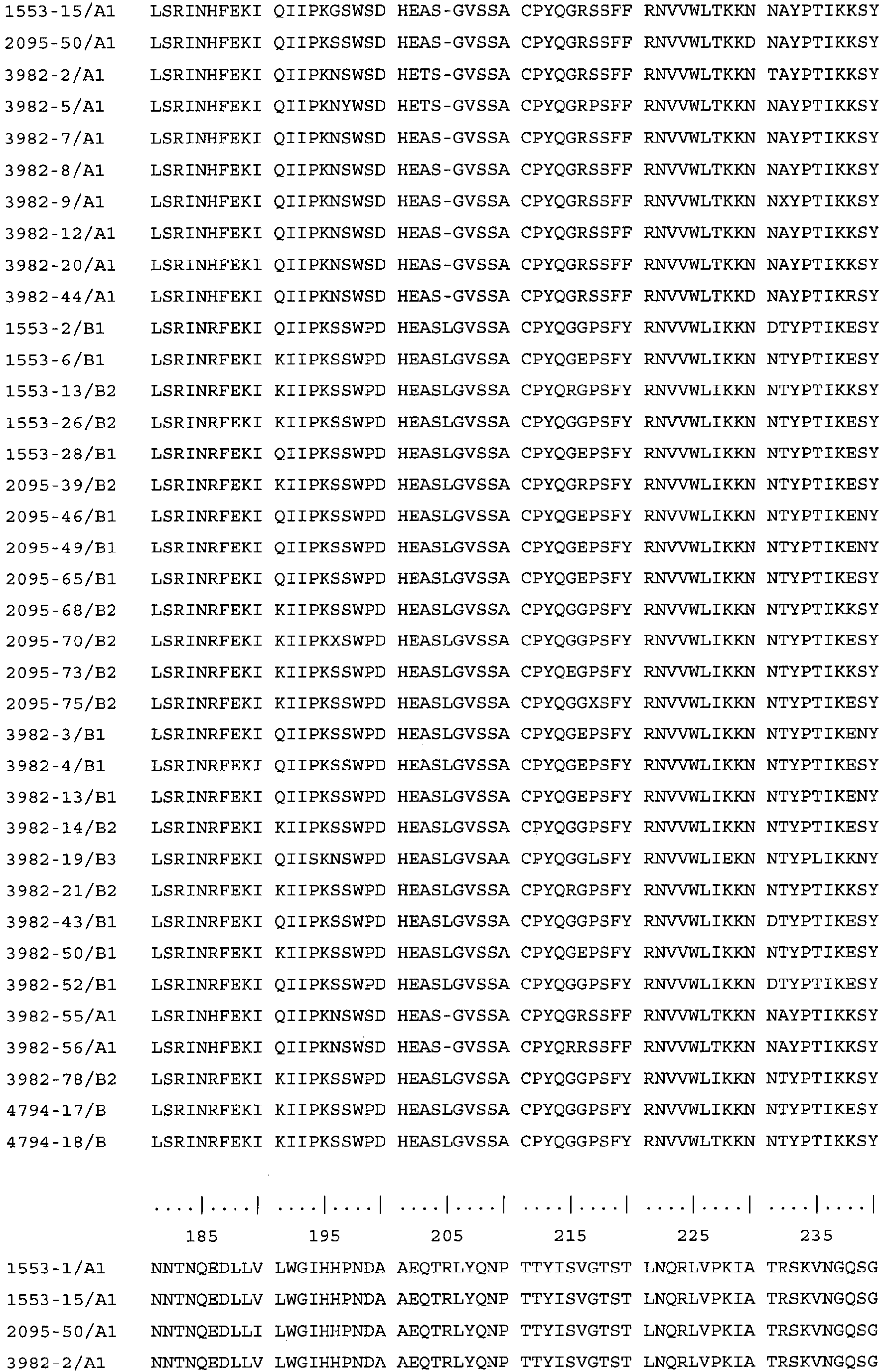

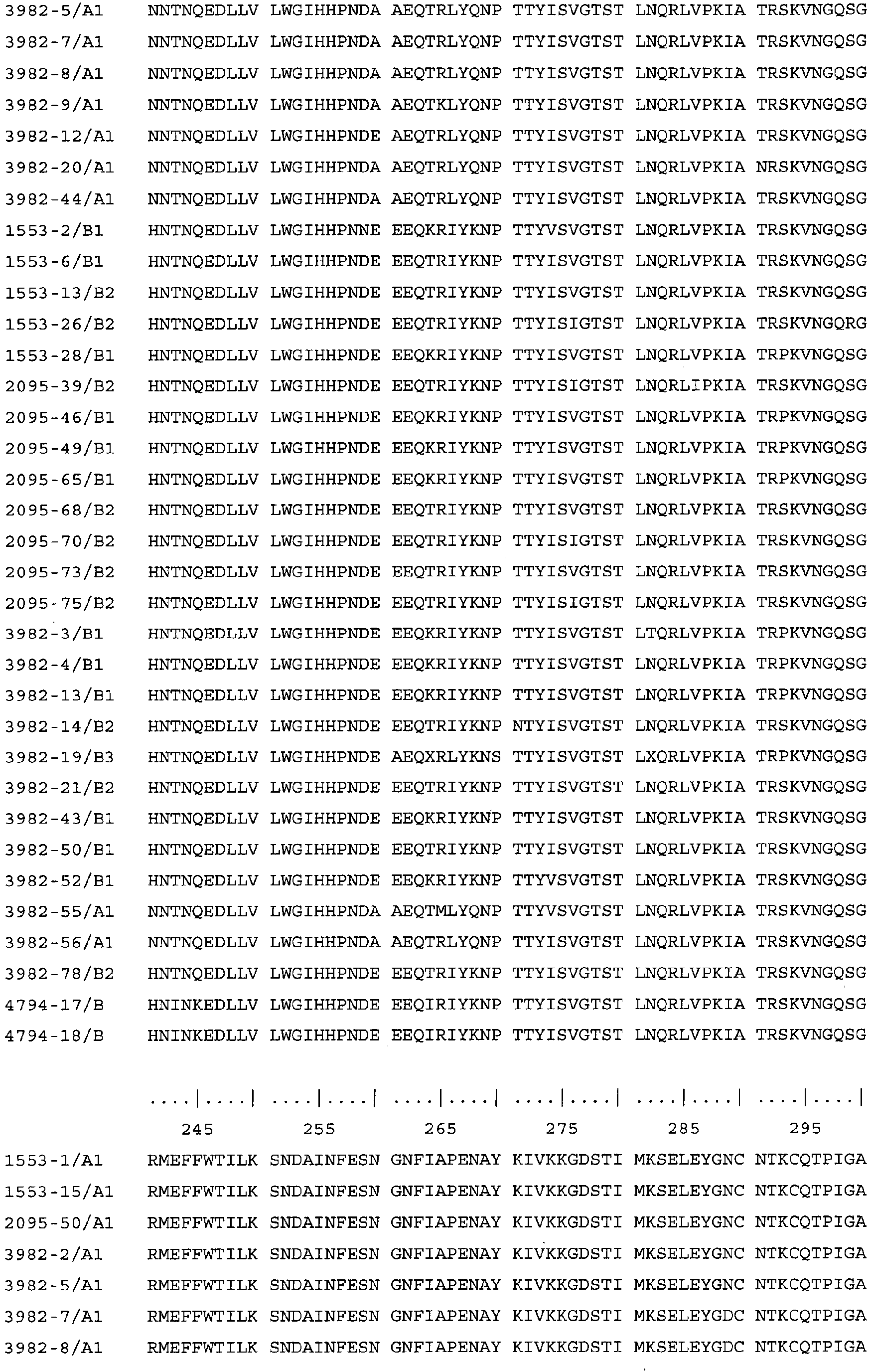

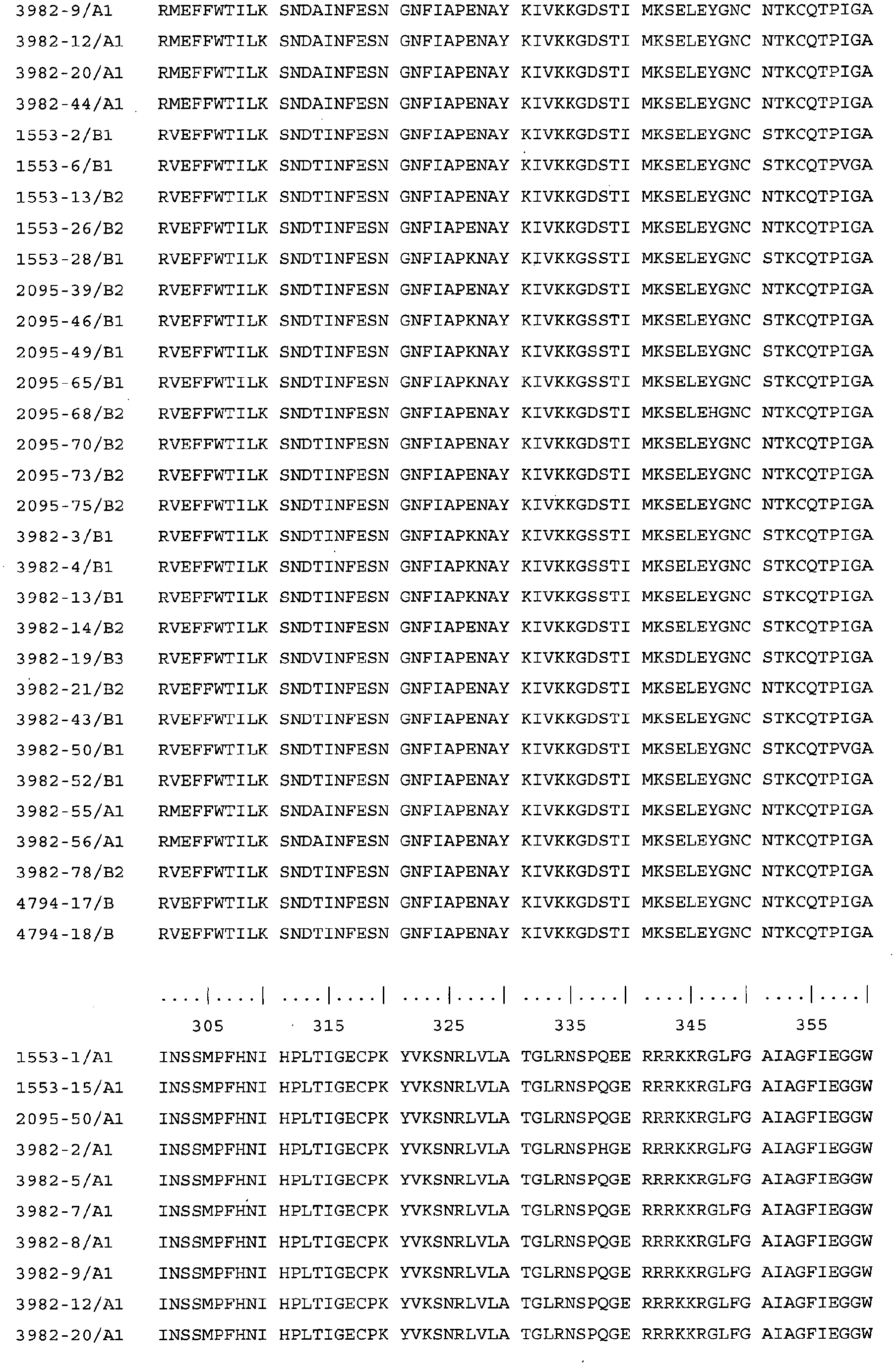

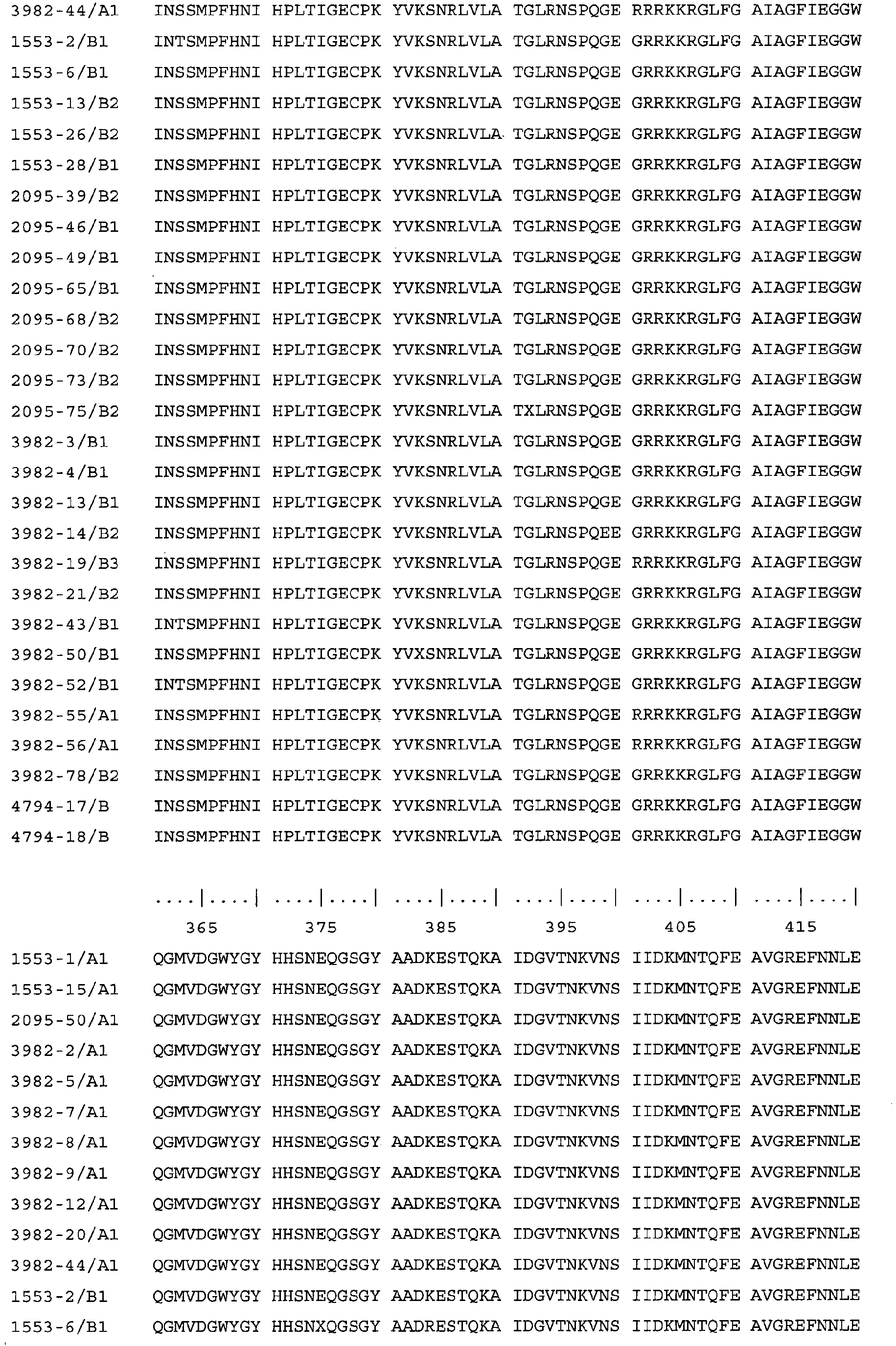

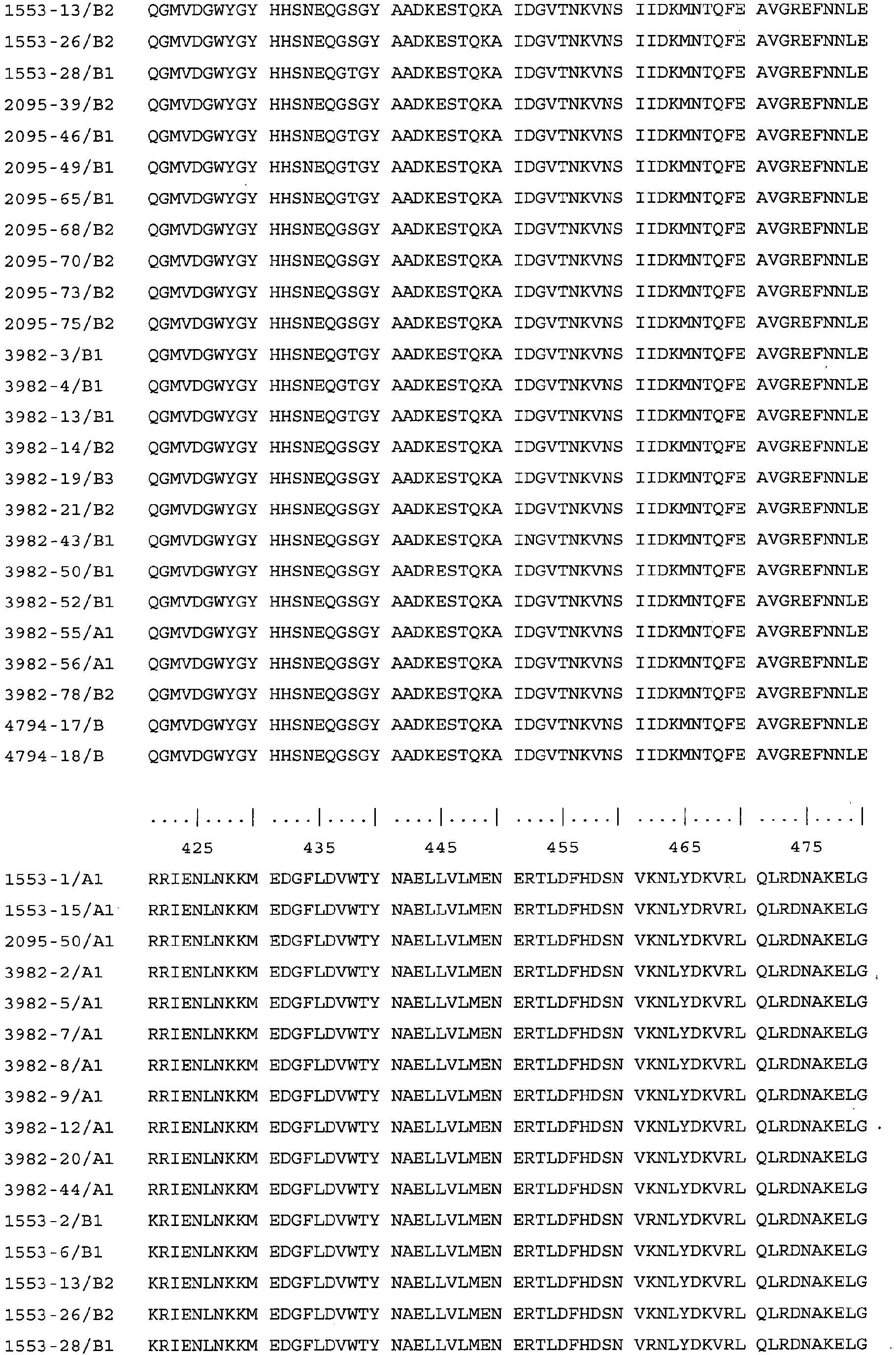

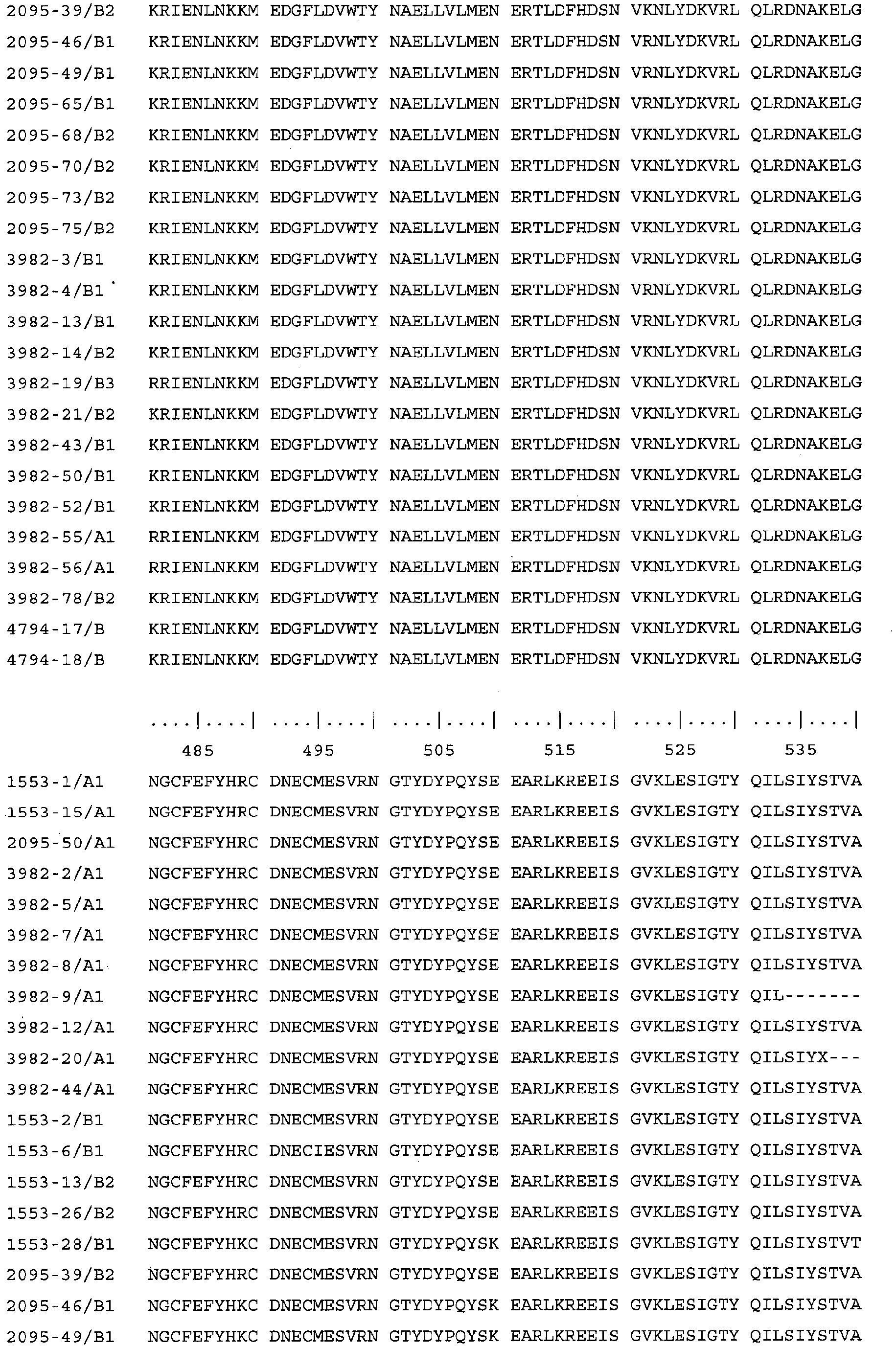

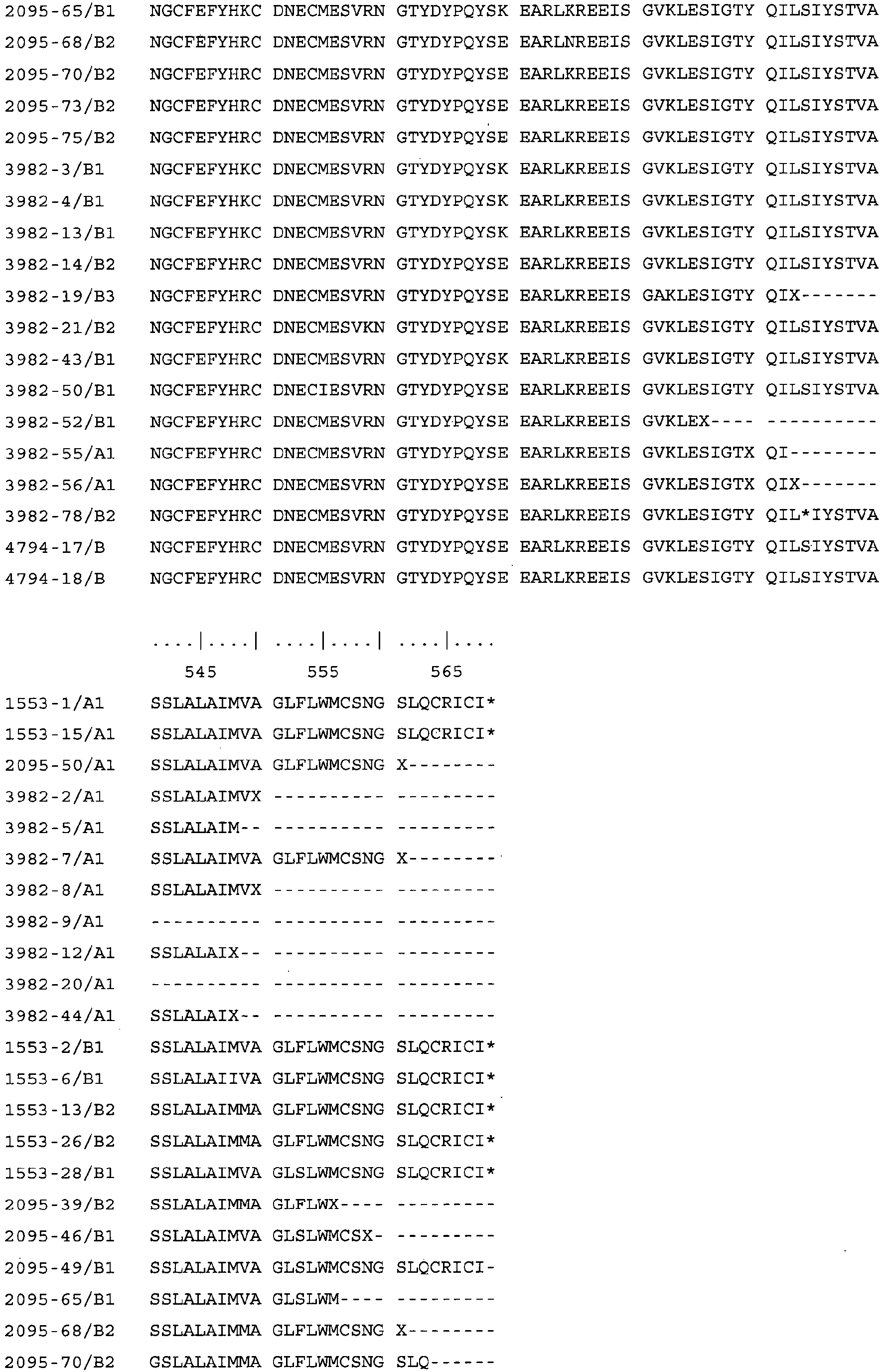

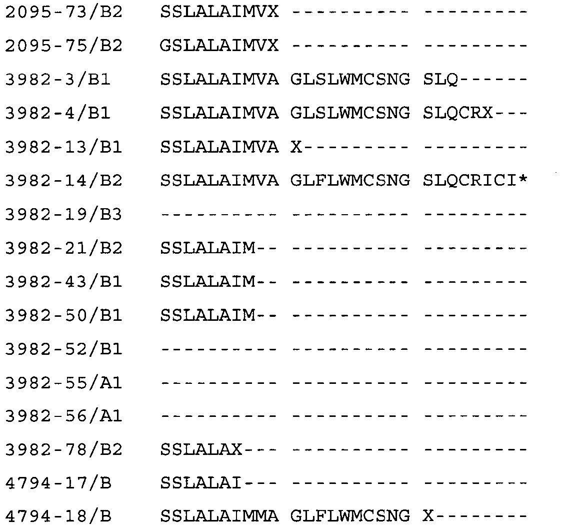

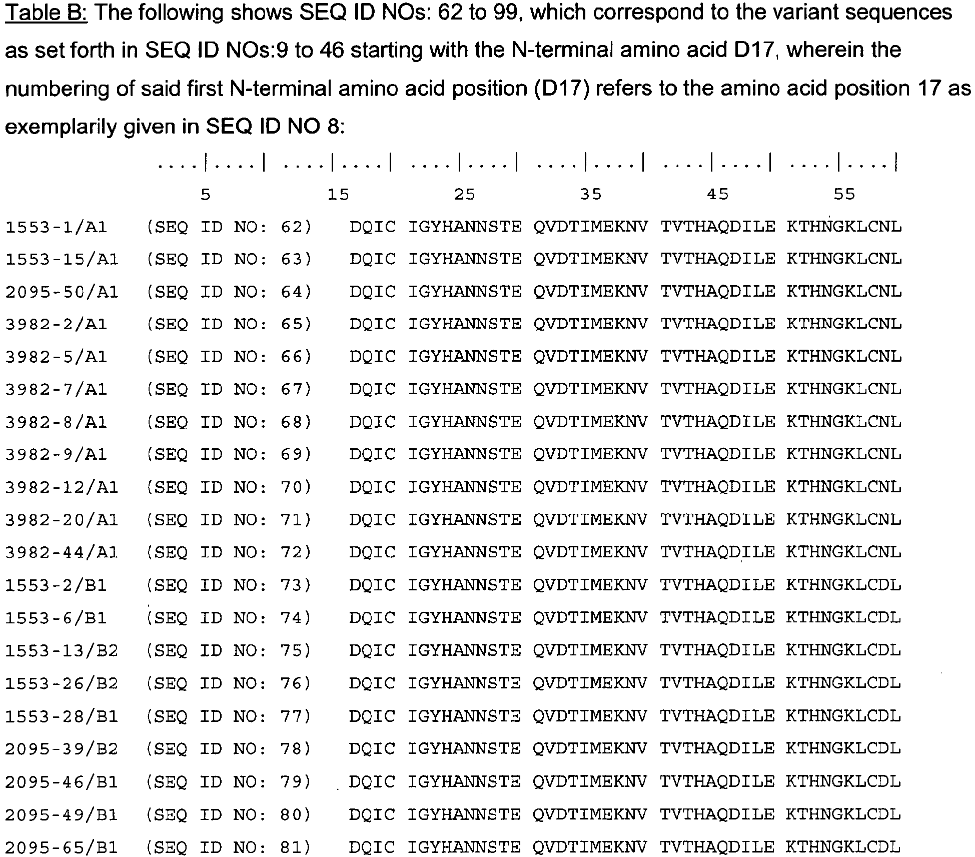

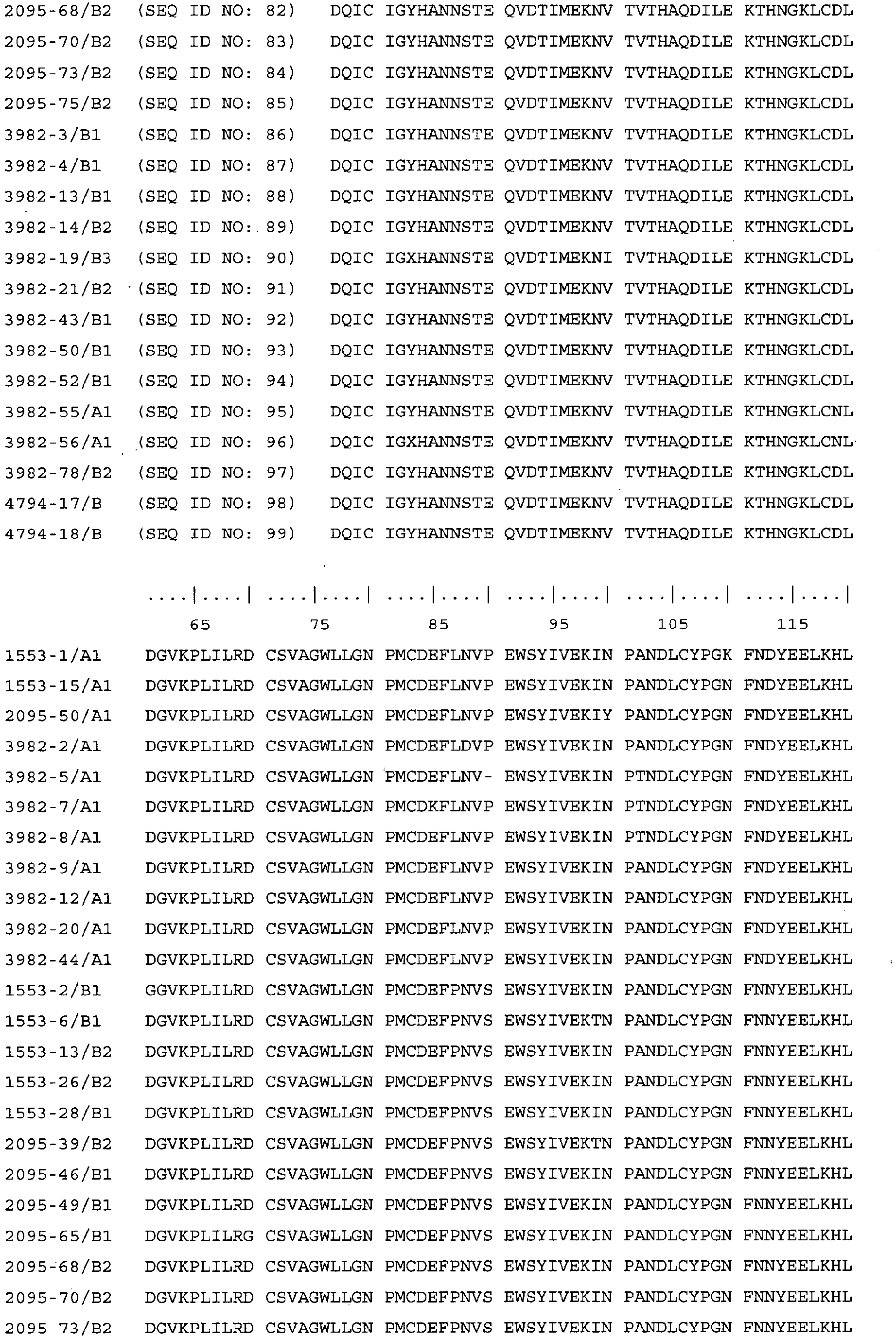

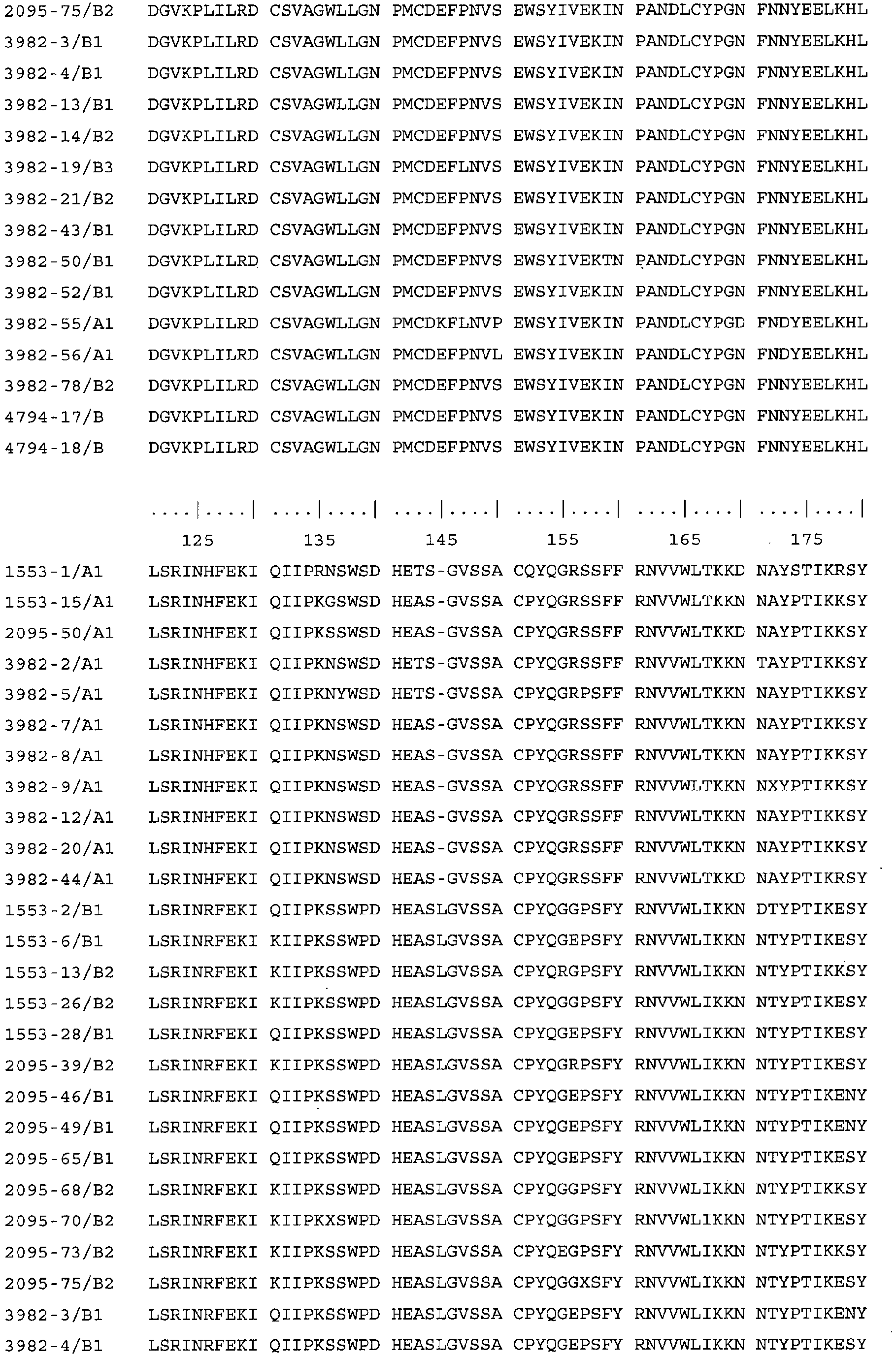

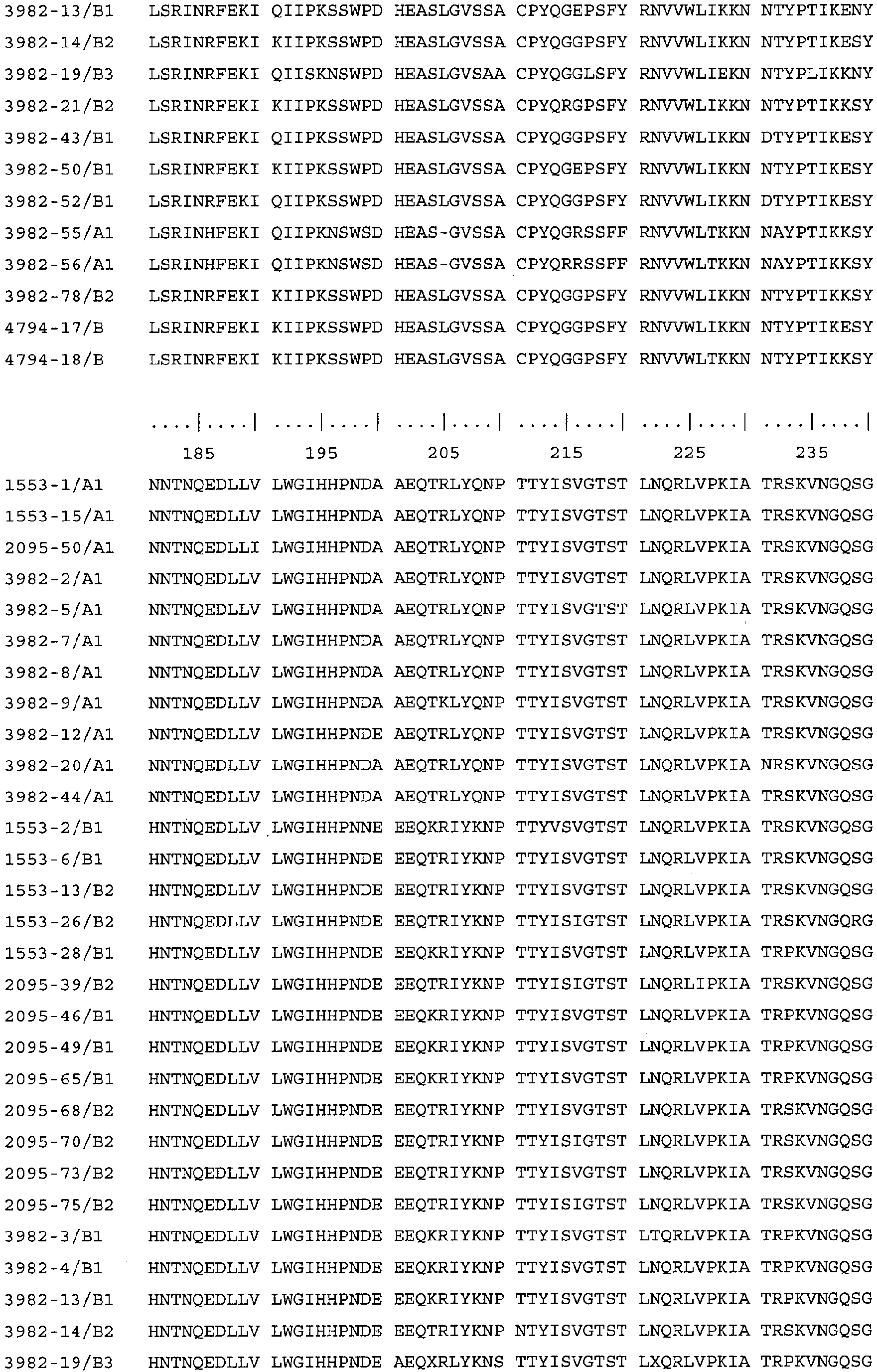

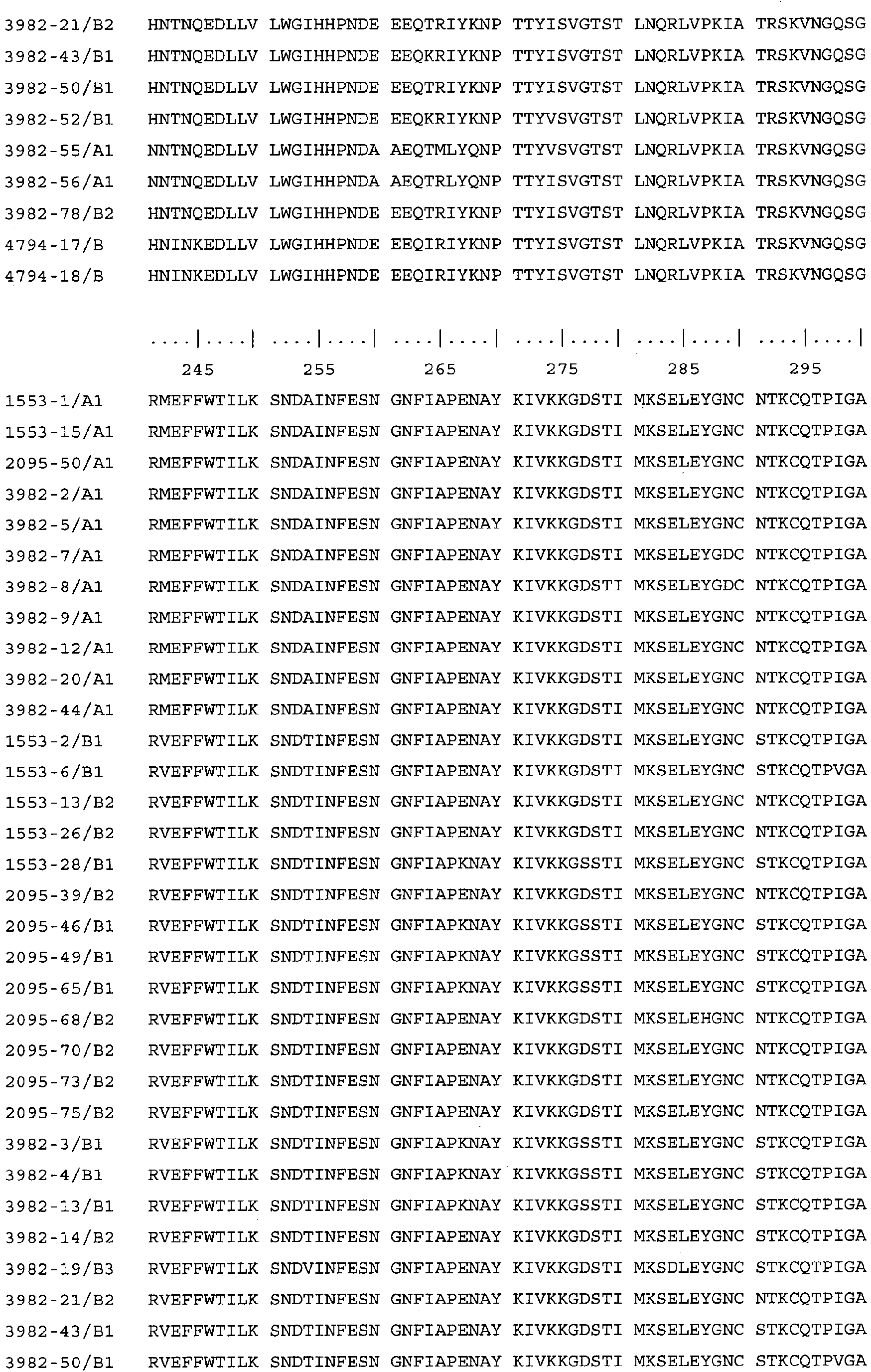

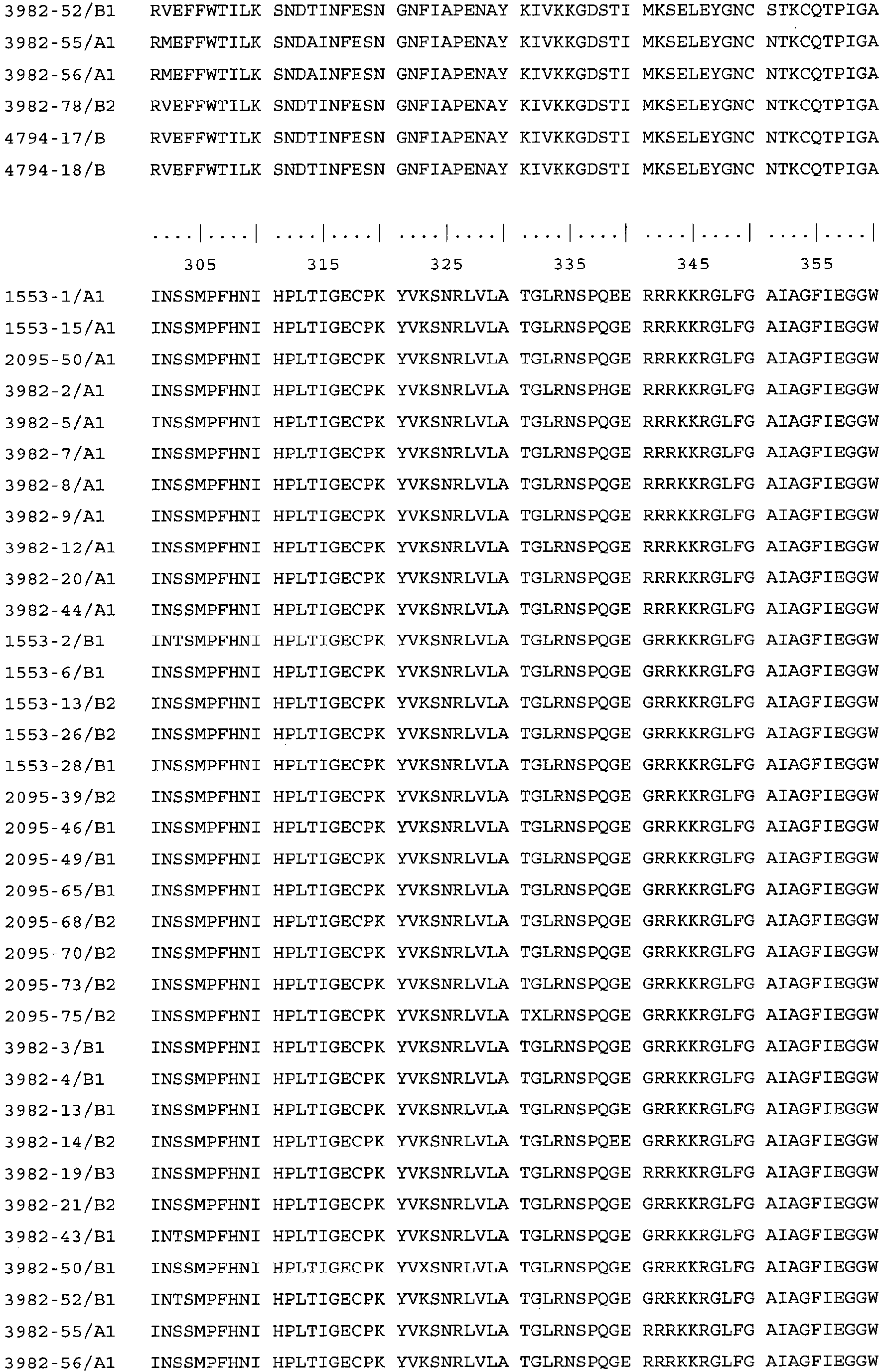

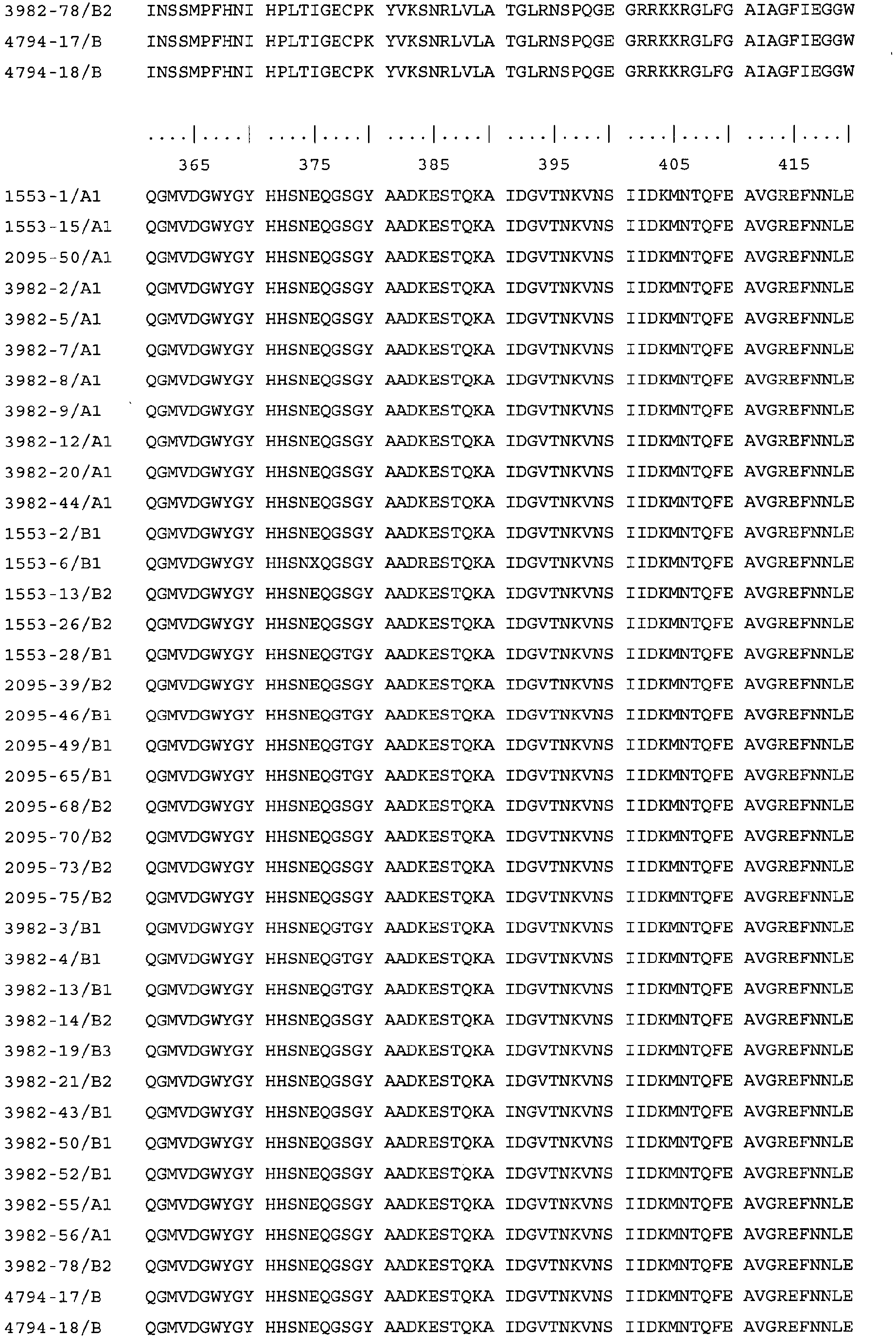

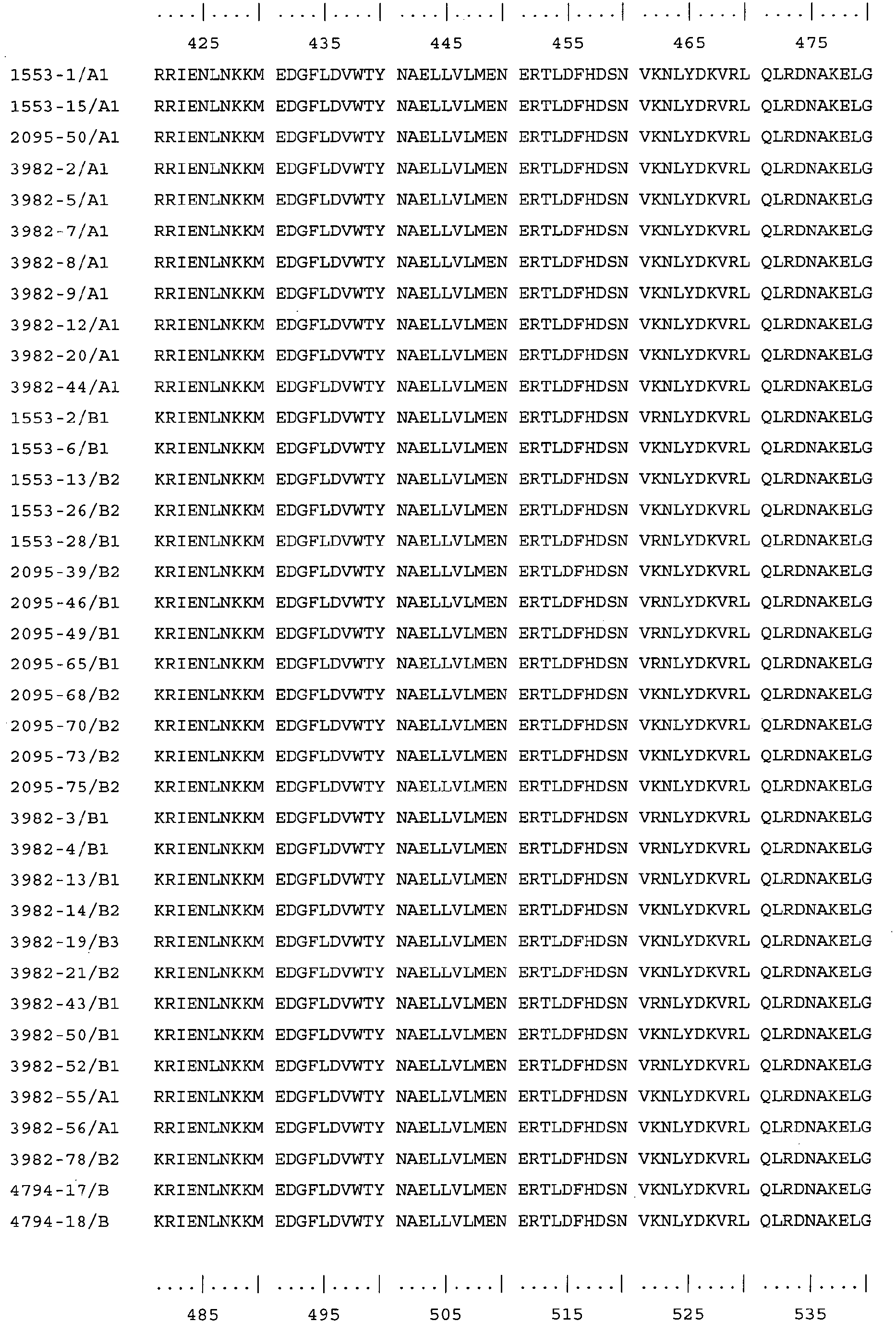

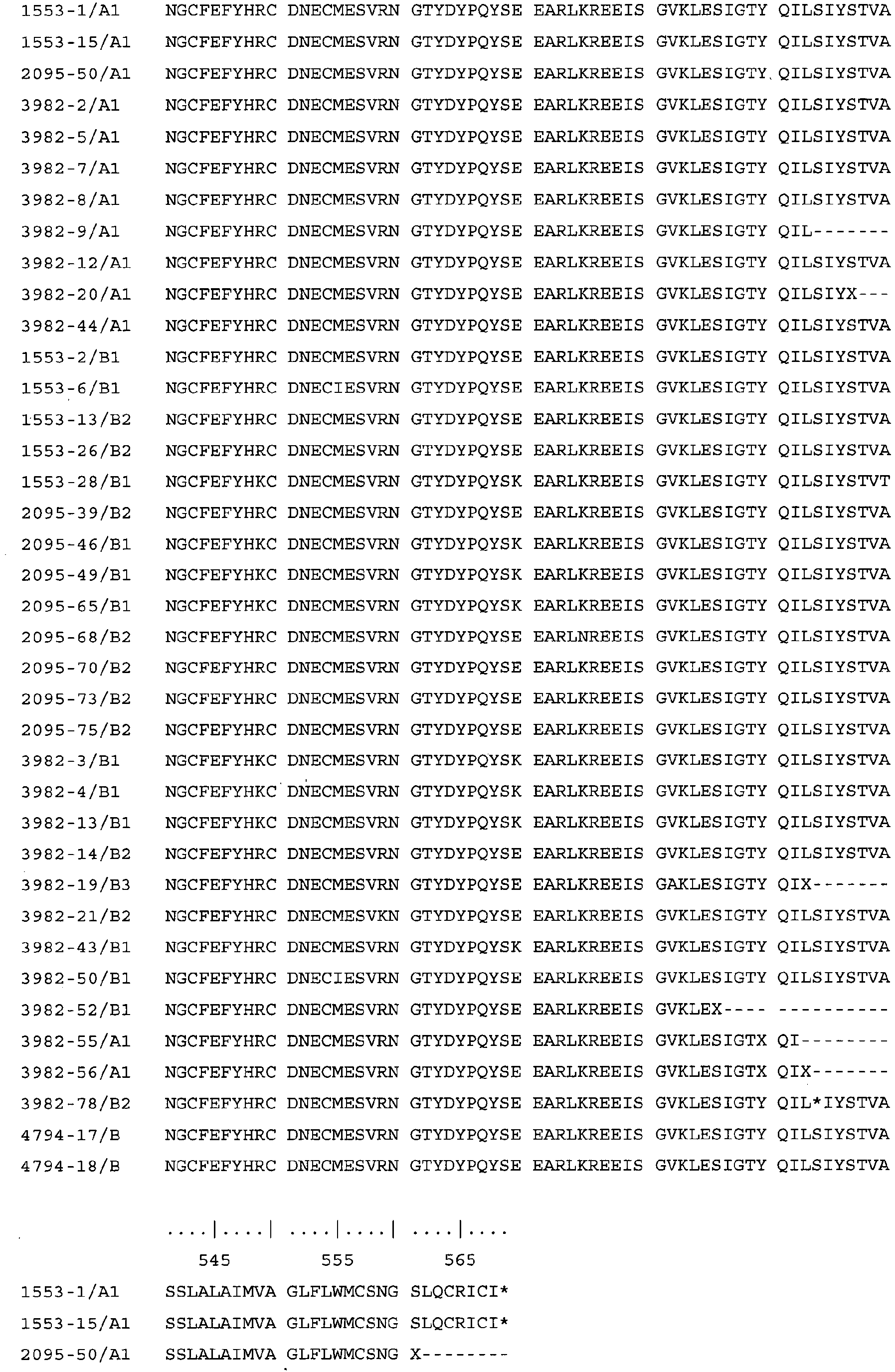

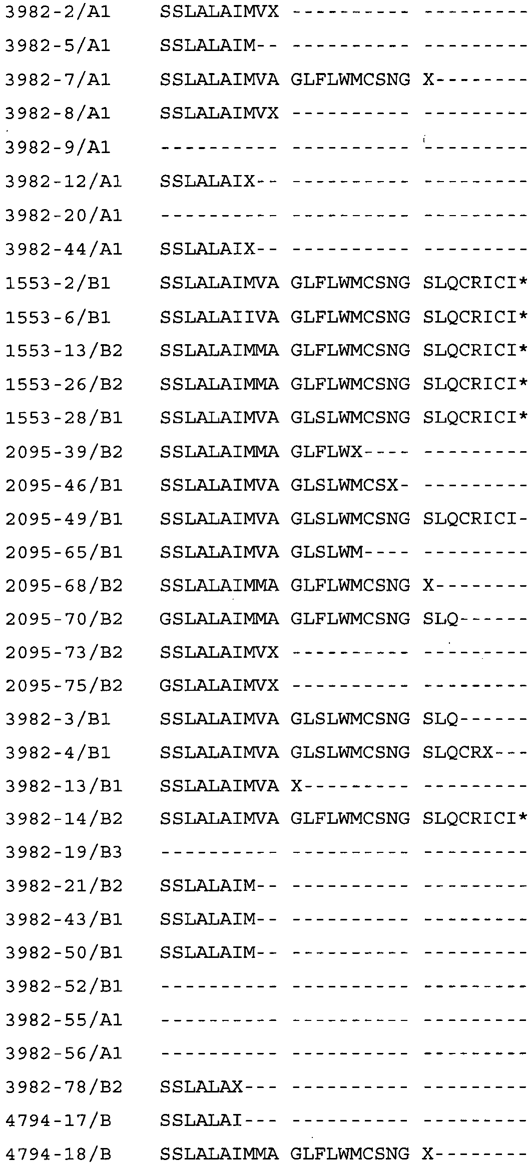

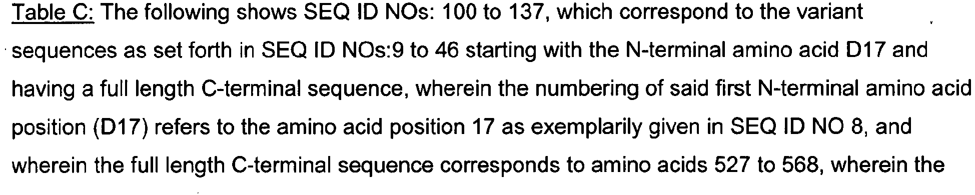

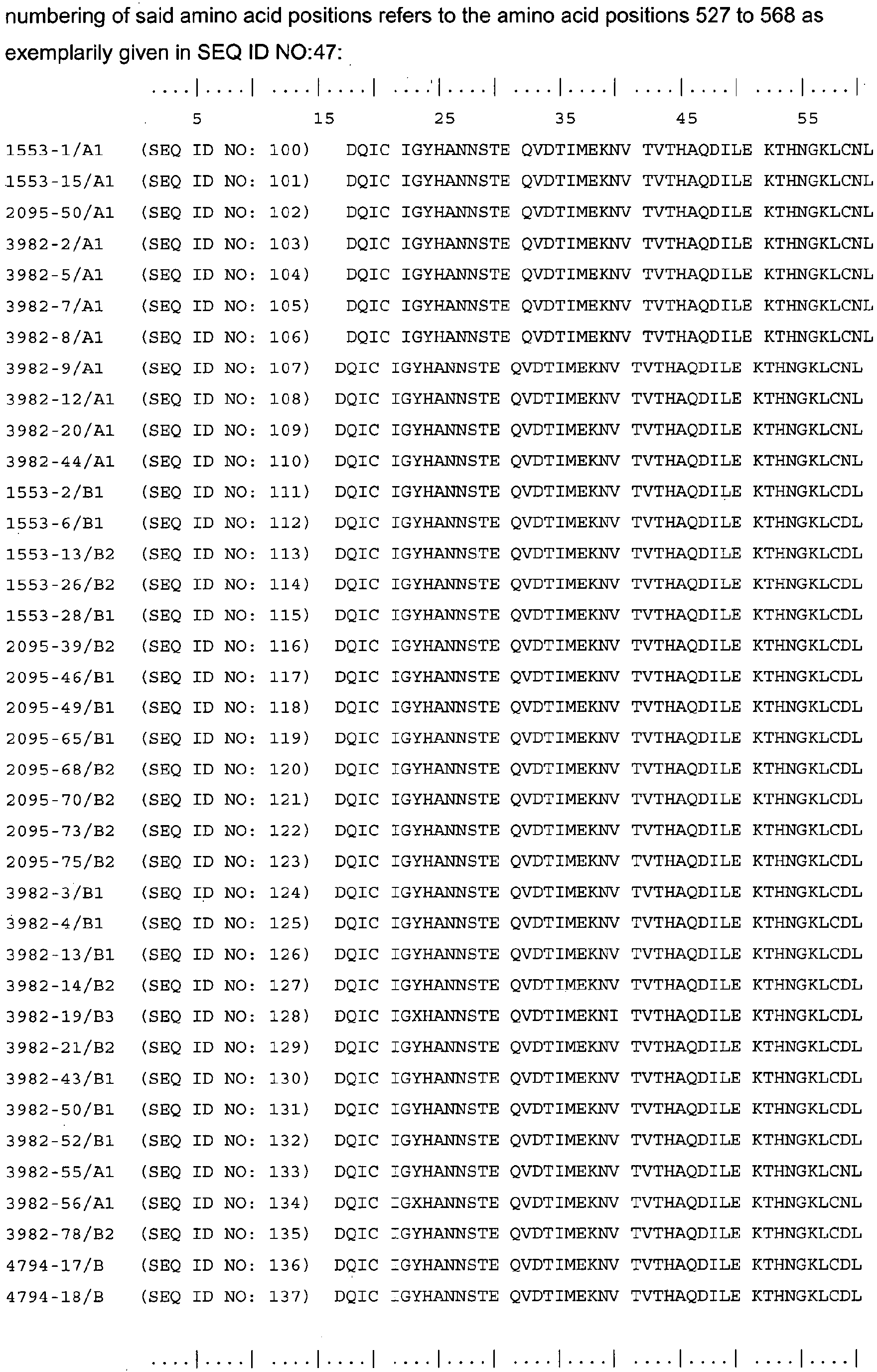

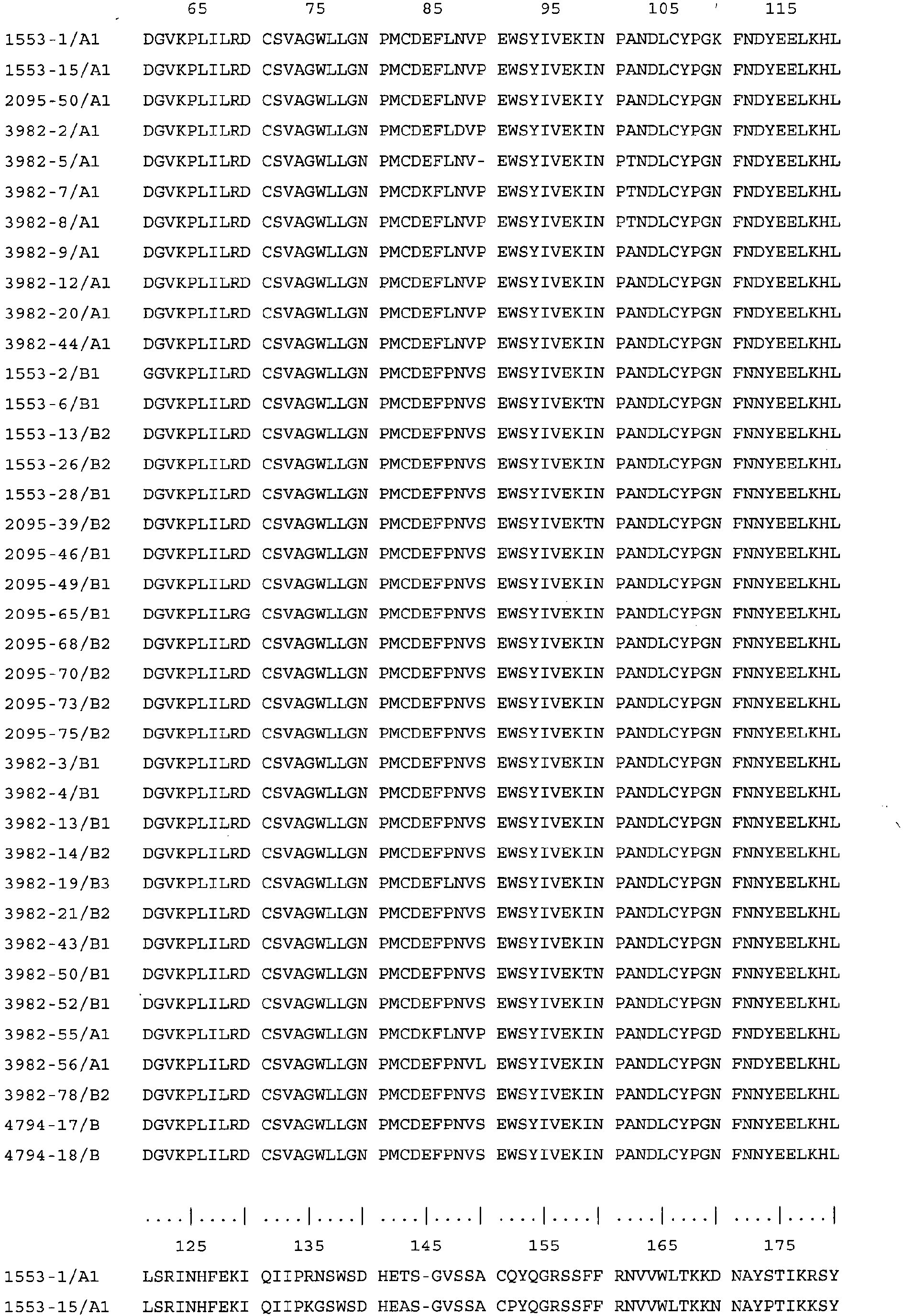

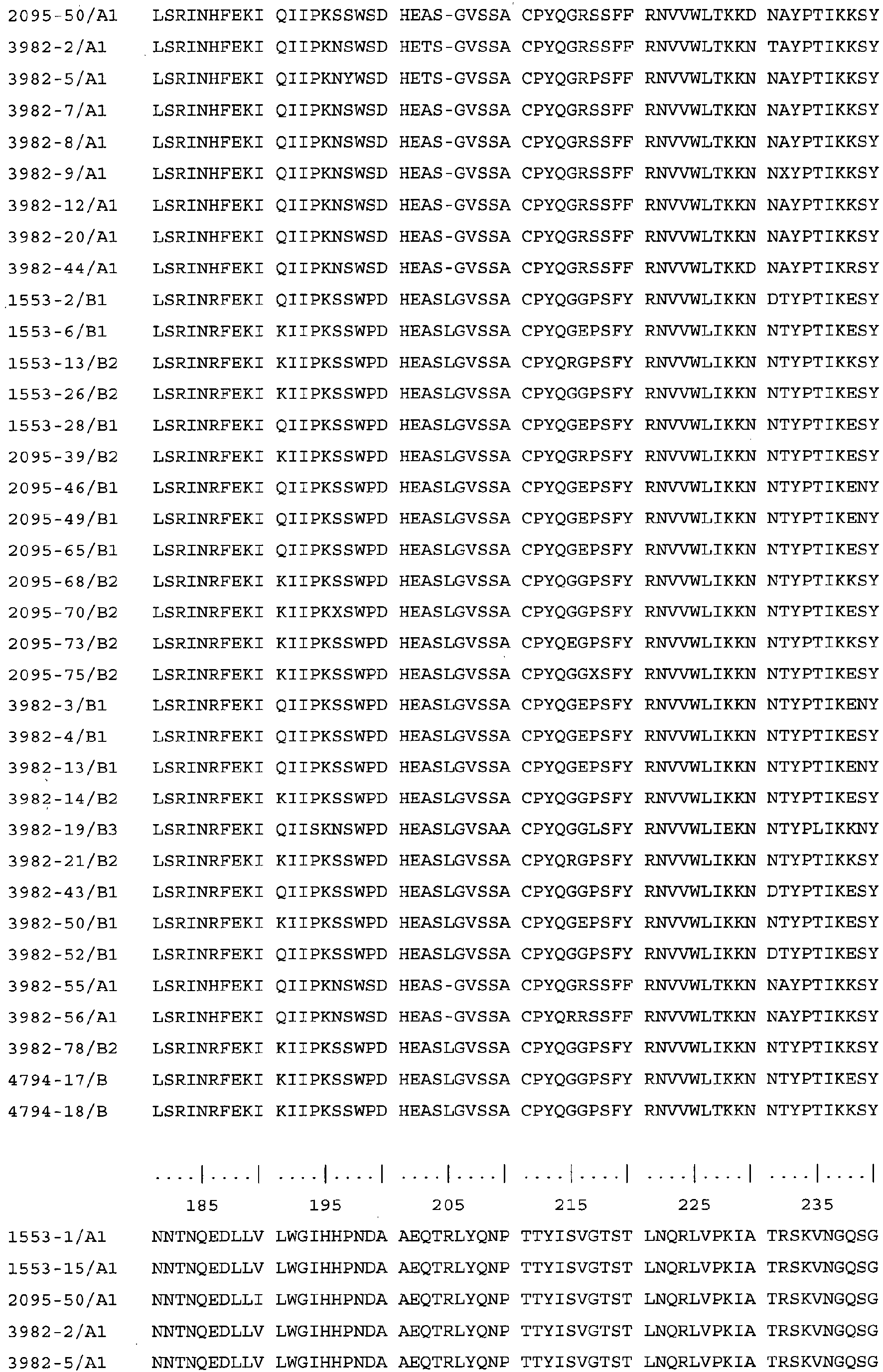

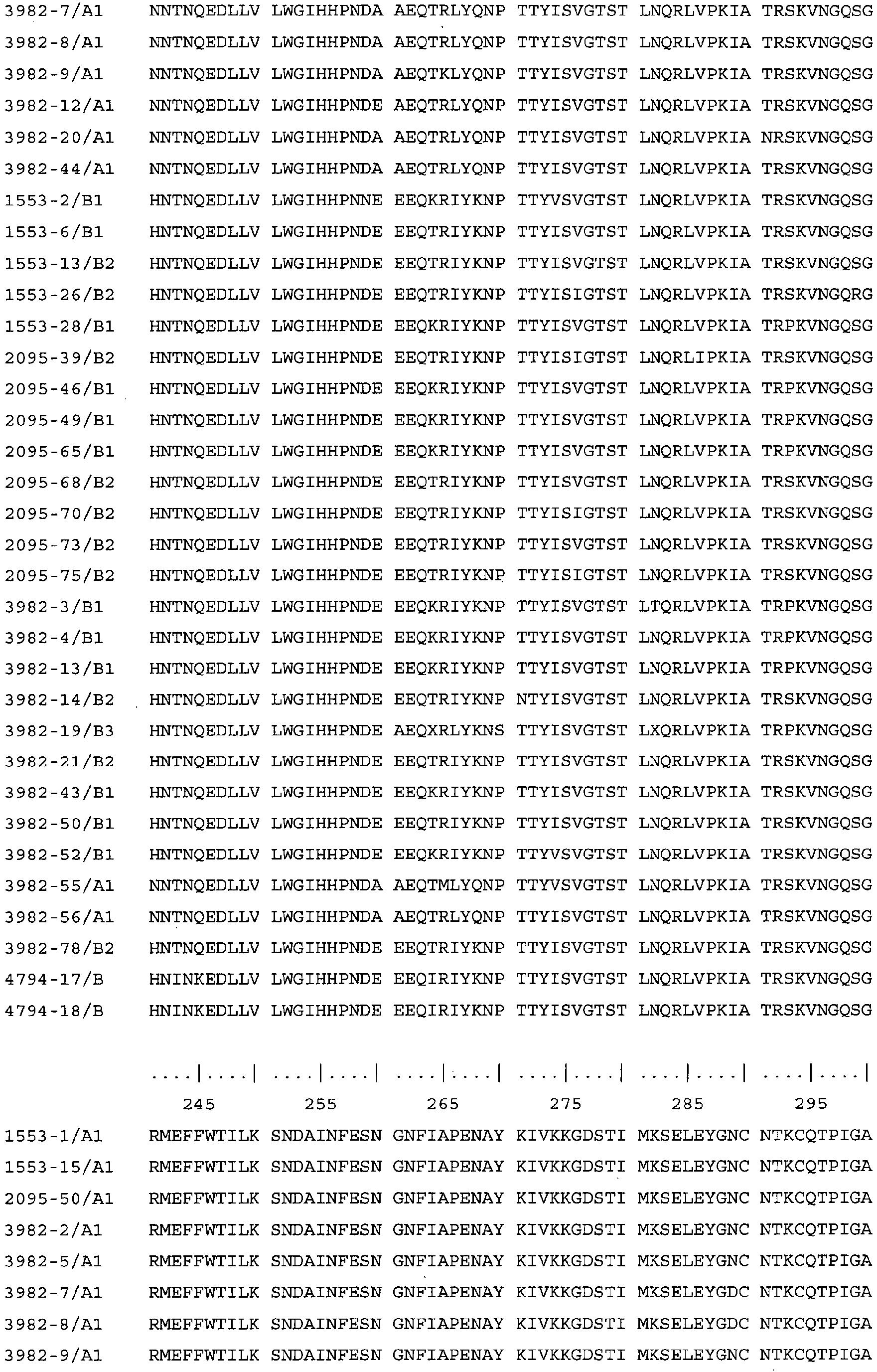

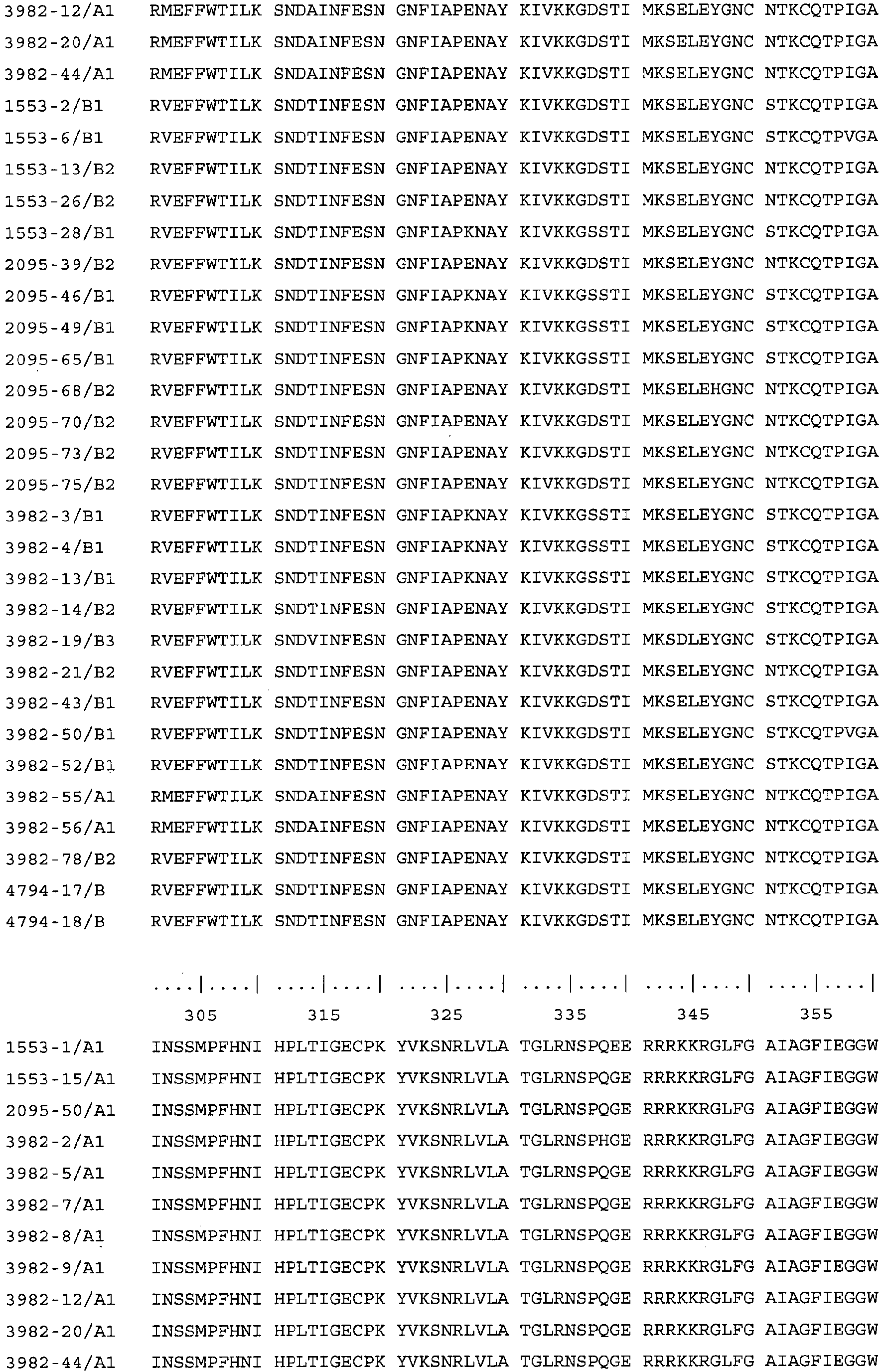

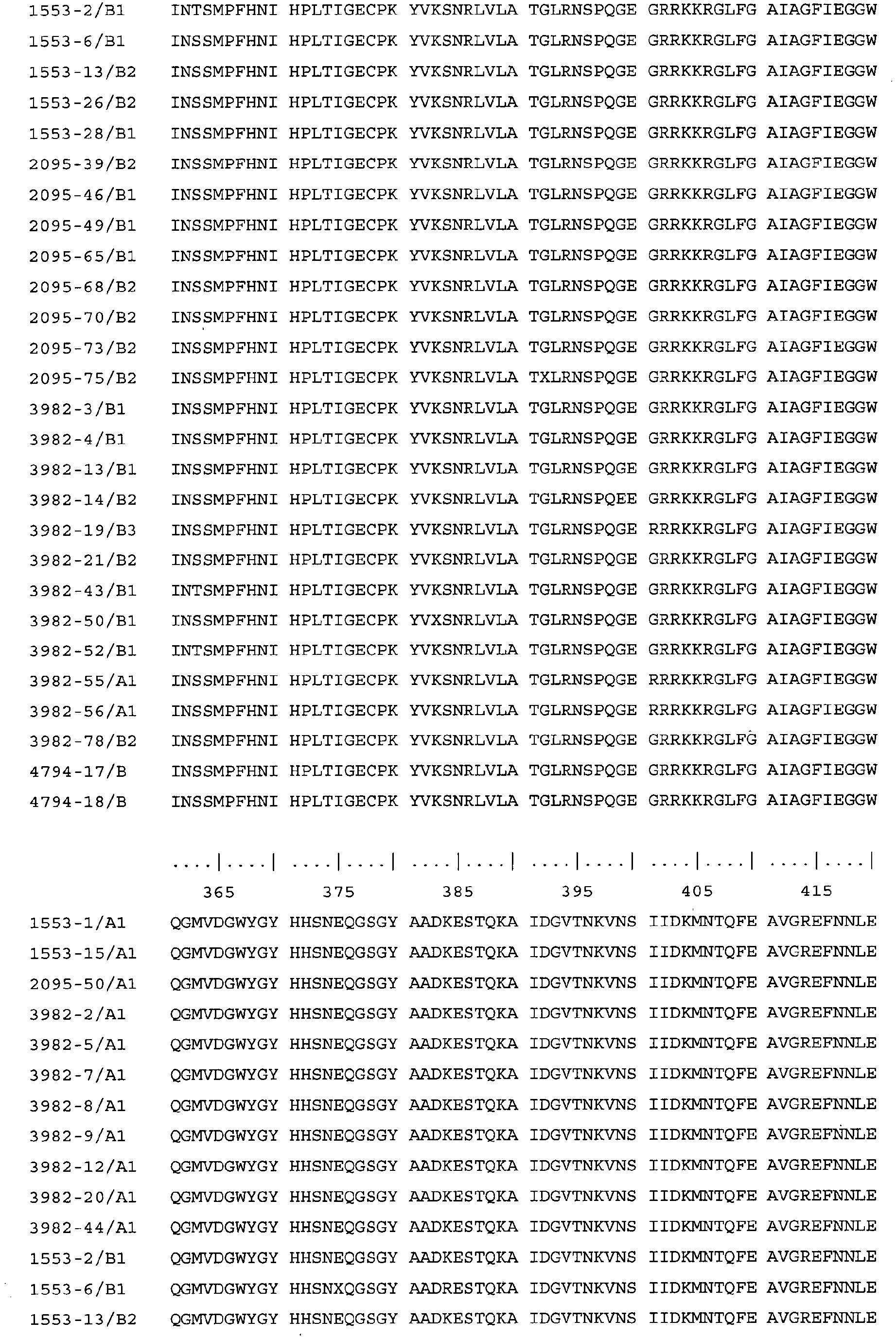

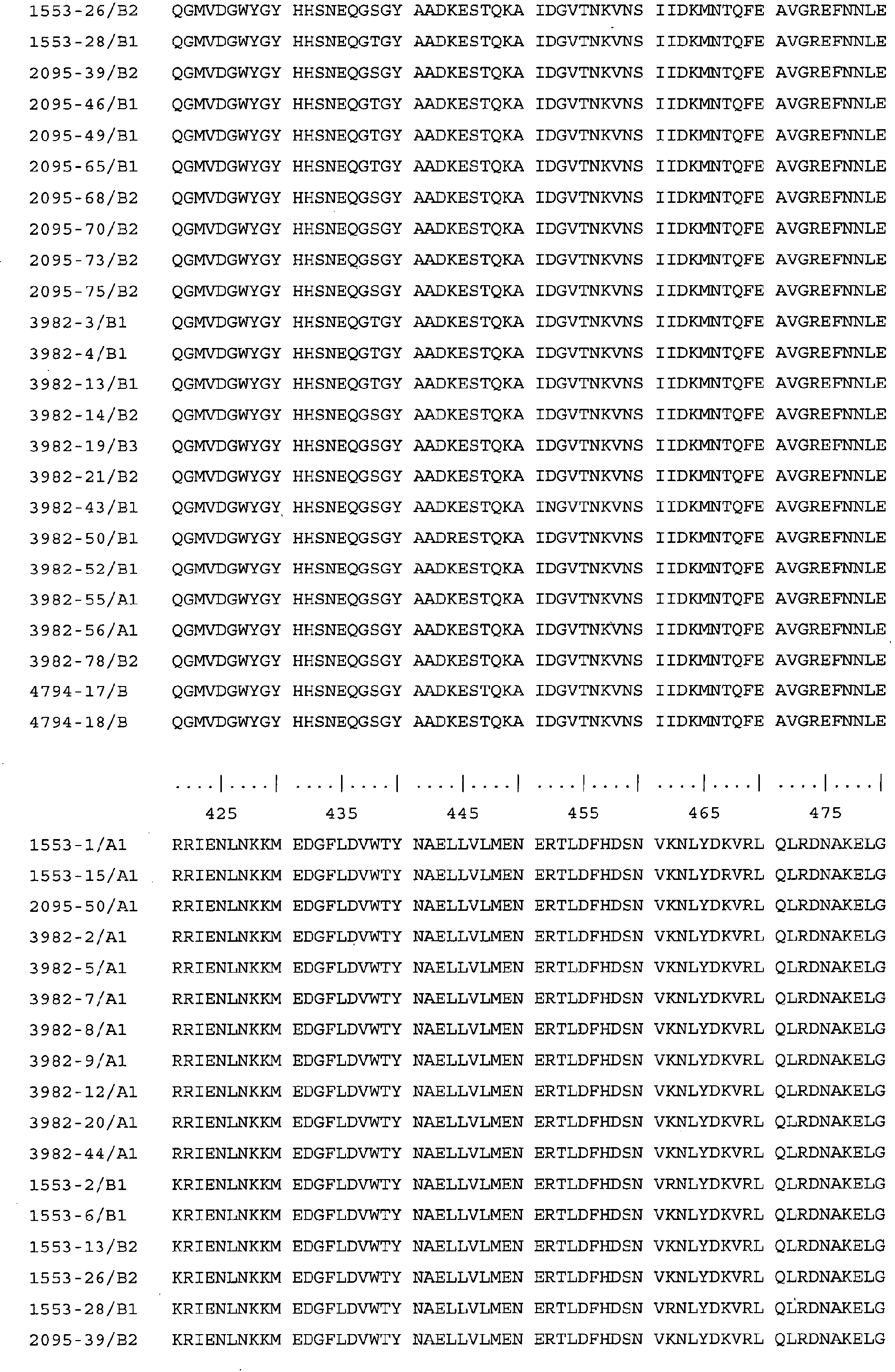

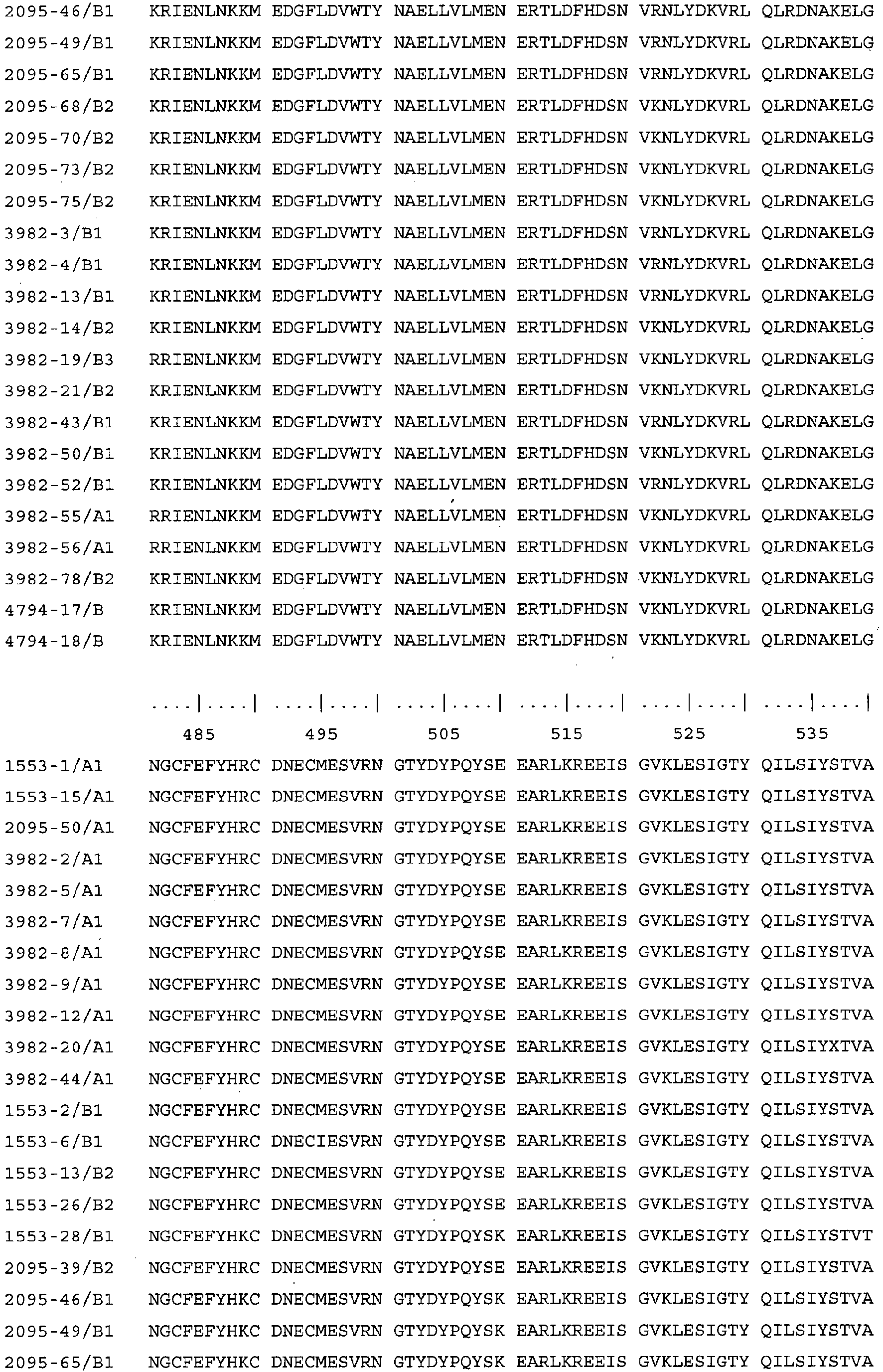

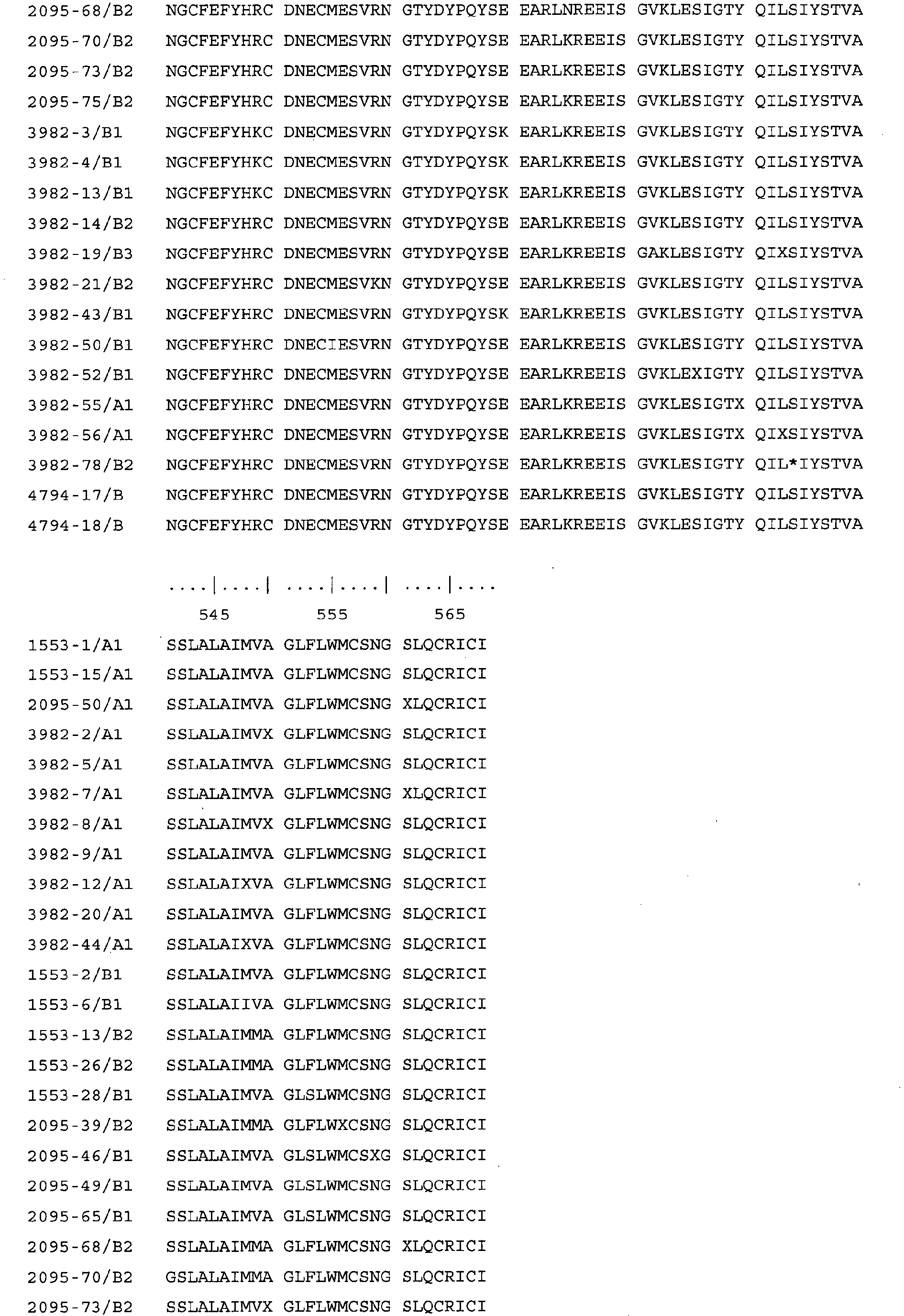

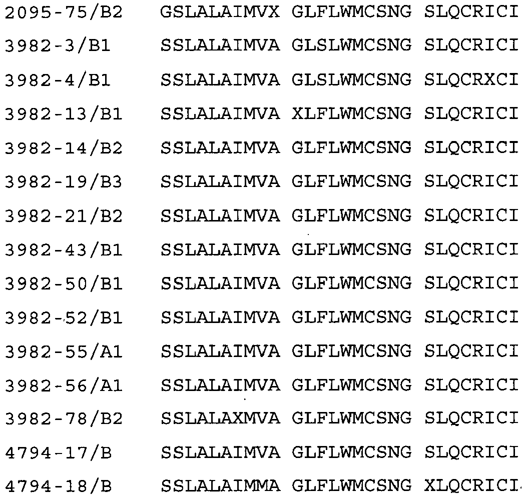

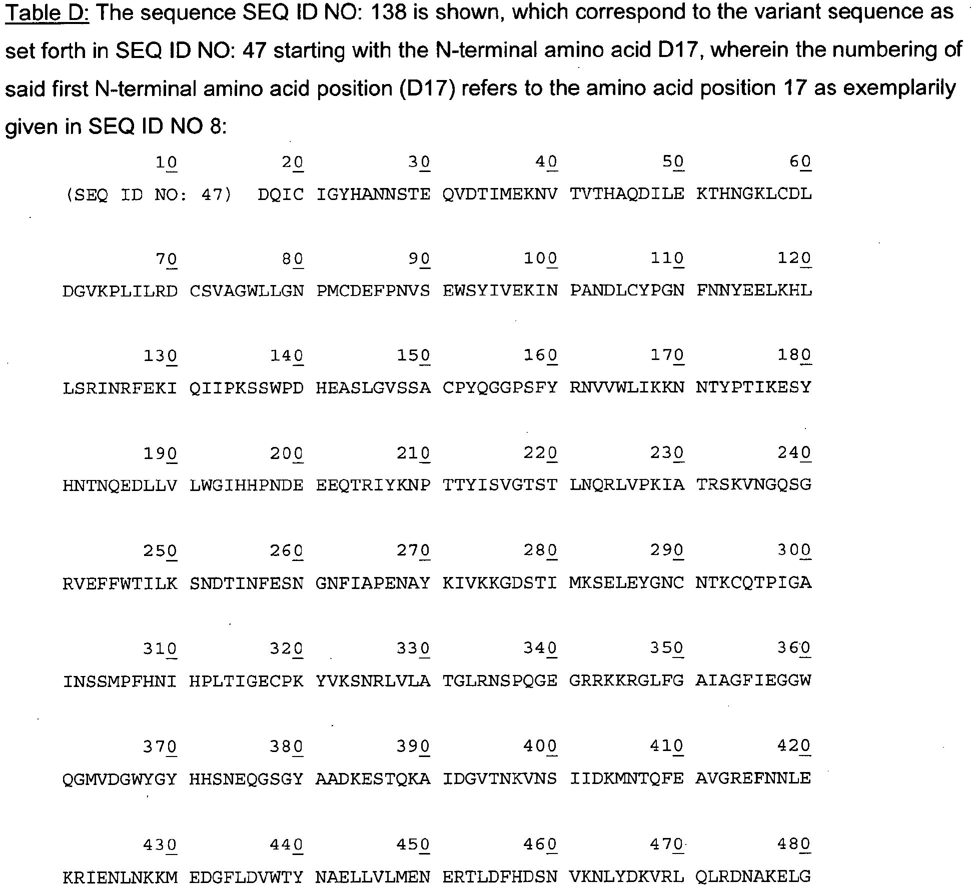

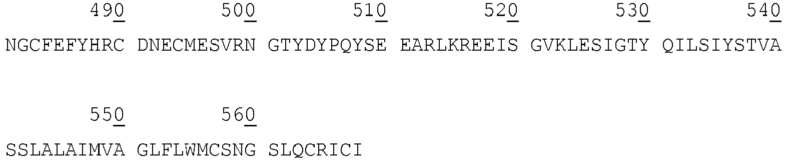

It is understood that the sequence provided in Table D (SEQ ID NO: 138) is a variant of the sequence SEQ ID NO: 47 starting with amino acid D17 and having the full length C-terminal or H5 protein.

## Claims

1. An H5 protein (1) of clade 1 H5N1 virus for use in a method of treating or preventing infections with H5N1 virus of a different clade, wherein said H5 protein (1) comprises or consists of the polypeptide sequence of SEQ ID NO:5,
wherein said H5N1 virus of a different clade is a H5N1 virus comprising a H5 protein (2) of influenza virus, wherein said H5 protein (2) consists of or comprises an amino acid sequence which has at least 98% sequence identity with any one of the sequences as set forth in SEQ ID NOs: 102 to 110, 116, 117, 119 to 126 and 128 to 137.

2. The H5 protein (1) for use according to claim 1, wherein said H5N1 virus of a different clade is a clade 2.2.1 H5N1 virus and/or, wherein said H5N1 virus of a different clade is a H5N1 virus of North African origin.

3. The H5 protein (1) for use according to claim 1 or 2,
wherein said H5N1 virus of a different clade comprises a polynucleotide encoding a H5 protein (2) having:
(a) the amino acids 87L, 113D, 126H, 145(-), 156R, 160F, 167T, and 181N, or
(b) the amino acids 87P, 113N, 126R, 145L, 160Y, 172T, 181H, 201E, 206I, 208K, 254T, 341G and 421K, or
(c) the amino acids 87L, 113N, 126R, 145L, 156G, 160Y, 172T, 181H, and 254V, and wherein the numbering of the amino acid positions of the H5 protein (2) refers to the amino acid position as exemplarily given in SEQ ID NO:8.

4. The H5 protein (1) for use according to any one of claims 1 to 3,
wherein said H5N1 virus of a different clade comprises a H5 protein (2) having:
(a) the amino acids 87L, 113D, 126H, 145(-), 156R, 160F, 167T, and 181N, or
(b) the amino acids 87P, 113N, 126R, 145L, 160Y, 172T, 181H, 201E, 206I, 208K, 254T, 341G and 421K, or
(c) the amino acids 87L, 113N, 126R, 145L, 156G, 160Y, 172T, 181H, and 254V
and wherein the numbering of the amino acid positions of the H5 protein (2) refers to the amino acid position as exemplarily given in SEQ ID NO:8.

5. The H5 protein (1) for use according to any one of claims 1 to 4, wherein said H5N1 virus of a different clade comprise a H5 protein (2) which consists of or comprises an amino acid sequence which has at least 98% sequence identity with the sequence as set forth in SEQ ID NO: 106, or the H5 protein (1) for use according to any one of claims 1 to 4, wherein said H5N1 virus of a different clade comprises a polynucleotide encoding a H5 protein (2) which consists of or comprises an amino acid sequence which has at least 98% sequence identity with the sequence as set forth in SEQ ID NO: 106.

6. The H5 protein (1) for use according to any one of claims 1 to 5 in a method of treating or preventing infections
(A) with Subclade A H5N1 virus of North African origin, namely an infection with a H5N1 virus comprising a polynucleotide encoding a H5 protein (2) having the amino acids according to (a) of claim 3 or 4 or an infection with a H5N1 virus comprising a polynucleotide encoding a H5 protein (2) according to claim 3 or 4 relating to any one of the sequences as set forth in SEQ ID NOs: 102 to 110, 133 or 134,
or
(B) with Subclade B H5N1 virus of North African origin, namely an infection with a H5N1 virus comprising a polynucleotide encoding a H5 protein (2) having the amino acids according to (b) or (c) of claim 3 or 4 or an infection with a H5N1 virus comprising a polynucleotide encoding a H5 protein (2) according to claim 3 or 4 relating to any one of the sequences as set forth in SEQ ID NOs: 116, 117, 119 to 126, 128 to 132 or 135 to 137.

7. The H5 protein (1) for use according to any one of claims 1 to 6 in a method of treating or preventing infections
(A) with Subclade A H5N1 virus of North African origin, namely an infection with a H5N1 virus comprising a H5 protein (2) having the amino acids according to (a) of claim 3 or 4 or comprising a H5 protein (2) according to claim 3 or 4 relating to any one of the sequences as set forth in SEQ ID NOs: 102 to 110, 133 or 134,
or
(B) with Subclade B H5N1 virus of North African origin, namely an infection with a H5N1 virus comprising a H5 protein (2) having the amino acids according to (b) or (c) of claim 3 or 4 or comprising a H5 protein (2) according to claim 3 or 4 relating to any one of the sequences as set forth in SEQ ID NOs: 116, 117, 119 to 126, 128 to 132 or 135 to 137.

8. A vaccine for use in a method of treating or preventing infections with H5N1 virus of a different clade as defined in any one of claims 1 to 7 comprising or consisting of:
a. the H5 protein (1) of claim 1, and
b. a pharmaceutical acceptable carrier and/or excipient.

9. The vaccine for use according to claim 8, wherein the excipient is an Emulsigen-based adjuvant.

10. The vaccine for use of claim 8 or 9, wherein the vaccine is a single-shot vaccine or a one dose vaccine.

11. The vaccine for use of any one of claims 8 to 10 for reducing viral shedding in a subject infected with or at risk of a viral infection with H5N1 virus of a clade other than clade 1.

12. A combination of
the H5 protein (1) of claim 1 and an inactivted Newcastle disease virus
for use in a method of treating or preventing infections with H5N1 virus of a different clade as defined in any one of claims 1 to 7.

13. The combination for use of claim 12, wherein the inactivated Newcastle disease virus is an inactivated Newcastle disease virus obtained by inactivation of a Newcastle disease virus comprising a RNA polynucleotide having at least 70% sequence identity with a RNA copy of the polynucleotide set forth in SEQ ID NO: 139, which has been inactivated.

## Patentansprüche

1. H5-Protein (1) des Klade 1 H5N1 -Virus zur Verwendung in einem Verfahren zur Behandlung von oder Vorbeugung vor Infektionen mit H5N1-Virus einer anderen Klade, wobei das H5-Protein (1) die Polypeptidsequenz von SEQ ID NO:5 umfasst oder daraus besteht,
wobei das H5N1-Virus einer anderen Klade ein H5N1-Virus ist, der ein H5-Protein (2) des Influenzavirus umfasst, wobei das H5-Protein (2) aus einer Aminosäuresequenz besteht oder diese umfasst, die mindestens 98% Sequenzidentität mit irgendeiner der Sequenzen aufweist, wie in SEQ ID NOs: 102 bis 110, 116, 117, 119 bis 126 und 128 bis 137 dargelegt.

2. H5-Protein (1) zur Verwendung nach Anspruch 1, wobei das H5N1 -Virus einer anderen Klade ein Klade 2.2.1 H5N1-Virus ist und/oder wobei das H5N1-Virus einer anderen Klade ein H5N1-Virus nordafrikanischen Ursprungs ist.

3. H5-Protein (1) zur Verwendung nach Anspruch 1 oder 2,
wobei das H5N1-Virus einer anderen Klade ein Polynukleotid umfasst, das ein H5-Protein (2) kodiert, das
(a) die Aminosäuren 87L, 113D, 126H, 145(-), 156R, 160F, 167T und 181N oder
(b) die Aminosäuren 87P, 113N, 126R, 145L, 160Y, 172T, 181H, 201E, 206I, 208K, 254T, 341G und 421K oder
(c) die Aminosäuren 87L, 113N, 126R, 145L, 156G, 160Y, 172T, 181H und 254V aufweist,
und wobei sich die Nummerierung der Aminosäurepositionen des H5-Proteins (2) auf die Aminosäureposition bezieht, wie beispielhaft in SEQ ID NO: 8 angegeben.

4. H5-Protein (1) zur Verwendung nach irgendeinem der Ansprüche 1 bis 3,
wobei das H5N1-Virus einer anderen Klade ein H5-Protein (2) umfasst, das
(a) die Aminosäuren 87L, 113D, 126H, 145(-), 156R, 160F, 167T und 181N oder
(b) die Aminosäuren 87P, 113N, 126R, 145L, 160Y, 172T, 181H, 201E, 206I, 208K, 254T, 341G und 421K oder
(c) die Aminosäuren 87L, 113N, 126R, 145L, 156G, 160Y, 172T, 181H und 254V aufweist,
und wobei sich die Nummerierung der Aminosäurepositionen des H5-Proteins (2) auf die Aminosäureposition bezieht, wie beispielhaft in SEQ ID NO: 8 angegeben.

5. H5-Protein (1) zur Verwendung nach irgendeinem der Ansprüche 1 bis 4, wobei das H5N1-Virus einer anderen Klade ein H5-Protein (2) umfasst, das aus einer Aminosäuresequenz besteht oder diese umfasst, die mindestens 98% Sequenzidentität mit der Sequenz wie in SEQ ID NO: 106 dargelegt aufweist,
oder
H5-Protein (1) zur Verwendung nach irgendeinem der Ansprüche 1 bis 4, wobei das H5N1-Virus einer anderen Klade ein Polynukleotid umfasst, das ein H5-Protein (2) kodiert, das aus einer Aminosäuresequenz besteht oder diese umfasst, die mindestens 98% Sequenzidentität mit der Sequenz wie in SEQ ID NO: 106 dargelegt aufweist.

6. H5-Protein (1) zur Verwendung nach irgendeinem der Ansprüche 1 bis 5 in einem Verfahren zur Behandlung von oder Vorbeugung vor Infektionen (A) mit Subklade A H5N1-Virus nordafrikanischen Ursprungs, nämlich einer Infektion mit einem H5N1-Virus, umfassend ein Polynukleotid, das ein H5-Protein (2) kodiert, das die Aminosäuren gemäß (a) von Anspruch 3 oder 4 aufweist, oder einer Infektion mit einem H5N1-Virus, umfassend ein Polynukleotid, das ein H5-Protein (2) gemäß Anspruch 3 oder 4 in Bezug auf irgendeine der Sequenzen wie in SEQ ID NOs: 102 bis 110, 133 oder 134 dargelegt kodiert,
oder
(B) mit Subklade B H5N1-Virus nordafrikanischen Ursprungs, nämlich einer Infektion mit einem H5N1-Virus, umfassend ein Polynukleotid, das ein H5-Protein (2) kodiert, das die Aminosäuren gemäß (b) oder (c) von Anspruch 3 oder 4 aufweist, oder einer Infektion mit einem H5N1-Virus, umfassend ein Polynukleotid, das ein H5-Protein (2) gemäß Anspruch 3 oder 4 in Bezug auf irgendeine der Sequenzen wie in SEQ ID NOs: 116, 117, 119 bis 126, 128 bis 132 oder 135 bis 137 dargelegt kodiert.

7. H5-Protein (1) zur Verwendung nach irgendeinem der Ansprüche 1 bis 6 in einem Verfahren zur Behandlung von oder Vorbeugung vor Infektionen
(A) mit Subklade A H5N1-Virus nordafrikanischen Ursprungs, nämlich einer Infektion mit einem H5N1-Virus, umfassend ein H5-Protein (2), das die Aminosäuren gemäß (a) von Anspruch 3 oder 4 aufweist, oder umfassend ein H5-Protein (2) gemäß Anspruch 3 oder 4 in Bezug auf irgendeine der Sequenzen wie in SEQ ID NOs: 102 bis 110, 133 oder 134 dargelegt,
oder
(B) mit Subklade B H5N1-Virus nordafrikanischen Ursprungs, nämlich einer Infektion mit einem H5N1-Virus, umfassend ein H5-Protein (2), das die Aminosäuren gemäß (b) oder (c) von Anspruch 3 oder 4 aufweist, oder umfassend ein H5-Protein (2) gemäß Anspruch 3 oder 4 in Bezug auf irgendeine der Sequenzen wie in SEQ ID NOs: 116, 117, 119 bis 126, 128 bis 132 oder 135 bis 137 dargelegt.

8. Impfstoff zur Verwendung in einem Verfahren zur Behandlung von oder Vorbeugung vor Infektionen mit H5N1 -Virus einer anderen Klade wie in irgendeinem der Ansprüche 1 bis 7 definiert, umfassend oder bestehend aus:
a. das bzw. dem H5-Protein (1) von Anspruch 1 und
b. einen bzw. einem pharmazeutisch geeigneten Träger und/oder Hilfsstoff.

9. Impfstoff zur Verwendung nach Anspruch 8, wobei der Hilfsstoff ein auf Emulsigen basierendes Adjuvans ist.

10. Impfstoff zur Verwendung nach Anspruch 8 oder 9, wobei der Impfstoff ein Single-Shot-Impfstoff oder ein Einzeldosis-Impfstoff ist.

11. Impfstoff zur Verwendung nach irgendeinem der Ansprüche 8 bis 10 zur Reduzierung der Virenfreisetzung in einem Patienten, der mit H5N1 -Virus einer von Klade 1 verschiedenen Klade infiziert ist oder bei dem das Risiko einer viralen Infektion damit besteht.

12. Kombination von dem H5-Protein (1) nach Anspruch 1 und einem inaktivierten Newcastle-Krankheit-Virus zur Verwendung in einem Verfahren zur Behandlung von oder Vorbeugung vor Infektionen mit H5N1-Virus einer anderen Klade wie in irgendeinem der Ansprüche 1 bis 7 definiert.

13. Kombination zur Verwendung nach Anspruch 12, wobei das inaktivierte Newcastle-Krankheit-Virus ein inaktiviertes Newcastle-Krankheit-Virus ist, das erhalten wird durch Inaktivierung eines Newcastle-Krankheit-Virus, umfassend ein RNA-Polynukleotid mit mindestens 70% Sequenzidentität mit einer RNA-Kopie des in SEQ ID NO: 139 dargelegten Polynukleotids, das inaktiviert worden ist.

## Revendications

1. Protéine H5 (1) du virus H5N1 du clade 1 pour son utilisation dans une méthode de traitement ou de prévention d'infections avec le virus H5N1 d'un clade différent, dans laquelle ladite protéine H5 (1) comprend ou consiste en la séquence polypeptidique de SEQ ID NO : 5,
dans laquelle ledit virus H5N1 d'un clade différent est un virus H5N1 comprenant une protéine H5 (2) du virus de la grippe, dans laquelle ladite protéine H5 (2) consiste en ou comprend une séquence d'acides aminés qui présente au moins 98 % d'identité de séquence avec l'une quelconque des séquences telles que représentées par SEQ ID NO : 102 à 110, 116, 117, 119 à 126 et 128 à 137.

2. Protéine H5 (1) pour son utilisation selon la revendication 1, dans laquelle ledit virus H5N1 d'un clade différent est un virus H5N1 du clade 2.2.1 et/ou, dans laquelle ledit virus H5N1 d'un clade différent est un virus H5N1 d'origine nord-africaine.

3. Protéine H5 (1) pour son utilisation selon la revendication 1 ou 2,
dans laquelle ledit virus H5N1 d'un clade différent comprend un polynucléotide codant une protéine H5 (2) ayant :
(a) les acides aminés 87L, 113D, 126H, 145(-), 156R, 160F, 167T, et 181N, ou
(b) les acides aminés 87P, 113N, 126R, 145L, 160Y, 172T, 181H, 201E, 206I, 208K, 254T, 341G et 421K, ou
(c) les acides aminés 87L, 113N, 126R, 145L, 156G, 160Y, 172T, 181H, et 254V,
et dans laquelle la numérotation des positions d'acides aminés de la protéine H5 (2) se rapporte à la position d'acide aminé telle que donnée à titre d'exemple dans SEQ ID NO : 8.

4. Protéine H5 (1) pour son utilisation selon l'une quelconque des revendications 1 à 3,
dans laquelle ledit virus H5N1 d'un clade différent comprend une protéine H5 (2) ayant :
(a) les acides aminés 87L, 113D, 126H, 145(-), 156R, 160F, 167T, et 181N, ou
(b) les acides aminés 87P, 113N, 126R, 145L, 160Y, 172T, 181H, 201E, 206I, 208K, 254T, 341G et 421K,
ou
(c) les acides aminés 87L, 113N, 126R, 145L, 156G, 160Y, 172T, 181H, et 254V,
et dans laquelle la numérotation des positions d'acides aminés de la protéine H5 (2) se rapporte à la position d'acide aminé telle que donnée à titre d'exemple dans SEQ ID NO : 8.

5. Protéine H5 (1) pour son utilisation selon l'une quelconque des revendications 1 à 4, dans laquelle ledit virus H5N1 d'un clade différent comprend une protéine H5 (2) qui consiste en ou comprend une séquence d'acides aminés qui présente au moins 98 % d'identité de séquence avec la séquence telle que représentée par SEQ ID NO : 106, ou
la protéine H5 (1) pour son utilisation selon l'une quelconque des revendications 1 à 4, dans laquelle ledit virus H5N1 d'un clade différent comprend un polynucléotide codant une protéine H5 (2) qui consiste en ou comprend une séquence d'acides aminés qui présente au moins 98 % d'identité de séquence avec la séquence telle que représentée par SEQ ID NO : 106.

6. Protéine H5 (1) pour son utilisation selon l'une quelconque des revendications 1 à 5 dans une méthode de traitement ou de prévention d'infections
(A) avec le virus H5N1 du sous-clade A d'origine nord-africaine, à savoir une infection avec un virus H5N1 comprenant un polynucléotide codant une protéine H5 (2) ayant les acides aminés selon (a) de la revendication 3 ou 4 ou une infection avec un virus H5N1 comprenant un polynucléotide codant une protéine H5 (2) selon la revendication 3 ou 4 relative à l'une quelconque des séquences telles que représentées par SEQ ID NO : 102 à 110, 133 ou 134,
ou
(B) avec le virus H5N1 du sous-clade B d'origine nord-africaine, à savoir une infection avec un virus H5N1 comprenant un polynucléotide codant une protéine H5 (2) ayant les acides aminés selon (b) ou (c) de la revendication 3 ou 4 ou une infection avec un virus H5N1 comprenant un polynucléotide codant une protéine H5 (2) selon la revendication 3 ou 4 relative à l'une quelconque des séquences telles que représentées par SEQ ID NO : 116, 117, 119 à 126, 128 à 132 ou 135 à 137.

7. Protéine H5 (1) pour son utilisation selon l'une quelconque des revendications 1 à 6 dans une méthode de traitement ou de prévention d'infections
(A) avec le virus H5N1 du sous-clade A d'origine nord-africaine, à savoir une infection avec un virus H5N1 comprenant une protéine H5 (2) ayant les acides aminés selon (a) de la revendication 3 ou 4 ou comprenant une protéine H5 (2) selon la revendication 3 ou 4 relative à l'une quelconque des séquences telles que représentées par SEQ ID NO : 102 à 110, 133 ou 134,
ou
(B) avec le virus H5N1 du sous-clade B d'origine nord-africaine, à savoir une infection avec un virus H5N1 comprenant une protéine H5 (2) ayant les acides aminés selon (b) ou (c) de la revendication 3 ou 4 ou comprenant une protéine H5 (2) selon la revendication 3 ou 4 relative à l'une quelconque des séquences telles que représentées par SEQ ID NO : 116, 117, 119 à 126, 128 à 132 ou 135 à 137.

8. Vaccin pour son utilisation dans une méthode de traitement ou de prévention d'infections avec le virus H5N1 d'un clade différent tel que défini dans l'une quelconque des revendications 1 à 7 comprenant ou consistant en :
a. la protéine H5 (1) selon la revendication 1, et
b. un support et/ou un excipient pharmaceutique acceptable.

9. Vaccin pour son utilisation selon la revendication 8, dans lequel l'excipient est un adjuvant à base de Emulsigen.

10. Vaccin pour son utilisation selon la revendication 8 ou 9, dans lequel le vaccin est un vaccin en une seule injection ou un vaccin en une dose.

11. Vaccin pour son utilisation selon l'une quelconque des revendications 8 à 10 pour la réduction de l'excrétion virale chez un sujet infecté ou à risque d'une infection virale avec le virus H5N1 d'un clade autre que le clade 1.

12. Combinaison de
la protéine H5 (1) selon la revendication 1 et d'un virus de la maladie de Newcastle inactivé pour son utilisation dans une méthode de traitement ou de prévention d'infections avec le virus H5N1 d'un clade différent tel que défini dans l'une quelconque des revendications 1 à 7.

13. Combinaison pour son utilisation selon la revendication 12, dans laquelle le virus de la maladie de Newcastle inactivé est un virus de la maladie de Newcastle inactivé obtenu par inactivation d'un virus de la maladie de Newcastle comprenant un polynucléotide d'ARN présentant au moins 70 % d'identité de séquence avec une copie d'ARN du polynucléotide tel que représenté par SEQ ID NO : 139, qui a été inactivé.


### SEQUENCE LISTING

<110> Boehringer Ingelheim Vetmedica GmbH
<120> H5 PROTEINS OF H5N1 INFLUENZA VIRUS FOR USE AS A MEDICAMENT
<130> Case 1-2906
<160> 140
<170> PatentIn version 3.3
<210> 1
   <211> 552
   <212> PRT
   <213> Avian influenza virus
<400> 1
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Ile Asn Val Pro Glu Trp Ser Tyr Ile Val
   65                  70                  75                  80
   Glu Lys Ala Asn Pro Ala Asn Asp Leu Cys Tyr Pro Gly Asp Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Ser His Glu Ala Ser
           115                 120                 125
   Leu Gly Val Ser Ser Ala Cys Pro Tyr Gln Gly Lys Ser Ser Phe Phe
       130                 135                 140
   Arg Asn Val Val Trp Leu Ile Lys Lys Asn Asn Ala Tyr Pro Thr Ile
   145                 150                 155                 160
   Lys Arg Ser Tyr Asn Asn Thr Asn Gln Glu Asp Leu Leu Val Leu Trp
                   165                 170                 175
   Gly Ile His His Pro Asn Asp Ala Ala Glu Gln Thr Arg Leu Tyr Gln
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Asn Gly
       210                 215                 220
   Arg Met Glu Phe Phe Trp Thr Ile Leu Lys Pro Asn Asp Ala Ile Asn
   225                 230                 235                 240
   Phe Glu Ser Asn Gly Asn Phe Ile Ala Pro Glu Tyr Ala Tyr Lys Ile
                   245                 250                 255
   Val Lys Lys Gly Asp Ser Ala Ile Met Lys Ser Glu Leu Glu Tyr Gly
               260                 265                 270
   Asn Cys Asn Thr Lys Cys Gln Thr Pro Met Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Arg Glu Arg Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Arg Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Ile Cys Ile
   545 550
<210> 2
   <211> 551
   <212> PRT
   <213> Avian influenza virus
<400> 2
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Ile Asn Val Pro Glu Trp Ser Tyr Ile Val
   65 70 75 80
   Glu Lys Ala Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
               100                 105                 110
   Lys Ile Gln Ile Ile Pro Lys Ser Ser Trp Ser Asp His Glu Ala Ser
           115                 120                 125
   Ser Gly Val Ser Ser Ala Cys Pro Tyr Gln Gly Ser Ser Ser Phe Phe
       130                 135                 140
   Arg Asn Val Val Trp Leu Ile Lys Lys Asn Asp Ala Tyr Pro Thr Ile
   145                 150                 155                 160
   Lys Arg Ser Tyr Asn Asn Thr Asn Gln Glu Asp Leu Leu Val Leu Trp
                   165                 170                 175
   Gly Ile His His Pro Asn Asp Ala Ala Glu Gln Thr Arg Leu Tyr Gln
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Met Asp Phe Phe Trp Thr Ile Leu Lys Pro Asn Asp Ala Ile Asn
   225                 230                 235                 240
   Phe Glu Ser Asn Gly Asn Phe Ile Ala Pro Glu Tyr Ala Tyr Lys Ile
                   245                 250                 255
   Val Lys Lys Gly Asp Ser Ala Ile Met Lys Ser Glu Val Glu Tyr Gly
               260                 265                 270
   Asn Cys Asn Thr Lys Cys Gln Thr Pro Met Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Lys Leu Val Leu Ala Thr Gly Leu Arg Asn Ser
   305                 310                 315                 320
   Pro Gln Arg Glu Arg Arg Arg Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Lys Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Ser Leu Trp Met Cys Ser Asn Gly Ser
   530 535 540
   Leu Gln Cys Arg Ile Cys Ile
   545 550
<210> 3
   <211> 567
   <212> PRT
   <213> Avian influenza virus
<400> 3
   Met Glu Lys Thr Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Asp His Glu Ala Ser
       130                 135                 140
   Ser Gly Val Ser Ser Ala Cys Pro Tyr Gln Gly Ser Ser Ser Phe Phe
   145                 150                 155                 160
   Arg Asn Val Val Trp Leu Ile Lys Lys Asn Asp Ala Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Arg Leu Tyr Gln
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Ser Gly
   225                 230                 235                 240
   Arg Met Asp Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Ala Ile Met Lys Ser Glu Val Glu Tyr Gly
           275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Lys Leu Val Leu Ala Thr Gly Leu Arg Asn Ser
                   325                 330                 335
   Pro Gln Arg Glu Arg Arg Arg Lys Arg Gly Leu Phe Gly Ala Ile Ala
               340                 345                 350
   Gly Phe Ile Glu Gly Gly Trp Gln Gly Met Val Asp Gly Trp Tyr Gly
           355                 360                 365
   Tyr His His Ser Asn Glu Gln Gly Ser Gly Tyr Ala Ala Asp Lys Glu
       370                 375                 380
   Ser Thr Gln Lys Ala Ile Asp Gly Val Thr Asn Lys Val Asn Ser Ile
   385                 390                 395                 400
   Ile Asp Lys Met Asn Thr Gln Phe Glu Ala Val Gly Arg Glu Phe Asn
                   405                 410                 415
   Asn Leu Glu Arg Arg Ile Glu Asn Leu Asn Lys Lys Met Glu Asp Gly
               420                 425                 430
   Phe Leu Asp Val Trp Thr Tyr Asn Ala Glu Leu Leu Val Leu Met Glu
           435                 440                 445
   Asn Glu Arg Thr Leu Asp Phe His Asp Ser Asn Val Lys Asn Leu Tyr
       450                 455                 460
   Asp Lys Val Arg Leu Gln Leu Arg Asp Asn Ala Lys Glu Leu Gly Asn
   465                 470                 475                 480
   Gly Cys Phe Glu Phe Tyr His Lys Cys Asp Asn Glu Cys Met Glu Ser
                   485                 490                 495
   Val Arg Asn Gly Thr Tyr Asp Tyr Pro Gln Tyr Ser Glu Glu Ala Arg
               500                 505                 510
   Leu Lys Arg Glu Glu Ile Ser Gly Val Lys Leu Glu Ser Ile Gly Thr
           515                 520                 525
   Tyr Gln Ile Leu Ser Ile Tyr Ser Thr Val Ala Ser Ser Leu Ala Leu
       530                 535                 540
   Ala Ile Met Val Ala Gly Leu Ser Leu Trp Met Cys Ser Asn Gly Ser
   545                 550                 555                 560
   Leu Gln Cys Arg Ile Cys Ile
                   565
<210> 4
   <211> 568
   <212> PRT
   <213> Avian influenza virus
<400> 4
   Met Glu Lys Ile Val Leu Leu Phe Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Ala Asn Asp Leu Cys Tyr Pro Gly Asp Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Asn Ser Trp Ser Ser His Glu Ala Ser
       130                 135                 140
   Leu Gly Val Ser Ser Ala Cys Pro Tyr Gln Gly Lys Ser Ser Phe Phe
   145                 150                 155                 160
   Arg Asn Val Val Trp Leu Ile Lys Lys Asn Asn Ala Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Arg Leu Tyr Gln
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Asn Gly
   225                 230                 235                 240
   Arg Met Glu Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Ala Ile Met Lys Ser Glu Leu Glu Tyr Gly
           275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Arg Glu Arg Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Arg Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Lys Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Val Ala Gly Leu Ser Leu Trp Met Cys Ser Asn Gly
   545                 550                 555                 560
   Ser Leu Gln Cys Arg Ile Cys Ile
                   565
<210> 5
   <211> 568
   <212> PRT
   <213> Avian influenza virus
<400> 5
   Met Glu Lys Thr Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Asn Ser Trp Ser Asp His Glu Ala Ser
       130                 135                 140
   Ser Gly Val Ser Ser Ala Cys Pro Tyr Gln Gly Ser Ser Ser Phe Phe
   145                 150                 155                 160
   Arg Asn Val Val Trp Leu Ile Lys Lys Asn Asn Ala Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Arg Leu Tyr Gln
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Asn Gly
   225                 230                 235                 240
   Arg Met Asp Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Ala Ile Met Lys Ser Glu Val Glu Tyr Gly
           275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Lys Leu Val Leu Ala Thr Gly Leu Arg Asn Ser
   325 330 335
   Pro Gln Arg Glu Arg Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Arg Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Met Val Ala Gly Leu Ser Leu Trp Met Cys Ser Asn Gly
   545 550 555 560
   Ser Leu Gln Cys Arg Ile Cys Ile
   565
<210> 6
   <211> 263
   <212> PRT
   <213> Avian influenza virus
<400> 6
   His Ala Asn Asn Trp Thr Glu Gln Val Asp Thr Ile Met Glu Lys Asn
   1               5                   10                  15
   Val Thr Val Thr His Ala Gln Asp Ile Leu Glu Lys Thr His Asn Gly
               20                  25                  30
   Lys Leu Cys Asp Leu Asp Gly Val Lys Pro Leu Ile Leu Arg Asp Cys
           35                  40                  45
   Ser Val Ala Gly Trp Leu Leu Gly Asn Pro Met Cys Asp Glu Phe Ile
       50                  55                  60
   Asn Val Pro Glu Trp Ser Tyr Ile Val Glu Lys Ala Asn Pro Ala Asn
   65                  70                  75                  80
   Asp Leu Cys Tyr Pro Gly Asp Phe Asn Asp Tyr Glu Glu Leu Lys His
                   85                  90                  95
   Leu Leu Ser Arg Ile Asn His Phe Glu Lys Ile Gln Ile Ile Pro Lys
               100                 105                 110
   Asn Ser Trp Ser Ser His Glu Ala Ser Leu Gly Val Ser Ser Ala Cys
           115                 120                 125
   Pro Tyr Gln Gly Lys Ser Ser Phe Phe Arg Asn Val Val Trp Leu Ile
       130                 135                 140
   Lys Lys Asn Asn Ala Tyr Pro Thr Ile Lys Arg Ser Tyr Asn Asn Thr
   145                 150                 155                 160
   Asn Gln Glu Asp Leu Leu Val Leu Trp Gly Ile His His Pro Asn Asp
                   165                 170                 175
   Ala Ala Glu Gln Thr Arg Leu Tyr Gln Asn Pro Thr Thr Tyr Ile Ser
               180                 185                 190
   Val Gly Thr Ser Thr Leu Asn Gln Arg Leu Val Pro Lys Ile Ala Thr
           195                 200                 205
   Arg Ser Lys Val Asn Gly Gln Asn Gly Arg Met Glu Phe Phe Trp Thr
   210 215 220
   Ile Leu Lys Pro Asn Asp Ala Ile Asn Phe Glu Ser Asn Gly Asn Phe
   225 230 235 240
   Ile Ala Pro Glu Tyr Ala Tyr Lys Ile Val Lys Lys Gly Asp Ser Ala
   245 250 255
   Ile Met Lys Ser Glu Leu Glu
   260
<210> 7
   <211> 290
   <212> PRT
   <213> Avian influenza virus
<400> 7
   Gly Ser Ala Thr Met Glu Lys Thr Val Leu Leu Leu Ala Ile Val Ser
   1               5                   10                  15
   Leu Val Lys Ser Asp Gln Ile Cys Ile Gly Tyr His Ala Asn Asn Ser
               20                  25                  30
   Thr Glu Gln Val Asp Thr Ile Met Glu Lys Asn Val Thr Val Thr His
           35                  40                  45
   Ala Gln Asp Ile Leu Glu Lys Thr His Asn Gly Lys Leu Cys Asp Leu
       50                  55                  60
   Asp Gly Val Lys Pro Leu Ile Leu Arg Asp Cys Ser Val Ala Gly Trp
   65                  70                  75                  80
   Leu Leu Gly Asn Pro Met Cys Asp Glu Phe Ile Asn Val Pro Glu Trp
                   85                  90                  95
   Ser Tyr Ile Val Glu Lys Ala Asn Pro Ala Asn Asp Leu Cys Tyr Pro
               100                 105                 110
   Gly Asn Phe Asn Asp Tyr Glu Glu Leu Lys His Leu Leu Ser Arg Ile
           115                 120                 125
   Asn His Phe Glu Lys Ile Gln Ile Ile Pro Lys Ser Ser Trp Ser Asp
       130                 135                 140
   His Glu Ala Ser Ser Gly Val Ser Ser Ala Cys Pro Tyr Gln Gly Ser
   145                 150                 155                 160
   Ser Ser Phe Phe Arg Asn Val Val Trp Leu Ile Lys Lys Asn Asp Ala
   165 170 175
   Tyr Pro Thr Ile Lys Arg Ser Tyr Asn Asn Thr Asn Gln Glu Asp Leu
   180 185 190
   Leu Val Leu Trp Gly Ile His His Pro Asn Asp Ala Ala Glu Gln Thr
   195 200 205
   Arg Leu Tyr Gln Asn Pro Thr Thr Tyr Ile Ser Val Gly Thr Ser Thr
   210 215 220
   Leu Asn Gln Arg Leu Val Pro Lys Ile Ala Thr Arg Ser Lys Val Asn
   225 230 235 240
   Gly Gln Ser Gly Arg Met Asp Phe Phe Trp Thr Ile Leu Lys Pro Asn
   245 250 255
   Asp Ala Ile Asn Phe Glu Ser Asn Gly Asn Phe Ile Ala Pro Glu Tyr
   260 265 270
   Ala Tyr Lys Ile Val Lys Lys Gly Asp Ser Ala Ile Met Lys Ser Glu
   275 280 285
   Val Glu
   290
<210> 8
   <211> 562
   <212> PRT
   <213> Influenza A virus
<220>
   <221> MISC_FEATURE
   <223> subtype H5N1
<400> 8
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65                  70                  75                  80
   Pro Met Cys Asp Glu Phe Leu Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Gln Ile Ile Pro Lys Ser Ser Trp Pro Asp His Glu Ala Ser
       130                 135                 140
   Ser Gly Val Ser Ser Ala Cys Pro Tyr Gln Gly Gly Pro Ser Phe Tyr
   145                 150                 155                 160
   Arg Asn Val Val Trp Leu Ile Lys Lys Asp Asn Ala Tyr Pro Thr Ile
                   165                 170                 175
   Lys Lys Ser Tyr His Asn Thr Asn Gln Glu Asp Leu Leu Val Leu Trp
               180                 185                 190
   Gly Ile His His Pro Asn Asp Glu Ala Glu Gln Thr Arg Leu Tyr Gln
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Ser Gly
   225                 230                 235                 240
   Arg Val Glu Phe Phe Trp Thr Ile Leu Lys Ser Asn Asp Ala Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
           275                 280                 285
   Asn Cys Asn Thr Lys Cys Gln Thr Pro Ile Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Gly Glu Arg Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Arg Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Arg Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Val Ala Gly Leu Phe Leu Trp Met Cys Ser Asn Gly
   545                 550                 555                 560
   Ser Leu
<210> 9
   <211> 567
   <212> PRT
   <213> Influenza A virus
<220>
   <221> MISC_FEATURE
   <223> subtype H5N1
<400> 9
   Met Glu Lys Ile Met Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asn Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Leu Asn Val Pro Glu Trp Ser Tyr Ile Val
                   85                  90                  95
   Glu Lys Ile Asn Pro Ala Asn Asp Leu Cys Tyr Pro Gly Lys Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Arg Asn Ser Trp Ser Asp His Glu Thr Ser
       130                 135                 140
   Gly Val Ser Ser Ala Cys Gln Tyr Gln Gly Arg Ser Ser Phe Phe Arg
   145                 150                 155                 160
   Asn Val Val Trp Leu Thr Lys Lys Asp Asn Ala Tyr Ser Thr Ile Lys
                   165                 170                 175
   Arg Ser Tyr Asn Asn Thr Asn Gln Glu Asp Leu Leu Val Leu Trp Gly
               180                 185                 190
   Ile His His Pro Asn Asp Ala Ala Glu Gln Thr Arg Leu Tyr Gln Asn
   195 200 205
   Pro Thr Thr Tyr Ile Ser Val Gly Thr Ser Thr Leu Asn Gln Arg Leu
   210 215 220
   Val Pro Lys Ile Ala Thr Arg Ser Lys Val Asn Gly Gln Ser Gly Arg
   225 230 235 240
   Met Glu Phe Phe Trp Thr Ile Leu Lys Ser Asn Asp Ala Ile Asn Phe
   245 250 255
   Glu Ser Asn Gly Asn Phe Ile Ala Pro Glu Asn Ala Tyr Lys Ile Val
   260 265 270
   Lys Lys Gly Asp Ser Thr Ile Met Lys Ser Glu Leu Glu Tyr Gly Asn
   275 280 285
   Cys Asn Thr Lys Cys Gln Thr Pro Ile Gly Ala Ile Asn Ser Ser Met
   290 295 300
   Pro Phe His Asn Ile His Pro Leu Thr Ile Gly Glu Cys Pro Lys Tyr
   305 310 315 320
   Val Lys Ser Asn Arg Leu Val Leu Ala Thr Gly Leu Arg Asn Ser Pro
   325 330 335
   Gln Glu Glu Arg Arg Arg Lys Lys Arg Gly Leu Phe Gly Ala Ile Ala
   340 345 350
   Gly Phe Ile Glu Gly Gly Trp Gln Gly Met Val Asp Gly Trp Tyr Gly
   355 360 365
   Tyr His His Ser Asn Glu Gln Gly Ser Gly Tyr Ala Ala Asp Lys Glu
   370 375 380
   Ser Thr Gln Lys Ala Ile Asp Gly Val Thr Asn Lys Val Asn Ser Ile
   385 390 395 400
   Ile Asp Lys Met Asn Thr Gln Phe Glu Ala Val Gly Arg Glu Phe Asn
   405 410 415
   Asn Leu Glu Arg Arg Ile Glu Asn Leu Asn Lys Lys Met Glu Asp Gly
   420 425 430
   Phe Leu Asp Val Trp Thr Tyr Asn Ala Glu Leu Leu Val Leu Met Glu
   435 440 445
   Asn Glu Arg Thr Leu Asp Phe His Asp Ser Asn Val Lys Asn Leu Tyr
       450                 455                 460
   Asp Lys Val Arg Leu Gln Leu Arg Asp Asn Ala Lys Glu Leu Gly Asn
   465                 470                 475                 480
   Gly Cys Phe Glu Phe Tyr His Arg Cys Asp Asn Glu Cys Met Glu Ser
                   485                 490                 495
   Val Arg Asn Gly Thr Tyr Asp Tyr Pro Gln Tyr Ser Glu Glu Ala Arg
               500                 505                 510
   Leu Lys Arg Glu Glu Ile Ser Gly Val Lys Leu Glu Ser Ile Gly Thr
           515                 520                 525
   Tyr Gln Ile Leu Ser Ile Tyr Ser Thr Val Ala Ser Ser Leu Ala Leu
       530                 535                 540
   Ala Ile Met Val Ala Gly Leu Phe Leu Trp Met Cys Ser Asn Gly Ser
   545                 550                 555                 560
   Leu Gln Cys Arg Ile Cys Ile
                   565
<210> 10
   <211> 567
   <212> PRT
   <213> Influenza A virus
<220>
   <221> MISC_FEATURE
   <223> subtype H5N1
<400> 10
   Met Glu Lys Ile Val Leu Leu Leu Ala Ile Val Ser Ile Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asn Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Leu Asn Val Pro Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Gly Ser Trp Ser Asp His Glu Ala Ser
       130                 135                 140
   Gly Val Ser Ser Ala Cys Pro Tyr Gln Gly Arg Ser Ser Phe Phe Arg
   145                 150                 155                 160
   Asn Val Val Trp Leu Thr Lys Lys Asn Asn Ala Tyr Pro Thr Ile Lys
                   165                 170                 175
   Lys Ser Tyr Asn Asn Thr Asn Gln Glu Asp Leu Leu Val Leu Trp Gly
               180                 185                 190
   Ile His His Pro Asn Asp Ala Ala Glu Gln Thr Arg Leu Tyr Gln Asn
           195                 200                 205
   Pro Thr Thr Tyr Ile Ser Val Gly Thr Ser Thr Leu Asn Gln Arg Leu
       210                 215                 220
   Val Pro Lys Ile Ala Thr Arg Ser Lys Val Asn Gly Gln Ser Gly Arg
   225                 230                 235                 240
   Met Glu Phe Phe Trp Thr Ile Leu Lys Ser Asn Asp Ala Ile Asn Phe
                   245                 250                 255
   Glu Ser Asn Gly Asn Phe Ile Ala Pro Glu Asn Ala Tyr Lys Ile Val
               260                 265                 270
   Lys Lys Gly Asp Ser Thr Ile Met Lys Ser Glu Leu Glu Tyr Gly Asn
           275                 280                 285
   Cys Asn Thr Lys Cys Gln Thr Pro Ile Gly Ala Ile Asn Ser Ser Met
       290                 295                 300
   Pro Phe His Asn Ile His Pro Leu Thr Ile Gly Glu Cys Pro Lys Tyr
   305                 310                 315                 320
   Val Lys Ser Asn Arg Leu Val Leu Ala Thr Gly Leu Arg Asn Ser Pro
                   325                 330                 335
   Gln Gly Glu Arg Arg Arg Lys Lys Arg Gly Leu Phe Gly Ala Ile Ala
               340                 345                 350
   Gly Phe Ile Glu Gly Gly Trp Gln Gly Met Val Asp Gly Trp Tyr Gly
           355                 360                 365
   Tyr His His Ser Asn Glu Gln Gly Ser Gly Tyr Ala Ala Asp Lys Glu
       370                 375                 380
   Ser Thr Gln Lys Ala Ile Asp Gly Val Thr Asn Lys Val Asn Ser Ile
   385                 390                 395                 400
   Ile Asp Lys Met Asn Thr Gln Phe Glu Ala Val Gly Arg Glu Phe Asn
                   405                 410                 415
   Asn Leu Glu Arg Arg Ile Glu Asn Leu Asn Lys Lys Met Glu Asp Gly
               420                 425                 430
   Phe Leu Asp Val Trp Thr Tyr Asn Ala Glu Leu Leu Val Leu Met Glu
           435                 440                 445
   Asn Glu Arg Thr Leu Asp Phe His Asp Ser Asn Val Lys Asn Leu Tyr
       450                 455                 460
   Asp Arg Val Arg Leu Gln Leu Arg Asp Asn Ala Lys Glu Leu Gly Asn
   465                 470                 475                 480
   Gly Cys Phe Glu Phe Tyr His Arg Cys Asp Asn Glu Cys Met Glu Ser
                   485                 490                 495
   Val Arg Asn Gly Thr Tyr Asp Tyr Pro Gln Tyr Ser Glu Glu Ala Arg
               500                 505                 510
   Leu Lys Arg Glu Glu Ile Ser Gly Val Lys Leu Glu Ser Ile Gly Thr
           515                 520                 525
   Tyr Gln Ile Leu Ser Ile Tyr Ser Thr Val Ala Ser Ser Leu Ala Leu
       530                 535                 540
   Ala Ile Met Val Ala Gly Leu Phe Leu Trp Met Cys Ser Asn Gly Ser
   545                 550                 555                 560
   Leu Gln Cys Arg Ile Cys Ile
                   565
<210> 11
   <211> 560
   <212> PRT
   <213> Influenza A virus
<220>
   <221> MISC_FEATURE
   <223> subtype H5N1
<220>
   <221> misc_feature
   <222> (560)..(560)
   <223> Xaa can be any naturally occurring amino acid
<400> 11
   Met Glu Lys Ile Val Leu Leu Leu Ala Ile Val Ser Leu Val Lys Gly
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asn Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Leu Asn Val Pro Glu Trp Ser Tyr Ile Val
                   85                  90                  95
   Glu Lys Ile Tyr Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Asp His Glu Ala Ser
       130                 135                 140
   Gly Val Ser Ser Ala Cys Pro Tyr Gln Gly Arg Ser Ser Phe Phe Arg
   145                 150                 155                 160
   Asn Val Val Trp Leu Thr Lys Lys Asp Asn Ala Tyr Pro Thr Ile Lys
                   165                 170                 175
   Lys Ser Tyr Asn Asn Thr Asn Gln Glu Asp Leu Leu Ile Leu Trp Gly
               180                 185                 190
   Ile His His Pro Asn Asp Ala Ala Glu Gln Thr Arg Leu Tyr Gln Asn
           195                 200                 205
   Pro Thr Thr Tyr Ile Ser Val Gly Thr Ser Thr Leu Asn Gln Arg Leu
       210                 215                 220
   Val Pro Lys Ile Ala Thr Arg Ser Lys Val Asn Gly Gln Ser Gly Arg
   225                 230                 235                 240
   Met Glu Phe Phe Trp Thr Ile Leu Lys Ser Asn Asp Ala Ile Asn Phe
                   245                 250                 255
   Glu Ser Asn Gly Asn Phe Ile Ala Pro Glu Asn Ala Tyr Lys Ile Val
               260                 265                 270
   Lys Lys Gly Asp Ser Thr Ile Met Lys Ser Glu Leu Glu Tyr Gly Asn
           275                 280                 285
   Cys Asn Thr Lys Cys Gln Thr Pro Ile Gly Ala Ile Asn Ser Ser Met
       290                 295                 300
   Pro Phe His Asn Ile His Pro Leu Thr Ile Gly Glu Cys Pro Lys Tyr
   305                 310                 315                 320
   Val Lys Ser Asn Arg Leu Val Leu Ala Thr Gly Leu Arg Asn Ser Pro
                   325                 330                 335
   Gln Gly Glu Arg Arg Arg Lys Lys Arg Gly Leu Phe Gly Ala Ile Ala
               340                 345                 350
   Gly Phe Ile Glu Gly Gly Trp Gln Gly Met Val Asp Gly Trp Tyr Gly
           355                 360                 365
   Tyr His His Ser Asn Glu Gln Gly Ser Gly Tyr Ala Ala Asp Lys Glu
       370                 375                 380
   Ser Thr Gln Lys Ala Ile Asp Gly Val Thr Asn Lys Val Asn Ser Ile
   385                 390                 395                 400
   Ile Asp Lys Met Asn Thr Gln Phe Glu Ala Val Gly Arg Glu Phe Asn
                   405                 410                 415
   Asn Leu Glu Arg Arg Ile Glu Asn Leu Asn Lys Lys Met Glu Asp Gly
               420                 425                 430
   Phe Leu Asp Val Trp Thr Tyr Asn Ala Glu Leu Leu Val Leu Met Glu
           435                 440                 445
   Asn Glu Arg Thr Leu Asp Phe His Asp Ser Asn Val Lys Asn Leu Tyr
   450 455 460
   Asp Lys Val Arg Leu Gln Leu Arg Asp Asn Ala Lys Glu Leu Gly Asn
   465 470 475 480
   Gly Cys Phe Glu Phe Tyr His Arg Cys Asp Asn Glu Cys Met Glu Ser
   485 490 495
   Val Arg Asn Gly Thr Tyr Asp Tyr Pro Gln Tyr Ser Glu Glu Ala Arg
   500 505 510
   Leu Lys Arg Glu Glu Ile Ser Gly Val Lys Leu Glu Ser Ile Gly Thr
   515 520 525
   Tyr Gln Ile Leu Ser Ile Tyr Ser Thr Val Ala Ser Ser Leu Ala Leu
   530 535 540
   Ala Ile Met Val Ala Gly Leu Phe Leu Trp Met Cys Ser Asn Gly Xaa
   545 550 555 560
<210> 12
   <211> 544
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (544)..(544)
   <223> Xaa can be any naturally occurring amino acid
<400> 12
   Xaa Leu Leu Ala Ile Val Ser Ile Val Lys Ser Asp Gln Ile Cys Ile
   1 5 10 15
   Gly Tyr His Ala Asn Asn Ser Thr Glu Gln Val Asp Thr Ile Met Glu
   20 25 30
   Lys Asn Val Thr Val Thr His Ala Gln Asp Ile Leu Glu Lys Thr His
   35 40 45
   Asn Gly Lys Leu Cys Asn Leu Asp Gly Val Lys Pro Leu Ile Leu Arg
       50                  55                  60
   Asp Cys Ser Val Ala Gly Trp Leu Leu Gly Asn Pro Met Cys Asp Glu
   65                  70                  75                  80
   Phe Leu Asp Val Pro Glu Trp Ser Tyr Ile Val Glu Lys Ile Asn Pro
                   85                  90                  95
   Ala Asn Asp Leu Cys Tyr Pro Gly Asn Phe Asn Asp Tyr Glu Glu Leu
               100                 105                 110
   Lys His Leu Leu Ser Arg Ile Asn His Phe Glu Lys Ile Gln Ile Ile
           115                 120                 125
   Pro Lys Asn Ser Trp Ser Asp His Glu Thr Ser Gly Val Ser Ser Ala
       130                 135                 140
   Cys Pro Tyr Gln Gly Arg Ser Ser Phe Phe Arg Asn Val Val Trp Leu
   145                 150                 155                 160
   Thr Lys Lys Asn Thr Ala Tyr Pro Thr Ile Lys Lys Ser Tyr Asn Asn
                   165                 170                 175
   Thr Asn Gln Glu Asp Leu Leu Val Leu Trp Gly Ile His His Pro Asn
               180                 185                 190
   Asp Ala Ala Glu Gln Thr Arg Leu Tyr Gln Asn Pro Thr Thr Tyr Ile
           195                 200                 205
   Ser Val Gly Thr Ser Thr Leu Asn Gln Arg Leu Val Pro Lys Ile Ala
       210                 215                 220
   Thr Arg Ser Lys Val Asn Gly Gln Ser Gly Arg Met Glu Phe Phe Trp
   225                 230                 235                 240
   Thr Ile Leu Lys Ser Asn Asp Ala Ile Asn Phe Glu Ser Asn Gly Asn
                   245                 250                 255
   Phe Ile Ala Pro Glu Asn Ala Tyr Lys Ile Val Lys Lys Gly Asp Ser
               260                 265                 270
   Thr Ile Met Lys Ser Glu Leu Glu Tyr Gly Asn Cys Asn Thr Lys Cys
           275                 280                 285
   Gln Thr Pro Ile Gly Ala Ile Asn Ser Ser Met Pro Phe His Asn Ile
       290                 295                 300
   His Pro Leu Thr Ile Gly Glu Cys Pro Lys Tyr Val Lys Ser Asn Arg
   305 310 315 320
   Leu Val Leu Ala Thr Gly Leu Arg Asn Ser Pro His Gly Glu Arg Arg
   325 330 335
   Arg Lys Lys Arg Gly Leu Phe Gly Ala Ile Ala Gly Phe Ile Glu Gly
   340 345 350
   Gly Trp Gln Gly Met Val Asp Gly Trp Tyr Gly Tyr His His Ser Asn
   355 360 365
   Glu Gln Gly Ser Gly Tyr Ala Ala Asp Lys Glu Ser Thr Gln Lys Ala
   370 375 380
   Ile Asp Gly Val Thr Asn Lys Val Asn Ser Ile Ile Asp Lys Met Asn
   385 390 395 400
   Thr Gln Phe Glu Ala Val Gly Arg Glu Phe Asn Asn Leu Glu Arg Arg
   405 410 415
   Ile Glu Asn Leu Asn Lys Lys Met Glu Asp Gly Phe Leu Asp Val Trp
   420 425 430
   Thr Tyr Asn Ala Glu Leu Leu Val Leu Met Glu Asn Glu Arg Thr Leu
   435 440 445
   Asp Phe His Asp Ser Asn Val Lys Asn Leu Tyr Asp Lys Val Arg Leu
   450 455 460
   Gln Leu Arg Asp Asn Ala Lys Glu Leu Gly Asn Gly Cys Phe Glu Phe
   465 470 475 480
   Tyr His Arg Cys Asp Asn Glu Cys Met Glu Ser Val Arg Asn Gly Thr
   485 490 495
   Tyr Asp Tyr Pro Gln Tyr Ser Glu Glu Ala Arg Leu Lys Arg Glu Glu
   500 505 510
   Ile Ser Gly Val Lys Leu Glu Ser Ile Gly Thr Tyr Gln Ile Leu Ser
   515 520 525
   Ile Tyr Ser Thr Val Ala Ser Ser Leu Ala Leu Ala Ile Met Val Xaa
   530 535 540
<210> 13
   <211> 534
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<400> 13
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asn Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Glu Phe Leu Asn Val Glu Trp Ser
   65                  70                  75                  80
   Tyr Ile Val Glu Lys Ile Asn Pro Thr Asn Asp Leu Cys Tyr Pro Gly
                   85                  90                  95
   Asn Phe Asn Asp Tyr Glu Glu Leu Lys His Leu Leu Ser Arg Ile Asn
               100                 105                 110
   His Phe Glu Lys Ile Gln Ile Ile Pro Lys Asn Tyr Trp Ser Asp His
           115                 120                 125
   Glu Thr Ser Gly Val Ser Ser Ala Cys Pro Tyr Gln Gly Arg Pro Ser
       130                 135                 140
   Phe Phe Arg Asn Val Val Trp Leu Thr Lys Lys Asn Asn Ala Tyr Pro
   145                 150                 155                 160
   Thr Ile Lys Lys Ser Tyr Asn Asn Thr Asn Gln Glu Asp Leu Leu Val
                   165                 170                 175
   Leu Trp Gly Ile His His Pro Asn Asp Ala Ala Glu Gln Thr Arg Leu
               180                 185                 190
   Tyr Gln Asn Pro Thr Thr Tyr Ile Ser Val Gly Thr Ser Thr Leu Asn
           195                 200                 205
   Gln Arg Leu Val Pro Lys Ile Ala Thr Arg Ser Lys Val Asn Gly Gln
       210                 215                 220
   Ser Gly Arg Met Glu Phe Phe Trp Thr Ile Leu Lys Ser Asn Asp Ala
   225                 230                 235                 240
   Ile Asn Phe Glu Ser Asn Gly Asn Phe Ile Ala Pro Glu Asn Ala Tyr
                   245                 250                 255
   Lys Ile Val Lys Lys Gly Asp Ser Thr Ile Met Lys Ser Glu Leu Glu
               260                 265                 270
   Tyr Gly Asn Cys Asn Thr Lys Cys Gln Thr Pro Ile Gly Ala Ile Asn
           275                 280                 285
   Ser Ser Met Pro Phe His Asn Ile His Pro Leu Thr Ile Gly Glu Cys
       290                 295                 300
   Pro Lys Tyr Val Lys Ser Asn Arg Leu Val Leu Ala Thr Gly Leu Arg
   305                 310                 315                 320
   Asn Ser Pro Gln Gly Glu Arg Arg Arg Lys Lys Arg Gly Leu Phe Gly
                   325                 330                 335
   Ala Ile Ala Gly Phe Ile Glu Gly Gly Trp Gln Gly Met Val Asp Gly
               340                 345                 350
   Trp Tyr Gly Tyr His His Ser Asn Glu Gln Gly Ser Gly Tyr Ala Ala
           355                 360                 365
   Asp Lys Glu Ser Thr Gln Lys Ala Ile Asp Gly Val Thr Asn Lys Val
       370                 375                 380
   Asn Ser Ile Ile Asp Lys Met Asn Thr Gln Phe Glu Ala Val Gly Arg
   385                 390                 395                 400
   Glu Phe Asn Asn Leu Glu Arg Arg Ile Glu Asn Leu Asn Lys Lys Met
                   405                 410                 415
   Glu Asp Gly Phe Leu Asp Val Trp Thr Tyr Asn Ala Glu Leu Leu Val
               420                 425                 430
   Leu Met Glu Asn Glu Arg Thr Leu Asp Phe His Asp Ser Asn Val Lys
           435                 440                 445
   Asn Leu Tyr Asp Lys Val Arg Leu Gln Leu Arg Asp Asn Ala Lys Glu
       450                 455                 460
   Leu Gly Asn Gly Cys Phe Glu Phe Tyr His Arg Cys Asp Asn Glu Cys
   465                 470                 475                 480
   Met Glu Ser Val Arg Asn Gly Thr Tyr Asp Tyr Pro Gln Tyr Ser Glu
                   485                 490                 495
   Glu Ala Arg Leu Lys Arg Glu Glu Ile Ser Gly Val Lys Leu Glu Ser
               500                 505                 510
   Ile Gly Thr Tyr Gln Ile Leu Ser Ile Tyr Ser Thr Val Ala Ser Ser
           515                 520                 525
   Leu Ala Leu Ala Ile Met
       530
<210> 14
   <211> 548
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (548)..(548)
   <223> Xaa can be any naturally occurring amino acid
<400> 14
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asn Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Lys Phe Leu Asn Val Pro Glu Trp
   65                  70                  75                  80
   Ser Tyr Ile Val Glu Lys Ile Asn Pro Thr Asn Asp Leu Cys Tyr Pro
                   85                  90                  95
   Gly Asn Phe Asn Asp Tyr Glu Glu Leu Lys His Leu Leu Ser Arg Ile
               100                 105                 110
   Asn His Phe Glu Lys Ile Gln Ile Ile Pro Lys Asn Ser Trp Ser Asp
           115                 120                 125
   His Glu Ala Ser Gly Val Ser Ser Ala Cys Pro Tyr Gln Gly Arg Ser
       130                 135                 140
   Ser Phe Phe Arg Asn Val Val Trp Leu Thr Lys Lys Asn Asn Ala Tyr
   145                 150                 155                 160
   Pro Thr Ile Lys Lys Ser Tyr Asn Asn Thr Asn Gln Glu Asp Leu Leu
                   165                 170                 175
   Val Leu Trp Gly Ile His His Pro Asn Asp Ala Ala Glu Gln Thr Arg
               180                 185                 190
   Leu Tyr Gln Asn Pro Thr Thr Tyr Ile Ser Val Gly Thr Ser Thr Leu
           195                 200                 205
   Asn Gln Arg Leu Val Pro Lys Ile Ala Thr Arg Ser Lys Val Asn Gly
       210                 215                 220
   Gln Ser Gly Arg Met Glu Phe Phe Trp Thr Ile Leu Lys Ser Asn Asp
   225                 230                 235                 240
   Ala Ile Asn Phe Glu Ser Asn Gly Asn Phe Ile Ala Pro Glu Asn Ala
                   245                 250                 255
   Tyr Lys Ile Val Lys Lys Gly Asp Ser Thr Ile Met Lys Ser Glu Leu
               260                 265                 270
   Glu Tyr Gly Asp Cys Asn Thr Lys Cys Gln Thr Pro Ile Gly Ala Ile
           275                 280                 285
   Asn Ser Ser Met Pro Phe His Asn Ile His Pro Leu Thr Ile Gly Glu
       290                 295                 300
   Cys Pro Lys Tyr Val Lys Ser Asn Arg Leu Val Leu Ala Thr Gly Leu
   305                 310                 315                 320
   Arg Asn Ser Pro Gln Gly Glu Arg Arg Arg Lys Lys Arg Gly Leu Phe
   325 330 335
   Gly Ala Ile Ala Gly Phe Ile Glu Gly Gly Trp Gln Gly Met Val Asp
   340 345 350
   Gly Trp Tyr Gly Tyr His His Ser Asn Glu Gln Gly Ser Gly Tyr Ala
   355 360 365
   Ala Asp Lys Glu Ser Thr Gln Lys Ala Ile Asp Gly Val Thr Asn Lys
   370 375 380
   Val Asn Ser Ile Ile Asp Lys Met Asn Thr Gln Phe Glu Ala Val Gly
   385 390 395 400
   Arg Glu Phe Asn Asn Leu Glu Arg Arg Ile Glu Asn Leu Asn Lys Lys
   405 410 415
   Met Glu Asp Gly Phe Leu Asp Val Trp Thr Tyr Asn Ala Glu Leu Leu
   420 425 430
   Val Leu Met Glu Asn Glu Arg Thr Leu Asp Phe His Asp Ser Asn Val
   435 440 445
   Lys Asn Leu Tyr Asp Lys Val Arg Leu Gln Leu Arg Asp Asn Ala Lys
   450 455 460
   Glu Leu Gly Asn Gly Cys Phe Glu Phe Tyr His Arg Cys Asp Asn Glu
   465 470 475 480
   Cys Met Glu Ser Val Arg Asn Gly Thr Tyr Asp Tyr Pro Gln Tyr Ser
   485 490 495
   Glu Glu Ala Arg Leu Lys Arg Glu Glu Ile Ser Gly Val Lys Leu Glu
   500 505 510
   Ser Ile Gly Thr Tyr Gln Ile Leu Ser Ile Tyr Ser Thr Val Ala Ser
   515 520 525
   Ser Leu Ala Leu Ala Ile Met Val Ala Gly Leu Phe Leu Trp Met Cys
   530 535 540
   Ser Asn Gly Xaa
   545
<210> 15
   <211> 541
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (541)..(541)
   <223> Xaa can be any naturally occurring amino acid
<400> 15
   Xaa Ile Val Ser Ile Val Lys Ser Asp Gln Ile Cys Ile Gly Tyr His
   1               5                   10                  15
   Ala Asn Asn Ser Thr Glu Gln Val Asp Thr Ile Met Glu Lys Asn Val
               20                  25                  30
   Thr Val Thr His Ala Gln Asp Ile Leu Glu Lys Thr His Asn Gly Lys
           35                  40                  45
   Leu Cys Asn Leu Asp Gly Val Lys Pro Leu Ile Leu Arg Asp Cys Ser
       50                  55                  60
   Val Ala Gly Trp Leu Leu Gly Asn Pro Met Cys Asp Glu Phe Leu Asn
   65                  70                  75                  80
   Val Pro Glu Trp Ser Tyr Ile Val Glu Lys Ile Asn Pro Thr Asn Asp
                   85                  90                  95
   Leu Cys Tyr Pro Gly Asn Phe Asn Asp Tyr Glu Glu Leu Lys His Leu
               100                 105                 110
   Leu Ser Arg Ile Asn His Phe Glu Lys Ile Gln Ile Ile Pro Lys Asn
           115                 120                 125
   Ser Trp Ser Asp His Glu Ala Ser Gly Val Ser Ser Ala Cys Pro Tyr
       130                 135                 140
   Gln Gly Arg Ser Ser Phe Phe Arg Asn Val Val Trp Leu Thr Lys Lys
   145                 150                 155                 160
   Asn Asn Ala Tyr Pro Thr Ile Lys Lys Ser Tyr Asn Asn Thr Asn Gln
                   165                 170                 175
   Glu Asp Leu Leu Val Leu Trp Gly Ile His His Pro Asn Asp Ala Ala
   180 185 190
   Glu Gln Thr Arg Leu Tyr Gln Asn Pro Thr Thr Tyr Ile Ser Val Gly
   195 200 205
   Thr Ser Thr Leu Asn Gln Arg Leu Val Pro Lys Ile Ala Thr Arg Ser
   210 215 220
   Lys Val Asn Gly Gln Ser Gly Arg Met Glu Phe Phe Trp Thr Ile Leu
   225 230 235 240
   Lys Ser Asn Asp Ala Ile Asn Phe Glu Ser Asn Gly Asn Phe Ile Ala
   245 250 255
   Pro Glu Asn Ala Tyr Lys Ile Val Lys Lys Gly Asp Ser Thr Ile Met
   260 265 270
   Lys Ser Glu Leu Glu Tyr Gly Asp Cys Asn Thr Lys Cys Gln Thr Pro
   275 280 285
   Ile Gly Ala Ile Asn Ser Ser Met Pro Phe His Asn Ile His Pro Leu
   290 295 300
   Thr Ile Gly Glu Cys Pro Lys Tyr Val Lys Ser Asn Arg Leu Val Leu
   305 310 315 320
   Ala Thr Gly Leu Arg Asn Ser Pro Gln Gly Glu Arg Arg Arg Lys Lys
   325 330 335
   Arg Gly Leu Phe Gly Ala Ile Ala Gly Phe Ile Glu Gly Gly Trp Gln
   340 345 350
   Gly Met Val Asp Gly Trp Tyr Gly Tyr His His Ser Asn Glu Gln Gly
   355 360 365
   Ser Gly Tyr Ala Ala Asp Lys Glu Ser Thr Gln Lys Ala Ile Asp Gly
   370 375 380
   Val Thr Asn Lys Val Asn Ser Ile Ile Asp Lys Met Asn Thr Gln Phe
   385 390 395 400
   Glu Ala Val Gly Arg Glu Phe Asn Asn Leu Glu Arg Arg Ile Glu Asn
   405 410 415
   Leu Asn Lys Lys Met Glu Asp Gly Phe Leu Asp Val Trp Thr Tyr Asn
   420 425 430
   Ala Glu Leu Leu Val Leu Met Glu Asn Glu Arg Thr Leu Asp Phe His
   435 440 445
   Asp Ser Asn Val Lys Asn Leu Tyr Asp Lys Val Arg Leu Gln Leu Arg
   450 455 460
   Asp Asn Ala Lys Glu Leu Gly Asn Gly Cys Phe Glu Phe Tyr His Arg
   465 470 475 480
   Cys Asp Asn Glu Cys Met Glu Ser Val Arg Asn Gly Thr Tyr Asp Tyr
   485 490 495
   Pro Gln Tyr Ser Glu Glu Ala Arg Leu Lys Arg Glu Glu Ile Ser Gly
   500 505 510
   Val Lys Leu Glu Ser Ile Gly Thr Tyr Gln Ile Leu Ser Ile Tyr Ser
   515 520 525
   Thr Val Ala Ser Ser Leu Ala Leu Ala Ile Met Val Xaa
   530 535 540
<210> 16
   <211> 520
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (159)..(159)
   <223> Xaa can be any naturally occurring amino acid
<400> 16
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asn Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Glu Phe Leu Asn Val Pro Glu Trp
   65                  70                  75                  80
   Ser Tyr Ile Val Glu Lys Ile Asn Pro Ala Asn Asp Leu Cys Tyr Pro
                   85                  90                  95
   Gly Asn Phe Asn Asp Tyr Glu Glu Leu Lys His Leu Leu Ser Arg Ile
               100                 105                 110
   Asn His Phe Glu Lys Ile Gln Ile Ile Pro Lys Asn Ser Trp Ser Asp
           115                 120                 125
   His Glu Ala Ser Gly Val Ser Ser Ala Cys Pro Tyr Gln Gly Arg Ser
       130                 135                 140
   Ser Phe Phe Arg Asn Val Val Trp Leu Thr Lys Lys Asn Asn Xaa Tyr
   145                 150                 155                 160
   Pro Thr Ile Lys Lys Ser Tyr Asn Asn Thr Asn Gln Glu Asp Leu Leu
                   165                 170                 175
   Val Leu Trp Gly Ile His His Pro Asn Asp Ala Ala Glu Gln Thr Lys
               180                 185                 190
   Leu Tyr Gln Asn Pro Thr Thr Tyr Ile Ser Val Gly Thr Ser Thr Leu
           195                 200                 205
   Asn Gln Arg Leu Val Pro Lys Ile Ala Thr Arg Ser Lys Val Asn Gly
       210                 215                 220
   Gln Ser Gly Arg Met Glu Phe Phe Trp Thr Ile Leu Lys Ser Asn Asp
   225                 230                 235                 240
   Ala Ile Asn Phe Glu Ser Asn Gly Asn Phe Ile Ala Pro Glu Asn Ala
                   245                 250                 255
   Tyr Lys Ile Val Lys Lys Gly Asp Ser Thr Ile Met Lys Ser Glu Leu
               260                 265                 270
   Glu Tyr Gly Asn Cys Asn Thr Lys Cys Gln Thr Pro Ile Gly Ala Ile
           275                 280                 285
   Asn Ser Ser Met Pro Phe His Asn Ile His Pro Leu Thr Ile Gly Glu
       290                 295                 300
   Cys Pro Lys Tyr Val Lys Ser Asn Arg Leu Val Leu Ala Thr Gly Leu
   305                 310                 315                 320
   Arg Asn Ser Pro Gln Gly Glu Arg Arg Arg Lys Lys Arg Gly Leu Phe
                   325                 330                 335
   Gly Ala Ile Ala Gly Phe Ile Glu Gly Gly Trp Gln Gly Met Val Asp
               340                 345                 350
   Gly Trp Tyr Gly Tyr His His Ser Asn Glu Gln Gly Ser Gly Tyr Ala
           355                 360                 365
   Ala Asp Lys Glu Ser Thr Gln Lys Ala Ile Asp Gly Val Thr Asn Lys
       370                 375                 380
   Val Asn Ser Ile Ile Asp Lys Met Asn Thr Gln Phe Glu Ala Val Gly
   385                 390                 395                 400
   Arg Glu Phe Asn Asn Leu Glu Arg Arg Ile Glu Asn Leu Asn Lys Lys
                   405                 410                 415
   Met Glu Asp Gly Phe Leu Asp Val Trp Thr Tyr Asn Ala Glu Leu Leu
               420                 425                 430
   Val Leu Met Glu Asn Glu Arg Thr Leu Asp Phe His Asp Ser Asn Val
           435                 440                 445
   Lys Asn Leu Tyr Asp Lys Val Arg Leu Gln Leu Arg Asp Asn Ala Lys
       450                 455                 460
   Glu Leu Gly Asn Gly Cys Phe Glu Phe Tyr His Arg Cys Asp Asn Glu
   465                 470                 475                 480
   Cys Met Glu Ser Val Arg Asn Gly Thr Tyr Asp Tyr Pro Gln Tyr Ser
                   485                 490                 495
   Glu Glu Ala Arg Leu Lys Arg Glu Glu Ile Ser Gly Val Lys Leu Glu
               500                 505                 510
   Ser Ile Gly Thr Tyr Gln Ile Leu
           515                 520
<210> 17
   <211> 537
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (537)..(537)
   <223> Xaa can be any naturally occurring amino acid
<400> 17
   Xaa Ser Ile Val Lys Ser Asp Gln Ile Cys Ile Gly Tyr His Ala Asn
   1               5                   10                  15
   Asn Ser Thr Glu Gln Val Asp Thr Ile Met Glu Lys Asn Val Thr Val
               20                  25                  30
   Thr His Ala Gln Asp Ile Leu Glu Lys Thr His Asn Gly Lys Leu Cys
           35                  40                  45
   Asn Leu Asp Gly Val Lys Pro Leu Ile Leu Arg Asp Cys Ser Val Ala
       50                  55                  60
   Gly Trp Leu Leu Gly Asn Pro Met Cys Asp Glu Phe Leu Asn Val Pro
   65                  70                  75                  80
   Glu Trp Ser Tyr Ile Val Glu Lys Ile Asn Pro Ala Asn Asp Leu Cys
                   85                  90                  95
   Tyr Pro Gly Asn Phe Asn Asp Tyr Glu Glu Leu Lys His Leu Leu Ser
               100                 105                 110
   Arg Ile Asn His Phe Glu Lys Ile Gln Ile Ile Pro Lys Asn Ser Trp
           115                 120                 125
   Ser Asp His Glu Ala Ser Gly Val Ser Ser Ala Cys Pro Tyr Gln Gly
       130                 135                 140
   Arg Ser Ser Phe Phe Arg Asn Val Val Trp Leu Thr Lys Lys Asn Asn
   145                 150                 155                 160
   Ala Tyr Pro Thr Ile Lys Lys Ser Tyr Asn Asn Thr Asn Gln Glu Asp
                   165                 170                 175
   Leu Leu Val Leu Trp Gly Ile His His Pro Asn Asp Glu Ala Glu Gln
   180 185 190
   Thr Arg Leu Tyr Gln Asn Pro Thr Thr Tyr Ile Ser Val Gly Thr Ser
   195 200 205
   Thr Leu Asn Gln Arg Leu Val Pro Lys Ile Ala Thr Arg Ser Lys Val
   210 215 220
   Asn Gly Gln Ser Gly Arg Met Glu Phe Phe Trp Thr Ile Leu Lys Ser
   225 230 235 240
   Asn Asp Ala Ile Asn Phe Glu Ser Asn Gly Asn Phe Ile Ala Pro Glu
   245 250 255
   Asn Ala Tyr Lys Ile Val Lys Lys Gly Asp Ser Thr Ile Met Lys Ser
   260 265 270
   Glu Leu Glu Tyr Gly Asn Cys Asn Thr Lys Cys Gln Thr Pro Ile Gly
   275 280 285
   Ala Ile Asn Ser Ser Met Pro Phe His Asn Ile His Pro Leu Thr Ile
   290 295 300
   Gly Glu Cys Pro Lys Tyr Val Lys Ser Asn Arg Leu Val Leu Ala Thr
   305 310 315 320
   Gly Leu Arg Asn Ser Pro Gln Gly Glu Arg Arg Arg Lys Lys Arg Gly
   325 330 335
   Leu Phe Gly Ala Ile Ala Gly Phe Ile Glu Gly Gly Trp Gln Gly Met
   340 345 350
   Val Asp Gly Trp Tyr Gly Tyr His His Ser Asn Glu Gln Gly Ser Gly
   355 360 365
   Tyr Ala Ala Asp Lys Glu Ser Thr Gln Lys Ala Ile Asp Gly Val Thr
   370 375 380
   Asn Lys Val Asn Ser Ile Ile Asp Lys Met Asn Thr Gln Phe Glu Ala
   385 390 395 400
   Val Gly Arg Glu Phe Asn Asn Leu Glu Arg Arg Ile Glu Asn Leu Asn
   405 410 415
   Lys Lys Met Glu Asp Gly Phe Leu Asp Val Trp Thr Tyr Asn Ala Glu
   420 425 430
   Leu Leu Val Leu Met Glu Asn Glu Arg Thr Leu Asp Phe His Asp Ser
   435 440 445
   Asn Val Lys Asn Leu Tyr Asp Lys Val Arg Leu Gln Leu Arg Asp Asn
   450 455 460
   Ala Lys Glu Leu Gly Asn Gly Cys Phe Glu Phe Tyr His Arg Cys Asp
   465 470 475 480
   Asn Glu Cys Met Glu Ser Val Arg Asn Gly Thr Tyr Asp Tyr Pro Gln
   485 490 495
   Tyr Ser Glu Glu Ala Arg Leu Lys Arg Glu Glu Ile Ser Gly Val Lys
   500 505 510
   Leu Glu Ser Ile Gly Thr Tyr Gln Ile Leu Ser Ile Tyr Ser Thr Val
   515 520 525
   Ala Ser Ser Leu Ala Leu Ala Ile Xaa
   530 535
<210> 18
   <211> 528
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (528)..(528)
   <223> Xaa can be any naturally occurring amino acid
<400> 18
   Xaa Ile Val Ser Ile Val Lys Ser Asp Gln Ile Cys Ile Gly Tyr His
   1               5                   10                  15
   Ala Asn Asn Ser Thr Glu Gln Val Asp Thr Ile Met Glu Lys Asn Val
               20                  25                  30
   Thr Val Thr His Ala Gln Asp Ile Leu Glu Lys Thr His Asn Gly Lys
           35                  40                  45
   Leu Cys Asn Leu Asp Gly Val Lys Pro Leu Ile Leu Arg Asp Cys Ser
       50                  55                  60
   Val Ala Gly Trp Leu Leu Gly Asn Pro Met Cys Asp Glu Phe Leu Asn
   65                  70                  75                  80
   Val Pro Glu Trp Ser Tyr Ile Val Glu Lys Ile Asn Pro Ala Asn Asp
                   85                  90                  95
   Leu Cys Tyr Pro Gly Asn Phe Asn Asp Tyr Glu Glu Leu Lys His Leu
               100                 105                 110
   Leu Ser Arg Ile Asn His Phe Glu Lys Ile Gln Ile Ile Pro Lys Asn
           115                 120                 125
   Ser Trp Ser Asp His Glu Ala Ser Gly Val Ser Ser Ala Cys Pro Tyr
       130                 135                 140
   Gln Gly Arg Ser Ser Phe Phe Arg Asn Val Val Trp Leu Thr Lys Lys
   145                 150                 155                 160
   Asn Asn Ala Tyr Pro Thr Ile Lys Lys Ser Tyr Asn Asn Thr Asn Gln
                   165                 170                 175
   Glu Asp Leu Leu Val Leu Trp Gly Ile His His Pro Asn Asp Ala Ala
               180                 185                 190
   Glu Gln Thr Arg Leu Tyr Gln Asn Pro Thr Thr Tyr Ile Ser Val Gly
           195                 200                 205
   Thr Ser Thr Leu Asn Gln Arg Leu Val Pro Lys Ile Ala Asn Arg Ser
       210                 215                 220
   Lys Val Asn Gly Gln Ser Gly Arg Met Glu Phe Phe Trp Thr Ile Leu
   225                 230                 235                 240
   Lys Ser Asn Asp Ala Ile Asn Phe Glu Ser Asn Gly Asn Phe Ile Ala
                   245                 250                 255
   Pro Glu Asn Ala Tyr Lys Ile Val Lys Lys Gly Asp Ser Thr Ile Met
               260                 265                 270
   Lys Ser Glu Leu Glu Tyr Gly Asn Cys Asn Thr Lys Cys Gln Thr Pro
           275                 280                 285
   Ile Gly Ala Ile Asn Ser Ser Met Pro Phe His Asn Ile His Pro Leu
       290                 295                 300
   Thr Ile Gly Glu Cys Pro Lys Tyr Val Lys Ser Asn Arg Leu Val Leu
   305 310 315 320
   Ala Thr Gly Leu Arg Asn Ser Pro Gln Gly Glu Arg Arg Arg Lys Lys
   325 330 335
   Arg Gly Leu Phe Gly Ala Ile Ala Gly Phe Ile Glu Gly Gly Trp Gln
   340 345 350
   Gly Met Val Asp Gly Trp Tyr Gly Tyr His His Ser Asn Glu Gln Gly
   355 360 365
   Ser Gly Tyr Ala Ala Asp Lys Glu Ser Thr Gln Lys Ala Ile Asp Gly
   370 375 380
   Val Thr Asn Lys Val Asn Ser Ile Ile Asp Lys Met Asn Thr Gln Phe
   385 390 395 400
   Glu Ala Val Gly Arg Glu Phe Asn Asn Leu Glu Arg Arg Ile Glu Asn
   405 410 415
   Leu Asn Lys Lys Met Glu Asp Gly Phe Leu Asp Val Trp Thr Tyr Asn
   420 425 430
   Ala Glu Leu Leu Val Leu Met Glu Asn Glu Arg Thr Leu Asp Phe His
   435 440 445
   Asp Ser Asn Val Lys Asn Leu Tyr Asp Lys Val Arg Leu Gln Leu Arg
   450 455 460
   Asp Asn Ala Lys Glu Leu Gly Asn Gly Cys Phe Glu Phe Tyr His Arg
   465 470 475 480
   Cys Asp Asn Glu Cys Met Glu Ser Val Arg Asn Gly Thr Tyr Asp Tyr
   485 490 495
   Pro Gln Tyr Ser Glu Glu Ala Arg Leu Lys Arg Glu Glu Ile Ser Gly
   500 505 510
   Val Lys Leu Glu Ser Ile Gly Thr Tyr Gln Ile Leu Ser Ile Tyr Xaa
   515 520 525
<210> 19
   <211> 535
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (535)..(535)
   <223> Xaa can be any naturally occurring amino acid
<400> 19
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asn Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Glu Phe Leu Asn Val Pro Glu Trp
   65                  70                  75                  80
   Ser Tyr Ile Val Glu Lys Ile Asn Pro Ala Asn Asp Leu Cys Tyr Pro
                   85                  90                  95
   Gly Asn Phe Asn Asp Tyr Glu Glu Leu Lys His Leu Leu Ser Arg Ile
               100                 105                 110
   Asn His Phe Glu Lys Ile Gln Ile Ile Pro Lys Asn Ser Trp Ser Asp
           115                 120                 125
   His Glu Ala Ser Gly Val Ser Ser Ala Cys Pro Tyr Gln Gly Arg Ser
       130                 135                 140
   Ser Phe Phe Arg Asn Val Val Trp Leu Thr Lys Lys Asp Asn Ala Tyr
   145                 150                 155                 160
   Pro Thr Ile Lys Arg Ser Tyr Asn Asn Thr Asn Gln Glu Asp Leu Leu
                   165                 170                 175
   Val Leu Trp Gly Ile His His Pro Asn Asp Ala Ala Glu Gln Thr Arg
               180                 185                 190
   Leu Tyr Gln Asn Pro Thr Thr Tyr Ile Ser Val Gly Thr Ser Thr Leu
   195 200 205
   Asn Gln Arg Leu Val Pro Lys Ile Ala Thr Arg Ser Lys Val Asn Gly
   210 215 220
   Gln Ser Gly Arg Met Glu Phe Phe Trp Thr Ile Leu Lys Ser Asn Asp
   225 230 235 240
   Ala Ile Asn Phe Glu Ser Asn Gly Asn Phe Ile Ala Pro Glu Asn Ala
   245 250 255
   Tyr Lys Ile Val Lys Lys Gly Asp Ser Thr Ile Met Lys Ser Glu Leu
   260 265 270
   Glu Tyr Gly Asn Cys Asn Thr Lys Cys Gln Thr Pro Ile Gly Ala Ile
   275 280 285
   Asn Ser Ser Met Pro Phe His Asn Ile His Pro Leu Thr Ile Gly Glu
   290 295 300
   Cys Pro Lys Tyr Val Lys Ser Asn Arg Leu Val Leu Ala Thr Gly Leu
   305 310 315 320
   Arg Asn Ser Pro Gln Gly Glu Arg Arg Arg Lys Lys Arg Gly Leu Phe
   325 330 335
   Gly Ala Ile Ala Gly Phe Ile Glu Gly Gly Trp Gln Gly Met Val Asp
   340 345 350
   Gly Trp Tyr Gly Tyr His His Ser Asn Glu Gln Gly Ser Gly Tyr Ala
   355 360 365
   Ala Asp Lys Glu Ser Thr Gln Lys Ala Ile Asp Gly Val Thr Asn Lys
   370 375 380
   Val Asn Ser Ile Ile Asp Lys Met Asn Thr Gln Phe Glu Ala Val Gly
   385 390 395 400
   Arg Glu Phe Asn Asn Leu Glu Arg Arg Ile Glu Asn Leu Asn Lys Lys
   405 410 415
   Met Glu Asp Gly Phe Leu Asp Val Trp Thr Tyr Asn Ala Glu Leu Leu
   420 425 430
   Val Leu Met Glu Asn Glu Arg Thr Leu Asp Phe His Asp Ser Asn Val
   435 440 445
   Lys Asn Leu Tyr Asp Lys Val Arg Leu Gln Leu Arg Asp Asn Ala Lys
   450 455 460
   Glu Leu Gly Asn Gly Cys Phe Glu Phe Tyr His Arg Cys Asp Asn Glu
   465 470 475 480
   Cys Met Glu Ser Val Arg Asn Gly Thr Tyr Asp Tyr Pro Gln Tyr Ser
   485 490 495
   Glu Glu Ala Arg Leu Lys Arg Glu Glu Ile Ser Gly Val Lys Leu Glu
   500 505 510
   Ser Ile Gly Thr Tyr Gln Ile Leu Ser Ile Tyr Ser Thr Val Ala Ser
   515 520 525
   Ser Leu Ala Leu Ala Ile Xaa
   530 535
<210> 20
   <211> 568
   <212> PRT
   <213> Influenza A virus
<220>
   <221> MISC_FEATURE
   <223> subtype H5N1
<400> 20
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Gly Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Pro Asn Val Ser Glu Trp Ser Tyr Ile Val
   85 90 95
   Glu Lys Ile Asn Pro Ala Asn Asp Leu Cys Tyr Pro Gly Asn Phe Asn
   100 105 110
   Asn Tyr Glu Glu Leu Lys His Leu Leu Ser Arg Ile Asn Arg Phe Glu
   115 120 125
   Lys Ile Gln Ile Ile Pro Lys Ser Ser Trp Pro Asp His Glu Ala Ser
   130 135 140
   Leu Gly Val Ser Ser Ala Cys Pro Tyr Gln Gly Gly Pro Ser Phe Tyr
   145 150 155 160
   Arg Asn Val Val Trp Leu Ile Lys Lys Asn Asp Thr Tyr Pro Thr Ile
   165 170 175
   Lys Glu Ser Tyr His Asn Thr Asn Gln Glu Asp Leu Leu Val Leu Trp
   180 185 190
   Gly Ile His His Pro Asn Asn Glu Glu Glu Gln Lys Arg Ile Tyr Lys
   195 200 205
   Asn Pro Thr Thr Tyr Val Ser Val Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Val Glu Phe Phe Trp Thr Ile Leu Lys Ser Asn Asp Thr Ile Asn
   245 250 255
   Phe Glu Ser Asn Gly Asn Phe Ile Ala Pro Glu Asn Ala Tyr Lys Ile
   260 265 270
   Val Lys Lys Gly Asp Ser Thr Ile Met Lys Ser Glu Leu Glu Tyr Gly
   275 280 285
   Asn Cys Ser Thr Lys Cys Gln Thr Pro Ile Gly Ala Ile Asn Thr Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Gly Glu Gly Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
               355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Lys Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Arg Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Arg Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Val Ala Gly Leu Phe Leu Trp Met Cys Ser Asn Gly
   545                 550                 555                 560
   Ser Leu Gln Cys Arg Ile Cys Ile
                   565
<210> 21
   <211> 568
   <212> PRT
   <213> Influenza A virus
<220>
   <221> MISC_FEATURE
   <223> subtype H5N1
<220>
   <221> misc_feature
   <222> (375)..(375)
   <223> Xaa can be any naturally occurring amino acid
<400> 21
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Pro Asn Val Ser Glu Trp Ser Tyr Ile Val
   85 90 95
   Glu Lys Thr Asn Pro Ala Asn Asp Leu Cys Tyr Pro Gly Asn Phe Asn
   100 105 110
   Asn Tyr Glu Glu Leu Lys His Leu Leu Ser Arg Ile Asn Arg Phe Glu
   115 120 125
   Lys Ile Lys Ile Ile Pro Lys Ser Ser Trp Pro Asp His Glu Ala Ser
   130 135 140
   Leu Gly Val Ser Ser Ala Cys Pro Tyr Gln Gly Glu Pro Ser Phe Tyr
   145 150 155 160
   Arg Asn Val Val Trp Leu Ile Lys Lys Asn Asn Thr Tyr Pro Thr Ile
   165 170 175
   Lys Glu Ser Tyr His Asn Thr Asn Gln Glu Asp Leu Leu Val Leu Trp
   180 185 190
   Gly Ile His His Pro Asn Asp Glu Glu Glu Gln Thr Arg Ile Tyr Lys
   195 200 205
   Asn Pro Thr Thr Tyr Ile Ser Val Gly Thr Ser Thr Leu Asn Gln Arg
   210 215 220
   Leu Val Pro Lys Ile Ala Thr Arg Ser Lys Val Asn Gly Gln Ser Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
           275                 280                 285
   Asn Cys Ser Thr Lys Cys Gln Thr Pro Val Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Gly Glu Gly Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Xaa Gln Gly Ser Gly Tyr Ala Ala Asp Arg
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Lys Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Arg Cys Asp Asn Glu Cys Ile Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Ile Val Ala Gly Leu Phe Leu Trp Met Cys Ser Asn Gly
   545                 550                 555                 560
   Ser Leu Gln Cys Arg Ile Cys Ile
                   565
<210> 22
   <211> 568
   <212> PRT
   <213> Influenza A virus
<220>
   <221> MISC_FEATURE
   <223> subtype H5N1
<400> 22
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Lys Ile Ile Pro Lys Ser Ser Trp Pro Asp His Glu Ala Ser
       130                 135                 140
   Leu Gly Val Ser Ser Ala Cys Pro Tyr Gln Arg Gly Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Lys Ser Tyr His Asn Thr Asn Gln Glu Asp Leu Leu Val Leu Trp
               180                 185                 190
   Gly Ile His His Pro Asn Asp Glu Glu Glu Gln Thr Arg Ile Tyr Lys
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Ser Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
           275                 280                 285
   Asn Cys Asn Thr Lys Cys Gln Thr Pro Ile Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Gly Glu Gly Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Arg Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Met Met Ala Gly Leu Phe Leu Trp Met Cys Ser Asn Gly
   545 550 555 560
   Ser Leu Gln Cys Arg Ile Cys Ile
   565
<210> 23
   <211> 568
   <212> PRT
   <213> Influenza A virus
<220>
   <221> MISC_FEATURE
   <223> subtype H5N1
<400> 23
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Lys Ile Ile Pro Lys Ser Ser Trp Pro Asp His Glu Ala Ser
       130                 135                 140
   Leu Gly Val Ser Ser Ala Cys Pro Tyr Gln Gly Gly Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Thr Arg Ile Tyr Lys
           195                 200                 205
   Asn Pro Thr Thr Tyr Ile Ser Ile Gly Thr Ser Thr Leu Asn Gln Arg
       210                 215                 220
   Leu Val Pro Lys Ile Ala Thr Arg Ser Lys Val Asn Gly Gln Arg Gly
   225 230 235 240
   Arg Val Glu Phe Phe Trp Thr Ile Leu Lys Ser Asn Asp Thr Ile Asn
   245 250 255
   Phe Glu Ser Asn Gly Asn Phe Ile Ala Pro Glu Asn Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Gly Glu Gly Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Arg Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Met Met Ala Gly Leu Phe Leu Trp Met Cys Ser Asn Gly
   545 550 555 560
   Ser Leu Gln Cys Arg Ile Cys Ile
   565
<210> 24
   <211> 568
   <212> PRT
   <213> Influenza A virus
<220>
   <221> MISC_FEATURE
   <223> subtype H5N1
<400> 24
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Pro Asn Val Ser Glu Trp Ser Tyr Ile Val
   85 90 95
   Glu Lys Ile Asn Pro Ala Asn Asp Leu Cys Tyr Pro Gly Asn Phe Asn
   100 105 110
   Asn Tyr Glu Glu Leu Lys His Leu Leu Ser Arg Ile Asn Arg Phe Glu
           115                 120                 125
   Lys Ile Gln Ile Ile Pro Lys Ser Ser Trp Pro Asp His Glu Ala Ser
       130                 135                 140
   Leu Gly Val Ser Ser Ala Cys Pro Tyr Gln Gly Glu Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Lys Arg Ile Tyr Lys
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Pro Lys Val Asn Gly Gln Ser Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Lys Asn Ala Tyr Lys Ile
               260                 265                 270
   Val Lys Lys Gly Ser Ser Thr Ile Met Lys Ser Glu Leu Glu Tyr Gly
           275                 280                 285
   Asn Cys Ser Thr Lys Cys Gln Thr Pro Ile Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Gly Glu Gly Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Thr Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Arg Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Lys Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Thr Ser Ser Leu Ala
   530 535 540
   Leu Ala Ile Met Val Ala Gly Leu Ser Leu Trp Met Cys Ser Asn Gly
   545 550 555 560
   Ser Leu Gln Cys Arg Ile Cys Ile
   565
<210> 25
   <211> 556
   <212> PRT
   <213> Influenza A virus
<220>
   <221> MISC_FEATURE
   <223> subtype H5N1
<220>
   <221> misc_feature
   <222> (556)..(556)
   <223> Xaa can be any naturally occurring amino acid
<400> 25
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Thr Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Lys Ile Ile Pro Lys Ser Ser Trp Pro Asp His Glu Ala Ser
       130                 135                 140
   Leu Gly Val Ser Ser Ala Cys Pro Tyr Gln Gly Arg Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Thr Arg Ile Tyr Lys
           195                 200                 205
   Asn Pro Thr Thr Tyr Ile Ser Ile Gly Thr Ser Thr Leu Asn Gln Arg
       210                 215                 220
   Leu Ile Pro Lys Ile Ala Thr Arg Ser Lys Val Asn Gly Gln Ser Gly
   225 230 235 240
   Arg Val Glu Phe Phe Trp Thr Ile Leu Lys Ser Asn Asp Thr Ile Asn
   245 250 255
   Phe Glu Ser Asn Gly Asn Phe Ile Ala Pro Glu Asn Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Gly Glu Gly Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Arg Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Met Met Ala Gly Leu Phe Leu Trp Xaa
   545 550 555
<210> 26
   <211> 554
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (554)..(554)
   <223> Xaa can be any naturally occurring amino acid
<400> 26
   Xaa Leu Leu Ala Ile Val Ser Leu Val Lys Ser Asp Gln Ile Cys Ile
   1 5 10 15
   Gly Tyr His Ala Asn Asn Ser Thr Glu Gln Val Asp Thr Ile Met Glu
   20 25 30
   Lys Asn Val Thr Val Thr His Ala Gln Asp Ile Leu Glu Lys Thr His
   35 40 45
   Asn Gly Lys Leu Cys Asp Leu Asp Gly Val Lys Pro Leu Ile Leu Arg
   50 55 60
   Asp Cys Ser Val Ala Gly Trp Leu Leu Gly Asn Pro Met Cys Asp Glu
   65 70 75 80
   Phe Pro Asn Val Ser Glu Trp Ser Tyr Ile Val Glu Lys Ile Asn Pro
                   85                  90                  95
   Ala Asn Asp Leu Cys Tyr Pro Gly Asn Phe Asn Asn Tyr Glu Glu Leu
               100                 105                 110
   Lys His Leu Leu Ser Arg Ile Asn Arg Phe Glu Lys Ile Gln Ile Ile
           115                 120                 125
   Pro Lys Ser Ser Trp Pro Asp His Glu Ala Ser Leu Gly Val Ser Ser
       130                 135                 140
   Ala Cys Pro Tyr Gln Gly Glu Pro Ser Phe Tyr Arg Asn Val Val Trp
   145                 150                 155                 160
   Leu Ile Lys Lys Asn Asn Thr Tyr Pro Thr Ile Lys Glu Asn Tyr His
                   165                 170                 175
   Asn Thr Asn Gln Glu Asp Leu Leu Val Leu Trp Gly Ile His His Pro
               180                 185                 190
   Asn Asp Glu Glu Glu Gln Lys Arg Ile Tyr Lys Asn Pro Thr Thr Tyr
           195                 200                 205
   Ile Ser Val Gly Thr Ser Thr Leu Asn Gln Arg Leu Val Pro Lys Ile
       210                 215                 220
   Ala Thr Arg Pro Lys Val Asn Gly Gln Ser Gly Arg Val Glu Phe Phe
   225                 230                 235                 240
   Trp Thr Ile Leu Lys Ser Asn Asp Thr Ile Asn Phe Glu Ser Asn Gly
                   245                 250                 255
   Asn Phe Ile Ala Pro Lys Asn Ala Tyr Lys Ile Val Lys Lys Gly Ser
               260                 265                 270
   Ser Thr Ile Met Lys Ser Glu Leu Glu Tyr Gly Asn Cys Ser Thr Lys
           275                 280                 285
   Cys Gln Thr Pro Ile Gly Ala Ile Asn Ser Ser Met Pro Phe His Asn
       290                 295                 300
   Ile His Pro Leu Thr Ile Gly Glu Cys Pro Lys Tyr Val Lys Ser Asn
   305                 310                 315                 320
   Arg Leu Val Leu Ala Thr Gly Leu Arg Asn Ser Pro Gln Gly Glu Gly
                   325                 330                 335
   Arg Arg Lys Lys Arg Gly Leu Phe Gly Ala Ile Ala Gly Phe Ile Glu
               340                 345                 350
   Gly Gly Trp Gln Gly Met Val Asp Gly Trp Tyr Gly Tyr His His Ser
           355                 360                 365
   Asn Glu Gln Gly Thr Gly Tyr Ala Ala Asp Lys Glu Ser Thr Gln Lys
       370                 375                 380
   Ala Ile Asp Gly Val Thr Asn Lys Val Asn Ser Ile Ile Asp Lys Met
   385                 390                 395                 400
   Asn Thr Gln Phe Glu Ala Val Gly Arg Glu Phe Asn Asn Leu Glu Lys
                   405                 410                 415
   Arg Ile Glu Asn Leu Asn Lys Lys Met Glu Asp Gly Phe Leu Asp Val
               420                 425                 430
   Trp Thr Tyr Asn Ala Glu Leu Leu Val Leu Met Glu Asn Glu Arg Thr
           435                 440                 445
   Leu Asp Phe His Asp Ser Asn Val Arg Asn Leu Tyr Asp Lys Val Arg
       450                 455                 460
   Leu Gln Leu Arg Asp Asn Ala Lys Glu Leu Gly Asn Gly Cys Phe Glu
   465                 470                 475                 480
   Phe Tyr His Lys Cys Asp Asn Glu Cys Met Glu Ser Val Arg Asn Gly
                   485                 490                 495
   Thr Tyr Asp Tyr Pro Gln Tyr Ser Lys Glu Ala Arg Leu Lys Arg Glu
               500                 505                 510
   Glu Ile Ser Gly Val Lys Leu Glu Ser Ile Gly Thr Tyr Gln Ile Leu
           515                 520                 525
   Ser Ile Tyr Ser Thr Val Ala Ser Ser Leu Ala Leu Ala Ile Met Val
       530                 535                 540
   Ala Gly Leu Ser Leu Trp Met Cys Ser Xaa
   545                 550
<210> 27
   <211> 564
   <212> PRT
   <213> Influenza A virus
<220>
   <221> MISC_FEATURE
   <223> subtype H5N1
<400> 27
   Val Leu Leu Leu Ala Ile Val Ser Leu Val Lys Ser Asp Gln Ile Cys
   1               5                   10                  15
   Ile Gly Tyr His Ala Asn Asn Ser Thr Glu Gln Val Asp Thr Ile Met
               20                  25                  30
   Glu Lys Asn Val Thr Val Thr His Ala Gln Asp Ile Leu Glu Lys Thr
           35                  40                  45
   His Asn Gly Lys Leu Cys Asp Leu Asp Gly Val Lys Pro Leu Ile Leu
       50                  55                  60
   Arg Asp Cys Ser Val Ala Gly Trp Leu Leu Gly Asn Pro Met Cys Asp
   65                  70                  75                  80
   Glu Phe Pro Asn Val Ser Glu Trp Ser Tyr Ile Val Glu Lys Ile Asn
                   85                  90                  95
   Pro Ala Asn Asp Leu Cys Tyr Pro Gly Asn Phe Asn Asn Tyr Glu Glu
               100                 105                 110
   Leu Lys His Leu Leu Ser Arg Ile Asn Arg Phe Glu Lys Ile Gln Ile
           115                 120                 125
   Ile Pro Lys Ser Ser Trp Pro Asp His Glu Ala Ser Leu Gly Val Ser
       130                 135                 140
   Ser Ala Cys Pro Tyr Gln Gly Glu Pro Ser Phe Tyr Arg Asn Val Val
   145                 150                 155                 160
   Trp Leu Ile Lys Lys Asn Asn Thr Tyr Pro Thr Ile Lys Glu Asn Tyr
                   165                 170                 175
   His Asn Thr Asn Gln Glu Asp Leu Leu Val Leu Trp Gly Ile His His
               180                 185                 190
   Pro Asn Asp Glu Glu Glu Gln Lys Arg Ile Tyr Lys Asn Pro Thr Thr
           195                 200                 205
   Tyr Ile Ser Val Gly Thr Ser Thr Leu Asn Gln Arg Leu Val Pro Lys
       210                 215                 220
   Ile Ala Thr Arg Pro Lys Val Asn Gly Gln Ser Gly Arg Val Glu Phe
   225                 230                 235                 240
   Phe Trp Thr Ile Leu Lys Ser Asn Asp Thr Ile Asn Phe Glu Ser Asn
                   245                 250                 255
   Gly Asn Phe Ile Ala Pro Lys Asn Ala Tyr Lys Ile Val Lys Lys Gly
               260                 265                 270
   Ser Ser Thr Ile Met Lys Ser Glu Leu Glu Tyr Gly Asn Cys Ser Thr
           275                 280                 285
   Lys Cys Gln Thr Pro Ile Gly Ala Ile Asn Ser Ser Met Pro Phe His
       290                 295                 300
   Asn Ile His Pro Leu Thr Ile Gly Glu Cys Pro Lys Tyr Val Lys Ser
   305                 310                 315                 320
   Asn Arg Leu Val Leu Ala Thr Gly Leu Arg Asn Ser Pro Gln Gly Glu
                   325                 330                 335
   Gly Arg Arg Lys Lys Arg Gly Leu Phe Gly Ala Ile Ala Gly Phe Ile
               340                 345                 350
   Glu Gly Gly Trp Gln Gly Met Val Asp Gly Trp Tyr Gly Tyr His His
           355                 360                 365
   Ser Asn Glu Gln Gly Thr Gly Tyr Ala Ala Asp Lys Glu Ser Thr Gln
       370                 375                 380
   Lys Ala Ile Asp Gly Val Thr Asn Lys Val Asn Ser Ile Ile Asp Lys
   385                 390                 395                 400
   Met Asn Thr Gln Phe Glu Ala Val Gly Arg Glu Phe Asn Asn Leu Glu
                   405                 410                 415
   Lys Arg Ile Glu Asn Leu Asn Lys Lys Met Glu Asp Gly Phe Leu Asp
               420                 425                 430
   Val Trp Thr Tyr Asn Ala Glu Leu Leu Val Leu Met Glu Asn Glu Arg
           435                 440                 445
   Thr Leu Asp Phe His Asp Ser Asn Val Arg Asn Leu Tyr Asp Lys Val
       450                 455                 460
   Arg Leu Gln Leu Arg Asp Asn Ala Lys Glu Leu Gly Asn Gly Cys Phe
   465                 470                 475                 480
   Glu Phe Tyr His Lys Cys Asp Asn Glu Cys Met Glu Ser Val Arg Asn
   485 490 495
   Gly Thr Tyr Asp Tyr Pro Gln Tyr Ser Lys Glu Ala Arg Leu Lys Arg
   500 505 510
   Glu Glu Ile Ser Gly Val Lys Leu Glu Ser Ile Gly Thr Tyr Gln Ile
   515 520 525
   Leu Ser Ile Tyr Ser Thr Val Ala Ser Ser Leu Ala Leu Ala Ile Met
   530 535 540
   Val Ala Gly Leu Ser Leu Trp Met Cys Ser Asn Gly Ser Leu Gln Cys
   545 550 555 560
   Arg Ile Cys Ile
<210> 28
   <211> 553
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<400> 28
   Xaa Val Leu Leu Leu Ala Ile Ile Ser Leu Val Lys Ser Asp Gln Ile
   1 5 10 15
   Cys Ile Gly Tyr His Ala Asn Asn Ser Thr Glu Gln Val Asp Thr Ile
   20 25 30
   Met Glu Lys Asn Val Thr Val Thr His Ala Gln Asp Ile Leu Glu Lys
   35 40 45
   Thr His Asn Gly Lys Leu Cys Asp Leu Asp Gly Val Lys Pro Leu Ile
   50 55 60
   Leu Arg Gly Cys Ser Val Ala Gly Trp Leu Leu Gly Asn Pro Met Cys
   65 70 75 80
   Asp Glu Phe Pro Asn Val Ser Glu Trp Ser Tyr Ile Val Glu Lys Ile
   85 90 95
   Asn Pro Ala Asn Asp Leu Cys Tyr Pro Gly Asn Phe Asn Asn Tyr Glu
   100 105 110
   Glu Leu Lys His Leu Leu Ser Arg Ile Asn Arg Phe Glu Lys Ile Gln
   115 120 125
   Ile Ile Pro Lys Ser Ser Trp Pro Asp His Glu Ala Ser Leu Gly Val
   130 135 140
   Ser Ser Ala Cys Pro Tyr Gln Gly Glu Pro Ser Phe Tyr Arg Asn Val
   145 150 155 160
   Val Trp Leu Ile Lys Lys Asn Asn Thr Tyr Pro Thr Ile Lys Glu Ser
   165 170 175
   Tyr His Asn Thr Asn Gln Glu Asp Leu Leu Val Leu Trp Gly Ile His
   180 185 190
   His Pro Asn Asp Glu Glu Glu Gln Lys Arg Ile Tyr Lys Asn Pro Thr
   195 200 205
   Thr Tyr Ile Ser Val Gly Thr Ser Thr Leu Asn Gln Arg Leu Val Pro
   210 215 220
   Lys Ile Ala Thr Arg Pro Lys Val Asn Gly Gln Ser Gly Arg Val Glu
   225 230 235 240
   Phe Phe Trp Thr Ile Leu Lys Ser Asn Asp Thr Ile Asn Phe Glu Ser
   245 250 255
   Asn Gly Asn Phe Ile Ala Pro Lys Asn Ala Tyr Lys Ile Val Lys Lys
   260 265 270
   Gly Ser Ser Thr Ile Met Lys Ser Glu Leu Glu Tyr Gly Asn Cys Ser
   275 280 285
   Thr Lys Cys Gln Thr Pro Ile Gly Ala Ile Asn Ser Ser Met Pro Phe
   290 295 300
   His Asn Ile His Pro Leu Thr Ile Gly Glu Cys Pro Lys Tyr Val Lys
   305 310 315 320
   Ser Asn Arg Leu Val Leu Ala Thr Gly Leu Arg Asn Ser Pro Gln Gly
   325 330 335
   Glu Gly Arg Arg Lys Lys Arg Gly Leu Phe Gly Ala Ile Ala Gly Phe
               340                 345                 350
   Ile Glu Gly Gly Trp Gln Gly Met Val Asp Gly Trp Tyr Gly Tyr His
           355                 360                 365
   His Ser Asn Glu Gln Gly Thr Gly Tyr Ala Ala Asp Lys Glu Ser Thr
       370                 375                 380
   Gln Lys Ala Ile Asp Gly Val Thr Asn Lys Val Asn Ser Ile Ile Asp
   385                 390                 395                 400
   Lys Met Asn Thr Gln Phe Glu Ala Val Gly Arg Glu Phe Asn Asn Leu
                   405                 410                 415
   Glu Lys Arg Ile Glu Asn Leu Asn Lys Lys Met Glu Asp Gly Phe Leu
               420                 425                 430
   Asp Val Trp Thr Tyr Asn Ala Glu Leu Leu Val Leu Met Glu Asn Glu
           435                 440                 445
   Arg Thr Leu Asp Phe His Asp Ser Asn Val Arg Asn Leu Tyr Asp Lys
       450                 455                 460
   Val Arg Leu Gln Leu Arg Asp Asn Ala Lys Glu Leu Gly Asn Gly Cys
   465                 470                 475                 480
   Phe Glu Phe Tyr His Lys Cys Asp Asn Glu Cys Met Glu Ser Val Arg
                   485                 490                 495
   Asn Gly Thr Tyr Asp Tyr Pro Gln Tyr Ser Lys Glu Ala Arg Leu Lys
               500                 505                 510
   Arg Glu Glu Ile Ser Gly Val Lys Leu Glu Ser Ile Gly Thr Tyr Gln
           515                 520                 525
   Ile Leu Ser Ile Tyr Ser Thr Val Ala Ser Ser Leu Ala Leu Ala Ile
       530                 535                 540
   Met Val Ala Gly Leu Ser Leu Trp Met
   545                 550
<210> 29
   <211> 558
   <212> PRT
   <213> Influenza A virus
<220>
   <221> MISC_FEATURE
   <223> subtype H5N1
<220>
   <221> misc_feature
   <222> (558)..(558)
   <223> Xaa can be any naturally occurring amino acid
<400> 29
   Ile Val Leu Leu Leu Ala Ile Val Ser Leu Val Lys Ser Asp Gln Ile
   1               5                   10                  15
   Cys Ile Gly Tyr His Ala Asn Asn Ser Thr Glu Gln Val Asp Thr Ile
               20                  25                  30
   Met Glu Lys Asn Val Thr Val Thr His Ala Gln Asp Ile Leu Glu Lys
           35                  40                  45
   Thr His Asn Gly Lys Leu Cys Asp Leu Asp Gly Val Lys Pro Leu Ile
       50                  55                  60
   Leu Arg Asp Cys Ser Val Ala Gly Trp Leu Leu Gly Asn Pro Met Cys
   65                  70                  75                  80
   Asp Glu Phe Pro Asn Val Ser Glu Trp Ser Tyr Ile Val Glu Lys Ile
                   85                  90                  95
   Asn Pro Ala Asn Asp Leu Cys Tyr Pro Gly Asn Phe Asn Asn Tyr Glu
               100                 105                 110
   Glu Leu Lys His Leu Leu Ser Arg Ile Asn Arg Phe Glu Lys Ile Lys
           115                 120                 125
   Ile Ile Pro Lys Ser Ser Trp Pro Asp His Glu Ala Ser Leu Gly Val
       130                 135                 140
   Ser Ser Ala Cys Pro Tyr Gln Gly Gly Pro Ser Phe Tyr Arg Asn Val
   145                 150                 155                 160
   Val Trp Leu Ile Lys Lys Asn Asn Thr Tyr Pro Thr Ile Lys Lys Ser
                   165                 170                 175
   Tyr His Asn Thr Asn Gln Glu Asp Leu Leu Val Leu Trp Gly Ile His
               180                 185                 190
   His Pro Asn Asp Glu Glu Glu Gln Thr Arg Ile Tyr Lys Asn Pro Thr
           195                 200                 205
   Thr Tyr Ile Ser Val Gly Thr Ser Thr Leu Asn Gln Arg Leu Val Pro
   210 215 220
   Lys Ile Ala Thr Arg Ser Lys Val Asn Gly Gln Ser Gly Arg Val Glu
   225 230 235 240
   Phe Phe Trp Thr Ile Leu Lys Ser Asn Asp Thr Ile Asn Phe Glu Ser
   245 250 255
   Asn Gly Asn Phe Ile Ala Pro Glu Asn Ala Tyr Lys Ile Val Lys Lys
   260 265 270
   Gly Asp Ser Thr Ile Met Lys Ser Glu Leu Glu His Gly Asn Cys Asn
   275 280 285
   Thr Lys Cys Gln Thr Pro Ile Gly Ala Ile Asn Ser Ser Met Pro Phe
   290 295 300
   His Asn Ile His Pro Leu Thr Ile Gly Glu Cys Pro Lys Tyr Val Lys
   305 310 315 320
   Ser Asn Arg Leu Val Leu Ala Thr Gly Leu Arg Asn Ser Pro Gln Gly
   325 330 335
   Glu Gly Arg Arg Lys Lys Arg Gly Leu Phe Gly Ala Ile Ala Gly Phe
   340 345 350
   Ile Glu Gly Gly Trp Gln Gly Met Val Asp Gly Trp Tyr Gly Tyr His
   355 360 365
   His Ser Asn Glu Gln Gly Ser Gly Tyr Ala Ala Asp Lys Glu Ser Thr
   370 375 380
   Gln Lys Ala Ile Asp Gly Val Thr Asn Lys Val Asn Ser Ile Ile Asp
   385 390 395 400
   Lys Met Asn Thr Gln Phe Glu Ala Val Gly Arg Glu Phe Asn Asn Leu
   405 410 415
   Glu Lys Arg Ile Glu Asn Leu Asn Lys Lys Met Glu Asp Gly Phe Leu
   420 425 430
   Asp Val Trp Thr Tyr Asn Ala Glu Leu Leu Val Leu Met Glu Asn Glu
   435 440 445
   Arg Thr Leu Asp Phe His Asp Ser Asn Val Lys Asn Leu Tyr Asp Lys
   450 455 460
   Val Arg Leu Gln Leu Arg Asp Asn Ala Lys Glu Leu Gly Asn Gly Cys
   465 470 475 480
   Phe Glu Phe Tyr His Arg Cys Asp Asn Glu Cys Met Glu Ser Val Arg
   485 490 495
   Asn Gly Thr Tyr Asp Tyr Pro Gln Tyr Ser Glu Glu Ala Arg Leu Asn
   500 505 510
   Arg Glu Glu Ile Ser Gly Val Lys Leu Glu Ser Ile Gly Thr Tyr Gln
   515 520 525
   Ile Leu Ser Ile Tyr Ser Thr Val Ala Ser Ser Leu Ala Leu Ala Ile
   530 535 540
   Met Met Ala Gly Leu Phe Leu Trp Met Cys Ser Asn Gly Xaa
   545 550 555
<210> 30
   <211> 557
   <212> PRT
   <213> Influenza A virus
<220>
   <221> MISC_FEATURE
   <223> subtype H5N1
<220>
   <221> misc_feature
   <222> (130)..(130)
   <223> Xaa can be any naturally occurring amino acid
<400> 30
   Leu Leu Ala Ile Val Ser Leu Val Lys Ser Asp Gln Ile Cys Ile Gly
   1               5                   10                  15
   Tyr His Ala Asn Asn Ser Thr Glu Gln Val Asp Thr Ile Met Glu Lys
               20                  25                  30
   Asn Val Thr Val Thr His Ala Gln Asp Ile Leu Glu Lys Thr His Asn
           35                  40                  45
   Gly Lys Leu Cys Asp Leu Asp Gly Val Lys Pro Leu Ile Leu Arg Asp
       50                  55                  60
   Cys Ser Val Ala Gly Trp Leu Leu Gly Asn Pro Met Cys Asp Glu Phe
   65                  70                  75                  80
   Pro Asn Val Ser Glu Trp Ser Tyr Ile Val Glu Lys Ile Asn Pro Ala
                   85                  90                  95
   Asn Asp Leu Cys Tyr Pro Gly Asn Phe Asn Asn Tyr Glu Glu Leu Lys
   100 105 110
   His Leu Leu Ser Arg Ile Asn Arg Phe Glu Lys Ile Lys Ile Ile Pro
   115 120 125
   Lys Xaa Ser Trp Pro Asp His Glu Ala Ser Leu Gly Val Ser Ser Ala
   130 135 140
   Cys Pro Tyr Gln Gly Gly Pro Ser Phe Tyr Arg Asn Val Val Trp Leu
   145 150 155 160
   Ile Lys Lys Asn Asn Thr Tyr Pro Thr Ile Lys Glu Ser Tyr His Asn
   165 170 175
   Thr Asn Gln Glu Asp Leu Leu Val Leu Trp Gly Ile His His Pro Asn
   180 185 190
   Asp Glu Glu Glu Gln Thr Arg Ile Tyr Lys Asn Pro Thr Thr Tyr Ile
   195 200 205
   Ser Ile Gly Thr Ser Thr Leu Asn Gln Arg Leu Val Pro Lys Ile Ala
   210 215 220
   Thr Arg Ser Lys Val Asn Gly Gln Ser Gly Arg Val Glu Phe Phe Trp
   225 230 235 240
   Thr Ile Leu Lys Ser Asn Asp Thr Ile Asn Phe Glu Ser Asn Gly Asn
   245 250 255
   Phe Ile Ala Pro Glu Asn Ala Tyr Lys Ile Val Lys Lys Gly Asp Ser
   260 265 270
   Thr Ile Met Lys Ser Glu Leu Glu Tyr Gly Asn Cys Asn Thr Lys Cys
   275 280 285
   Gln Thr Pro Ile Gly Ala Ile Asn Ser Ser Met Pro Phe His Asn Ile
   290 295 300
   His Pro Leu Thr Ile Gly Glu Cys Pro Lys Tyr Val Lys Ser Asn Arg
   305 310 315 320
   Leu Val Leu Ala Thr Gly Leu Arg Asn Ser Pro Gln Gly Glu Gly Arg
   325 330 335
   Arg Lys Lys Arg Gly Leu Phe Gly Ala Ile Ala Gly Phe Ile Glu Gly
                   340                 345                 350
   Gly Trp Gln Gly Met Val Asp Gly Trp Tyr Gly Tyr His His Ser Asn
               355                 360                 365
   Glu Gln Gly Ser Gly Tyr Ala Ala Asp Lys Glu Ser Thr Gln Lys Ala
       370                 375                 380
   Ile Asp Gly Val Thr Asn Lys Val Asn Ser Ile Ile Asp Lys Met Asn
   385                 390                 395                 400
   Thr Gln Phe Glu Ala Val Gly Arg Glu Phe Asn Asn Leu Glu Lys Arg
                   405                 410                 415
   Ile Glu Asn Leu Asn Lys Lys Met Glu Asp Gly Phe Leu Asp Val Trp
               420                 425                 430
   Thr Tyr Asn Ala Glu Leu Leu Val Leu Met Glu Asn Glu Arg Thr Leu
           435                 440                 445
   Asp Phe His Asp Ser Asn Val Lys Asn Leu Tyr Asp Lys Val Arg Leu
       450                 455                 460
   Gln Leu Arg Asp Asn Ala Lys Glu Leu Gly Asn Gly Cys Phe Glu Phe
   465                 470                 475                 480
   Tyr His Arg Cys Asp Asn Glu Cys Met Glu Ser Val Arg Asn Gly Thr
                   485                 490                 495
   Tyr Asp Tyr Pro Gln Tyr Ser Glu Glu Ala Arg Leu Lys Arg Glu Glu
               500                 505                 510
   Ile Ser Gly Val Lys Leu Glu Ser Ile Gly Thr Tyr Gln Ile Leu Ser
           515                 520                 525
   Ile Tyr Ser Thr Val Ala Gly Ser Leu Ala Leu Ala Ile Met Met Ala
       530                 535                 540
   Gly Leu Phe Leu Trp Met Cys Ser Asn Gly Ser Leu Gln
   545                 550                 555
<210> 31
   <211> 547
   <212> PRT
   <213> Influenza A virus
<220>
   <221> MISC_FEATURE
   <223> subtype H5N1
<220>
   <221> misc_feature
   <222> (547)..(547)
   <223> Xaa can be any naturally occurring amino acid
<400> 31
   Ile Val Leu Leu Leu Ala Ile Val Ser Leu Val Lys Ser Asp Gln Ile
   1               5                   10                  15
   Cys Ile Gly Tyr His Ala Asn Asn Ser Thr Glu Gln Val Asp Thr Ile
               20                  25                  30
   Met Glu Lys Asn Val Thr Val Thr His Ala Gln Asp Ile Leu Glu Lys
           35                  40                  45
   Thr His Asn Gly Lys Leu Cys Asp Leu Asp Gly Val Lys Pro Leu Ile
       50                  55                  60
   Leu Arg Asp Cys Ser Val Ala Gly Trp Leu Leu Gly Asn Pro Met Cys
   65                  70                  75                  80
   Asp Glu Phe Pro Asn Val Ser Glu Trp Ser Tyr Ile Val Glu Lys Ile
                   85                  90                  95
   Asn Pro Ala Asn Asp Leu Cys Tyr Pro Gly Asn Phe Asn Asn Tyr Glu
               100                 105                 110
   Glu Leu Lys His Leu Leu Ser Arg Ile Asn Arg Phe Glu Lys Ile Lys
           115                 120                 125
   Ile Ile Pro Lys Ser Ser Trp Pro Asp His Glu Ala Ser Leu Gly Val
       130                 135                 140
   Ser Ser Ala Cys Pro Tyr Gln Glu Gly Pro Ser Phe Tyr Arg Asn Val
   145                 150                 155                 160
   Val Trp Leu Ile Lys Lys Asn Asn Thr Tyr Pro Thr Ile Lys Lys Ser
                   165                 170                 175
   Tyr His Asn Thr Asn Gln Glu Asp Leu Leu Val Leu Trp Gly Ile His
               180                 185                 190
   His Pro Asn Asp Glu Glu Glu Gln Thr Arg Ile Tyr Lys Asn Pro Thr
           195                 200                 205
   Thr Tyr Ile Ser Val Gly Thr Ser Thr Leu Asn Gln Arg Leu Val Pro
       210                 215                 220
   Lys Ile Ala Thr Arg Ser Lys Val Asn Gly Gln Ser Gly Arg Val Glu
   225                 230                 235                 240
   Phe Phe Trp Thr Ile Leu Lys Ser Asn Asp Thr Ile Asn Phe Glu Ser
                   245                 250                 255
   Asn Gly Asn Phe Ile Ala Pro Glu Asn Ala Tyr Lys Ile Val Lys Lys
               260                 265                 270
   Gly Asp Ser Thr Ile Met Lys Ser Glu Leu Glu Tyr Gly Asn Cys Asn
           275                 280                 285
   Thr Lys Cys Gln Thr Pro Ile Gly Ala Ile Asn Ser Ser Met Pro Phe
       290                 295                 300
   His Asn Ile His Pro Leu Thr Ile Gly Glu Cys Pro Lys Tyr Val Lys
   305                 310                 315                 320
   Ser Asn Arg Leu Val Leu Ala Thr Gly Leu Arg Asn Ser Pro Gln Gly
                   325                 330                 335
   Glu Gly Arg Arg Lys Lys Arg Gly Leu Phe Gly Ala Ile Ala Gly Phe
               340                 345                 350
   Ile Glu Gly Gly Trp Gln Gly Met Val Asp Gly Trp Tyr Gly Tyr His
           355                 360                 365
   His Ser Asn Glu Gln Gly Ser Gly Tyr Ala Ala Asp Lys Glu Ser Thr
       370                 375                 380
   Gln Lys Ala Ile Asp Gly Val Thr Asn Lys Val Asn Ser Ile Ile Asp
   385                 390                 395                 400
   Lys Met Asn Thr Gln Phe Glu Ala Val Gly Arg Glu Phe Asn Asn Leu
                   405                 410                 415
   Glu Lys Arg Ile Glu Asn Leu Asn Lys Lys Met Glu Asp Gly Phe Leu
               420                 425                 430
   Asp Val Trp Thr Tyr Asn Ala Glu Leu Leu Val Leu Met Glu Asn Glu
           435                 440                 445
   Arg Thr Leu Asp Phe His Asp Ser Asn Val Lys Asn Leu Tyr Asp Lys
       450                 455                 460
   Val Arg Leu Gln Leu Arg Asp Asn Ala Lys Glu Leu Gly Asn Gly Cys
   465 470 475 480
   Phe Glu Phe Tyr His Arg Cys Asp Asn Glu Cys Met Glu Ser Val Arg
   485 490 495
   Asn Gly Thr Tyr Asp Tyr Pro Gln Tyr Ser Glu Glu Ala Arg Leu Lys
   500 505 510
   Arg Glu Glu Ile Ser Gly Val Lys Leu Glu Ser Ile Gly Thr Tyr Gln
   515 520 525
   Ile Leu Ser Ile Tyr Ser Thr Val Ala Ser Ser Leu Ala Leu Ala Ile
   530 535 540
   Met Val Xaa
   545
<210> 32
   <211> 547
   <212> PRT
   <213> Influenza A virus
<220>
   <221> MISC_FEATURE
   <223> subtype H5N1
<220>
   <221> misc_feature
   <222> (154)..(154)
   <223> Xaa can be any naturally occurring amino acid
<220>
   <221> misc_feature
   <222> (329)..(329)
   <223> Xaa can be any naturally occurring amino acid
<220>
   <221> misc_feature
   <222> (547)..(547)
   <223> Xaa can be any naturally occurring amino acid
<400> 32
   Ile Val Leu Leu Leu Ala Ile Val Ser Leu Val Lys Ser Asp Gln Ile
   1               5                   10                  15
   Cys Ile Gly Tyr His Ala Asn Asn Ser Thr Glu Gln Val Asp Thr Ile
               20                  25                  30
   Met Glu Lys Asn Val Thr Val Thr His Ala Gln Asp Ile Leu Glu Lys
           35                  40                  45
   Thr His Asn Gly Lys Leu Cys Asp Leu Asp Gly Val Lys Pro Leu Ile
       50                  55                  60
   Leu Arg Asp Cys Ser Val Ala Gly Trp Leu Leu Gly Asn Pro Met Cys
   65                  70                  75                  80
   Asp Glu Phe Pro Asn Val Ser Glu Trp Ser Tyr Ile Val Glu Lys Ile
                   85                  90                  95
   Asn Pro Ala Asn Asp Leu Cys Tyr Pro Gly Asn Phe Asn Asn Tyr Glu
               100                 105                 110
   Glu Leu Lys His Leu Leu Ser Arg Ile Asn Arg Phe Glu Lys Ile Lys
           115                 120                 125
   Ile Ile Pro Lys Ser Ser Trp Pro Asp His Glu Ala Ser Leu Gly Val
       130                 135                 140
   Ser Ser Ala Cys Pro Tyr Gln Gly Gly Xaa Ser Phe Tyr Arg Asn Val
   145                 150                 155                 160
   Val Trp Leu Ile Lys Lys Asn Asn Thr Tyr Pro Thr Ile Lys Glu Ser
                   165                 170                 175
   Tyr His Asn Thr Asn Gln Glu Asp Leu Leu Val Leu Trp Gly Ile His
               180                 185                 190
   His Pro Asn Asp Glu Glu Glu Gln Thr Arg Ile Tyr Lys Asn Pro Thr
           195                 200                 205
   Thr Tyr Ile Ser Ile Gly Thr Ser Thr Leu Asn Gln Arg Leu Val Pro
       210                 215                 220
   Lys Ile Ala Thr Arg Ser Lys Val Asn Gly Gln Ser Gly Arg Val Glu
   225                 230                 235                 240
   Phe Phe Trp Thr Ile Leu Lys Ser Asn Asp Thr Ile Asn Phe Glu Ser
                   245                 250                 255
   Asn Gly Asn Phe Ile Ala Pro Glu Asn Ala Tyr Lys Ile Val Lys Lys
               260                 265                 270
   Gly Asp Ser Thr Ile Met Lys Ser Glu Leu Glu Tyr Gly Asn Cys Asn
           275                 280                 285
   Thr Lys Cys Gln Thr Pro Ile Gly Ala Ile Asn Ser Ser Met Pro Phe
       290                 295                 300
   His Asn Ile His Pro Leu Thr Ile Gly Glu Cys Pro Lys Tyr Val Lys
   305 310 315 320
   Ser Asn Arg Leu Val Leu Ala Thr Xaa Leu Arg Asn Ser Pro Gln Gly
   325 330 335
   Glu Gly Arg Arg Lys Lys Arg Gly Leu Phe Gly Ala Ile Ala Gly Phe
   340 345 350
   Ile Glu Gly Gly Trp Gln Gly Met Val Asp Gly Trp Tyr Gly Tyr His
   355 360 365
   His Ser Asn Glu Gln Gly Ser Gly Tyr Ala Ala Asp Lys Glu Ser Thr
   370 375 380
   Gln Lys Ala Ile Asp Gly Val Thr Asn Lys Val Asn Ser Ile Ile Asp
   385 390 395 400
   Lys Met Asn Thr Gln Phe Glu Ala Val Gly Arg Glu Phe Asn Asn Leu
   405 410 415
   Glu Lys Arg Ile Glu Asn Leu Asn Lys Lys Met Glu Asp Gly Phe Leu
   420 425 430
   Asp Val Trp Thr Tyr Asn Ala Glu Leu Leu Val Leu Met Glu Asn Glu
   435 440 445
   Arg Thr Leu Asp Phe His Asp Ser Asn Val Lys Asn Leu Tyr Asp Lys
   450 455 460
   Val Arg Leu Gln Leu Arg Asp Asn Ala Lys Glu Leu Gly Asn Gly Cys
   465 470 475 480
   Phe Glu Phe Tyr His Arg Cys Asp Asn Glu Cys Met Glu Ser Val Arg
   485 490 495
   Asn Gly Thr Tyr Asp Tyr Pro Gln Tyr Ser Glu Glu Ala Arg Leu Lys
   500 505 510
   Arg Glu Glu Ile Ser Gly Val Lys Leu Glu Ser Ile Gly Thr Tyr Gln
   515 520 525
   Ile Leu Ser Ile Tyr Ser Thr Val Ala Gly Ser Leu Ala Leu Ala Ile
   530 535 540
   Met Val Xaa
   545
<210> 33
   <211> 559
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<400> 33
   Xaa Leu Leu Leu Ala Ile Val Ser Leu Val Lys Ser Asp Gln Ile Cys
   1               5                   10                  15
   Ile Gly Tyr His Ala Asn Asn Ser Thr Glu Gln Val Asp Thr Ile Met
               20                  25                  30
   Glu Lys Asn Val Thr Val Thr His Ala Gln Asp Ile Leu Glu Lys Thr
           35                  40                  45
   His Asn Gly Lys Leu Cys Asp Leu Asp Gly Val Lys Pro Leu Ile Leu
       50                  55                  60
   Arg Asp Cys Ser Val Ala Gly Trp Leu Leu Gly Asn Pro Met Cys Asp
   65                  70                  75                  80
   Glu Phe Pro Asn Val Ser Glu Trp Ser Tyr Ile Val Glu Lys Ile Asn
                   85                  90                  95
   Pro Ala Asn Asp Leu Cys Tyr Pro Gly Asn Phe Asn Asn Tyr Glu Glu
               100                 105                 110
   Leu Lys His Leu Leu Ser Arg Ile Asn Arg Phe Glu Lys Ile Gln Ile
           115                 120                 125
   Ile Pro Lys Ser Ser Trp Pro Asp His Glu Ala Ser Leu Gly Val Ser
       130                 135                 140
   Ser Ala Cys Pro Tyr Gln Gly Glu Pro Ser Phe Tyr Arg Asn Val Val
   145                 150                 155                 160
   Trp Leu Ile Lys Lys Asn Asn Thr Tyr Pro Thr Ile Lys Glu Asn Tyr
                   165                 170                 175
   His Asn Thr Asn Gln Glu Asp Leu Leu Val Leu Trp Gly Ile His His
   180 185 190
   Pro Asn Asp Glu Glu Glu Gln Lys Arg Ile Tyr Lys Asn Pro Thr Thr
   195 200 205
   Tyr Ile Ser Val Gly Thr Ser Thr Leu Thr Gln Arg Leu Val Pro Lys
   210 215 220
   Ile Ala Thr Arg Pro Lys Val Asn Gly Gln Ser Gly Arg Val Glu Phe
   225 230 235 240
   Phe Trp Thr Ile Leu Lys Ser Asn Asp Thr Ile Asn Phe Glu Ser Asn
   245 250 255
   Gly Asn Phe Ile Ala Pro Lys Asn Ala Tyr Lys Ile Val Lys Lys Gly
   260 265 270
   Ser Ser Thr Ile Met Lys Ser Glu Leu Glu Tyr Gly Asn Cys Ser Thr
   275 280 285
   Lys Cys Gln Thr Pro Ile Gly Ala Ile Asn Ser Ser Met Pro Phe His
   290 295 300
   Asn Ile His Pro Leu Thr Ile Gly Glu Cys Pro Lys Tyr Val Lys Ser
   305 310 315 320
   Asn Arg Leu Val Leu Ala Thr Gly Leu Arg Asn Ser Pro Gln Gly Glu
   325 330 335
   Gly Arg Arg Lys Lys Arg Gly Leu Phe Gly Ala Ile Ala Gly Phe Ile
   340 345 350
   Glu Gly Gly Trp Gln Gly Met Val Asp Gly Trp Tyr Gly Tyr His His
   355 360 365
   Ser Asn Glu Gln Gly Thr Gly Tyr Ala Ala Asp Lys Glu Ser Thr Gln
   370 375 380
   Lys Ala Ile Asp Gly Val Thr Asn Lys Val Asn Ser Ile Ile Asp Lys
   385 390 395 400
   Met Asn Thr Gln Phe Glu Ala Val Gly Arg Glu Phe Asn Asn Leu Glu
   405 410 415
   Lys Arg Ile Glu Asn Leu Asn Lys Lys Met Glu Asp Gly Phe Leu Asp
   420 425 430
   Val Trp Thr Tyr Asn Ala Glu Leu Leu Val Leu Met Glu Asn Glu Arg
           435                 440                 445
   Thr Leu Asp Phe His Asp Ser Asn Val Arg Asn Leu Tyr Asp Lys Val
       450                 455                 460
   Arg Leu Gln Leu Arg Asp Asn Ala Lys Glu Leu Gly Asn Gly Cys Phe
   465                 470                 475                 480
   Glu Phe Tyr His Lys Cys Asp Asn Glu Cys Met Glu Ser Val Arg Asn
                   485                 490                 495
   Gly Thr Tyr Asp Tyr Pro Gln Tyr Ser Lys Glu Ala Arg Leu Lys Arg
               500                 505                 510
   Glu Glu Ile Ser Gly Val Lys Leu Glu Ser Ile Gly Thr Tyr Gln Ile
           515                 520                 525
   Leu Ser Ile Tyr Ser Thr Val Ala Ser Ser Leu Ala Leu Ala Ile Met
       530                 535                 540
   Val Ala Gly Leu Ser Leu Trp Met Cys Ser Asn Gly Ser Leu Gln
   545                 550                 555
<210> 34
   <211> 554
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (554)..(554)
   <223> Xaa can be any naturally occurring amino acid
<400> 34
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asp Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Glu Phe Pro Asn Val Ser Glu Trp
   65                  70                  75                  80
   Ser Tyr Ile Val Glu Lys Ile Asn Pro Ala Asn Asp Leu Cys Tyr Pro
                   85                  90                  95
   Gly Asn Phe Asn Asn Tyr Glu Glu Leu Lys His Leu Leu Ser Arg Ile
               100                 105                 110
   Asn Arg Phe Glu Lys Ile Gln Ile Ile Pro Lys Ser Ser Trp Pro Asp
           115                 120                 125
   His Glu Ala Ser Leu Gly Val Ser Ser Ala Cys Pro Tyr Gln Gly Glu
       130                 135                 140
   Pro Ser Phe Tyr Arg Asn Val Val Trp Leu Ile Lys Lys Asn Asn Thr
   145                 150                 155                 160
   Tyr Pro Thr Ile Lys Glu Ser Tyr His Asn Thr Asn Gln Glu Asp Leu
                   165                 170                 175
   Leu Val Leu Trp Gly Ile His His Pro Asn Asp Glu Glu Glu Gln Lys
               180                 185                 190
   Arg Ile Tyr Lys Asn Pro Thr Thr Tyr Ile Ser Val Gly Thr Ser Thr
           195                 200                 205
   Leu Asn Gln Arg Leu Val Pro Lys Ile Ala Thr Arg Pro Lys Val Asn
       210                 215                 220
   Gly Gln Ser Gly Arg Val Glu Phe Phe Trp Thr Ile Leu Lys Ser Asn
   225                 230                 235                 240
   Asp Thr Ile Asn Phe Glu Ser Asn Gly Asn Phe Ile Ala Pro Lys Asn
                   245                 250                 255
   Ala Tyr Lys Ile Val Lys Lys Gly Ser Ser Thr Ile Met Lys Ser Glu
               260                 265                 270
   Leu Glu Tyr Gly Asn Cys Ser Thr Lys Cys Gln Thr Pro Ile Gly Ala
           275                 280                 285
   Ile Asn Ser Ser Met Pro Phe His Asn Ile His Pro Leu Thr Ile Gly
       290                 295                 300
   Glu Cys Pro Lys Tyr Val Lys Ser Asn Arg Leu Val Leu Ala Thr Gly
   305                 310                 315                 320
   Leu Arg Asn Ser Pro Gln Gly Glu Gly Arg Arg Lys Lys Arg Gly Leu
                   325                 330                 335
   Phe Gly Ala Ile Ala Gly Phe Ile Glu Gly Gly Trp Gln Gly Met Val
               340                 345                 350
   Asp Gly Trp Tyr Gly Tyr His His Ser Asn Glu Gln Gly Thr Gly Tyr
           355                 360                 365
   Ala Ala Asp Lys Glu Ser Thr Gln Lys Ala Ile Asp Gly Val Thr Asn
       370                 375                 380
   Lys Val Asn Ser Ile Ile Asp Lys Met Asn Thr Gln Phe Glu Ala Val
   385                 390                 395                 400
   Gly Arg Glu Phe Asn Asn Leu Glu Lys Arg Ile Glu Asn Leu Asn Lys
                   405                 410                 415
   Lys Met Glu Asp Gly Phe Leu Asp Val Trp Thr Tyr Asn Ala Glu Leu
               420                 425                 430
   Leu Val Leu Met Glu Asn Glu Arg Thr Leu Asp Phe His Asp Ser Asn
           435                 440                 445
   Val Arg Asn Leu Tyr Asp Lys Val Arg Leu Gln Leu Arg Asp Asn Ala
       450                 455                 460
   Lys Glu Leu Gly Asn Gly Cys Phe Glu Phe Tyr His Lys Cys Asp Asn
   465                 470                 475                 480
   Glu Cys Met Glu Ser Val Arg Asn Gly Thr Tyr Asp Tyr Pro Gln Tyr
                   485                 490                 495
   Ser Lys Glu Ala Arg Leu Lys Arg Glu Glu Ile Ser Gly Val Lys Leu
               500                 505                 510
   Glu Ser Ile Gly Thr Tyr Gln Ile Leu Ser Ile Tyr Ser Thr Val Ala
           515                 520                 525
   Ser Ser Leu Ala Leu Ala Ile Met Val Ala Gly Leu Ser Leu Trp Met
       530                 535                 540
   Cys Ser Asn Gly Ser Leu Gln Cys Arg Xaa
   545 550
<210> 35
   <211> 537
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (537)..(537)
   <223> Xaa can be any naturally occurring amino acid
<400> 35
   Xaa Ser Asp Gln Ile Cys Ile Gly Tyr His Ala Asn Asn Ser Thr Glu
   1 5 10 15
   Gln Val Asp Thr Ile Met Glu Lys Asn Val Thr Val Thr His Ala Gln
   20 25 30
   Asp Ile Leu Glu Lys Thr His Asn Gly Lys Leu Cys Asp Leu Asp Gly
   35 40 45
   Val Lys Pro Leu Ile Leu Arg Asp Cys Ser Val Ala Gly Trp Leu Leu
   50 55 60
   Gly Asn Pro Met Cys Asp Glu Phe Pro Asn Val Ser Glu Trp Ser Tyr
   65 70 75 80
   Ile Val Glu Lys Ile Asn Pro Ala Asn Asp Leu Cys Tyr Pro Gly Asn
   85 90 95
   Phe Asn Asn Tyr Glu Glu Leu Lys His Leu Leu Ser Arg Ile Asn Arg
   100 105 110
   Phe Glu Lys Ile Gln Ile Ile Pro Lys Ser Ser Trp Pro Asp His Glu
   115 120 125
   Ala Ser Leu Gly Val Ser Ser Ala Cys Pro Tyr Gln Gly Glu Pro Ser
   130 135 140
   Phe Tyr Arg Asn Val Val Trp Leu Ile Lys Lys Asn Asn Thr Tyr Pro
   145                 150                 155                 160
   Thr Ile Lys Glu Asn Tyr His Asn Thr Asn Gln Glu Asp Leu Leu Val
                   165                 170                 175
   Leu Trp Gly Ile His His Pro Asn Asp Glu Glu Glu Gln Lys Arg Ile
               180                 185                 190
   Tyr Lys Asn Pro Thr Thr Tyr Ile Ser Val Gly Thr Ser Thr Leu Asn
           195                 200                 205
   Gln Arg Leu Val Pro Lys Ile Ala Thr Arg Pro Lys Val Asn Gly Gln
       210                 215                 220
   Ser Gly Arg Val Glu Phe Phe Trp Thr Ile Leu Lys Ser Asn Asp Thr
   225                 230                 235                 240
   Ile Asn Phe Glu Ser Asn Gly Asn Phe Ile Ala Pro Lys Asn Ala Tyr
                   245                 250                 255
   Lys Ile Val Lys Lys Gly Ser Ser Thr Ile Met Lys Ser Glu Leu Glu
               260                 265                 270
   Tyr Gly Asn Cys Ser Thr Lys Cys Gln Thr Pro Ile Gly Ala Ile Asn
           275                 280                 285
   Ser Ser Met Pro Phe His Asn Ile His Pro Leu Thr Ile Gly Glu Cys
       290                 295                 300
   Pro Lys Tyr Val Lys Ser Asn Arg Leu Val Leu Ala Thr Gly Leu Arg
   305                 310                 315                 320
   Asn Ser Pro Gln Gly Glu Gly Arg Arg Lys Lys Arg Gly Leu Phe Gly
                   325                 330                 335
   Ala Ile Ala Gly Phe Ile Glu Gly Gly Trp Gln Gly Met Val Asp Gly
               340                 345                 350
   Trp Tyr Gly Tyr His His Ser Asn Glu Gln Gly Thr Gly Tyr Ala Ala
           355                 360                 365
   Asp Lys Glu Ser Thr Gln Lys Ala Ile Asp Gly Val Thr Asn Lys Val
       370                 375                 380
   Asn Ser Ile Ile Asp Lys Met Asn Thr Gln Phe Glu Ala Val Gly Arg
   385                 390                 395                 400
   Glu Phe Asn Asn Leu Glu Lys Arg Ile Glu Asn Leu Asn Lys Lys Met
   405 410 415
   Glu Asp Gly Phe Leu Asp Val Trp Thr Tyr Asn Ala Glu Leu Leu Val
   420 425 430
   Leu Met Glu Asn Glu Arg Thr Leu Asp Phe His Asp Ser Asn Val Arg
   435 440 445
   Asn Leu Tyr Asp Lys Val Arg Leu Gln Leu Arg Asp Asn Ala Lys Glu
   450 455 460
   Leu Gly Asn Gly Cys Phe Glu Phe Tyr His Lys Cys Asp Asn Glu Cys
   465 470 475 480
   Met Glu Ser Val Arg Asn Gly Thr Tyr Asp Tyr Pro Gln Tyr Ser Lys
   485 490 495
   Glu Ala Arg Leu Lys Arg Glu Glu Ile Ser Gly Val Lys Leu Glu Ser
   500 505 510
   Ile Gly Thr Tyr Gln Ile Leu Ser Ile Tyr Ser Thr Val Ala Ser Ser
   515 520 525
   Leu Ala Leu Ala Ile Met Val Ala Xaa
   530 535
<210> 36
   <211> 554
   <212> PRT
   <213> Influenza A virus
<220>
   <221> MISC_FEATURE
   <223> subtype H5N1
<400> 36
   Lys Gly Asp Gln Ile Cys Ile Gly Tyr His Ala Asn Asn Ser Thr Glu
   1 5 10 15
   Gln Val Asp Thr Ile Met Glu Lys Asn Val Thr Val Thr His Ala Gln
   20 25 30
   Asp Ile Leu Glu Lys Thr His Asn Gly Lys Leu Cys Asp Leu Asp Gly
   35 40 45
   Val Lys Pro Leu Ile Leu Arg Asp Cys Ser Val Ala Gly Trp Leu Leu
   50 55 60
   Gly Asn Pro Met Cys Asp Glu Phe Pro Asn Val Ser Glu Trp Ser Tyr
   65                  70                  75                  80
   Ile Val Glu Lys Ile Asn Pro Ala Asn Asp Leu Cys Tyr Pro Gly Asn
                   85                  90                  95
   Phe Asn Asn Tyr Glu Glu Leu Lys His Leu Leu Ser Arg Ile Asn Arg
               100                 105                 110
   Phe Glu Lys Ile Lys Ile Ile Pro Lys Ser Ser Trp Pro Asp His Glu
           115                 120                 125
   Ala Ser Leu Gly Val Ser Ser Ala Cys Pro Tyr Gln Gly Gly Pro Ser
       130                 135                 140
   Phe Tyr Arg Asn Val Val Trp Leu Ile Lys Lys Asn Asn Thr Tyr Pro
   145                 150                 155                 160
   Thr Ile Lys Glu Ser Tyr His Asn Thr Asn Gln Glu Asp Leu Leu Val
                   165                 170                 175
   Leu Trp Gly Ile His His Pro Asn Asp Glu Glu Glu Gln Thr Arg Ile
               180                 185                 190
   Tyr Lys Asn Pro Asn Thr Tyr Ile Ser Val Gly Thr Ser Thr Leu Asn
           195                 200                 205
   Gln Arg Leu Val Pro Lys Ile Ala Thr Arg Ser Lys Val Asn Gly Gln
       210                 215                 220
   Ser Gly Arg Val Glu Phe Phe Trp Thr Ile Leu Lys Ser Asn Asp Thr
   225                 230                 235                 240
   Ile Asn Phe Glu Ser Asn Gly Asn Phe Ile Ala Pro Glu Asn Ala Tyr
                   245                 250                 255
   Lys Ile Val Lys Lys Gly Asp Ser Thr Ile Met Lys Ser Glu Leu Glu
               260                 265                 270
   Tyr Gly Asn Cys Ser Thr Lys Cys Gln Thr Pro Ile Gly Ala Ile Asn
           275                 280                 285
   Ser Ser Met Pro Phe His Asn Ile His Pro Leu Thr Ile Gly Glu Cys
       290                 295                 300
   Pro Lys Tyr Val Lys Ser Asn Arg Leu Val Leu Ala Thr Gly Leu Arg
   305                 310                 315                 320
   Asn Ser Pro Gln Glu Glu Gly Arg Arg Lys Lys Arg Gly Leu Phe Gly
                   325                 330                 335
   Ala Ile Ala Gly Phe Ile Glu Gly Gly Trp Gln Gly Met Val Asp Gly
               340                 345                 350
   Trp Tyr Gly Tyr His His Ser Asn Glu Gln Gly Ser Gly Tyr Ala Ala
           355                 360                 365
   Asp Lys Glu Ser Thr Gln Lys Ala Ile Asp Gly Val Thr Asn Lys Val
       370                 375                 380
   Asn Ser Ile Ile Asp Lys Met Asn Thr Gln Phe Glu Ala Val Gly Arg
   385                 390                 395                 400
   Glu Phe Asn Asn Leu Glu Lys Arg Ile Glu Asn Leu Asn Lys Lys Met
                   405                 410                 415
   Glu Asp Gly Phe Leu Asp Val Trp Thr Tyr Asn Ala Glu Leu Leu Val
               420                 425                 430
   Leu Met Glu Asn Glu Arg Thr Leu Asp Phe His Asp Ser Asn Val Lys
           435                 440                 445
   Asn Leu Tyr Asp Lys Val Arg Leu Gln Leu Arg Asp Asn Ala Lys Glu
       450                 455                 460
   Leu Gly Asn Gly Cys Phe Glu Phe Tyr His Arg Cys Asp Asn Glu Cys
   465                 470                 475                 480
   Met Glu Ser Val Arg Asn Gly Thr Tyr Asp Tyr Pro Gln Tyr Ser Glu
                   485                 490                 495
   Glu Ala Arg Leu Lys Arg Glu Glu Ile Ser Gly Val Lys Leu Glu Ser
               500                 505                 510
   Ile Gly Thr Tyr Gln Ile Leu Ser Ile Tyr Ser Thr Val Ala Ser Ser
           515                 520                 525
   Leu Ala Leu Ala Ile Met Val Ala Gly Leu Phe Leu Trp Met Cys Ser
       530                 535                 540
   Asn Gly Ser Leu Gln Cys Arg Ile Cys Ile
   545                 550
<210> 37
   <211> 511
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (182)..(182)
   <223> Xaa can be any naturally occurring amino acid
<220>
   <221> misc_feature
   <222> (200)..(200)
   <223> Xaa can be any naturally occurring amino acid
<220>
   <221> misc_feature
   <222> (511)..(511)
   <223> Xaa can be any naturally occurring amino acid
<400> 37
   Xaa His Ala Asn Asn Ser Thr Glu Gln Val Asp Thr Ile Met Glu Lys
   1               5                   10                  15
   Asn Ile Thr Val Thr His Ala Gln Asp Ile Leu Glu Lys Thr His Asn
               20                  25                  30
   Gly Lys Leu Cys Asp Leu Asp Gly Val Lys Pro Leu Ile Leu Arg Asp
           35                  40                  45
   Cys Ser Val Ala Gly Trp Leu Leu Gly Asn Pro Met Cys Asp Glu Phe
       50                  55                  60
   Leu Asn Val Ser Glu Trp Ser Tyr Ile Val Glu Lys Ile Asn Pro Ala
   65                  70                  75                  80
   Asn Asp Leu Cys Tyr Pro Gly Asn Phe Asn Asn Tyr Glu Glu Leu Lys
                   85                  90                  95
   His Leu Leu Ser Arg Ile Asn Arg Phe Glu Lys Ile Gln Ile Ile Ser
               100                 105                 110
   Lys Asn Ser Trp Pro Asp His Glu Ala Ser Leu Gly Val Ser Ala Ala
           115                 120                 125
   Cys Pro Tyr Gln Gly Gly Leu Ser Phe Tyr Arg Asn Val Val Trp Leu
       130                 135                 140
   Ile Glu Lys Asn Asn Thr Tyr Pro Leu Ile Lys Lys Asn Tyr His Asn
   145                 150                 155                 160
   Thr Asn Gln Glu Asp Leu Leu Val Leu Trp Gly Ile His His Pro Asn
                   165                 170                 175
   Asp Glu Ala Glu Gln Xaa Arg Leu Tyr Lys Asn Ser Thr Thr Tyr Ile
               180                 185                 190
   Ser Val Gly Thr Ser Thr Leu Xaa Gln Arg Leu Val Pro Lys Ile Ala
           195                 200                 205
   Thr Arg Pro Lys Val Asn Gly Gln Ser Gly Arg Val Glu Phe Phe Trp
       210                 215                 220
   Thr Ile Leu Lys Ser Asn Asp Val Ile Asn Phe Glu Ser Asn Gly Asn
   225                 230                 235                 240
   Phe Ile Ala Pro Glu Asn Ala Tyr Lys Ile Val Lys Lys Gly Asp Ser
                   245                 250                 255
   Thr Ile Met Lys Ser Asp Leu Glu Tyr Gly Asn Cys Ser Thr Lys Cys
               260                 265                 270
   Gln Thr Pro Ile Gly Ala Ile Asn Ser Ser Met Pro Phe His Asn Ile
           275                 280                 285
   His Pro Leu Thr Ile Gly Glu Cys Pro Lys Tyr Val Lys Ser Asn Arg
       290                 295                 300
   Leu Val Leu Ala Thr Gly Leu Arg Asn Ser Pro Gln Gly Glu Arg Arg
   305                 310                 315                 320
   Arg Lys Lys Arg Gly Leu Phe Gly Ala Ile Ala Gly Phe Ile Glu Gly
                   325                 330                 335
   Gly Trp Gln Gly Met Val Asp Gly Trp Tyr Gly Tyr His His Ser Asn
               340                 345                 350
   Glu Gln Gly Ser Gly Tyr Ala Ala Asp Lys Glu Ser Thr Gln Lys Ala
           355                 360                 365
   Ile Asp Gly Val Thr Asn Lys Val Asn Ser Ile Ile Asp Lys Met Asn
       370                 375                 380
   Thr Gln Phe Glu Ala Val Gly Arg Glu Phe Asn Asn Leu Glu Arg Arg
   385 390 395 400
   Ile Glu Asn Leu Asn Lys Lys Met Glu Asp Gly Phe Leu Asp Val Trp
   405 410 415
   Thr Tyr Asn Ala Glu Leu Leu Val Leu Met Glu Asn Glu Arg Thr Leu
   420 425 430
   Asp Phe His Asp Ser Asn Val Lys Asn Leu Tyr Asp Lys Val Arg Leu
   435 440 445
   Gln Leu Arg Asp Asn Ala Lys Glu Leu Gly Asn Gly Cys Phe Glu Phe
   450 455 460
   Tyr His Arg Cys Asp Asn Glu Cys Met Glu Ser Val Arg Asn Gly Thr
   465 470 475 480
   Tyr Asp Tyr Pro Gln Tyr Ser Glu Glu Ala Arg Leu Lys Arg Glu Glu
   485 490 495
   Ile Ser Gly Ala Lys Leu Glu Ser Ile Gly Thr Tyr Gln Ile Xaa
   500 505 510
<210> 38
   <211> 537
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<400> 38
   Xaa Leu Val Lys Ser Asp Gln Ile Cys Ile Gly Tyr His Ala Asn Asn
   1 5 10 15
   Ser Thr Glu Gln Val Asp Thr Ile Met Glu Lys Asn Val Thr Val Thr
   20 25 30
   His Ala Gln Asp Ile Leu Glu Lys Thr His Asn Gly Lys Leu Cys Asp
   35 40 45
   Leu Asp Gly Val Lys Pro Leu Ile Leu Arg Asp Cys Ser Val Ala Gly
       50                  55                  60
   Trp Leu Leu Gly Asn Pro Met Cys Asp Glu Phe Pro Asn Val Ser Glu
   65                  70                  75                  80
   Trp Ser Tyr Ile Val Glu Lys Ile Asn Pro Ala Asn Asp Leu Cys Tyr
                   85                  90                  95
   Pro Gly Asn Phe Asn Asn Tyr Glu Glu Leu Lys His Leu Leu Ser Arg
               100                 105                 110
   Ile Asn Arg Phe Glu Lys Ile Lys Ile Ile Pro Lys Ser Ser Trp Pro
           115                 120                 125
   Asp His Glu Ala Ser Leu Gly Val Ser Ser Ala Cys Pro Tyr Gln Arg
       130                 135                 140
   Gly Pro Ser Phe Tyr Arg Asn Val Val Trp Leu Ile Lys Lys Asn Asn
   145                 150                 155                 160
   Thr Tyr Pro Thr Ile Lys Lys Ser Tyr His Asn Thr Asn Gln Glu Asp
                   165                 170                 175
   Leu Leu Val Leu Trp Gly Ile His His Pro Asn Asp Glu Glu Glu Gln
               180                 185                 190
   Thr Arg Ile Tyr Lys Asn Pro Thr Thr Tyr Ile Ser Val Gly Thr Ser
           195                 200                 205
   Thr Leu Asn Gln Arg Leu Val Pro Lys Ile Ala Thr Arg Ser Lys Val
       210                 215                 220
   Asn Gly Gln Ser Gly Arg Val Glu Phe Phe Trp Thr Ile Leu Lys Ser
   225                 230                 235                 240
   Asn Asp Thr Ile Asn Phe Glu Ser Asn Gly Asn Phe Ile Ala Pro Glu
                   245                 250                 255
   Asn Ala Tyr Lys Ile Val Lys Lys Gly Asp Ser Thr Ile Met Lys Ser
               260                 265                 270
   Glu Leu Glu Tyr Gly Asn Cys Asn Thr Lys Cys Gln Thr Pro Ile Gly
           275                 280                 285
   Ala Ile Asn Ser Ser Met Pro Phe His Asn Ile His Pro Leu Thr Ile
       290                 295                 300
   Gly Glu Cys Pro Lys Tyr Val Lys Ser Asn Arg Leu Val Leu Ala Thr
   305 310 315 320
   Gly Leu Arg Asn Ser Pro Gln Gly Glu Gly Arg Arg Lys Lys Arg Gly
   325 330 335
   Leu Phe Gly Ala Ile Ala Gly Phe Ile Glu Gly Gly Trp Gln Gly Met
   340 345 350
   Val Asp Gly Trp Tyr Gly Tyr His His Ser Asn Glu Gln Gly Ser Gly
   355 360 365
   Tyr Ala Ala Asp Lys Glu Ser Thr Gln Lys Ala Ile Asp Gly Val Thr
   370 375 380
   Asn Lys Val Asn Ser Ile Ile Asp Lys Met Asn Thr Gln Phe Glu Ala
   385 390 395 400
   Val Gly Arg Glu Phe Asn Asn Leu Glu Lys Arg Ile Glu Asn Leu Asn
   405 410 415
   Lys Lys Met Glu Asp Gly Phe Leu Asp Val Trp Thr Tyr Asn Ala Glu
   420 425 430
   Leu Leu Val Leu Met Glu Asn Glu Arg Thr Leu Asp Phe His Asp Ser
   435 440 445
   Asn Val Lys Asn Leu Tyr Asp Lys Val Arg Leu Gln Leu Arg Asp Asn
   450 455 460
   Ala Lys Glu Leu Gly Asn Gly Cys Phe Glu Phe Tyr His Arg Cys Asp
   465 470 475 480
   Asn Glu Cys Met Glu Ser Val Lys Asn Gly Thr Tyr Asp Tyr Pro Gln
   485 490 495
   Tyr Ser Glu Glu Ala Arg Leu Lys Arg Glu Glu Ile Ser Gly Val Lys
   500 505 510
   Leu Glu Ser Ile Gly Thr Tyr Gln Ile Leu Ser Ile Tyr Ser Thr Val
   515 520 525
   Ala Ser Ser Leu Ala Leu Ala Ile Met
   530 535
<210> 39
   <211> 534
   <212> PRT
   <213> Influenza A virus
<220>
   <221> MISC_FEATURE
   <223> subtype H5N1
<400> 39
   Lys Ser Asp Gln Ile Cys Ile Gly Tyr His Ala Asn Asn Ser Thr Glu
   1               5                   10                  15
   Gln Val Asp Thr Ile Met Glu Lys Asn Val Thr Val Thr His Ala Gln
               20                  25                  30
   Asp Ile Leu Glu Lys Thr His Asn Gly Lys Leu Cys Asp Leu Asp Gly
           35                  40                  45
   Val Lys Pro Leu Ile Leu Arg Asp Cys Ser Val Ala Gly Trp Leu Leu
       50                  55                  60
   Gly Asn Pro Met Cys Asp Glu Phe Pro Asn Val Ser Glu Trp Ser Tyr
   65                  70                  75                  80
   Ile Val Glu Lys Ile Asn Pro Ala Asn Asp Leu Cys Tyr Pro Gly Asn
                   85                  90                  95
   Phe Asn Asn Tyr Glu Glu Leu Lys His Leu Leu Ser Arg Ile Asn Arg
               100                 105                 110
   Phe Glu Lys Ile Gln Ile Ile Pro Lys Ser Ser Trp Pro Asp His Glu
           115                 120                 125
   Ala Ser Leu Gly Val Ser Ser Ala Cys Pro Tyr Gln Gly Gly Pro Ser
       130                 135                 140
   Phe Tyr Arg Asn Val Val Trp Leu Ile Lys Lys Asn Asp Thr Tyr Pro
   145                 150                 155                 160
   Thr Ile Lys Glu Ser Tyr His Asn Thr Asn Gln Glu Asp Leu Leu Val
                   165                 170                 175
   Leu Trp Gly Ile His His Pro Asn Asp Glu Glu Glu Gln Lys Arg Ile
               180                 185                 190
   Tyr Lys Asn Pro Thr Thr Tyr Ile Ser Val Gly Thr Ser Thr Leu Asn
           195                 200                 205
   Gln Arg Leu Val Pro Lys Ile Ala Thr Arg Ser Lys Val Asn Gly Gln
       210                 215                 220
   Ser Gly Arg Val Glu Phe Phe Trp Thr Ile Leu Lys Ser Asn Asp Thr
   225                 230                 235                 240
   Ile Asn Phe Glu Ser Asn Gly Asn Phe Ile Ala Pro Glu Asn Ala Tyr
                   245                 250                 255
   Lys Ile Val Lys Lys Gly Asp Ser Thr Ile Met Lys Ser Glu Leu Glu
               260                 265                 270
   Tyr Gly Asn Cys Ser Thr Lys Cys Gln Thr Pro Ile Gly Ala Ile Asn
           275                 280                 285
   Thr Ser Met Pro Phe His Asn Ile His Pro Leu Thr Ile Gly Glu Cys
       290                 295                 300
   Pro Lys Tyr Val Lys Ser Asn Arg Leu Val Leu Ala Thr Gly Leu Arg
   305                 310                 315                 320
   Asn Ser Pro Gln Gly Glu Gly Arg Arg Lys Lys Arg Gly Leu Phe Gly
                   325                 330                 335
   Ala Ile Ala Gly Phe Ile Glu Gly Gly Trp Gln Gly Met Val Asp Gly
               340                 345                 350
   Trp Tyr Gly Tyr His His Ser Asn Glu Gln Gly Ser Gly Tyr Ala Ala
           355                 360                 365
   Asp Lys Glu Ser Thr Gln Lys Ala Ile Asn Gly Val Thr Asn Lys Val
       370                 375                 380
   Asn Ser Ile Ile Asp Lys Met Asn Thr Gln Phe Glu Ala Val Gly Arg
   385                 390                 395                 400
   Glu Phe Asn Asn Leu Glu Lys Arg Ile Glu Asn Leu Asn Lys Lys Met
                   405                 410                 415
   Glu Asp Gly Phe Leu Asp Val Trp Thr Tyr Asn Ala Glu Leu Leu Val
               420                 425                 430
   Leu Met Glu Asn Glu Arg Thr Leu Asp Phe His Asp Ser Asn Val Arg
           435                 440                 445
   Asn Leu Tyr Asp Lys Val Arg Leu Gln Leu Arg Asp Asn Ala Lys Glu
       450                 455                 460
   Leu Gly Asn Gly Cys Phe Glu Phe Tyr His Arg Cys Asp Asn Glu Cys
   465 470 475 480
   Met Glu Ser Val Arg Asn Gly Thr Tyr Asp Tyr Pro Gln Tyr Ser Lys
   485 490 495
   Glu Ala Arg Leu Lys Arg Glu Glu Ile Ser Gly Val Lys Leu Glu Ser
   500 505 510
   Ile Gly Thr Tyr Gln Ile Leu Ser Ile Tyr Ser Thr Val Ala Ser Ser
   515 520 525
   Leu Ala Leu Ala Ile Met
   530
<210> 40
   <211> 536
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (311)..(311)
   <223> Xaa can be any naturally occurring amino acid
<400> 40
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asp Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Glu Phe Pro Asn Val Ser Glu Trp
   65                  70                  75                  80
   Ser Tyr Ile Val Glu Lys Thr Asn Pro Ala Asn Asp Leu Cys Tyr Pro
   85 90 95
   Gly Asn Phe Asn Asn Tyr Glu Glu Leu Lys His Leu Leu Ser Arg Ile
   100 105 110
   Asn Arg Phe Glu Lys Ile Lys Ile Ile Pro Lys Ser Ser Trp Pro Asp
   115 120 125
   His Glu Ala Ser Leu Gly Val Ser Ser Ala Cys Pro Tyr Gln Gly Glu
   130 135 140
   Pro Ser Phe Tyr Arg Asn Val Val Trp Leu Ile Lys Lys Asn Asn Thr
   145 150 155 160
   Tyr Pro Thr Ile Lys Glu Ser Tyr His Asn Thr Asn Gln Glu Asp Leu
   165 170 175
   Leu Val Leu Trp Gly Ile His His Pro Asn Asp Glu Glu Glu Gln Thr
   180 185 190
   Arg Ile Tyr Lys Asn Pro Thr Thr Tyr Ile Ser Val Gly Thr Ser Thr
   195 200 205
   Leu Asn Gln Arg Leu Val Pro Lys Ile Ala Thr Arg Ser Lys Val Asn
   210 215 220
   Gly Gln Ser Gly Arg Val Glu Phe Phe Trp Thr Ile Leu Lys Ser Asn
   225 230 235 240
   Asp Thr Ile Asn Phe Glu Ser Asn Gly Asn Phe Ile Ala Pro Glu Asn
   245 250 255
   Ala Tyr Lys Ile Val Lys Lys Gly Asp Ser Thr Ile Met Lys Ser Glu
   260 265 270
   Leu Glu Tyr Gly Asn Cys Ser Thr Lys Cys Gln Thr Pro Val Gly Ala
   275 280 285
   Ile Asn Ser Ser Met Pro Phe His Asn Ile His Pro Leu Thr Ile Gly
   290 295 300
   Glu Cys Pro Lys Tyr Val Xaa Ser Asn Arg Leu Val Leu Ala Thr Gly
   305 310 315 320
   Leu Arg Asn Ser Pro Gln Gly Glu Gly Arg Arg Lys Lys Arg Gly Leu
   325 330 335
   Phe Gly Ala Ile Ala Gly Phe Ile Glu Gly Gly Trp Gln Gly Met Val
   340 345 350
   Asp Gly Trp Tyr Gly Tyr His His Ser Asn Glu Gln Gly Ser Gly Tyr
   355 360 365
   Ala Ala Asp Arg Glu Ser Thr Gln Lys Ala Ile Asp Gly Val Thr Asn
   370 375 380
   Lys Val Asn Ser Ile Ile Asp Lys Met Asn Thr Gln Phe Glu Ala Val
   385 390 395 400
   Gly Arg Glu Phe Asn Asn Leu Glu Lys Arg Ile Glu Asn Leu Asn Lys
   405 410 415
   Lys Met Glu Asp Gly Phe Leu Asp Val Trp Thr Tyr Asn Ala Glu Leu
   420 425 430
   Leu Val Leu Met Glu Asn Glu Arg Thr Leu Asp Phe His Asp Ser Asn
   435 440 445
   Val Lys Asn Leu Tyr Asp Lys Val Arg Leu Gln Leu Arg Asp Asn Ala
   450 455 460
   Lys Glu Leu Gly Asn Gly Cys Phe Glu Phe Tyr His Arg Cys Asp Asn
   465 470 475 480
   Glu Cys Ile Glu Ser Val Arg Asn Gly Thr Tyr Asp Tyr Pro Gln Tyr
   485 490 495
   Ser Glu Glu Ala Arg Leu Lys Arg Glu Glu Ile Ser Gly Val Lys Leu
   500 505 510
   Glu Ser Ile Gly Thr Tyr Gln Ile Leu Ser Ile Tyr Ser Thr Val Ala
   515 520 525
   Ser Ser Leu Ala Leu Ala Ile Met
   530 535
<210> 41
   <211> 515
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (515)..(515)
   <223> Xaa can be any naturally occurring amino acid
<400> 41
   Xaa Leu Val Lys Ser Asp Gln Ile Cys Ile Gly Tyr His Ala Asn Asn
   1               5                   10                  15
   Ser Thr Glu Gln Val Asp Thr Ile Met Glu Lys Asn Val Thr Val Thr
               20                  25                  30
   His Ala Gln Asp Ile Leu Glu Lys Thr His Asn Gly Lys Leu Cys Asp
           35                  40                  45
   Leu Asp Gly Val Lys Pro Leu Ile Leu Arg Asp Cys Ser Val Ala Gly
       50                  55                  60
   Trp Leu Leu Gly Asn Pro Met Cys Asp Glu Phe Pro Asn Val Ser Glu
   65                  70                  75                  80
   Trp Ser Tyr Ile Val Glu Lys Ile Asn Pro Ala Asn Asp Leu Cys Tyr
                   85                  90                  95
   Pro Gly Asn Phe Asn Asn Tyr Glu Glu Leu Lys His Leu Leu Ser Arg
               100                 105                 110
   Ile Asn Arg Phe Glu Lys Ile Gln Ile Ile Pro Lys Ser Ser Trp Pro
           115                 120                 125
   Asp His Glu Ala Ser Leu Gly Val Ser Ser Ala Cys Pro Tyr Gln Gly
       130                 135                 140
   Gly Pro Ser Phe Tyr Arg Asn Val Val Trp Leu Ile Lys Lys Asn Asp
   145                 150                 155                 160
   Thr Tyr Pro Thr Ile Lys Glu Ser Tyr His Asn Thr Asn Gln Glu Asp
                   165                 170                 175
   Leu Leu Val Leu Trp Gly Ile His His Pro Asn Asp Glu Glu Glu Gln
               180                 185                 190
   Lys Arg Ile Tyr Lys Asn Pro Thr Thr Tyr Val Ser Val Gly Thr Ser
           195                 200                 205
   Thr Leu Asn Gln Arg Leu Val Pro Lys Ile Ala Thr Arg Ser Lys Val
       210                 215                 220
   Asn Gly Gln Ser Gly Arg Val Glu Phe Phe Trp Thr Ile Leu Lys Ser
   225                 230                 235                 240
   Asn Asp Thr Ile Asn Phe Glu Ser Asn Gly Asn Phe Ile Ala Pro Glu
                   245                 250                 255
   Asn Ala Tyr Lys Ile Val Lys Lys Gly Asp Ser Thr Ile Met Lys Ser
               260                 265                 270
   Glu Leu Glu Tyr Gly Asn Cys Ser Thr Lys Cys Gln Thr Pro Ile Gly
           275                 280                 285
   Ala Ile Asn Thr Ser Met Pro Phe His Asn Ile His Pro Leu Thr Ile
       290                 295                 300
   Gly Glu Cys Pro Lys Tyr Val Lys Ser Asn Arg Leu Val Leu Ala Thr
   305                 310                 315                 320
   Gly Leu Arg Asn Ser Pro Gln Gly Glu Gly Arg Arg Lys Lys Arg Gly
                   325                 330                 335
   Leu Phe Gly Ala Ile Ala Gly Phe Ile Glu Gly Gly Trp Gln Gly Met
               340                 345                 350
   Val Asp Gly Trp Tyr Gly Tyr His His Ser Asn Glu Gln Gly Ser Gly
           355                 360                 365
   Tyr Ala Ala Asp Lys Glu Ser Thr Gln Lys Ala Ile Asp Gly Val Thr
       370                 375                 380
   Asn Lys Val Asn Ser Ile Ile Asp Lys Met Asn Thr Gln Phe Glu Ala
   385                 390                 395                 400
   Val Gly Arg Glu Phe Asn Asn Leu Glu Lys Arg Ile Glu Asn Leu Asn
                   405                 410                 415
   Lys Lys Met Glu Asp Gly Phe Leu Asp Val Trp Thr Tyr Asn Ala Glu
               420                 425                 430
   Leu Leu Val Leu Met Glu Asn Glu Arg Thr Leu Asp Phe His Asp Ser
           435                 440                 445
   Asn Val Arg Asn Leu Tyr Asp Lys Val Arg Leu Gln Leu Arg Asp Asn
       450                 455                 460
   Ala Lys Glu Leu Gly Asn Gly Cys Phe Glu Phe Tyr His Arg Cys Asp
   465 470 475 480
   Asn Glu Cys Met Glu Ser Val Arg Asn Gly Thr Tyr Asp Tyr Pro Gln
   485 490 495
   Tyr Ser Glu Glu Ala Arg Leu Lys Arg Glu Glu Ile Ser Gly Val Lys
   500 505 510
   Leu Glu Xaa
   515
<210> 42
   <211> 519
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (517)..(517)
   <223> Xaa can be any naturally occurring amino acid
<400> 42
   Xaa Val Lys Ser Asp Gln Ile Cys Ile Gly Tyr His Ala Asn Asn Ser
   1 5 10 15
   Thr Glu Gln Val Asp Thr Ile Met Glu Lys Asn Val Thr Val Thr His
   20 25 30
   Ala Gln Asp Ile Leu Glu Lys Thr His Asn Gly Lys Leu Cys Asn Leu
   35 40 45
   Asp Gly Val Lys Pro Leu Ile Leu Arg Asp Cys Ser Val Ala Gly Trp
   50 55 60
   Leu Leu Gly Asn Pro Met Cys Asp Lys Phe Leu Asn Val Pro Glu Trp
   65 70 75 80
   Ser Tyr Ile Val Glu Lys Ile Asn Pro Ala Asn Asp Leu Cys Tyr Pro
   85 90 95
   Gly Asp Phe Asn Asp Tyr Glu Glu Leu Lys His Leu Leu Ser Arg Ile
               100                 105                 110
   Asn His Phe Glu Lys Ile Gln Ile Ile Pro Lys Asn Ser Trp Ser Asp
           115                 120                 125
   His Glu Ala Ser Gly Val Ser Ser Ala Cys Pro Tyr Gln Gly Arg Ser
       130                 135                 140
   Ser Phe Phe Arg Asn Val Val Trp Leu Thr Lys Lys Asn Asn Ala Tyr
   145                 150                 155                 160
   Pro Thr Ile Lys Lys Ser Tyr Asn Asn Thr Asn Gln Glu Asp Leu Leu
                   165                 170                 175
   Val Leu Trp Gly Ile His His Pro Asn Asp Ala Ala Glu Gln Thr Met
               180                 185                 190
   Leu Tyr Gln Asn Pro Thr Thr Tyr Val Ser Val Gly Thr Ser Thr Leu
           195                 200                 205
   Asn Gln Arg Leu Val Pro Lys Ile Ala Thr Arg Ser Lys Val Asn Gly
       210                 215                 220
   Gln Ser Gly Arg Met Glu Phe Phe Trp Thr Ile Leu Lys Ser Asn Asp
   225                 230                 235                 240
   Ala Ile Asn Phe Glu Ser Asn Gly Asn Phe Ile Ala Pro Glu Asn Ala
                   245                 250                 255
   Tyr Lys Ile Val Lys Lys Gly Asp Ser Thr Ile Met Lys Ser Glu Leu
               260                 265                 270
   Glu Tyr Gly Asn Cys Asn Thr Lys Cys Gln Thr Pro Ile Gly Ala Ile
           275                 280                 285
   Asn Ser Ser Met Pro Phe His Asn Ile His Pro Leu Thr Ile Gly Glu
       290                 295                 300
   Cys Pro Lys Tyr Val Lys Ser Asn Arg Leu Val Leu Ala Thr Gly Leu
   305                 310                 315                 320
   Arg Asn Ser Pro Gln Gly Glu Arg Arg Arg Lys Lys Arg Gly Leu Phe
                   325                 330                 335
   Gly Ala Ile Ala Gly Phe Ile Glu Gly Gly Trp Gln Gly Met Val Asp
               340                 345                 350
   Gly Trp Tyr Gly Tyr His His Ser Asn Glu Gln Gly Ser Gly Tyr Ala
   355 360 365
   Ala Asp Lys Glu Ser Thr Gln Lys Ala Ile Asp Gly Val Thr Asn Lys
   370 375 380
   Val Asn Ser Ile Ile Asp Lys Met Asn Thr Gln Phe Glu Ala Val Gly
   385 390 395 400
   Arg Glu Phe Asn Asn Leu Glu Arg Arg Ile Glu Asn Leu Asn Lys Lys
   405 410 415
   Met Glu Asp Gly Phe Leu Asp Val Trp Thr Tyr Asn Ala Glu Leu Leu
   420 425 430
   Val Leu Met Glu Asn Glu Arg Thr Leu Asp Phe His Asp Ser Asn Val
   435 440 445
   Lys Asn Leu Tyr Asp Lys Val Arg Leu Gln Leu Arg Asp Asn Ala Lys
   450 455 460
   Glu Leu Gly Asn Gly Cys Phe Glu Phe Tyr His Arg Cys Asp Asn Glu
   465 470 475 480
   Cys Met Glu Ser Val Arg Asn Gly Thr Tyr Asp Tyr Pro Gln Tyr Ser
   485 490 495
   Glu Glu Ala Arg Leu Lys Arg Glu Glu Ile Ser Gly Val Lys Leu Glu
   500 505 510
   Ser Ile Gly Thr Xaa Gln Ile
   515
<210> 43
   <211> 510
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (507)..(507)
   <223> Xaa can be any naturally occurring amino acid
<220>
   <221> misc_feature
   <222> (510)..(510)
   <223> Xaa can be any naturally occurring amino acid
<400> 43
   Xaa His Ala Asn Asn Ser Thr Glu Gln Val Asp Thr Ile Met Glu Lys
   1               5                   10                  15
   Asn Val Thr Val Thr His Ala Gln Asp Ile Leu Glu Lys Thr His Asn
               20                  25                  30
   Gly Lys Leu Cys Asn Leu Asp Gly Val Lys Pro Leu Ile Leu Arg Asp
           35                  40                  45
   Cys Ser Val Ala Gly Trp Leu Leu Gly Asn Pro Met Cys Asp Glu Phe
       50                  55                  60
   Pro Asn Val Leu Glu Trp Ser Tyr Ile Val Glu Lys Ile Asn Pro Ala
   65                  70                  75                  80
   Asn Asp Leu Cys Tyr Pro Gly Asn Phe Asn Asp Tyr Glu Glu Leu Lys
                   85                  90                  95
   His Leu Leu Ser Arg Ile Asn His Phe Glu Lys Ile Gln Ile Ile Pro
               100                 105                 110
   Lys Asn Ser Trp Ser Asp His Glu Ala Ser Gly Val Ser Ser Ala Cys
           115                 120                 125
   Pro Tyr Gln Arg Arg Ser Ser Phe Phe Arg Asn Val Val Trp Leu Thr
       130                 135                 140
   Lys Lys Asn Asn Ala Tyr Pro Thr Ile Lys Lys Ser Tyr Asn Asn Thr
   145                 150                 155                 160
   Asn Gln Glu Asp Leu Leu Val Leu Trp Gly Ile His His Pro Asn Asp
                   165                 170                 175
   Ala Ala Glu Gln Thr Arg Leu Tyr Gln Asn Pro Thr Thr Tyr Ile Ser
               180                 185                 190
   Val Gly Thr Ser Thr Leu Asn Gln Arg Leu Val Pro Lys Ile Ala Thr
           195                 200                 205
   Arg Ser Lys Val Asn Gly Gln Ser Gly Arg Met Glu Phe Phe Trp Thr
       210                 215                 220
   Ile Leu Lys Ser Asn Asp Ala Ile Asn Phe Glu Ser Asn Gly Asn Phe
   225 230 235 240
   Ile Ala Pro Glu Asn Ala Tyr Lys Ile Val Lys Lys Gly Asp Ser Thr
   245 250 255
   Ile Met Lys Ser Glu Leu Glu Tyr Gly Asn Cys Asn Thr Lys Cys Gln
   260 265 270
   Thr Pro Ile Gly Ala Ile Asn Ser Ser Met Pro Phe His Asn Ile His
   275 280 285
   Pro Leu Thr Ile Gly Glu Cys Pro Lys Tyr Val Lys Ser Asn Arg Leu
   290 295 300
   Val Leu Ala Thr Gly Leu Arg Asn Ser Pro Gln Gly Glu Arg Arg Arg
   305 310 315 320
   Lys Lys Arg Gly Leu Phe Gly Ala Ile Ala Gly Phe Ile Glu Gly Gly
   325 330 335
   Trp Gln Gly Met Val Asp Gly Trp Tyr Gly Tyr His His Ser Asn Glu
   340 345 350
   Gln Gly Ser Gly Tyr Ala Ala Asp Lys Glu Ser Thr Gln Lys Ala Ile
   355 360 365
   Asp Gly Val Thr Asn Lys Val Asn Ser Ile Ile Asp Lys Met Asn Thr
   370 375 380
   Gln Phe Glu Ala Val Gly Arg Glu Phe Asn Asn Leu Glu Arg Arg Ile
   385 390 395 400
   Glu Asn Leu Asn Lys Lys Met Glu Asp Gly Phe Leu Asp Val Trp Thr
   405 410 415
   Tyr Asn Ala Glu Leu Leu Val Leu Met Glu Asn Glu Arg Thr Leu Asp
   420 425 430
   Phe His Asp Ser Asn Val Lys Asn Leu Tyr Asp Lys Val Arg Leu Gln
   435 440 445
   Leu Arg Asp Asn Ala Lys Glu Leu Gly Asn Gly Cys Phe Glu Phe Tyr
   450 455 460
   His Arg Cys Asp Asn Glu Cys Met Glu Ser Val Arg Asn Gly Thr Tyr
   465 470 475 480
   Asp Tyr Pro Gln Tyr Ser Glu Glu Ala Arg Leu Lys Arg Glu Glu Ile
   485 490 495
   Ser Gly Val Lys Leu Glu Ser Ile Gly Thr Xaa Gln Ile Xaa
   500 505 510
<210> 44
   <211> 523
   <212> PRT
   <213> Influenza A virus
<220>
   <221> MISC_FEATURE
   <223> subtype H5N1
<220>
   <221> misc_feature
   <222> (523)..(523)
   <223> Xaa can be any naturally occurring amino acid
<400> 44
   His Ala Asn Asn Ser Thr Glu Gln Val Asp Thr Ile Met Glu Lys Asn
   1 5 10 15
   Val Thr Val Thr His Ala Gln Asp Ile Leu Glu Lys Thr His Asn Gly
   20 25 30
   Lys Leu Cys Asp Leu Asp Gly Val Lys Pro Leu Ile Leu Arg Asp Cys
   35 40 45
   Ser Val Ala Gly Trp Leu Leu Gly Asn Pro Met Cys Asp Glu Phe Pro
   50 55 60
   Asn Val Ser Glu Trp Ser Tyr Ile Val Glu Lys Ile Asn Pro Ala Asn
   65 70 75 80
   Asp Leu Cys Tyr Pro Gly Asn Phe Asn Asn Tyr Glu Glu Leu Lys His
   85 90 95
   Leu Leu Ser Arg Ile Asn Arg Phe Glu Lys Ile Lys Ile Ile Pro Lys
   100 105 110
   Ser Ser Trp Pro Asp His Glu Ala Ser Leu Gly Val Ser Ser Ala Cys
   115 120 125
   Pro Tyr Gln Gly Gly Pro Ser Phe Tyr Arg Asn Val Val Trp Leu Ile
   130 135 140
   Lys Lys Asn Asn Thr Tyr Pro Thr Ile Lys Lys Ser Tyr His Asn Thr
   145                 150                 155                 160
   Asn Gln Glu Asp Leu Leu Val Leu Trp Gly Ile His His Pro Asn Asp
                   165                 170                 175
   Glu Glu Glu Gln Thr Arg Ile Tyr Lys Asn Pro Thr Thr Tyr Ile Ser
               180                 185                 190
   Val Gly Thr Ser Thr Leu Asn Gln Arg Leu Val Pro Lys Ile Ala Thr
           195                 200                 205
   Arg Ser Lys Val Asn Gly Gln Ser Gly Arg Val Glu Phe Phe Trp Thr
       210                 215                 220
   Ile Leu Lys Ser Asn Asp Thr Ile Asn Phe Glu Ser Asn Gly Asn Phe
   225                 230                 235                 240
   Ile Ala Pro Glu Asn Ala Tyr Lys Ile Val Lys Lys Gly Asp Ser Thr
                   245                 250                 255
   Ile Met Lys Ser Glu Leu Glu Tyr Gly Asn Cys Asn Thr Lys Cys Gln
               260                 265                 270
   Thr Pro Ile Gly Ala Ile Asn Ser Ser Met Pro Phe His Asn Ile His
           275                 280                 285
   Pro Leu Thr Ile Gly Glu Cys Pro Lys Tyr Val Lys Ser Asn Arg Leu
       290                 295                 300
   Val Leu Ala Thr Gly Leu Arg Asn Ser Pro Gln Gly Glu Gly Arg Arg
   305                 310                 315                 320
   Lys Lys Arg Gly Leu Phe Gly Ala Ile Ala Gly Phe Ile Glu Gly Gly
                   325                 330                 335
   Trp Gln Gly Met Val Asp Gly Trp Tyr Gly Tyr His His Ser Asn Glu
               340                 345                 350
   Gln Gly Ser Gly Tyr Ala Ala Asp Lys Glu Ser Thr Gln Lys Ala Ile
           355                 360                 365
   Asp Gly Val Thr Asn Lys Val Asn Ser Ile Ile Asp Lys Met Asn Thr
       370                 375                 380
   Gln Phe Glu Ala Val Gly Arg Glu Phe Asn Asn Leu Glu Lys Arg Ile
   385                 390                 395                 400
   Glu Asn Leu Asn Lys Lys Met Glu Asp Gly Phe Leu Asp Val Trp Thr
   405 410 415
   Tyr Asn Ala Glu Leu Leu Val Leu Met Glu Asn Glu Arg Thr Leu Asp
   420 425 430
   Phe His Asp Ser Asn Val Lys Asn Leu Tyr Asp Lys Val Arg Leu Gln
   435 440 445
   Leu Arg Asp Asn Ala Lys Glu Leu Gly Asn Gly Cys Phe Glu Phe Tyr
   450 455 460
   His Arg Cys Asp Asn Glu Cys Met Glu Ser Val Arg Asn Gly Thr Tyr
   465 470 475 480
   Asp Tyr Pro Gln Tyr Ser Glu Glu Ala Arg Leu Lys Arg Glu Glu Ile
   485 490 495
   Ser Gly Val Lys Leu Glu Ser Ile Gly Thr Tyr Gln Ile Leu Ile Tyr
   500 505 510
   Ser Thr Val Ala Ser Ser Leu Ala Leu Ala Xaa
   515 520
<210> 45
   <211> 541
   <212> PRT
   <213> Influenza A virus
<220>
   <221> MISC_FEATURE
   <223> subtype H5N1
<400> 45
   Leu Leu Ala Ile Val Ser Leu Val Lys Ser Asp Gln Ile Cys Ile Gly
   1 5 10 15
   Tyr His Ala Asn Asn Ser Thr Glu Gln Val Asp Thr Ile Met Glu Lys
   20 25 30
   Asn Val Thr Val Thr His Ala Gln Asp Ile Leu Glu Lys Thr His Asn
   35 40 45
   Gly Lys Leu Cys Asp Leu Asp Gly Val Lys Pro Leu Ile Leu Arg Asp
   50 55 60
   Cys Ser Val Ala Gly Trp Leu Leu Gly Asn Pro Met Cys Asp Glu Phe
   65                  70                  75                  80
   Pro Asn Val Ser Glu Trp Ser Tyr Ile Val Glu Lys Ile Asn Pro Ala
                   85                  90                  95
   Asn Asp Leu Cys Tyr Pro Gly Asn Phe Asn Asn Tyr Glu Glu Leu Lys
               100                 105                 110
   His Leu Leu Ser Arg Ile Asn Arg Phe Glu Lys Ile Lys Ile Ile Pro
           115                 120                 125
   Lys Ser Ser Trp Pro Asp His Glu Ala Ser Leu Gly Val Ser Ser Ala
       130                 135                 140
   Cys Pro Tyr Gln Gly Gly Pro Ser Phe Tyr Arg Asn Val Val Trp Leu
   145                 150                 155                 160
   Ile Lys Lys Asn Asn Thr Tyr Pro Thr Ile Lys Glu Ser Tyr His Asn
                   165                 170                 175
   Ile Asn Lys Glu Asp Leu Leu Val Leu Trp Gly Ile His His Pro Asn
               180                 185                 190
   Asp Glu Glu Glu Gln Ile Arg Ile Tyr Lys Asn Pro Thr Thr Tyr Ile
           195                 200                 205
   Ser Val Gly Thr Ser Thr Leu Asn Gln Arg Leu Val Pro Lys Ile Ala
       210                 215                 220
   Thr Arg Ser Lys Val Asn Gly Gln Ser Gly Arg Val Glu Phe Phe Trp
   225                 230                 235                 240
   Thr Ile Leu Lys Ser Asn Asp Thr Ile Asn Phe Glu Ser Asn Gly Asn
                   245                 250                 255
   Phe Ile Ala Pro Glu Asn Ala Tyr Lys Ile Val Lys Lys Gly Asp Ser
               260                 265                 270
   Thr Ile Met Lys Ser Glu Leu Glu Tyr Gly Asn Cys Asn Thr Lys Cys
           275                 280                 285
   Gln Thr Pro Ile Gly Ala Ile Asn Ser Ser Met Pro Phe His Asn Ile
       290                 295                 300
   His Pro Leu Thr Ile Gly Glu Cys Pro Lys Tyr Val Lys Ser Asn Arg
   305                 310                 315                 320
   Leu Val Leu Ala Thr Gly Leu Arg Asn Ser Pro Gln Gly Glu Gly Arg
                   325                 330                 335
   Arg Lys Lys Arg Gly Leu Phe Gly Ala Ile Ala Gly Phe Ile Glu Gly
               340                 345                 350
   Gly Trp Gln Gly Met Val Asp Gly Trp Tyr Gly Tyr His His Ser Asn
           355                 360                 365
   Glu Gln Gly Ser Gly Tyr Ala Ala Asp Lys Glu Ser Thr Gln Lys Ala
       370                 375                 380
   Ile Asp Gly Val Thr Asn Lys Val Asn Ser Ile Ile Asp Lys Met Asn
   385                 390                 395                 400
   Thr Gln Phe Glu Ala Val Gly Arg Glu Phe Asn Asn Leu Glu Lys Arg
                   405                 410                 415
   Ile Glu Asn Leu Asn Lys Lys Met Glu Asp Gly Phe Leu Asp Val Trp
               420                 425                 430
   Thr Tyr Asn Ala Glu Leu Leu Val Leu Met Glu Asn Glu Arg Thr Leu
           435                 440                 445
   Asp Phe His Asp Ser Asn Val Lys Asn Leu Tyr Asp Lys Val Arg Leu
       450                 455                 460
   Gln Leu Arg Asp Asn Ala Lys Glu Leu Gly Asn Gly Cys Phe Glu Phe
   465                 470                 475                 480
   Tyr His Arg Cys Asp Asn Glu Cys Met Glu Ser Val Arg Asn Gly Thr
                   485                 490                 495
   Tyr Asp Tyr Pro Gln Tyr Ser Glu Glu Ala Arg Leu Lys Arg Glu Glu
               500                 505                 510
   Ile Ser Gly Val Lys Leu Glu Ser Ile Gly Thr Tyr Gln Ile Leu Ser
           515                 520                 525
   Ile Tyr Ser Thr Val Ala Ser Ser Leu Ala Leu Ala Ile
       530                 535                 540
<210> 46
   <211> 555
   <212> PRT
   <213> Influenza A virus
<220>
   <221> MISC_FEATURE
   <223> subtype H5N1
<220>
   <221> misc_feature
   <222> (1)..(1)
   <223> Xaa can be any naturally occurring amino acid
<220>
   <221> misc_feature
   <222> (555)..(555)
   <223> Xaa can be any naturally occurring amino acid
<400> 46
   Xaa Leu Ala Ile Val Ser Leu Val Lys Ser Asp Gln Ile Cys Ile Gly
   1               5                   10                  15
   Tyr His Ala Asn Asn Ser Thr Glu Gln Val Asp Thr Ile Met Glu Lys
               20                  25                  30
   Asn Val Thr Val Thr His Ala Gln Asp Ile Leu Glu Lys Thr His Asn
           35                  40                  45
   Gly Lys Leu Cys Asp Leu Asp Gly Val Lys Pro Leu Ile Leu Arg Asp
       50                  55                  60
   Cys Ser Val Ala Gly Trp Leu Leu Gly Asn Pro Met Cys Asp Glu Phe
   65                  70                  75                  80
   Pro Asn Val Ser Glu Trp Ser Tyr Ile Val Glu Lys Ile Asn Pro Ala
                   85                  90                  95
   Asn Asp Leu Cys Tyr Pro Gly Asn Phe Asn Asn Tyr Glu Glu Leu Lys
               100                 105                 110
   His Leu Leu Ser Arg Ile Asn Arg Phe Glu Lys Ile Lys Ile Ile Pro
           115                 120                 125
   Lys Ser Ser Trp Pro Asp His Glu Ala Ser Leu Gly Val Ser Ser Ala
       130                 135                 140
   Cys Pro Tyr Gln Gly Gly Pro Ser Phe Tyr Arg Asn Val Val Trp Leu
   145                 150                 155                 160
   Thr Lys Lys Asn Asn Thr Tyr Pro Thr Ile Lys Lys Ser Tyr His Asn
                   165                 170                 175
   Ile Asn Lys Glu Asp Leu Leu Val Leu Trp Gly Ile His His Pro Asn
               180                 185                 190
   Asp Glu Glu Glu Gln Ile Arg Ile Tyr Lys Asn Pro Thr Thr Tyr Ile
           195                 200                 205
   Ser Val Gly Thr Ser Thr Leu Asn Gln Arg Leu Val Pro Lys Ile Ala
       210                 215                 220
   Thr Arg Ser Lys Val Asn Gly Gln Ser Gly Arg Val Glu Phe Phe Trp
   225                 230                 235                 240
   Thr Ile Leu Lys Ser Asn Asp Thr Ile Asn Phe Glu Ser Asn Gly Asn
                   245                 250                 255
   Phe Ile Ala Pro Glu Asn Ala Tyr Lys Ile Val Lys Lys Gly Asp Ser
               260                 265                 270
   Thr Ile Met Lys Ser Glu Leu Glu Tyr Gly Asn Cys Asn Thr Lys Cys
           275                 280                 285
   Gln Thr Pro Ile Gly Ala Ile Asn Ser Ser Met Pro Phe His Asn Ile
       290                 295                 300
   His Pro Leu Thr Ile Gly Glu Cys Pro Lys Tyr Val Lys Ser Asn Arg
   305                 310                 315                 320
   Leu Val Leu Ala Thr Gly Leu Arg Asn Ser Pro Gln Gly Glu Gly Arg
                   325                 330                 335
   Arg Lys Lys Arg Gly Leu Phe Gly Ala Ile Ala Gly Phe Ile Glu Gly
               340                 345                 350
   Gly Trp Gln Gly Met Val Asp Gly Trp Tyr Gly Tyr His His Ser Asn
           355                 360                 365
   Glu Gln Gly Ser Gly Tyr Ala Ala Asp Lys Glu Ser Thr Gln Lys Ala
       370                 375                 380
   Ile Asp Gly Val Thr Asn Lys Val Asn Ser Ile Ile Asp Lys Met Asn
   385                 390                 395                 400
   Thr Gln Phe Glu Ala Val Gly Arg Glu Phe Asn Asn Leu Glu Lys Arg
                   405                 410                 415
   Ile Glu Asn Leu Asn Lys Lys Met Glu Asp Gly Phe Leu Asp Val Trp
               420                 425                 430
   Thr Tyr Asn Ala Glu Leu Leu Val Leu Met Glu Asn Glu Arg Thr Leu
           435                 440                 445
   Asp Phe His Asp Ser Asn Val Lys Asn Leu Tyr Asp Lys Val Arg Leu
       450                 455                 460
   Gln Leu Arg Asp Asn Ala Lys Glu Leu Gly Asn Gly Cys Phe Glu Phe
   465                 470                 475                 480
   Tyr His Arg Cys Asp Asn Glu Cys Met Glu Ser Val Arg Asn Gly Thr
                   485                 490                 495
   Tyr Asp Tyr Pro Gln Tyr Ser Glu Glu Ala Arg Leu Lys Arg Glu Glu
               500                 505                 510
   Ile Ser Gly Val Lys Leu Glu Ser Ile Gly Thr Tyr Gln Ile Leu Ser
           515                 520                 525
   Ile Tyr Ser Thr Val Ala Ser Ser Leu Ala Leu Ala Ile Met Met Ala
       530                 535                 540
   Gly Leu Phe Leu Trp Met Cys Ser Asn Gly Xaa
   545                 550                 555
<210> 47
   <211> 568
   <212> PRT
   <213> Influenza A virus
<220>
   <221> MISC_FEATURE
   <223> subtype H5N1
<400> 47
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Gln Ile Ile Pro Lys Ser Ser Trp Pro Asp His Glu Ala Ser
       130                 135                 140
   Leu Gly Val Ser Ser Ala Cys Pro Tyr Gln Gly Gly Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Thr Arg Ile Tyr Lys
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Ser Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
           275                 280                 285
   Asn Cys Asn Thr Lys Cys Gln Thr Pro Ile Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Gly Glu Gly Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Lys Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Arg Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Val Ala Gly Leu Phe Leu Trp Met Cys Ser Asn Gly
   545                 550                 555                 560
   Ser Leu Gln Cys Arg Ile Cys Ile
                   565
<210> 48
   <211> 1620
   <212> DNA
   <213> Influenza A virus
<220>
   <221> misc_feature
   <223> subtype H5N1
<400> 48
   caatagtcag tattgttaaa agtgatcaga tttgcattgg ttaccatgca aacaactcga      60
   cagagcaggt tgacacaata atggaaaaga acgtcactgt tacacacgcc caagacatac     120
   tggaaaagac acacaacggg aaactctgca atctagatgg agtgaagcct ctaattttaa     180
   gagattgtag tgtagctgga tggctcctag ggaacccaat gtgcgacgaa ttcctcaatg     240
   tgccggaatg gtcttacata gtggagaaga tcaatccaac caatgacctc tgttatccag     300
   ggaatttcaa cgactatgaa gaactgaaac acctattgag cagaataaac cattttgaga     360
   aaattcagat cattcccaaa aattcttggt cagatcatga agcctcagga gtgagctcag     420
   catgtccata ccagggaaga tcctcctttt ttagaaatgt ggtatggctt accaaaaaga     480
   acaatgcata cccaacaata aagaaaagtt acaataatac caaccaagaa gatcttttgg     540
   tattatgggg gattcaccat ccaaatgatg cggcagagca gacaaggctt tatcaaaacc     600
   caactaccta tatttccgtt gggacatcaa cactaaacca gagattggta cccaaaatag     660
   ctactagatc taaggtaaac gggcaaagtg gaaggatgga gttcttttgg acaattttaa     720
   aatcgaatga tgcaataaac tttgagagta atggaaattt cattgctcca gaaaatgcat     780
   acaaaattgt caagaaaggg gactcaacaa ttatgaaaag tgagttggaa tatggtgact     840
   gcaacaccaa gtgtcagact ccaatagggg cgataaactc cagtatgcca ttccacaaca     900
   tccaccctct caccatcggg gaatgcccca aatatgtgaa atcaaacaga ttagtccttg     960
   ctactgggct cagaaatagc cctcaaggag agagaagaag aaaaaagaga ggactatttg    1020
   gagctatagc aggttttata gagggaggat ggcagggaat ggtagatggt tggtatgggt    1080
   accaccatag caacgagcag gggagtgggt acgctgcaga caaagaatcc actcaaaagg    1140
   caatagatgg agtcaccaat aaggtcaact cgatcattga caaaatgaac actcagtttg    1200
   aggctgttgg gagggaattt aataacttag aaaggagaat agaaaattta aacaagaaga    1260
   tggaagacgg attcctagat gtctggactt ataatgctga acttctggtt ctcatggaaa    1320
   atgagagaac tctagacttt catgactcaa atgtcaagaa cctttatgac aaggtccgac    1380
   tacagcttag ggataatgca aaggagcttg gtaacggttg tttcgagttc tatcacagat    1440
   gtgataatga atgtatggaa agtgtaagaa acggtacgta tgactacccg cagtattcag    1500
   aagaagcaag attaaaaaga gaggaaataa gtggagtaaa attggagtca ataggaactt    1560
   accaaatact gtcaatttat tcaacagtgg cgagctccct agcactggca atcatggtgg    1620
<210> 49
   <211> 1707
   <212> DNA
   <213> Influenza A virus
<220>
   <221> misc_feature
   <223> subtype H5N1
<400> 49
   atggagaaaa tagtgcttct tcttgcaata gtcagtcttg ttaaaagtga tcagatttgc 60
   attggttacc atgcaaacaa ctcaacagag caggttgaca caataatgga aaagaacgtc 120
   actgttacac acgctcaaga catactggaa aagacacaca atgggaaact ctgcgatcta 180
   ggtggagtga agcctctaat tttaagagat tgtagtgtag ctggatggct cctcgggaac 240
   ccaatgtgtg acgaattccc caatgtgtcg gaatggtcct acatagtgga gaagatcaat 300
   ccagccaatg acctctgtta cccagggaat ttcaacaact atgaagaact gaaacatcta 360
   ttgagcagaa taaaccggtt tgagaaaatt cagatcatcc ccaaaagttc ttggccagat 420
   catgaagcct cattaggagt gagctcagca tgtccatacc agggaggacc ctccttttat 480
   agaaatgtgg tatggcttat caaaaagaac gatacatacc caacaataaa ggaaagttac 540
   cataatacca atcaagaaga tcttttggtg ctgtggggga tccaccatcc aaataatgag 600
   gaagaacaga aaaggatcta taaaaaccca actacctatg tttccgttgg gacatcaaca 660
   ctaaaccaga gattggtacc gaagatagcc actagatcta aggtaaacgg gcaaagtgga 720
   agagtggagt tcttttggac aattttaaaa tcaaatgata caataaactt tgagagtaat 780
   ggaaatttca ttgctccaga aaatgcatac aaaattgtca agaaagggga ctcaacaatt 840
   atgaaaagtg agttggaata tggtaactgc agcaccaagt gtcaaactcc aataggggcg 900
   ataaacacca gtatgccatt ccacaacatc caccctctca ccatcgggga atgccccaaa 960
   tatgtgaaat caaacagatt agtccttgct actgggctta gaaatagccc tcaaggagag 1020
   ggaagaagaa aaaagagagg actatttgga gctatagcag gttttataga gggaggatgg 1080
   cagggaatgg tagatggttg gtatgggtac caccatagta acgagcaggg gagtgggtac 1140
   gctgcagaca aagaatccac tcaaaaggca atagatggag tcaccaataa ggtcaactcg 1200
   atcattgaca aaatgaatac tcagtttgag gctgttggga gggaatttaa taacttggaa 1260
   aagagaatag aaaatttaaa caagaagatg gaagacgggt tcctagatgt ctggacttat 1320
   aatgctgaac ttctggttct catggaaaat gagagaactc tagactttca tgactcaaat 1380
   gtcaggaacc tttacgacaa ggtgcgacta cagcttaggg acaatgcaaa ggagcttggt 1440
   aacggttgtt tcgagttcta tcacagatgc gataatgaat gtatggaaag tgtaagaaac 1500
   ggaacgtatg actacccgca gtattcagaa gaagcaagat taaaaagaga ggaaataagt 1560
   ggagtaaaat tggaatcaat aggaacttac caaatactat caatttattc aacagtggca 1620
   agttccctag cactggcaat catggtggct ggtctatttt tatggatgtg ctccaatgga 1680
   tcgttacaat gcagaatttg catttaa 1707
<210> 50
   <211> 1707
   <212> DNA
   <213> Influenza A virus
<220>
   <221> misc_feature
   <223> subtype H5N1
<400> 50
   atggagaaaa tagtgcttct tcttgcaata gtcagtcttg ttaaaagtga tcagatttgc     60
   attggttacc atgcaaacaa ctcaacagag caggttgaca caataatgga aaagaacgtc    120
   actgttacac acgctcaaga catactggaa aagacacaca acgggaaact ctgcgatcta    180
   gatggagtga agcctctaat tttaagagat tgtagtgtag ctggatggct cctcgggaac    240
   ccaatgtgtg acgaattccc caatgtgtcg gaatggtcct acatagtgga gaagatcaat    300
   ccagccaatg acctctgtta cccagggaat ttcaacaact atgaagaact gaaacaccta    360
   ttgagcagaa taaaccggtt tgagaaaatt cagatcatcc ccaaaagttc ttggccagat    420
   catgaagcct cattaggagt gagctcagca tgtccatacc agggaggacc ctccttttat    480
   agaaatgtgg tatggcttat caaaaagaac aatacatacc caacaataaa ggaaagttac    540
   cataatacca atcaagaaga tcttttggtg ctgtggggga ttcaccatcc aaatgatgag    600
   gaagagcaga caaggatcta taaaaaccca actacctata tttccgttgg gacatcaaca    660
   ctaaaccaga gattggtacc aaagatagcc actagatcta aggtaaacgg gcaaagtgga    720
   agagtggagt tcttttggac aattttaaaa tcaaatgata caataaactt tgagagtaat    780
   ggaaatttca ttgctccaga aaatgcatac aaaattgtca agaaagggga ctcaacaatt    840
   atgaaaagtg agttggaata tggtaactgc aacaccaagt gtcaaactcc aataggggcg    900
   ataaactcca gtatgccatt ccacaacatc caccctctca ccatcgggga atgccccaaa    960
   tatgtgaaat caaacagatt agtccttgct actgggctca gaaatagccc tcaaggagag   1020
   ggaagaagaa aaaagagagg actatttgga gctatagcag gttttataga gggaggatgg   1080
   cagggaatgg tagatggttg gtatgggtac caccatagca acgagcaggg gagtgggtac   1140
   gctgcagaca aagaatccac tcaaaaggca atagatggag tcaccaataa ggtcaactcg   1200
   atcattgaca aaatgaatac tcagtttgag gctgttggga gggaatttaa taacttggaa   1260
   aagagaatag aaaatttaaa caagaagatg gaagacgggt tcctagatgt ctggacttat   1320
   aatgctgaac ttctggttct catggaaaat gagagaactc tagactttca tgactcaaat   1380
   gtcaagaacc tttacgacaa ggtgcgacta cagcttaggg acaatgcaaa ggagcttggt 1440
   aacggttgtt tcgagttcta tcacagatgc gataatgaat gtatggaaag tgtaagaaac 1500
   ggaacgtatg actacccgca gtattcagaa gaagcaagat taaaaagaga ggaaataagt 1560
   ggagtaaaat tggaatcaat aggaacttac caaatactat caatttattc aacagtggca 1620
   agttccctag cactggcaat catggtggct ggtctatttt tatggatgtg ctccaatgga 1680
   tcgttacaat gcagaatttg catttaa 1707
<210> 51
   <211> 16
   <212> PRT
   <213> Influenza A virus
<220>
   <221> MISC_FEATURE
   <223> subtype H5N1
<400> 51
   Met Glu Lys Ile Val Leu Leu Leu Ala Ile Val Ser Leu Val Lys Ser
   1 5 10 15
<210> 52
   <211> 532
   <212> PRT
   <213> Influenza A virus
<220>
   <221> MISC_FEATURE
   <223> subtype H5N1
<400> 52
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Thr Asn Asp Leu Cys Tyr Pro Gly Asn Phe Asn
   85 90 95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asp
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val
   530
<210> 53
   <211> 552
   <212> PRT
   <213> Influenza A virus
<220>
   <221> MISC_FEATURE
   <223> subtype H5N1
<400> 53
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Gly Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asn Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 54
   <211> 552
   <212> PRT
   <213> Influenza A virus
<220>
   <221> MISC_FEATURE
   <223> subtype H5N1
<400> 54
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 55
   <211> 42
   <212> PRT
   <213> Influenza A virus
<220>
   <221> MISC_FEATURE
   <223> subtype H5N1
<400> 55
   Ile Gly Thr Tyr Gln Ile Leu Ser Ile Tyr Ser Thr Val Ala Ser Ser
   1               5                   10                  15
   Leu Ala Leu Ala Ile Met Val Ala Gly Leu Phe Leu Trp Met Cys Ser
               20                  25                  30
   Asn Gly Ser Leu Gln Cys Arg Ile Cys Ile
           35                  40
<210> 56
   <211> 4
   <212> PRT
   <213> Influenza A virus
<220>
   <221> MISC_FEATURE
   <223> subtype H5N1
<400> 56
   Ile Met Val Ala
   1
<210> 57
   <211> 4
   <212> PRT
   <213> Influenza A virus
<220>
   <221> MISC_FEATURE
   <223> subtype H5N1
<400> 57
   Ile Met Met Ala
   1
<210> 58
   <211> 42
   <212> PRT
   <213> Influenza A virus
<220>
   <221> MISC_FEATURE
   <223> subtype H5N1
<400> 58
   Ile Gly Thr Tyr Gln Ile Leu Ser Ile Tyr Ser Thr Val Ala Ser Ser
   1               5                   10                  15
   Leu Ala Leu Ala Ile Met Val Ala Gly Leu Phe Leu Trp Met Cys Ser
               20                  25                  30
   Asn Gly Ser Leu Gln Cys Arg Ile Cys Ile
           35                  40
<210> 59
   <211> 42
   <212> **PRT**
   <213> **Influenza A** virus
<220>
   <221> MISC_FEATURE
   <223> subtype H5N1
<400> 59
   Ile Gly Thr Tyr Gln Ile Leu Ser Ile Tyr Ser Thr Val Ala Ser Ser
   1               5                   10                  15
   Leu Ala Leu Ala Ile Met Val Ala Gly Leu Ser Leu Trp Met Cys Ser
               20                  25                  30
   Asn Gly Ser Leu Gln Cys Arg Ile Cys Ile
           35                  40
<210> 60
   <211> 42
   <212> PRT
   <213> Influenza A virus
<220>
   <221> MISC_FEATURE
   <223> subtype H5N1
<400> 60
   Ile Gly Thr Tyr Gln Ile Leu Ser Ile Tyr Ser Thr Val Ala Ser Ser
   1               5                   10                  15
   Leu Ala Leu Ala Ile Met Met Ala Gly Leu Ser Leu Trp Met Cys Ser
               20                  25                  30
   Asn Gly Ser Leu Gln Cys Arg Ile Cys Ile
           35                  40
<210> 61
   <211> 4
   <212> PRT
   <213> Influenza A virus
<220>
   <221> MISC_FEATURE
   <223> subtype H5N1
<400> 61
   Ala Ser Gly Val
   1
<210> 62
   <211> 551
   <212> PRT
   <213> Influenza A virus
<220>
   <221> MISC_FEATURE
   <223> subtype H5N1
<400> 62
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Lys Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Arg Asn Ser Trp Ser Asp His Glu Thr Ser
           115                 120                 125
   Gly Val Ser Ser Ala Cys Gln Tyr Gln Gly Arg Ser Ser Phe Phe Arg
       130                 135                 140
   Asn Val Val Trp Leu Thr Lys Lys Asp Asn Ala Tyr Ser Thr Ile Lys
   145                 150                 155                 160
   Arg Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Glu Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 63
   <211> 551
   <212> PRT
   <213> Influenza A virus
<220>
   <221> MISC_FEATURE
   <223> subtype H5N1
<400> 63
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Gly Ser Trp Ser Asp His Glu Ala Ser
   115 120 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Arg Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 64
   <211> 544
   <212> PRT
   <213> Influenza A virus
<220>
   <221> MISC_FEATURE
   <223> subtype H5N1
<220>
   <221> misc_feature
   <222> (544)..(544)
   <223> Xaa can be any naturally occurring amino acid
<400> 64
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Tyr Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Ser Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asp Asn Ala Tyr Pro Thr Ile Lys
   145                 150                 155                 160
   Lys Ser Tyr Asn Asn Thr Asn Gln Glu Asp Leu Leu Ile Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Xaa
   530 535 540
<210> 65
   <211> 533
   <212> PRT
   <213> Influenza A virus
<220>
   <221> MISC_FEATURE
   <223> subtype H5N1
<220>
   <221> misc_feature
   <222> (533)..(533)
   <223> Xaa can be any naturally occurring amino acid
<400> 65
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asp Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Thr Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Thr Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   His Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Xaa
   530
<210> 66
   <211> 530
   <212> PRT
   <213> Influenza A virus
<220>
   <221> MISC_FEATURE
   <223> subtype H5N1
<400> 66
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
       50                  55                  60
   Pro Met Cys Asp Glu Phe Leu Asn Val Glu Trp Ser Tyr Ile Val Glu
   65                  70                  75                  80
   Lys Ile Asn Pro Thr Asn Asp Leu Cys Tyr Pro Gly Asn Phe Asn Asp
                   85                  90                  95
   Tyr Glu Glu Leu Lys His Leu Leu Ser Arg Ile Asn His Phe Glu Lys
               100                 105                 110
   Ile Gln Ile Ile Pro Lys Asn Tyr Trp Ser Asp His Glu Thr Ser Gly
           115                 120                 125
   Val Ser Ser Ala Cys Pro Tyr Gln Gly Arg Pro Ser Phe Phe Arg Asn
       130                 135                 140
   Val Val Trp Leu Thr Lys Lys Asn Asn Ala Tyr Pro Thr Ile Lys Lys
   145                 150                 155                 160
   Ser Tyr Asn Asn Thr Asn Gln Glu Asp Leu Leu Val Leu Trp Gly Ile
                   165                 170                 175
   His His Pro Asn Asp Ala Ala Glu Gln Thr Arg Leu Tyr Gln Asn Pro
               180                 185                 190
   Thr Thr Tyr Ile Ser Val Gly Thr Ser Thr Leu Asn Gln Arg Leu Val
           195                 200                 205
   Pro Lys Ile Ala Thr Arg Ser Lys Val Asn Gly Gln Ser Gly Arg Met
       210                 215                 220
   Glu Phe Phe Trp Thr Ile Leu Lys Ser Asn Asp Ala Ile Asn Phe Glu
   225                 230                 235                 240
   Ser Asn Gly Asn Phe Ile Ala Pro Glu Asn Ala Tyr Lys Ile Val Lys
                   245                 250                 255
   Lys Gly Asp Ser Thr Ile Met Lys Ser Glu Leu Glu Tyr Gly Asn Cys
               260                 265                 270
   Asn Thr Lys Cys Gln Thr Pro Ile Gly Ala Ile Asn Ser Ser Met Pro
           275                 280                 285
   Phe His Asn Ile His Pro Leu Thr Ile Gly Glu Cys Pro Lys Tyr Val
       290                 295                 300
   Lys Ser Asn Arg Leu Val Leu Ala Thr Gly Leu Arg Asn Ser Pro Gln
   305                 310                 315                 320
   Gly Glu Arg Arg Arg Lys Lys Arg Gly Leu Phe Gly Ala Ile Ala Gly
                   325                 330                 335
   Phe Ile Glu Gly Gly Trp Gln Gly Met Val Asp Gly Trp Tyr Gly Tyr
               340                 345                 350
   His His Ser Asn Glu Gln Gly Ser Gly Tyr Ala Ala Asp Lys Glu Ser
           355                 360                 365
   Thr Gln Lys Ala Ile Asp Gly Val Thr Asn Lys Val Asn Ser Ile Ile
       370                 375                 380
   Asp Lys Met Asn Thr Gln Phe Glu Ala Val Gly Arg Glu Phe Asn Asn
   385                 390                 395                 400
   Leu Glu Arg Arg Ile Glu Asn Leu Asn Lys Lys Met Glu Asp Gly Phe
                   405                 410                 415
   Leu Asp Val Trp Thr Tyr Asn Ala Glu Leu Leu Val Leu Met Glu Asn
               420                 425                 430
   Glu Arg Thr Leu Asp Phe His Asp Ser Asn Val Lys Asn Leu Tyr Asp
           435                 440                 445
   Lys Val Arg Leu Gln Leu Arg Asp Asn Ala Lys Glu Leu Gly Asn Gly
       450                 455                 460
   Cys Phe Glu Phe Tyr His Arg Cys Asp Asn Glu Cys Met Glu Ser Val
   465                 470                 475                 480
   Arg Asn Gly Thr Tyr Asp Tyr Pro Gln Tyr Ser Glu Glu Ala Arg Leu
                   485                 490                 495
   Lys Arg Glu Glu Ile Ser Gly Val Lys Leu Glu Ser Ile Gly Thr Tyr
               500                 505                 510
   Gln Ile Leu Ser Ile Tyr Ser Thr Val Ala Ser Ser Leu Ala Leu Ala
           515                 520                 525
   Ile Met
       530
<210> 67
   <211> 544
   <212> PRT
   <213> Influenza A virus
<220>
   <221> MISC_FEATURE
   <223> subtype H5N1
<220>
   <221> misc_feature
   <222> (544)..(544)
   <223> Xaa can be any naturally occurring amino acid
<400> 67
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Lys Phe Leu Asn Val Pro Glu Trp Ser Tyr Ile Val
   65                  70                  75                  80
   Glu Lys Ile Asn Pro Thr Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asp
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Ala Gly Leu Phe Leu Trp Met Cys Ser Asn Gly Xaa
       530                 535                 540
<210> 68
   <211> 533
   <212> PRT
   <213> Influenza A virus
<220>
   <221> MISC_FEATURE
   <223> subtype H5N1
<220>
   <221> misc_feature
   <222> (533)..(533)
   <223> Xaa can be any naturally occurring amino acid
<400> 68
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Thr Asn Asp Leu Cys Tyr Pro Gly Asn Phe Asn
                   85                  90                  95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asp
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Xaa
   530
<210> 69
   <211> 516
   <212> PRT
   <213> Influenza A virus
<220>
   <221> MISC_FEATURE
   <223> subtype H5N1
<220>
   <221> misc_feature
   <222> (155)..(155)
   <223> Xaa can be any naturally occurring amino acid
<400> 69
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Xaa Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Lys Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu
   515
<210> 70
   <211> 531
   <212> PRT
   <213> Influenza A virus
<220>
   <221> MISC_FEATURE
   <223> subtype H5N1
<220>
   <221> misc_feature
   <222> (531)..(531)
   <223> Xaa can be any naturally occurring amino acid
<400> 70
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Glu Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Xaa
   530
<210> 71
   <211> 520
   <212> PRT
   <213> Influenza A virus
<220>
   <221> MISC_FEATURE
   <223> subtype H5N1
<220>
   <221> misc_feature
   <222> (520)..(520)
   <223> Xaa can be any naturally occurring amino acid
<400> 71
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Asn Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Xaa
           515                 520
<210> 72
   <211> 531
   <212> PRT
   <213> Influenza A virus
<220>
   <221> MISC_FEATURE
   <223> subtype H5N1
<220>
   <221> misc_feature
   <222> (531)..(531)
   <223> Xaa can be any naturally occurring amino acid
<400> 72
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asp Asn Ala Tyr Pro Thr Ile Lys
   145                 150                 155                 160
   Arg Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Xaa
       530
<210> 73
   <211> 552
   <212> PRT
   <213> Influenza A virus
<220>
   <221> MISC_FEATURE
   <223> subtype H5N1
<400> 73
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Gly Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asn Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 74
   <211> 552
   <212> PRT
   <213> Influenza A virus
<220>
   <221> MISC_FEATURE
   <223> subtype H5N1
<220>
   <221> misc_feature
   <222> (359)..(359)
   <223> Xaa can be any naturally occurring amino acid
<400> 74
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Ser Thr Lys Cys Gln Thr Pro Val Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Xaa Gln Gly Ser Gly Tyr Ala Ala Asp Arg
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Ile Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Ile Val Ala Gly Leu Phe Leu Trp Met Cys Ser Asn Gly
   530 535 540
   Ser Leu Gln Cys Arg Ile Cys Ile
   545 550
<210> 75
   <211> 552
   <212> PRT
   <213> Influenza A virus
<220>
   <221> MISC_FEATURE
   <223> subtype H5N1
<400> 75
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Arg Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Ser Leu Gln Cys Arg Ile Cys Ile
   545 550
<210> 76
   <211> 552
   <212> PRT
   <213> Influenza A virus
<220>
   <221> MISC_FEATURE
   <223> subtype H5N1
<400> 76
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Arg Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Met Cys Ser Asn Gly
   530 535 540
   Ser Leu Gln Cys Arg Ile Cys Ile
   545 550
<210> 77
   <211> 552
   <212> PRT
   <213> Influenza A virus
<220>
   <221> MISC_FEATURE
   <223> subtype H5N1
<400> 77
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Thr Ser Ser Leu Ala
           515                 520                 525
   Leu Ala Ile Met Val Ala Gly Leu Ser Leu Trp Met Cys Ser Asn Gly
       530                 535                 540
   Ser Leu Gln Cys Arg Ile Cys Ile
   545                 550
<210> 78
   <211> 540
   <212> PRT
   <213> Influenza A virus
<220>
   <221> MISC_FEATURE
   <223> subtype H5N1
<220>
   <221> misc_feature
   <222> (540)..(540)
   <223> Xaa can be any naturally occurring amino acid
<400> 78
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Arg Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Ile Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Xaa
       530                 535                 540
<210> 79
   <211> 543
   <212> PRT
   <213> Influenza A virus
<220>
   <221> MISC_FEATURE
   <223> subtype H5N1
<220>
   <221> misc_feature
   <222> (543)..(543)
   <223> Xaa can be any naturally occurring amino acid
<400> 79
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
   195 200 205
   Leu Val Pro Lys Ile Ala Thr Arg Pro Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Lys Asn Ala Tyr Lys Ile
   245 250 255
   Val Lys Lys Gly Ser Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Xaa
   530 535 540
<210> 80
   <211> 552
   <212> PRT
   <213> Influenza A virus
<220>
   <221> MISC_FEATURE
   <223> subtype H5N1
<400> 80
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Ile Cys Ile
   545 550
<210> 81
   <211> 540
   <212> PRT
   <213> Influenza A virus
<220>
   <221> MISC_FEATURE
   <223> subtype H5N1
<400> 81
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Gly Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met
   530 535 540
<210> 82
   <211> 545
   <212> PRT
   <213> Influenza A virus
<220>
   <221> MISC_FEATURE
   <223> subtype H5N1
<220>
   <221> misc_feature
   <222> (545)..(545)
   <223> Xaa can be any naturally occurring amino acid
<400> 82
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu His Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Asn Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Xaa
   545
<210> 83
   <211> 547
   <212> PRT
   <213> Influenza A virus
<220>
   <221> MISC_FEATURE
   <223> subtype H5N1
<220>
   <221> misc_feature
   <222> (120)..(120)
   <223> Xaa can be any naturally occurring amino acid
<400> 83
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Xaa Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Gly Ser Leu Ala
           515                 520                 525
   Leu Ala Ile Met Met Ala Gly Leu Phe Leu Trp Met Cys Ser Asn Gly
       530                 535                 540
   Ser Leu Gln
   545
<210> 84
   <211> 534
   <212> PRT
   <213> Influenza A virus
<220>
   <221> MISC_FEATURE
   <223> subtype H5N1
<220>
   <221> misc_feature
   <222> (534)..(534)
   <223> Xaa can be any naturally occurring amino acid
<400> 84
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Glu Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Xaa
   530
<210> 85
   <211> 534
   <212> PRT
   <213> Influenza A virus
<220>
   <221> MISC_FEATURE
   <223> subtype H5N1
<220>
   <221> misc_feature
   <222> (141)..(141)
   <223> Xaa can be any naturally occurring amino acid
<220>
   <221> misc_feature
   <222> (316)..(316)
   <223> Xaa can be any naturally occurring amino acid
<220>
   <221> misc_feature
   <222> (534)..(534)
   <223> Xaa can be any naturally occurring amino acid
<400> 85
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Xaa Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Xaa Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Gly Ser Leu Ala
           515                 520                 525
   Leu Ala Ile Met Val Xaa
       530
<210> 86
   <211> 547
   <212> PRT
   <213> Influenza A virus
<220>
   <221> MISC_FEATURE
   <223> subtype H5N1
<400> 86
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Thr Gln Arg
   195 200 205
   Leu Val Pro Lys Ile Ala Thr Arg Pro Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Lys Asn Ala Tyr Lys Ile
   245 250 255
   Val Lys Lys Gly Ser Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln
   545
<210> 87
   <211> 550
   <212> PRT
   <213> Influenza A virus
<220>
   <221> MISC_FEATURE
   <223> subtype H5N1
<220>
   <221> misc_feature
   <222> (550)..(550)
   <223> Xaa can be any naturally occurring amino acid
<400> 87
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Xaa
   545 550
<210> 88
   <211> 535
   <212> PRT
   <213> Influenza A virus
<220>
   <221> MISC_FEATURE
   <223> subtype H5N1
<220>
   <221> misc_feature
   <222> (535)..(535)
   <223> Xaa can be any naturally occurring amino acid
<400> 88
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Xaa
       530                 535
<210> 89
   <211> 552
   <212> PRT
   <213> Influenza A virus
<220>
   <221> MISC_FEATURE
   <223> subtype H5N1
<400> 89
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Asn Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Glu Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 90
   <211> 517
   <212> PRT
   <213> Influenza A virus
<220>
   <221> MISC_FEATURE
   <223> subtype H5N1
<220>
   <221> misc_feature
   <222> (7)..(7)
   <223> Xaa can be any naturally occurring amino acid
<220>
   <221> misc_feature
   <222> (188)..(188)
   <223> Xaa can be any naturally occurring amino acid
<220>
   <221> misc_feature
   <222> (206)..(206)
   <223> Xaa can be any naturally occurring amino acid
<220>
   <221> misc_feature
   <222> (517)..(517)
   <223> Xaa can be any naturally occurring amino acid
<400> 90
   Asp Gln Ile Cys Ile Gly Xaa His Ala Asn Asn Ser Thr Glu Gln Val
   1               5                   10                  15
   Asp Thr Ile Met Glu Lys Asn Ile Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Ser Lys Asn Ser Trp Pro Asp His Glu Ala Ser
           115                 120                 125
   Leu Gly Val Ser Ala Ala Cys Pro Tyr Gln Gly Gly Leu Ser Phe Tyr
       130                 135                 140
   Arg Asn Val Val Trp Leu Ile Glu Lys Asn Asn Thr Tyr Pro Leu Ile
   145 150 155 160
   Lys Lys Asn Tyr His Asn Thr Asn Gln Glu Asp Leu Leu Val Leu Trp
   165 170 175
   Gly Ile His His Pro Asn Asp Glu Ala Glu Gln Xaa Arg Leu Tyr Lys
   180 185 190
   Asn Ser Thr Thr Tyr Ile Ser Val Gly Thr Ser Thr Leu Xaa Gln Arg
   195 200 205
   Leu Val Pro Lys Ile Ala Thr Arg Pro Lys Val Asn Gly Gln Ser Gly
   210 215 220
   Arg Val Glu Phe Phe Trp Thr Ile Leu Lys Ser Asn Asp Val Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Asp Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Arg Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Arg Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Ala Lys Leu Glu Ser Ile Gly
   500 505 510
   Thr Tyr Gln Ile Xaa
   515
<210> 91
   <211> 532
   <212> PRT
   <213> Influenza A virus
<220>
   <221> MISC_FEATURE
   <223> subtype H5N1
<400> 91
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Arg Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Lys Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met
   530
<210> 92
   <211> 532
   <212> PRT
   <213> Influenza A virus
<220>
   <221> MISC_FEATURE
   <223> subtype H5N1
<400> 92
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asn Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met
   530
<210> 93
   <211> 532
   <212> PRT
   <213> Influenza A virus
<220>
   <221> MISC_FEATURE
   <223> subtype H5N1
<220>
   <221> misc_feature
   <222> (307)..(307)
   <223> Xaa can be any naturally occurring amino acid
<400> 93
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Val Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Xaa Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Arg
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Ile Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met
       530
<210> 94
   <211> 510
   <212> PRT
   <213> Influenza A virus
<220>
   <221> MISC_FEATURE
   <223> subtype H5N1
<220>
   <221> misc_feature
   <222> (510)..(510)
   <223> Xaa can be any naturally occurring amino acid
<400> 94
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Xaa
               500                 505                 510
<210> 95
   <211> 515
   <212> PRT
   <213> Influenza A virus
<220>
   <221> MISC_FEATURE
   <223> subtype H5N1
<220>
   <221> misc_feature
   <222> (513)..(513)
   <223> Xaa can be any naturally occurring amino acid
<400> 95
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Lys Phe Leu Asn Val Pro Glu Trp Ser Tyr Ile Val
   65                  70                  75                  80
   Glu Lys Ile Asn Pro Ala Asn Asp Leu Cys Tyr Pro Gly Asp Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Met Leu Tyr Gln Asn
               180                 185                 190
   Pro Thr Thr Tyr Val Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
           450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Xaa Gln Ile
           515
<210> 96
   <211> 516
   <212> PRT
   <213> Influenza A virus
<220>
   <221> MISC_FEATURE
   <223> subtype H5N1
<220>
   <221> misc_feature
   <222> (7)..(7)
   <223> Xaa can be any naturally occurring amino acid
<220>
   <221> misc_feature
   <222> (513)..(513)
   <223> Xaa can be any naturally occurring amino acid
<220>
   <221> misc_feature
   <222> (516)..(516)
   <223> Xaa can be any naturally occurring amino acid
<400> 96
   Asp Gln Ile Cys Ile Gly Xaa His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Leu Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Arg Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Xaa Gln Ile Xaa
   515
<210> 97
   <211> 530
   <212> PRT
   <213> Influenza A virus
<220>
   <221> MISC_FEATURE
   <223> subtype H5N1
<220>
   <221> misc_feature
   <222> (530)..(530)
   <223> Xaa can be any naturally occurring amino acid
<400> 97
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ile Tyr Ser Thr Val Ala Ser Ser Leu Ala Leu
   515 520 525
   Ala Xaa
   530
<210> 98
   <211> 531
   <212> PRT
   <213> Influenza A virus
<220>
   <221> MISC_FEATURE
   <223> subtype H5N1
<400> 98
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Ile Asn Lys Glu Asp Leu Leu Val Leu Trp
   165 170 175
   Gly Ile His His Pro Asn Asp Glu Glu Glu Gln Ile Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile
   530
<210> 99
   <211> 545
   <212> PRT
   <213> Influenza A virus
<220>
   <221> MISC_FEATURE
   <223> subtype H5N1
<220>
   <221> misc_feature
   <222> (545)..(545)
   <223> Xaa can be any naturally occurring amino acid
<400> 99 Xaa
545
<210> 100
   <211> 551
   <212> PRT
   <213> Influenza A virus
<220>
   <221> MISC_FEATURE
   <223> subtype H5N1
<400> 100
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Lys Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Arg Asn Ser Trp Ser Asp His Glu Thr Ser
           115                 120                 125
   Gly Val Ser Ser Ala Cys Gln Tyr Gln Gly Arg Ser Ser Phe Phe Arg
       130                 135                 140
   Asn Val Val Trp Leu Thr Lys Lys Asp Asn Ala Tyr Ser Thr Ile Lys
   145                 150                 155                 160
   Arg Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Glu Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Ala Gly Leu Phe Leu Trp Met Cys Ser Asn Gly Ser
       530                 535                 540
   Leu Gln Cys Arg Ile Cys Ile
   545                 550
<210> 101
   <211> 551
   <212> PRT
   <213> Influenza A virus
<220>
   <221> MISC_FEATURE
   <223> subtype H5N1
<400> 101
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Gly Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Arg Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Ala Gly Leu Phe Leu Trp Met Cys Ser Asn Gly Ser
       530                 535                 540
   Leu Gln Cys Arg Ile Cys Ile
   545                 550
<210> 102
   <211> 551
   <212> PRT
   <213> Influenza A virus
<220>
   <221> MISC_FEATURE
   <223> subtype H5N1
<220>
   <221> misc_feature
   <222> (544)..(544)
   <223> Xaa can be any naturally occurring amino acid
<400> 102
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Tyr Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Ser Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asp Asn Ala Tyr Pro Thr Ile Lys
   145                 150                 155                 160
   Lys Ser Tyr Asn Asn Thr Asn Gln Glu Asp Leu Leu Ile Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Xaa
   530 535 540
   Leu Gln Cys Arg Ile Cys Ile
   545 550
<210> 103
   <211> 551
   <212> PRT
   <213> Influenza A virus
<220>
   <221> MISC_FEATURE
   <223> subtype H5N1
<220>
   <221> misc_feature
   <222> (533)..(533)
   <223> Xaa can be any naturally occurring amino acid
<400> 103
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asp Val Pro Glu Trp Ser Tyr Ile Val
   65 70 75 80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Thr Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Thr Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   His Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Xaa Gly Leu Phe Leu Trp Met Cys Ser Asn Gly Ser
   530 535 540
   Leu Gln Cys Arg Ile Cys Ile
   545 550
<210> 104
   <211> 550
   <212> PRT
   <213> Influenza A virus
<220>
   <221> MISC_FEATURE
   <223> subtype H5N1
<400> 104
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Glu Trp Ser Tyr Ile Val Glu
   65                  70                  75                  80
   Lys Ile Asn Pro Thr Asn Asp Leu Cys Tyr Pro Gly Asn Phe Asn Asp
                   85                  90                  95
   Tyr Glu Glu Leu Lys His Leu Leu Ser Arg Ile Asn His Phe Glu Lys
               100                 105                 110
   Ile Gln Ile Ile Pro Lys Asn Tyr Trp Ser Asp His Glu Thr Ser Gly
           115                 120                 125
   Val Ser Ser Ala Cys Pro Tyr Gln Gly Arg Pro Ser Phe Phe Arg Asn
       130                 135                 140
   Val Val Trp Leu Thr Lys Lys Asn Asn Ala Tyr Pro Thr Ile Lys Lys
   145                 150                 155                 160
   Ser Tyr Asn Asn Thr Asn Gln Glu Asp Leu Leu Val Leu Trp Gly Ile
                   165                 170                 175
   His His Pro Asn Asp Ala Ala Glu Gln Thr Arg Leu Tyr Gln Asn Pro
               180                 185                 190
   Thr Thr Tyr Ile Ser Val Gly Thr Ser Thr Leu Asn Gln Arg Leu Val
           195                 200                 205
   Pro Lys Ile Ala Thr Arg Ser Lys Val Asn Gly Gln Ser Gly Arg Met
       210                 215                 220
   Glu Phe Phe Trp Thr Ile Leu Lys Ser Asn Asp Ala Ile Asn Phe Glu
   225 230 235 240
   Ser Asn Gly Asn Phe Ile Ala Pro Glu Asn Ala Tyr Lys Ile Val Lys
   245 250 255
   Lys Gly Asp Ser Thr Ile Met Lys Ser Glu Leu Glu Tyr Gly Asn Cys
   260 265 270
   Asn Thr Lys Cys Gln Thr Pro Ile Gly Ala Ile Asn Ser Ser Met Pro
   275 280 285
   Phe His Asn Ile His Pro Leu Thr Ile Gly Glu Cys Pro Lys Tyr Val
   290 295 300
   Lys Ser Asn Arg Leu Val Leu Ala Thr Gly Leu Arg Asn Ser Pro Gln
   305 310 315 320
   Gly Glu Arg Arg Arg Lys Lys Arg Gly Leu Phe Gly Ala Ile Ala Gly
   325 330 335
   Phe Ile Glu Gly Gly Trp Gln Gly Met Val Asp Gly Trp Tyr Gly Tyr
   340 345 350
   His His Ser Asn Glu Gln Gly Ser Gly Tyr Ala Ala Asp Lys Glu Ser
   355 360 365
   Thr Gln Lys Ala Ile Asp Gly Val Thr Asn Lys Val Asn Ser Ile Ile
   370 375 380
   Asp Lys Met Asn Thr Gln Phe Glu Ala Val Gly Arg Glu Phe Asn Asn
   385 390 395 400
   Leu Glu Arg Arg Ile Glu Asn Leu Asn Lys Lys Met Glu Asp Gly Phe
   405 410 415
   Leu Asp Val Trp Thr Tyr Asn Ala Glu Leu Leu Val Leu Met Glu Asn
   420 425 430
   Glu Arg Thr Leu Asp Phe His Asp Ser Asn Val Lys Asn Leu Tyr Asp
   435 440 445
   Lys Val Arg Leu Gln Leu Arg Asp Asn Ala Lys Glu Leu Gly Asn Gly
   450 455 460
   Cys Phe Glu Phe Tyr His Arg Cys Asp Asn Glu Cys Met Glu Ser Val
   465 470 475 480
   Arg Asn Gly Thr Tyr Asp Tyr Pro Gln Tyr Ser Glu Glu Ala Arg Leu
                   485                 490                 495
   Lys Arg Glu Glu Ile Ser Gly Val Lys Leu Glu Ser Ile Gly Thr Tyr
               500                 505                 510
   Gln Ile Leu Ser Ile Tyr Ser Thr Val Ala Ser Ser Leu Ala Leu Ala
           515                 520                 525
   Ile Met Val Ala Gly Leu Phe Leu Trp Met Cys Ser Asn Gly Ser Leu
       530                 535                 540
   Gln Cys Arg Ile Cys Ile
   545                 550
<210> 105
   <211> 551
   <212> PRT
   <213> Influenza A virus
<220>
   <221> MISC_FEATURE
   <223> subtype H5N1
<220>
   <221> misc_feature
   <222> (544)..(544)
   <223> Xaa can be any naturally occurring amino acid
<400> 105
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Lys Phe Leu Asn Val Pro Glu Trp Ser Tyr Ile Val
   65                  70                  75                  80
   Glu Lys Ile Asn Pro Thr Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asp
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
               355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Ala Gly Leu Phe Leu Trp Met Cys Ser Asn Gly Xaa
       530                 535                 540
   Leu Gln Cys Arg Ile Cys Ile
   545                 550
<210> 106
   <211> 551
   <212> PRT
   <213> Influenza A virus
<220>
   <221> MISC_FEATURE
   <223> subtype H5N1
<220>
   <221> misc_feature
   <222> (533)..(533)
   <223> Xaa can be any naturally occurring amino acid
<400> 106
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Thr Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asp
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Xaa Gly Leu Phe Leu Trp Met Cys Ser Asn Gly Ser
       530                 535                 540
   Leu Gln Cys Arg Ile Cys Ile
   545                 550
<210> 107
   <211> 551
   <212> PRT
   <213> Influenza A virus
<220>
   <221> MISC_FEATURE
   <223> subtype H5N1
<220>
   <221> misc_feature
   <222> (155)..(155)
   <223> Xaa can be any naturally occurring amino acid
<400> 107
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Xaa Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Lys Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Ala Gly Leu Phe Leu Trp Met Cys Ser Asn Gly Ser
       530                 535                 540
   Leu Gln Cys Arg Ile Cys Ile
   545                 550
<210> 108
   <211> 551
   <212> PRT
   <213> Influenza A virus
<220>
   <221> MISC_FEATURE
   <223> subtype H5N1
<220>
   <221> misc_feature
   <222> (531)..(531)
   <223> Xaa can be any naturally occurring amino acid
<400> 108
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Glu Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile Gln Gly Met Val Asp Gly Trp Tyr Gly Tyr His
       290                 295                 300
   His Ser Asn Glu Gln Gly Ser Gly Tyr Ala Ala Asp Lys Glu Ser Thr
   305                 310                 315                 320
   Gln Lys Ala Ile Asp Gly Val Thr Asn Lys Val Asn Ser Ile Ile Asp
                   325                 330                 335
   Lys Met Asn Thr Gln Phe Glu Ala Val Gly Arg Glu Phe Asn Asn Leu
               340                 345                 350
   Glu His Pro Leu Thr Ile Gly Glu Cys Pro Lys Tyr Val Lys Ser Asn
           355                 360                 365
   Arg Leu Val Leu Ala Thr Gly Leu Arg Asn Ser Pro Gln Gly Glu Arg
       370                 375                 380
   Arg Arg Lys Lys Arg Gly Leu Phe Gly Ala Ile Ala Gly Phe Ile Glu
   385                 390                 395                 400
   Gly Gly Trp Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Xaa Val Ala Gly Leu Phe Leu Trp Met Cys Ser Asn Gly Ser
       530                 535                 540
   Leu Gln Cys Arg Ile Cys Ile
   545                 550
<210> 109
   <211> 551
   <212> PRT
   <213> Influenza A virus
<220>
   <221> MISC_FEATURE
   <223> subtype H5N1
<220>
   <221> misc_feature
   <222> (520)..(520)
   <223> Xaa can be any naturally occurring amino acid
<400> 109
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
               115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Asn Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Xaa Thr Val Ala Ser Ser Leu Ala Leu
           515                 520                 525
   Ala Ile Met Val Ala Gly Leu Phe Leu Trp Met Cys Ser Asn Gly Ser
       530                 535                 540
   Leu Gln Cys Arg Ile Cys Ile
   545                 550
<210> 110
   <211> 551
   <212> PRT
   <213> Influenza A virus
<220>
   <221> MISC_FEATURE
   <223> subtype H5N1
<220>
   <221> misc_feature
   <222> (531)..(531)
   <223> Xaa can be any naturally occurring amino acid
<400> 110
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asp Asn Ala Tyr Pro Thr Ile Lys
   145                 150                 155                 160
   Arg Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Xaa Val Ala Gly Leu Phe Leu Trp Met Cys Ser Asn Gly Ser
   530 535 540
   Leu Gln Cys Arg Ile Cys Ile
   545 550
<210> 111
   <211> 552
   <212> PRT
   <213> Influenza A virus
<220>
   <221> MISC_FEATURE
   <223> subtype H5N1
<400> 111
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Gly Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asn Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 112
   <211> 552
   <212> PRT
   <213> Influenza A virus
<220>
   <221> MISC_FEATURE
   <223> subtype H5N1
<220>
   <221> misc_feature
   <222> (359)..(359)
   <223> Xaa can be any naturally occurring amino acid
<400> 112
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Thr Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Ser Thr Lys Cys Gln Thr Pro Val Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Xaa Gln Gly Ser Gly Tyr Ala Ala Asp Arg
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Ile Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
   515 520 525
   Leu Ala Ile Ile Val Ala Gly Leu Phe Leu Trp Met Cys Ser Asn Gly
   530 535 540
   Ser Leu Gln Cys Arg Ile Cys Ile
   545 550
<210> 113
   <211> 552
   <212> PRT
   <213> Influenza A virus
<220>
   <221> MISC_FEATURE
   <223> subtype H5N1
<400> 113
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Arg Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Ser Leu Gln Cys Arg Ile Cys Ile
   545 550
<210> 114
   <211> 552
   <212> PRT
   <213> Influenza A virus
<220>
   <221> MISC_FEATURE
   <223> subtype H5N1
<400> 114
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Ile Gly Thr Ser Thr Leu Asn Gln Arg
   195 200 205
   Leu Val Pro Lys Ile Ala Thr Arg Ser Lys Val Asn Gly Gln Arg Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Met Cys Ser Asn Gly
       530                 535                 540
   Ser Leu Gln Cys Arg Ile Cys Ile
   545                 550
<210> 115
   <211> 552
   <212> PRT
   <213> Influenza A virus
<220>
   <221> MISC_FEATURE
   <223> subtype H5N1
<400> 115
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Thr Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Ile Cys Ile
   545 550
<210> 116
   <211> 552
   <212> PRT
   <213> Influenza A virus
<220>
   <221> MISC_FEATURE
   <223> subtype H5N1
<220>
   <221> misc_feature
   <222> (540)..(540)
   <223> Xaa can be any naturally occurring amino acid
<400> 116
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Arg Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Ile Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Xaa Cys Ser Asn Gly
       530                 535                 540
   Ser Leu Gln Cys Arg Ile Cys Ile
   545                 550
<210> 117
   <211> 552
   <212> PRT
   <213> Influenza A virus
<220>
   <221> MISC_FEATURE
   <223> subtype H5N1
<220>
   <221> misc_feature
   <222> (543)..(543)
   <223> Xaa can be any naturally occurring amino acid
<400> 117
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Ser Leu Trp Met Cys Ser Xaa Gly
       530                 535                 540
   Ser Leu Gln Cys Arg Ile Cys Ile
   545                 550
<210> 118
   <211> 552
   <212> PRT
   <213> Influenza A virus
<220>
   <221> MISC_FEATURE
   <223> subtype H5N1
<400> 118
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
                   355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Ser Leu Trp Met Cys Ser Asn Gly
       530                 535                 540
   Ser Leu Gln Cys Arg Ile Cys Ile
   545                 550
<210> 119
   <211> 552
   <212> PRT
   <213> Influenza A virus
<220>
   <221> MISC_FEATURE
   <223> subtype H5N1
<400> 119
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Gly Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Ser Leu Trp Met Cys Ser Asn Gly
       530                 535                 540
   Ser Leu Gln Cys Arg Ile Cys Ile
   545                 550
<210> 120
   <211> 552
   <212> PRT
   <213> Influenza A virus
<220>
   <221> MISC_FEATURE
   <223> subtype H5N1
<220>
   <221> misc_feature
   <222> (545)..(545)
   <223> Xaa can be any naturally occurring amino acid
<400> 120
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu His Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Asn Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Xaa Leu Gln Cys Arg Ile Cys Ile
   545 550
<210> 121
   <211> 552
   <212> PRT
   <213> Influenza A virus
<220>
   <221> MISC_FEATURE
   <223> subtype H5N1
<220>
   <221> misc_feature
   <222> (120)..(120)
   <223> Xaa can be any naturally occurring amino acid
<400> 121
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Xaa Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Gly Ser Leu Ala
   515 520 525
   Leu Ala Ile Met Met Ala Gly Leu Phe Leu Trp Met Cys Ser Asn Gly
   530 535 540
   Ser Leu Gln Cys Arg Ile Cys Ile
   545 550
<210> 122
   <211> 552
   <212> PRT
   <213> Influenza A virus
<220>
   <221> MISC_FEATURE
   <223> subtype H5N1
<220>
   <221> misc_feature
   <222> (534)..(534)
   <223> Xaa can be any naturally occurring amino acid
<400> 122
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Glu Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Xaa Gly Leu Phe Leu Trp Met Cys Ser Asn Gly
       530                 535                 540
   Ser Leu Gln Cys Arg Ile Cys Ile
   545                 550
<210> 123
   <211> 552
   <212> PRT
   <213> Influenza A virus
<220>
   <221> MISC_FEATURE
   <223> subtype H5N1
<220>
   <221> misc_feature
   <222> (141)..(141)
   <223> Xaa can be any naturally occurring amino acid
<220>
   <221> misc_feature
   <222> (316)..(316)
   <223> Xaa can be any naturally occurring amino acid
<220>
   <221> misc_feature
   <222> (534)..(534)
   <223> Xaa can be any naturally occurring amino acid
<400> 123
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Xaa Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Xaa Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Gly Ser Leu Ala
   515 520 525
   Leu Ala Ile Met Val Xaa Gly Leu Phe Leu Trp Met Cys Ser Asn Gly
   530 535 540
   Ser Leu Gln Cys Arg Ile Cys Ile
   545 550
<210> 124
   <211> 552
   <212> PRT
   <213> Influenza A virus
<220>
   <221> MISC_FEATURE
   <223> subtype H5N1
<400> 124
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Thr Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Ser Leu Trp Met Cys Ser Asn Gly
       530                 535                 540
   Ser Leu Gln Cys Arg Ile Cys Ile
   545                 550
<210> 125
   <211> 552
   <212> PRT
   <213> Influenza A virus
<220>
   <221> MISC_FEATURE
   <223> subtype H5N1
<220>
   <221> misc_feature
   <222> (550)..(550)
   <223> Xaa can be any naturally occurring amino acid
<400> 125
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Pro Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Xaa Cys Ile
   545 550
<210> 126
   <211> 552
   <212> PRT
   <213> Influenza A virus
<220>
   <221> MISC_FEATURE
   <223> subtype H5N1
<220>
   <221> misc_feature
   <222> (535)..(535)
   <223> Xaa can be any naturally occurring amino acid
<400> 126
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Xaa Leu Phe Leu Trp Met Cys Ser Asn Gly
   530 535 540
   Ser Leu Gln Cys Arg Ile Cys Ile
   545 550
<210> 127
   <211> 552
   <212> PRT
   <213> Influenza A virus
<220>
   <221> MISC_FEATURE
   <223> subtype H5N1
<400> 127
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Asn Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Glu Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 128
   <211> 552
   <212> PRT
   <213> Influenza A virus
<220>
   <221> MISC_FEATURE
   <223> subtype H5N1
<220>
   <221> misc_feature
   <222> (7)..(7)
   <223> Xaa can be any naturally occurring amino acid
<220>
   <221> misc_feature
   <222> (188)..(188)
   <223> Xaa can be any naturally occurring amino acid
<220>
   <221> misc_feature
   <222> (206)..(206)
   <223> Xaa can be any naturally occurring amino acid
<220>
   <221> misc_feature
   <222> (517)..(517)
   <223> Xaa can be any naturally occurring amino acid
<400> 128
   Asp Gln Ile Cys Ile Gly Xaa His Ala Asn Asn Ser Thr Glu Gln Val
   1               5                   10                  15
   Asp Thr Ile Met Glu Lys Asn Ile Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Ser Lys Asn Ser Trp Pro Asp His Glu Ala Ser
           115                 120                 125
   Leu Gly Val Ser Ala Ala Cys Pro Tyr Gln Gly Gly Leu Ser Phe Tyr
       130                 135                 140
   Arg Asn Val Val Trp Leu Ile Glu Lys Asn Asn Thr Tyr Pro Leu Ile
   145                 150                 155                 160
   Lys Lys Asn Tyr His Asn Thr Asn Gln Glu Asp Leu Leu Val Leu Trp
                   165                 170                 175
   Gly Ile His His Pro Asn Asp Glu Ala Glu Gln Xaa Arg Leu Tyr Lys
               180                 185                 190
   Asn Ser Thr Thr Tyr Ile Ser Val Gly Thr Ser Thr Leu Xaa Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Val Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Asp Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Arg Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Arg Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Ala Lys Leu Glu Ser Ile Gly
               500                 505                 510
   Thr Tyr Gln Ile Xaa Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 129
   <211> 552
   <212> PRT
   <213> Influenza A virus
<220>
   <221> MISC_FEATURE
   <223> subtype H5N1
<400> 129
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Arg Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Lys Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 130
   <211> 552
   <212> PRT
   <213> Influenza A virus
<220>
   <221> MISC_FEATURE
   <223> subtype H5N1
<400> 130
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asn Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 131
   <211> 552
   <212> PRT
   <213> Influenza A virus
<220>
   <221> MISC_FEATURE
   <223> subtype H5N1
<220>
   <221> misc_feature
   <222> (307)..(307)
   <223> Xaa can be any naturally occurring amino acid
<400> 131
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Val Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Xaa Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Arg
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Ile Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 132
   <211> 552
   <212> PRT
   <213> Influenza A virus
<220>
   <221> MISC_FEATURE
   <223> subtype H5N1
<220>
   <221> misc_feature
   <222> (510)..(510)
   <223> Xaa can be any naturally occurring amino acid
<400> 132
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Xaa Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 133
   <211> 551
   <212> PRT
   <213> Influenza A virus
<220>
   <221> MISC_FEATURE
   <223> subtype H5N1
<220>
   <221> misc_feature
   <222> (513)..(513)
   <223> Xaa can be any naturally occurring amino acid
<400> 133
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Lys Phe Leu Asn Val Pro Glu Trp Ser Tyr Ile Val
   65                  70                  75                  80
   Glu Lys Ile Asn Pro Ala Asn Asp Leu Cys Tyr Pro Gly Asp Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Met Leu Tyr Gln Asn
               180                 185                 190
   Pro Thr Thr Tyr Val Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Xaa Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 134
   <211> 551
   <212> PRT
   <213> Influenza A virus
<220>
   <221> MISC_FEATURE
   <223> subtype H5N1
<220>
   <221> misc_feature
   <222> (7)..(7)
   <223> Xaa can be any naturally occurring amino acid
<220>
   <221> misc_feature
   <222> (513)..(513)
   <223> Xaa can be any naturally occurring amino acid
<220>
   <221> misc_feature
   <222> (516)..(516)
   <223> Xaa can be any naturally occurring amino acid
<400> 134
   Asp Gln Ile Cys Ile Gly Xaa His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Leu Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Arg Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Xaa Gln Ile Xaa Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 135
   <211> 551
   <212> PRT
   <213> Influenza A virus
<220>
   <221> MISC_FEATURE
   <223> subtype H5N1
<220>
   <221> misc_feature
   <222> (530)..(530)
   <223> Xaa can be any naturally occurring amino acid
<400> 135
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ile Tyr Ser Thr Val Ala Ser Ser Leu Ala Leu
   515 520 525
   Ala Xaa Met Val Ala Gly Leu Phe Leu Trp Met Cys Ser Asn Gly Ser
   530 535 540
   Leu Gln Cys Arg Ile Cys Ile
   545 550
<210> 136
   <211> 552
   <212> PRT
   <213> Influenza A virus
<220>
   <221> MISC_FEATURE
   <223> subtype H5N1
<400> 136
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Ile Asn Lys Glu Asp Leu Leu Val Leu Trp
                   165                 170                 175
   Gly Ile His His Pro Asn Asp Glu Glu Glu Gln Ile Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 137
   <211> 552
   <212> PRT
   <213> Influenza A virus
<220>
   <221> MISC_FEATURE
   <223> subtype H5N1
<220>
   <221> misc_feature
   <222> (545)..(545)
   <223> Xaa can be any naturally occurring amino acid
<400> 137
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Thr Lys Lys Asn Asn Thr Tyr Pro Thr Ile
   145                 150                 155                 160
   Lys Lys Ser Tyr His Asn Ile Asn Lys Glu Asp Leu Leu Val Leu Trp
                   165                 170                 175
   Gly Ile His His Pro Asn Asp Glu Glu Glu Gln Ile Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
                   500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Met Cys Ser Asn Gly
       530                 535                 540
   Xaa Leu Gln Cys Arg Ile Cys Ile
   545                 550
<210> 138
   <211> 552
   <212> PRT
   <213> Influenza A virus
<220>
   <221> MISC_FEATURE
   <223> subtype H5N1
<400> 138
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 139
   <211> 15186
   <212> DNA
   <213> Newcastle disease virus
<400> 139
   accaaacaga gaatccgtga gttacgataa aaggcgaagg agcaattgaa gtcgcacggg      60
   tagaaggtgt gaatctcgag tgcgagcccg aagcacaaac tcgagaaagc cttctgccaa     120
   catgtcttcc gtatttgatg agtacgaaca gctcctcgcg gctcagactc gccccaatgg     180
   agctcatgga gggggagaaa aagggagtac cttaaaagta gacgtcccgg tattcactct     240
   taacagtgat gacccagaag atagatggag ctttgtggta ttctgcctcc ggattgctgt     300
   tagcgaagat gccaacaaac cactcaggca aggtgctctc atatctcttt tatgctccca     360
   ctcacaggta atgaggaacc atgttgccct tgcagggaaa cagaatgaag ccacattggc     420
   cgtgcttgag attgatggct ttgccaacgg cacgccccag ttcaacaata ggagtggagt     480
   gtctgaagag agagcacaga gatttgcgat gatagcagga tctctccctc gggcatgcag 540
   caacggaacc ccgttcgtca cagccggggc cgaagatgat gcaccagaag acatcaccga 600
   taccctggag aggatcctct ctatccaggc tcaagtatgg gtcacagtag caaaagccat 660
   gactgcgtat gagactgcag atgagtcgga aacaaggcga atcaataagt atatgcagca 720
   aggcagggtc caaaagaaat acatcctcta ccccgtatgc aggagcacaa tccaactcac 780
   gatcagacag tctcttgcag tccgcatctt tttggttagc gagctcaaga gaggccgcaa 840
   cacggcaggt ggtacctcta cttattataa cctggtaggg gacgtagact catacatcag 900
   gaataccggg cttactgcat tcttcttgac actcaagtac ggaatcaaca ccaagacatc 960
   agcccttgca cttagtagcc tctcaggcga catccagaag atgaagcagc tcatgcgttt 1020
   gtatcggatg aaaggagata atgcgccgta catgacatta cttggtgata gtgaccagat 1080
   gagctttgcg cctgccgagt atgcacaact ttactccttt gccatgggta tggcatcagt 1140
   cctagataaa ggtactggga aataccaatt tgccagggac tttatgagca catcattctg 1200
   gagacttgga gtagagtacg ctcaggctca gggaagtagc attaacgagg atatggctgc 1260
   cgagctaaag ctaaccccag cagcaaggag gggcctggca gctgctgccc aacgggtctc 1320
   cgaggagacc agcagcatag acatgcctac tcaacaagtc ggagtcctca ctgggcttag 1380
   cgaggggggg tcccaagctc tacaaggcgg atcgaataga tcgcaagggc aaccagaagc 1440
   cggggatggg gagacccaat tcctggatct gatgagagcg gtagcaaata gcatgaggga 1500
   ggcgccaaac tctgcacagg gcactcccca atcggggcct cccccaactc ctgggccatc 1560
   ccaagataac gacaccgact gggggtattg ataaacaaaa cccagcctgc ttccacaaaa 1620
   acatcccaat gccctcaccc gtagtcgacc cctcgatttg cggctctata tgaccacacc 1680
   ctcaaacaaa catccccctc tttcctccct ccccctgctg tacaactccg cacgccctag 1740
   ataccacagg cacaatgcgg ctcactaaca atcaaaacag agccgaggga attagaaaaa 1800
   agtacgggta gaagagggat attcagagat cagggcaagt ctcccgagtc tctgctctct 1860
   cctctacctg atagaccagg acaaacatgg ccacctttac ggatgcagag atcgacgagc 1920
   tatttgagac aagtggaact gtcattgaca acataattac agcccagggt aaaccagcag 1980
   agactgttgg aaggagtgca atcccacaag gcaagaccaa ggtgctgagc gcagcatggg 2040
   agaagcatgg gagcatccag ccaccggcca gtcaagacaa ccccgatcga caggacagat 2100
   ctgacaaaca accatccaca cccgagcaaa cgaccccgca tgacagcccg ccggccacat 2160
   ccgccgacca gccccccacc caggccacag acgaagccgt cgacacacag ctcaggaccg 2220
   gagcaagcaa ctctctgctg ttgatgcttg acaagctcag caataaatcg tccaatgcta 2280
   aaaagggccc atggtcgagc ccccaagagg ggaatcacca acgtccgact caacagcagg 2340
   ggagtcaacc cagtcgcgga aacagtcagg aaagaccgca gaaccaagtc aaggccgccc 2400
   ctggaaacca gggcacagac gtgaacacag catatcatgg acaatgggag gagtcacaac   2460
   tatcagctgg tgcaacccct catgctctcc gatcaaggca gagccaagac aatacccttg   2520
   tatctgcgga tcatgtccag ccacctgtag actttgtgca agcgatgatg tctatgatgg 2580
   aggcgatatc acagagagta agtaaggttg actatcagct agatcttgtc ttgaaacaga 2640
   catcctccat ccctatgatg cggtccgaaa tccaacagct gaaaacatct gttgcagtca 2700
   tggaagccaa cttgggaatg atgaagattc tggatcccgg ttgtgccaac atttcatctc 2760
   tgagtgatct acgggcagtt gcccgatctc acccggtttt agtttcaggc cctggagacc 2820
   cctctcccta tgtgacacaa ggaggcgaaa tggcacttaa taaactttcg caaccagtgc 2880
   cacatccatc tgaattgatt aaacccgcca ctgcatgcgg gcctgatata ggagtggaaa 2940
   aggacactgt ccgtgcattg atcatgtcac gcccaatgca cccgagttct tcagccaagc 3000
   tcctaagcaa gttagatgca gccgggtcga tcgaggaaat caggaaaatc aagcgccttg 3060
   ctctaaatgg ctaattacta ctgccacacg cagcgggtcc ctgtccactc ggcatcacac 3120
   ggaatctgca ccgagttccc ccccgcagac ccaaggtcca actctccaag cggcaatcct 3180
   ctctcgcttc ctcagcccca ctgaatggac gcgtaaccgt aattaatcta gctacattta 3240
   agattaagaa aaaatacggg tagaattgga gtgccccaat tgtgccaaga tggactcatc 3300
   taggacaatt gggctgtact ttgattctgc ccattcttct agcaacctgt tagcatttcc 3360
   gatcgtccta caaggcacag gagatgggaa gaagcaaatc gccccgcaat ataggatcca 3420
   gcgccttgac ttgtggactg atagtaagga ggactcagta ttcatcacca cctatggatt 3480
   catctttcaa gttgggaatg aagaagccac tgtcggcatg atcgatgata aacccaagcg 3540
   cgagttactt tccgctgcga tgctctgcct aggaagcgtc ccaaataccg gagaccttat 3600
   tgagctggca agggcctgtc tcactatgat agtcacatgc aagaagagtg caactaatac 3660
   tgagagaatg gttttctcag tagtgcaggc accccaagtg ctgcaaagct gtagggttgt 3720
   ggcaaacaaa tactcatcag tgaatgcagt caagcacgtg aaagcgccag agaagattcc 3780
   cgggagtgga accctagaat ataaggtgaa ctttgtctcc ttgactgtgg taccgaagaa 3840
   ggatgtctac aagatcccag ctgcagtatt gaaggtttct ggctcgagtc tgtacaatct 3900
   tgcgctcaat gtcactatta atgtggaggt tgacccgagg agtcctttgg ttaaatctct 3960
   gtctaagtct gacagcggat actatgctaa cctcttcttg catattggac ttatgaccac 4020
   cgtagatagg aaggggaaga aagtgacatt tgacaagctg gaaaagaaaa taaggagcct 4080
   tgatctatct gtcgggctca gtgatgtgct cgggccttcc gtgttggtaa aagcaagagg 4140
   tgcacggact aagcttttgg cacctttctt ctctagcagt gggacagcct gctatcccat 4200
   agcaaatgct tctcctcagg tggccaagat actctggagt caaaccgcgt gcctgcggag 4260
   cgttaaaatc attatccaag caggtaccca acgcgctgtc gcagtgaccg ccgaccacga 4320
   ggttacctct actaagctgg agaaggggca cacccttgcc aaatacaatc cttttaagaa 4380
   ataagctgcg tctctgagat tgcgctccgc ccactcaccc agatcatcat gacacaaaaa 4440
   actaatctgt cttgattatt tacagttagt ttacctgcct atcaagttag aaaaaacacg 4500
   ggtagaagat tctggatccc ggttggcgcc ctccaggtgc aagatgggct ccagaccttc 4560
   taccaagaac ccagcaccta tgatgctgac tatccgggtt gcgctggtac tgagttgcat 4620
   ctgtccggca aactccattg atggcaggcc tcttgcagct gcaggaattg tggttacagg 4680
   agacaaagcc gtcaacatat acacctcatc ccagacagga tcaatcatag ttaagctcct 4740
   cccgaatctg cccaaggata aggaggcatg tgcgaaagcc cccttggatg catacaacag 4800
   gacattgacc actttgctca ccccccttgg tgactctatc cgtaggatac aagagtctgt 4860
   gactacatct ggagggggga gacaggggcg ccttataggc gccattattg gcggtgtggc 4920
   tcttggggtt gcaactgccg cacaaataac agcggccgca gctctgatac aagccaaaca 4980
   aaatgctgcc aacatcctcc gacttaaaga gagcattgcc gcaaccaatg aggctgtgca 5040
   tgaggtcact gacggattat cgcaactagc agtggcagtt gggaagatgc agcagtttgt 5100
   taatgaccaa cttaataaaa cagctcagga attagactgc atcaaaattg cacagcaagt 5160
   tggtgtagag ctcaacctgt acctaaccga attgactaca gtattcggac cacaaatcac 5220
   ttcacctgct ttaaacaagc tgactattca ggcactttac aatctagctg gtggaaatat 5280
   ggattactta ttgactaagt taggtgtagg gaacaatcaa ctcagctcat taatcggtag 5340
   cggcttaatc accggtaacc ctattctata cgactcacag actcaactct tgggtatacg 5400
   ggtaactcta ccttcagtcg ggaacctaaa taatatgcgt gccacctact tggaaacctt 5460
   atccgtaagc acaaccaggg gatttgcctc ggcacttgtc cccaaagtgg tgacacaggt 5520
   cggttctgtg atagaagaac ttgacacctc atactgtata gaaactgact tagatttata 5580
   ttgtacaaga atagtaacgt tccctatgtc ccctggtatt tattcctgct tgagcggcaa 5640
   tacgtcggcc tgtatgtact caaagaccga aggcgcactt actacaccat acatgactat 5700
   caaaggttca gtcatcgcca actgcaagat gacaacatgt agatgtgtaa accccccggg 5760
   tatcatatcg caaaactatg gagaagccgt gtctctaata gataaacaat catgcaatgt 5820
   tttatcctta ggcgggataa ctttaaggct cagtggggaa ttcgatgtaa cttatcagaa 5880
   gaatatctca atacaagatt ctcaagtaat aataacaggc aatcttgata tctcaactga 5940
   gcttgggaat gtcaacaact cgatcagtaa tgctttgaat aagttagagg aaagcaacag 6000
   aaaactagac aaagtcaatg tcaaactgac tagcacatct gctctcatta cctatatcgt 6060
   tttgactatc atatctcttg tttttggtat acttagcctg attctagcat gctacctaat 6120
   gtacaagcaa aaggcgcaac aaaagacctt attatggctt gggaataata ctctagatca 6180
   gatgagagcc actacaaaaa tgtgaacaca gatgaggaac gaaggtttcc ctaatagtaa 6240
   tttgtgtgaa agttctggta gtctgtcagt tcagagggtt aagaaaaaac taccggttgt 6300
   agatgaccaa aggacgatat acgggtagaa cggtaagaga ggccgcccct caattgcgag 6360
   ccaggcttca caacctccgt tctaccgctt caccgacaac ggtcctcaat catggaccgc 6420
   gccgttagcc aagttgcgtt agagaatgat gaaagagagg caaaaaatac atggcgcttg 6480
   atattccgga ttgcaatctt attcttaaca gtagtgacct tggctatatc tgtagcctcc 6540
   cttttatata gcatgggggc tagcacacct agcgatcttg taggcatacc gactaggaat 6600
   tccagggcag aagaaaagat tacatctaca cttggttcca atcaagatgt agtagatagg 6660
   atatataagc aagtggccct tgagtctccg ttggcattgt taaaaactga gaccacaatt 6720
   atgaacgcaa taacatctct ctcttatcag attaatggag ctgcaaacaa cagtgggtgg 6780
   ggggcactta tccatgaccc agattatata ggggggatag gcaaagaact cattgtagat 6840
   gatgctagtg atgtcacatc attctatccc tctgcatttc aagaacatct gaattttatc 6900
   ccggcgccta ctacaggatc aggttgcact cgaataccct catttgacat gagtgctacc 6960
   cattactgct acacccataa tgtaatattg tctggatgca gagatcactc acattcatat 7020
   cagtatttag cacttggtgt gctccggaca tctgcaacag ggagggtatt cttttctact 7080
   ctgcgttcca tcaacctgga cgacacccaa aatcggaagt cttgcagtgt gagtgcaact 7140
   cccctgggtt gtgatatgct gtgctcgaaa gtcacggaga cagaggaaga agattataac 7200
   tcagctgtcc ctacgcggat ggtacatggg aggttagggt tcgacggcca gtaccacgaa 7260
   aaggacctag atgtcacaac attattcggg gactgggtgg ccaactaccc aggagtaggg 7320
   ggtggatctt ttattgacag ccgcgtatgg ttctcagtct acggagggtt aaaacccaat 7380
   tcacccagtg acactgtaca ggaagggaaa tatgtgatat acaagcgata caatgacaca 7440
   tgcccagatg agcaagacta ccagattcga atggccaggt cttcgtataa gcctggacgg 7500
   tttggtggga aacgcataca gcaggctatc ttatctatca aggtgtcaac atccttaggc 7560
   gaagacccgg tactgactgt accgcccaac acagtcacac tcatgggggc cgaaggcaga 7620
   attctcacag tagggacatc tcatttcttg tatcaacgag ggtcatcata cttctctccc 7680
   gcgttattat atcctatgac agtcagcaac aaaacagcca ctcttcatag tccttataca 7740
   ttcaatgcct tcactcggcc aggtagtatc ccttgccagg cttcagcaag atgccccaac 7800
   ccgtgtgtta ctggagtcta tacagatcca catcccctaa tcttctatag aaaccacacc 7860
   ttgcgagggg tattcgggac aatgcttgat ggtgtacaag caagacttaa ccctgcgtct 7920
   gcagtattcg atagcacatc ccgcagtcgc attactcgag tgagttcaag cagtaccaaa 7980
   gcagcataca caacatcaac ttgttttaaa gtggtcaaga ctaataagac ctattgtctc 8040
   agcattgctg aaatatctaa tactctcttc ggagaattca gaatcgtccc gttactagtt 8100
   gagatcctca aagatgacgg ggttagagaa gccaggtctg gctagttgag tcaattataa 8160
   aggagttgga aagatggcat tgtatcacct atcttctgcg acatcaagaa tcaaaccgaa 8220
   tgccggcgcg tgctcgaatt ccatgttgcc agttgaccac aatcagccag tgctcatgcg 8280
   atcagattaa gccttgtcaa tagtctcttg attaagaaaa aatgtaagtg gcaatgagat 8340
   acaaggcaaa acagctcatg gtaaataata cgggtagaac atggcgagct ccggtcctga 8400
   aagggcagag catcagatta tcctaccaga gtcacacctg tcttcaccat tggtcaagca 8460
   caaactactc tattactgga aattaactgg gctaccgctt cctgatgaat gtgacttcga 8520
   ccacctcatt ctcagccgac aatggaaaaa aatacttgaa tcggcctctc ctgatactga 8580
   gagaatgata aaactcggaa gggcagtaca ccaaactctt aaccacaatt ccagaataac 8640
   cggagtgctc caccccaggt gtttagaaga actggctaat attgaggtcc cagattcaac 8700
   caacaaattt cggaagattg agaagaagat ccaaattcac aacacgagat atggagaact 8760
   gttcacaagg ctgtgtacgc atatagagaa gaaactgctg gggtcatctt ggtctaacaa 8820
   tgtcccccgg tcagaggagt tcagcagcat ccgtacggac ccggcattct ggtttcactc 8880
   aaaatggtcc acagccaagt ttgcatggct ccatataaaa cagatccaga ggcatctgat 8940
   ggtggcagct aggacaaggt ctgcggccaa caaattggtg atgctaaccc ataaggtagg 9000
   ccaagtcttt gtcactcctg aacttgtcgt tgtgacgcat acgaatgaga acaagttcac 9060
   atgtcttacc caggaacttg tattgatgta tgcagatatg atggagggca gagatatggt 9120
   caacataata tcaaccacgg cggtgcatct cagaagctta tcagagaaaa ttgatgacat 9180
   tttgcggtta atagacgctc tggcaaaaga cttgggtaat caagtctacg atgttgtatc 9240
   actaatggag ggatttgcat acggagctgt ccagctactc gagccgtcag gtacatttgc 9300
   aggagatttc ttcgcattca acctgcagga gcttaaagac attctaattg gcctcctccc 9360
   caatgatata gcagaatccg tgactcatgc aatcgctact gtattctctg gtttagaaca 9420
   gaatcaagca gctgagatgt tgtgtctgtt gcgtctgtgg ggtcacccac tgcttgagtc 9480
   ccgtattgcc gcaaaggcag tcaggagcca aatgtgcgca ccgaaaatgg tagactttga 9540
   tatgatcctt caggtactgt ctttcttcaa gggaacaatc atcaacgggt acagaaagaa 9600
   gaatgcaggt gtgtggccgc gagtcaaagt ggatacaata tatgggaagg tcattgggca 9660
   actacatgca gattcagcag agatttcaca cgatatcatg ttgagagagt ataagagttt 9720
   atctgcactt gaatttgagc catgtataga atatgaccct gtcaccaacc tgagcatgtt 9780
   cctaaaagac aaggcaatcg cacaccccaa cgataattgg cttgcctcgt ttaggcggaa 9840
   ccttctctcc gaagaccaga agaaacatgt aaaagaagca acttcgacta atcgcctctt 9900
   gatagagttt ttagagtcaa atgattttga tccatataaa gagatggaat atctgacgac 9960
   ccttgagtac cttagagatg acaatgtggc agtatcatac tcgctcaagg agaaggaagt 10020
   gaaagttaat ggacggatct tcgctaagct gacaaagaag ttaaggaact gtcaggtgat 10080
   ggcggaaggg atcctagccg atcagattgc acctttcttt cagggaaatg gagtcattca 10140
   ggatagcata tccttgacca agagtatgct agcgatgagt caactgtctt ttaacagcaa 10200
   taagaaacgt atcactgact gtaaagaaag agtatcttca aaccgcaatc atgatccgaa 10260
   aagcaagaac cgtcggagag ttgcaacctt cataacaact gacctgcaaa agtactgtct 10320
   taattggaga tatcagacaa tcaaattgtt cgctcatgcc atcaatcagt tgatgggcct 10380
   acctcacttc ttcgaatgga ttcacctaag actgatggac actacgatgt tcgtaggaga 10440
   ccctttcaat cctccaagtg accctactga ctgtgacctc tcaagagtcc ctaatgatga 10500
   catatatatt gtcagtgcca gagggggtat cgaaggatta tgccagaagc tatggacaat 10560
   gatctcaatt gctgcaatcc aacttgctgc agctagatcg cattgtcgtg ttgcctgtat 10620
   ggtacagggt gataatcaag taatagcagt aacgagagag gtaagatcag acgactctcc 10680
   ggagatggtg ttgacacagt tgcatcaagc cagtgataat ttcttcaagg aattaattca 10740
   tgtcaatcat ttgattggcc ataatttgaa ggatcgtgaa accatcaggt cagacacatt 10800
   cttcatatac agcaaacgaa tcttcaaaga tggagcaatc ctcagtcaag tcctcaaaaa 10860
   ttcatctaaa ttagtgctag tgtcaggtga tctcagtgaa aacaccgtaa tgtcctgtgc 10920
   caacattgcc tctactgtag cacggctatg cgagaacggg cttcccaaag acttctgtta 10980
   ctatttaaac tatataatga gttgtgtgca gacatacttt gactctgagt tctccatcac 11040
   caacaattcg caccccgatc ttaatcagtc gtggattgag gacatctctt ttgtgcactc 11100
   atatgttctg actcctgccc aattaggggg actgagtaac cttcaatact caaggctcta 11160
   cactagaaat atcggtgacc cggggactac tgcttttgca gagatccagc gactagaagc 11220
   agtgggatta ctgagtccta acattatgac taatatctta actaggccgc ctgggaatgg 11280
   agattgggcc agtctgtgca acgacccata ctctttcaat tttgagactg ttgcaagccc 11340
   aaatattgtt cttaagaaac atacgcgaag agtcctattt gaaacttgtt caaatccctt 11400
   attgtctgga gtgcacacag aggataatga ggcagaagag aaggcattgg ctgaattctt 11460
   gcttaatcaa gaggtgattc atccccgcgt tgcgcatgcc atcatggagg caagctctgt 11520
   aggtaggaga aagcaaattc aagggcttgt tgacacaaca aacaccgtaa ttaagattgc 11580
   gcttactagg aggccattag gcataaagag gctgatgcgg atagtcaatt attctagcat 11640
   gcatgcaatg ctgtttagag acgatgtttt ttcctccagt agatccaacc accccttagt 11700
   ctcttctaat atgtgttctc tgacactggc agactatgca cggaatagaa gctggtcacc 11760
   tttgacggga ggcaggaaaa tactgggtgt atctaatcct gatacgatag aactcgtaga 11820
   gggtgagatt cttagtgtaa gcggagggtg tacaagatgt gacagcggag atgaacaatt 11880
   tacttggttc catcttccaa gcaatataga attgaccgat gacaccagca agaatcctcc 11940
   gatgagggta ccatatctcg ggtcgaagac acaggagagg agagctgcct cacttgcaaa 12000
   aatagctcat atgtcgccac atgtaaaggc tgccctaagg gcatcatccg tgttgatctg 12060
   ggcttatggg gataatgacg taaattggac tgctgctctt acgattgcaa aatctcggtg 12120
   taatgtaaac ttagagtatc ttcggttact gtccccttta cccacggctg ggaatcttca 12180
   acatagacta gatgatggta taactcagat gacattcacc cctgcatctc tctacaggtg 12240
   tcaccttaca ttcacatatc caatgattct caaaggctgt tcactgaaga aggagtcaaa 12300
   gaggggaatg tggtttacca acagagtcat gctcttgggt ttatctctaa tcgaatcgat 12360
   atttccaatg acagcaacca ggacatatga tgagatcaca ctgcacctac atagtaaatt 12420
   tagttgctgt atcagagaag cacctgttgc ggttcctttc gagctacttg gggtggtacc 12480
   ggaactgagg acagtgacct caaataagtt tatgtatgat cctagccctg gatcggaggg 12540
   agactttgcg agacttgact tagctatctt caagagttat gagcttaatc tggagtcata 12600
   tcccacgata gagctaatga acattctttc aatatccagc gggaagttga ttggccagtc 12660
   tgtggtttct tatgatgaag atacctccat aaagaatgac gccataatag tgtacgacaa 12720
   tacccgaaat tggatcagtg aagctcagaa ttcagatgtg gtccgcctat ttgaatatgc 12780
   agcacttgaa gtgctcctca actgttctta ccaactctat tacctgagag taagaggcct 12840
   agacaatatt gtcttatata tgggtgattt atacaagaat atgccaggaa ttctactttc 12900
   caacattgca gctacaatat ctcatcccgt cattcattca aggttacatg cagtgggcct 12960
   ggtcaaccat gacggatcac accaacttgc agatacggat tttatcgaaa tgtctgcaaa 13020
   actattagta tcttgcaccc gacgtgtgat ctccggctta tattcaggaa ataagtatga 13080
   tctgctgttc ccatctgtct tagatgataa cctgaatgag aagatgcttc agctgatatc 13140
   ccggttatgc tgtctgtaca cggtactctt tgctacaaca agagaaatcc cgaaaataag 13200
   aggcttaact acaaaagaga aatgttcaat acccactgag tatttactgt cggatgctgt 13260
   gaaaccatta cttagccccg atcaagtgag ctctatcatg tctcctaaca taattacatt 13320
   cccagctaat ccgtactaca tgtctcggaa gagcctcaat ttgatcaggg aaagggagga 13380
   cagggatact atcctggtgt tgttgttccc ccaagagcca ttattagagt tcccttctgt 13440
   gcaagatatt ggtgctcgag tgaaagatcc attcacccga caacctgcgg catttttgca 13500
   agagttagat ttgagtgctc cagcaaggta tgacgcattc acacttagtc agattcatcc 13560
   tgaactcaca tctccaaatc cggaggaaga ctacttagta cgatacttgt tcagagggat 13620
   agggactgca tcttcctctt ggtataaggc atctcatctc ctttctgtac ccgaggtaag 13680
   atgtgcaaga cacgggaact ccttatactt agctgaaggg agcggagcca tcatgagtct 13740
   tctcgaactg catgtaccac atgaaactat ctattacaat acgctctttt caaatgagat  13800
   gaaccccccg caacgacatt tcgggccgac cccaactcag tttttgaatt cggttgttta  13860
   taggaatcta caggcggagg taacatgcaa agatggattt gtccaagagt tccgtccatt  13920
   atggagagaa aatacagagg aaagtgacct gacctcagat aaagcagtgg ggtatattac  13980
   atctgcagtg ccctacagat ctgtatcatt gctgcattgt gacattgaaa ctcctccagg  14040
   gtccaatcaa agcttactag atcaactagc tatcaattta tctctgattg ccatgcattc  14100
   tgtaagggag ggcggggtag taatcatcaa agtgttgtat gcaatgggat actactttca  14160
   tctactcatg aacttgtttg ctccgtgttc cacaaaagga tatattctct ctaatggtta  14220
   tgcatgtcga ggagatatgg agtgttacct ggtatttgtc atgggttacc tgggcgggcc  14280
   tacatttgta catgaggtgg tgaggatggc aaaaactctg gtgcagcggc acggtacgct  14340
   cttgtctaaa tcagatgaga tcacactgac caggttattc acctcacagc ggcagcgtgt  14400
   gacagacatc ctatccagtc ctttaccaag attaataaag tacttgagga agaatattga  14460
   cactgcgctg attgaagccg ggggacagcc cgtccgtcca ttctgtgcgg agagtctggt  14520
   gagcacgcta gcgaacataa ctcagataac ccagattatc gctagtcaca ttgacacagt  14580
   tatccggtct gtgatatata tggaagctga gggtgatctc gctgacacag tatttctatt  14640
   taccccttac aatctctcta ctgacgggaa aaagaggaca tcacttaaac agtgcacgag  14700
   acagatccta gaggttacaa tactaggtct tagagtcgaa aatctcaata aaataggcga  14760
   tataatcagt ctagagctta aaggcatgat ctccatggag gaccttatcc cactaaggac  14820
   atacttgaag catagtacct gccctaaata tttgaaggct gtcctaggta ttaccaaact  14880
   caaagaaatg tttacagaca cttctgtact gtacttgact cgtgctcaac aaaaattcta  14940
   catgaaaact ataggcaatg cagtcaacgg atattacagt aactgtgact cttaacgaaa  15000
   atcacatatt aataggctcc ttttttggcc aattgtattc ttgttgattt aatcatatta  15060
   tgttagaaaa aagttgaacc ctgactcctt aggactcgaa ttcgaactca aataaatgtc  15120
   ttaaaaaaag gttgcgcaca attattcttg agtgtagtct cgtcattcac caaatctttg  15180
   tttggt                                                              15186
<210> 140
   <211> 546
   <212> PRT
   <213> Avian influenza virus
<400> 140
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Ser Gly Val Ser Ser Ala Cys Pro Tyr Gln Gly Gly Pro Ser Phe Tyr
       130                 135                 140
   Arg Asn Val Val Trp Leu Ile Lys Lys Asp Asn Ala Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Ala Glu Gln Thr Arg Leu Tyr Gln
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Ala Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Arg Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Arg Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu
   545